# EUROPEAN PATENT SPECIFICATION

(11) **EP 3 897 689 B1**
(45) Date of publication and mention of the grant of the patent: **05.02.2025**
(21) Application number: 19842987.0
(22) Date of filing: 20.12.2019
(51) Int. Cl.: A61K 38/17, A61P 21/00

(54) **USE OF ANNEXINS IN PREVENTING AND TREATING MUSCLE MEMBRANE INJURY**
VERWENDUNG VON ANNEXINEN ZUR VORBEUGUNG UND BEHANDLUNG VON MUSKELMEMBRANVERLETZUNGEN
UTILISATION D'ANNEXINES DANS LA PRÉVENTION ET LE TRAITEMENT D'UNE LÉSION DE LA MEMBRANE MUSCULAIRE

(30) Priority: 21.12.2018 US 201862783619 P; 20.09.2019 US 201962903525 P
(43) Date of publication of application: 27.10.2021
(73) Proprietor: Northwestern University, Evanston, IL 60208 (US)
(72) Inventor: DEMONBREUN, Alexis R., Mokena, IL 60448 (US); MCNALLY, Elizabeth M., Oak Park, IL 60302 (US)
(74) Representative: Stolmár & Partner Patentanwälte PartG mbB
(86) International application number: PCT/US2019/068168
(87) International publication number: WO 2020/132647

(56) References cited:
- WO-A1-2016/059146
- RAMAZI SHAHIN ET AL: "Post-translational modifications in proteins: resources, tools and prediction methods", DATABASE, vol. 2021, 7 April 2021 (2021-04-07), XP093098036, Retrieved from the Internet <URL:http://academic.oup.com/database/article-pdf/doi/10.1093/database/baab012/37044588/baab012.pdf> DOI: 10.1093/database/baab012
- ALEXIS R. DEMONBREUN ET AL: "Recombinant annexin A6 promotes membrane repair and protects against muscle injury", JOURNAL OF CLINICAL INVESTIGATION, vol. 129, no. 11, 23 September 2019 (2019-09-23), GB, pages 4657 - 4670, XP055675771, ISSN: 0021-9738, DOI: 10.1172/JCI128840
- K. A. SWAGGART ET AL: "Annexin A6 modifies muscular dystrophy by mediating sarcolemmal repair", PROCEEDINGS OF THE NATIONAL ACADEMY OF SCIENCES, vol. 111, no. 16, 22 April 2014 (2014-04-22), US, pages 6004 - 6009, XP055675778, ISSN: 0027-8424, DOI: 10.1073/pnas.1324242111
- ALEXIS R. DEMONBREUN ET AL: "An actin-dependent annexin complex mediates plasma membrane repair in muscle", THE JOURNAL OF CELL BIOLOGY : JCB, vol. 213, no. 6, 13 June 2016 (2016-06-13), US, pages 705 - 718, XP055675784, ISSN: 0021-9525, DOI: 10.1083/jcb.201512022
- MATTIA QUATTROCELLI ET AL: "Intermittent glucocorticoid steroid dosing enhances muscle repair without eliciting muscle atrophy", JOURNAL OF CLINICAL INVESTIGATION, vol. 127, no. 6, 1 June 2017 (2017-06-01), GB, pages 2418 - 2432, XP055675785, ISSN: 0021-9738, DOI: 10.1172/JCI91445
- MATTIA QUATTROCELLI ET AL: "Genetic modifiers of muscular dystrophy act on sarcolemmal resealing and recovery from injury", PLOS GENETICS, vol. 13, no. 10, 24 October 2017 (2017-10-24), pages e1007070, XP055675790, DOI: 10.1371/journal.pgen.1007070
- THERESA LOUISE BOYE ET AL: "Annexin A4 and A6 induce membrane curvature and constriction during cell membrane repair", NATURE COMMUNICATIONS, vol. 8, no. 1, 20 November 2017 (2017-11-20), XP055675840, DOI: 10.1038/s41467-017-01743-6
- LAURITZEN STINE PREHN ET AL: "Annexins are instrumental for efficient plasma membrane repair in cancer cells", SEMINARS IN CELL AND DEVELOPMENTAL BIOLOGY, vol. 45, 20 October 2015 (2015-10-20), pages 32 - 38, XP029332316, ISSN: 1084-9521, DOI: 10.1016/J.SEMCDB.2015.10.028
- CARMEILLE ROMAIN ET AL: "Annexin-A5 promotes membrane resealing in human trophoblasts", BIOCHIMICA ET BIOPHYSICA ACTA. MOLECULAR CELL RESEARCH, ELSEVIER SCIENCE PUBLISHERS, AMSTERDAM, NL, vol. 1853, no. 9, 14 January 2015 (2015-01-14), pages 2033 - 2044, XP029254151, ISSN: 0167-4889, DOI: 10.1016/J.BBAMCR.2014.12.038
- LA ET AL: "Annexin 1 peptides protect against experimental myocardial ischemia-reperfusion: analysis of their mechanism of action", THE FASEB JOURNAL, vol. 15, no. 12, 1 October 2001 (2001-10-01), pages 2247 - 2256, XP055000646, ISSN: 0892-6638, DOI: 10.1096/fj.01-0196com
- WADDELL LEIGH B ET AL: "Dysferlin, Annexin A1, and Mitsugumin 53 Are Upregulated in Muscular Dystrophy and Localize to Longitudinal Tubules of the T-System With Stretch", JOURNAL OF NEUROPATHOLOGY AND EXPERIMENTAL NEUROLOGY, LIPPINCOTT WILLIAMS AND WILKINS, NEW YORK, NY, vol. 70, no. 4, 1 April 2011 (2011-04-01), pages 302 - 313, XP009176985, ISSN: 0022-3069, DOI: 10.1097/NEN.0B013E31821350B0
- KOERDT SOPHIA NINA ET AL: "Annexin A2 is involved in Ca2+-dependent plasma membrane repair in primary human endothelial cells", BIOCHIMICA ET BIOPHYSICA ACTA. MOLECULAR CELL RESEARCH, ELSEVIER SCIENCE PUBLISHERS, AMSTERDAM, NL, vol. 1864, no. 6, 9 December 2016 (2016-12-09), pages 1046 - 1053, XP085007921, ISSN: 0167-4889, DOI: 10.1016/J.BBAMCR.2016.12.007
- HIDETO ISHII ET AL: "Recombinant Annexin II Modulates Impaired Fibrinolytic Activity In Vitro and in Rat Carotid Artery", CIRCULATION RESEARCH, vol. 89, no. 12, 7 December 2001 (2001-12-07), US, pages 1240 - 1245, XP055676228, ISSN: 0009-7330, DOI: 10.1161/hh2401.101066

## Description

### STATEMENT OF GOVERNMENT INTEREST

This invention was made with government support under grant numbers U54 AR052646 and R01 NS047726 awarded by the National Institutes of Health. The government has certain rights in the invention.

### BACKGROUND

Plasma membrane repair occurs after membrane disruption and is a highly conserved process. The active process required for resealing membrane disruptions is thought to rely on Ca²⁺-dependent vesicle fusion and local cytoskeletal remodeling (McNeil and Khakee, 1992; McNeil and Kirchhausen, 2005). Other models suggest that membrane repair is mediated through the fusion of lysosomal vesicles and/or lateral diffusion of membrane to the site of injury (Demonbreun *et al.,* 2016b; McDade *et al.,* 2014; Reddy *et al*., 2001; Rodriguez *et al*., 1997). These models are not mutually exclusive and may depend on the type and extent of damage. Skeletal muscle is highly dependent on plasma membrane repair as mutation in genes encoding repair proteins lead to muscle disease (Bansal *et al.,* 2003; Bashir *et al.,* 1998; Cai *et al.,* 2009; Defour *et al*., 2017; Demonbreun and McNally, 2016; Demonbreun *et al.,* 2015).

Annexins are Ca²⁺-binding proteins that regulate lipid binding, cytoskeletal reorganization, and bleb formation, steps necessary for membrane repair (Bizzarro *et al.,* 2012; Boye *et al.,* 2018; Boye *et al.,* 2017; Grewal *et al.,* 2017; Jimenez and Perez, 2017; Lauritzen *et al.,* 2015). Individual annexin repeat domains coordinate Ca²⁺ binding with unique annexin-specific type II or type III binding sites. Differential Ca²⁺ affinity of the type II and type III binding sites provides each annexin a unique ability to respond to a range of intracellular Ca²⁺ levels and phospholipid binding (Blackwood and Ernst, 1990). Annexins have the ability to self- and hetero-oligomerize (Zaks and Creutz, 1991). Typical annexins like A1 and A2 contain one annexin core composed of four annexin repeat domains. In contrast, annexin A6 contains two annexin cores and thus eight annexin repeat domains (Benz *et al.,* 1996). Annexin A6's duplicated structure makes it possible for the amino- and carboxyl-terminal annexin core domains to bind one or two distinct membranes making annexin A6 a prime target for facilitating membrane coalescence and folding required during membrane repair (Boye *et al.,* 2018; Boye *et al.,* 2017; Buzhynskyy *et al.,* 2009).

Annexins have a high affinity for phosphatidylserine, phosphatidylinositol, and cholesterol, which are highly enriched in the sarcolemma (Fiehn *et al.,* 1971; Gerke *et al.,* 2005). Multiple annexins have been implicated in membrane repair in skeletal muscle, as well as Xenopus oocytes, human trophoblasts, and HeLa cancer cells, suggesting a conserved mechanism (Babbin *et al.,* 2008; Bement *et al.,* 1999; Carmeille *et al.,* 2015; Davenport *et al.,* 2016; Demonbreun *et al.,* 2016b; Lennon *et al.,* 2003; McNeil *et al.,* 2006; Roostalu and Strahle, 2012). Annexins are recruited to the injured membrane in a sequential manner forming a macromolecular repair complex at the membrane lesion (Boye *et al.,* 2017; Demonbreun *et al.,* 2016b; Roostalu and Strahle, 2012).
Annexin A6 and its role in sarcolemma repair has been previously described (K. A. SWAGGART ET AL, "Annexin A6 modifies muscular dystrophy by mediating sarcolemmal repair", PROCEEDINGS OF THE NATIONAL ACADEMY OF SCIENCES, US, (20140422), vol. 111, no. 16, doi:10.1073/pnas.1324242111, ISSN 0027-8424, pages 6004 - 6009. LAURITZEN STINE PREHN ET AL, "Annexins are instrumental for efficient plasma membrane repair in cancer cells", SEMINARS IN CELL AND DEVELOPMENTAL BIOLOGY, (20151020), vol. 45, doi:10.1016/J.SEMCDB.2015.10.028, ISSN 1084-9521, pages 32 - 38).

### SUMMARY

Defects in the ability of a cell to repair the plasma membrane lead to numerous diseases, including muscular dystrophy and other pathological conditions that result in cellular death. Membrane injury can result from events such as overuse, trauma, burn, chemical exposure and chronic disease. Currently, there is a lack of agents that prevent or treat membrane damage (Demonbreun and McNally, 2016). Annexins are calcium-binding proteins that have a high affinity for membrane lipids. Annexins have been implicated in membrane repair in many cell types including muscle, trophoblasts, HeLa cells, and oocytes, suggesting a widespread role for annexins (Babbin *et al.,* 2008; Bement *et al.,* 1999; Carmeille *et al.,* 2015; Davenport *et al.,* 2016; Demonbreun *et al.,* 2016b; McNeil *et al.,* 2006; Roostalu and Strahle, 2012). Annexin A1 ("A1"), annexin A2 ("A2"), annexin A5 ("A5"), and annexin A6 ("A6") are sequentially recruited to the injury site forming a large repair complex (Demonbreun *et al.,* 2016b; Roostalu and Strahle, 2012). A polymorphism in *Anxa6* was identified that created a truncated form of annexin A6 (Swaggart *et al.,* 2014). Expression of truncated annexin A6 acted in a dominant-negative fashion, decreasing annexin repair complex formation. This correlated with impaired membrane repair and more severe muscle disease (Demonbreun *et al.,* 2016a; Demonbreun *et al.,* 2016b; Quattrocelli *et al.,* 2017b; Swaggart *et al.,* 2014). Conversely, overexpression of annexin A6 was sufficient to improve repair and decrease susceptibility to membrane injury (Quattrocelli *et al.,* 2017a). Moreover, treatment with recombinant annexin A6 was sufficient to reduce muscle damage in cell based and whole animal studie

In one aspect, the invention relates to a composition for use in treating a cellular membrane injury comprising administering to a patient in need thereof a therapeutically effective amount of the composition, the composition comprising:
a protein having at least 90% sequence identity to annexin A6 as depicted in SEQ ID NO: 7, a protein having at least 90% sequence identity to annexin A6 as depicted in SEQ ID NO: 8 or a combination thereof, or a post-translationally modified form thereof, wherein the post-translationally modified form is biotinylated, glycosylated, PEGylated, and/or polysialylated, or
a polynucleotide capable of expressing an extracellular protein having at least 90% sequence identity to annexin A6 as depicted in SEQ ID NO: 7, a protein having at least 90% sequence identity to annexin A6 as depicted in SEQ ID NO: 8, or a combination thereof, or a modified form thereof.

In another aspect, the invention relates to a composition for use in delaying onset, enhancing recovery from cellular membrane injury, or preventing a cellular membrane injury comprising administering to a patient in need thereof a therapeutically effective amount of the composition, the composition comprising:
a protein having at least 90% sequence identity to annexin A6 as depicted in SEQ ID NO: 7, a protein having at least 90% sequence identity to annexin A6 as depicted in SEQ ID NO: 8, or a combination thereof, or a post-translationally modified form thereof, wherein the post-translationally modified form is biotinylated, glycosylated, PEGylated, and/or polysialylated, or
a polynucleotide capable of expressing an extracellular protein having at least 90% sequence identity to annexin A6 (SEQ ID NO: 7, SEQ ID NO: 8, or a combination thereof), or a modified form thereof.

Further preferred embodiments of the invention are described in the dependent claims.
The references to the methods of treatment by therapy or surgery in this description are to be interpreted as references to compounds, pharmaceutical compositions, and medicaments of the present invention for use in those methods.

In some embodiments of the methods disclosed herein, the polynucleotide is associated with a nanoparticle. In some embodiments, the polynucleotide is contained in a vector. In some embodiments, the vector is within a chloroplast. In some embodiments, the vector is a viral vector. In some embodiments, the viral vector is selected from the group consisting of a herpes virus vector, an adeno-associated virus (AAV) vector, an adeno virus vector, and a lentiviral vector. In some embodiments, the AAV vector is recombinant AAV5, AAV6, AAV8, AAV9, or AAV74, including embodiments wherein the AAV74 vector is AAVrh74.

In some embodiments of the methods disclosed herein, the composition increases the activity of annexin A1 (SEQ ID NO: 1), annexin A2 (SEQ ID NO: 2 or SEQ ID NO: 3), annexin A3 (SEQ ID NO: 4), annexin A4 (SEQ ID NO: 5), annexin A5 (SEQ ID NO: 6), annexin A6 (SEQ ID NO: 7, SEQ ID NO: 8, SEQ ID NO: 45, or a combination thereof), annexin A7 (SEQ ID NO: 9 or SEQ ID NO: 10), annexin A8 (SEQ ID NO: 11 or SEQ ID NO: 12), annexin A9 (SEQ ID NO: 13), annexin A10 (SEQ ID NO: 14), annexin A11 (SEQ ID NO: 15 or SEQ ID NO: 16), annexin A13 (SEQ ID NO: 17 or SEQ ID NO: 18), or a combination thereof. In some embodiments, the composition increases the activity of annexin A1 (SEQ ID NO: 1), annexin A2 (SEQ ID NO: 2 or SEQ ID NO: 3), and annexin A6 (SEQ ID NO: 7, SEQ ID NO: 8, SEQ ID NO: 45, or a combination thereof). In some embodiments, the composition increases the activity of annexin A2 (SEQ ID NO: 2 or SEQ ID NO: 3) and annexin A6 (SEQ ID NO: 7, SEQ ID NO: 8, SEQ ID NO: 45, or a combination thereof). In some embodiments, the composition increases the activity of annexin A1 (SEQ ID NO: 1) and annexin A6 (SEQ ID NO: 7, SEQ ID NO: 8, SEQ ID NO: 45, or a combination thereof). In some embodiments, the composition increases the activity of annexin A6 (SEQ ID NO: 7, SEQ ID NO: 8, SEQ ID NO: 45, or a combination thereof). In any of the aspects or embodiments of the disclosure, the composition for use in any of the methods described herein is a pharmaceutical composition as disclosed herein.

In some aspects, the disclosure provides a pharmaceutical composition comprising a modified annexin protein and a pharmaceutically acceptable carrier, buffer, and/or diluent. In some aspects, the disclosure provides a pharmaceutical composition comprising an annexin protein and a pharmaceutically acceptable carrier, buffer, and/or diluent. In some embodiments, the annexin protein or modified annexin protein is annexin A1 (SEQ ID NO: 1), annexin A2 (SEQ ID NO: 2 or SEQ ID NO: 3), annexin A3 (SEQ ID NO: 4), annexin A4 (SEQ ID NO: 5), annexin A5 (SEQ ID NO: 6), annexin A6 (SEQ ID NO: 7, SEQ ID NO: 8, SEQ ID NO: 45, or a combination thereof), annexin A7 (SEQ ID NO: 9 or SEQ ID NO: 10), annexin A8 (SEQ ID NO: 11 or SEQ ID NO: 12), annexin A9 (SEQ ID NO: 13), annexin A10 (SEQ ID NO: 14), annexin A11 (SEQ ID NO: 15 or SEQ ID NO: 16), annexin A13 (SEQ ID NO: 17 or SEQ ID NO: 18), or a combination thereof. In some embodiments, the annexin protein or modified annexin protein is annexin A6 (SEQ ID NO: 7, SEQ ID NO: 8, SEQ ID NO: 45, or a combination thereof). In further embodiments, the pharmaceutical composition comprises annexin A1 (SEQ ID NO: 1), annexin A2 (SEQ ID NO: 2 or SEQ ID NO: 3), and annexin A6 (SEQ ID NO: 7, SEQ ID NO: 8, SEQ ID NO: 45, or a combination thereof). In some embodiments, the pharmaceutical composition comprises annexin A2 (SEQ ID NO: 2 or SEQ ID NO: 3) and annexin A6 (SEQ ID NO: 7, SEQ ID NO: 8, SEQ ID NO: 45, or a combination thereof). In further embodiments, the pharmaceutical composition comprises annexin A1 (SEQ ID NO: 1) and annexin A6 (SEQ ID NO: 7, SEQ ID NO: 8, SEQ ID NO: 45, or a combination thereof). In some embodiments, the pharmaceutical composition further comprises a steroid. In further embodiments, the steroid is a corticosteroid or a glucocorticoid. In some embodiments, the pharmaceutical composition further comprises an effective amount of a second agent, wherein the second agent is selected from the group consisting of mitsugumin 53 (MG53), a modulator of latent TGF-β binding protein 4 (LTBP4), a modulator of transforming growth factor β(TGF-β) activity, a modulator of androgen response, a modulator of an inflammatory response, a promoter of muscle growth, a chemotherapeutic agent, a modulator of fibrosis, and a combination thereof. In further embodiments, purity of the annexin protein in the composition is about 90% or higher as measured by standard release assay, including but not limited to SDS-PAGE, SEC-HPLC, and immunoblot analysis. In some embodiments, the composition has an endotoxin level that is less than about 0.50000 endotoxin units per milligram (EU/mg) A280 annexin protein. In some embodiments, the modified annexin protein is produced in a prokaryotic cell.

In some aspects, the disclosure provides a pharmaceutical composition comprising modified annexin A6 (SEQ ID NO: 7, SEQ ID NO: 8, SEQ ID NO: 45, or a combination thereof) and a pharmaceutically acceptable carrier, buffer, and/or diluent. In some aspects, the disclosure provides a pharmaceutical composition comprising annexin A6 (SEQ ID NO: 7, SEQ ID NO: 8, SEQ ID NO: 45, or a combination thereof) and a pharmaceutically acceptable carrier, buffer, and/or diluent. In some embodiments, the pharmaceutical composition further comprises annexin A1 (SEQ ID NO: 1) and annexin A2 (SEQ ID NO: 2 or SEQ ID NO: 3). In some embodiments, the pharmaceutical composition further comprises annexin A2 (SEQ ID NO: 2 or SEQ ID NO: 3). In some embodiments, the pharmaceutical composition further comprises annexin A1 (SEQ ID NO: 1). In some embodiments, the pharmaceutical composition further comprises a steroid. In further embodiments, the steroid is a corticosteroid or a glucocorticoid. In some embodiments, the pharmaceutical composition further comprises an effective amount of a second agent, wherein the second agent is selected from the group consisting of mitsugumin 53 (MG53), a modulator of latent TGF-β binding protein 4 (LTBP4), a modulator of transforming growth

### BRIEF DESCRIPTION OF THE FIGURES

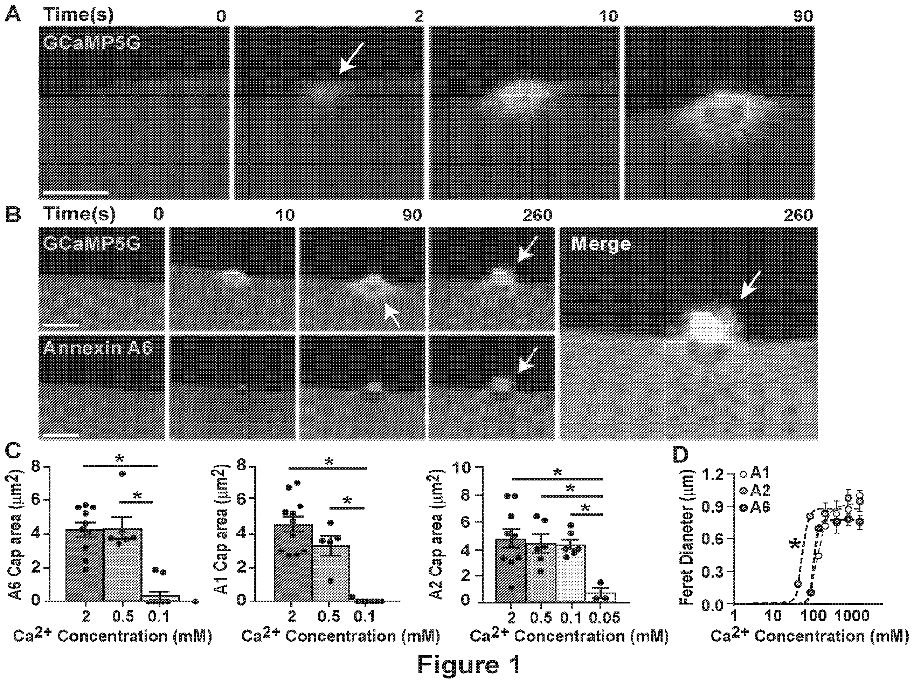

**Figure 1** shows that Ca²⁺ dynamics influenced annexin repair cap recruitment at the site of injury. Myofibers were generated to express the Ca²⁺ indicator GCaMP5G, and time-lapse single slice images were assessed at time points after membrane disruption. A) GCaMP5G fluorescence was present at the site of injury, at 2 seconds, indicating the presence of Ca²⁺ immediately after damage at the site of injury. B) Time-lapse images of myofibers co-electroporated with GCaMP5G and annexin A6-tdTomato. GCaMP5G fluorescence was present at the site of injury localized around the annexin A6-free zone (arrowhead) and at the annexin A6 cap (arrow). GCaMP5G colocalized (merge, arrow) with the annexin A6 repair cap. Scale bar 5µm. C) Myofibers expressing fluorescently tagged annexin A1, A2 or A6 were injured at multiple Ca²⁺ concentrations. Annexin A1 and A6 repair cap size was reduced at 0.1mM Ca²⁺ compared to 2mM and 0.5mM. Annexin A2 repair cap area was significantly reduced at 0.05mM Ca²⁺ compared to 2mM, 0.5mM, and 0.1mM Ca²⁺. D) Cap kinetics were plotted as cap feret diameter over a range of Ca²⁺ concentrations. Annexin A2 had a statistically significant leftward shift in Km_{1/2} followed by annexin A6 then A1. Scale 5µm.

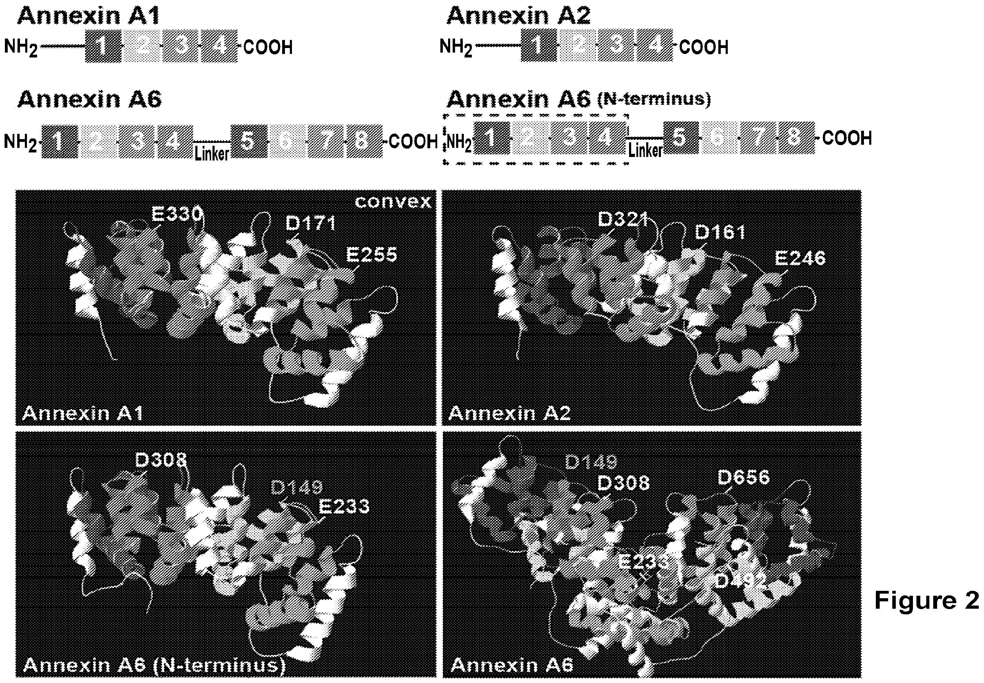

**Figure 2** shows domain modeling of annexin's multiple Ca²⁺ binding sites. Annexins typically contain four annexin domains with the exception of annexin A6, which is duplicated to have 8 annexin domains. Annexin A1, A2, and A6 coordinate multiple Ca²⁺ ions and bind membrane on the convex face, positioned facing the top of each ribbon diagram. The D171 residue in annexin A1 and the D161 residue in annexin A2 were previously described as necessary residues to coordinate Ca²⁺ binding (Jost *et al.,* 1992). Both D171 and D161 localize in the second (yellow) annexin domain in annexin A1 and A2, respectively. The orthologous residue in annexin A6, D149, which falls into the first half of annexin A6, was not observed to coordinate Ca²⁺ in bovine A6 (Avila-Sakar *et al.,* 1998). In this study, D149 was examined in annexin A6 as well as E233, which fall into the second and third annexin domains, respectively.

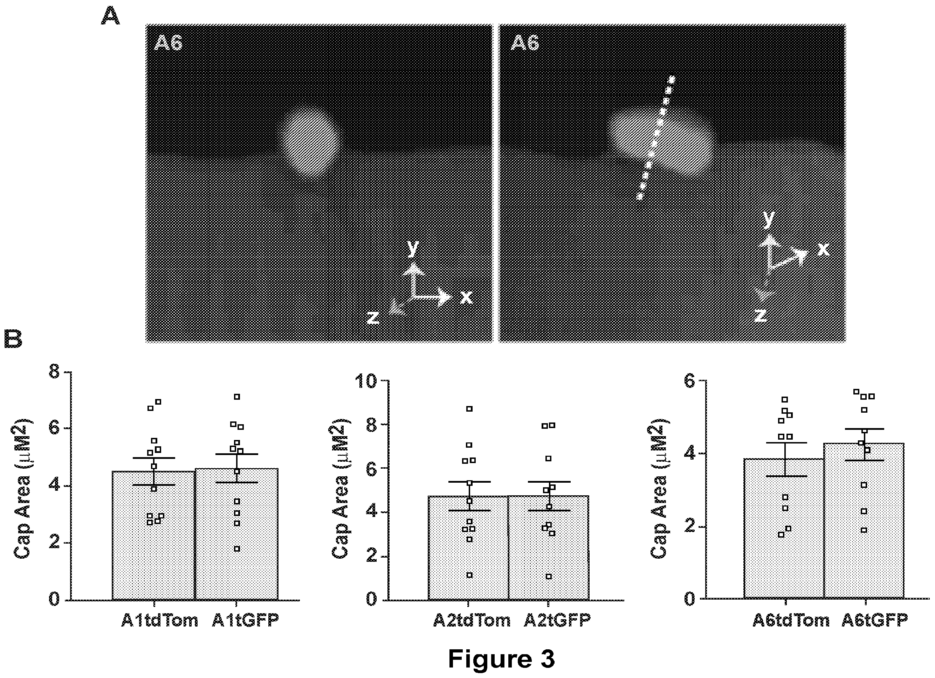

**Figure 3** shows A) Max projection of Z-stack imaging of repair cap (left). Rotated Z-stack projection (right). Cap size is measured from the center z-stack 2-D image represented by the white dotted line. B) Myofibers were co-electroporated with wildtype annexin-tdTomato and wildtype annexin-turboGFP. Cap size was assessed in both the red and green channels. The type of fluorescent tag did not influence cap size. Data are expressed as mean ± SEM. Differences were tested by two-tailed t-test, (n≥10 fibers per condition).

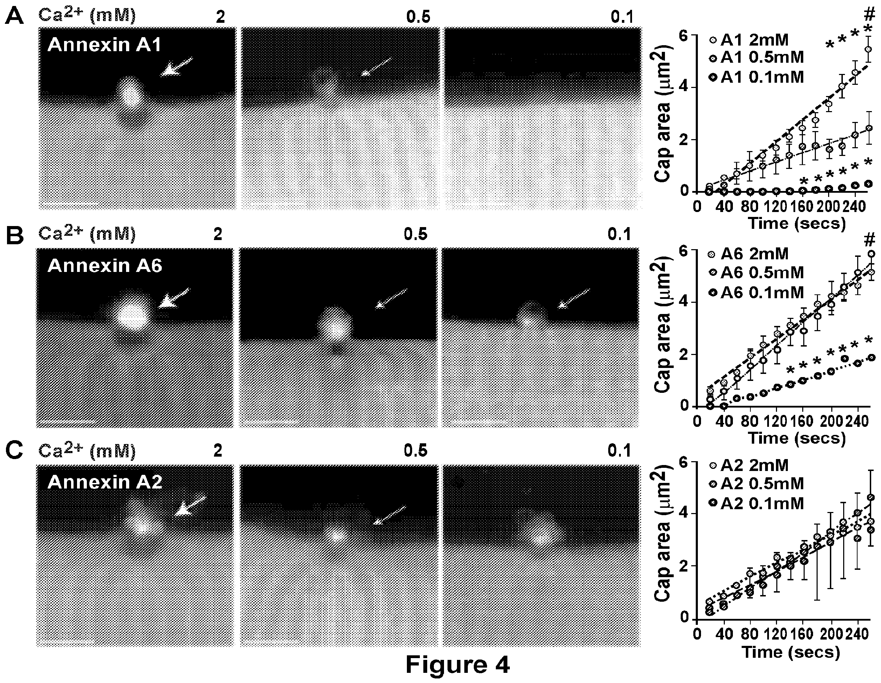

**Figure 4** shows differential Ca²⁺ sensitivity of annexin A1, A2 and A6 in repair cap formation. Myofibers expressing fluorescently tagged annexin A1, A2 or A6 were injured at multiple Ca²⁺ concentrations. A & B) Annexin A1 and A6 repair cap size was reduced with decreasing Ca²⁺ concentrations. Annexin A1 and A6 repair cap area and rate of repair cap formation was reduced at 0.5mM Ca²⁺ and 0.1mM compared to 2mM. C) Annexin A2 repair cap area was significantly reduced at 0.05mM Ca²⁺ compared to 2mM, 0.5mµM, and 0.1mM Ca²⁺. The rate of annexin A2 repair cap formation was equivalent at the Ca²⁺ concentrations tested. Data from the 0.05mM concentration was not plotted as caps were too small to measure over time. Large arrow indicates large cap. Small arrow indicates small cap. * p<0.05 cap area or time, # p<0.05 statistically different slopes. Scale bar 5µm. Data are expressed as mean ± SEM. Differences were tested by 2-way ANOVA with Bonferroni's multiple comparisons test. * p<0.05 cap area or time, # p<0.05 statistically different slopes (n= 4-7 myofibers per condition).

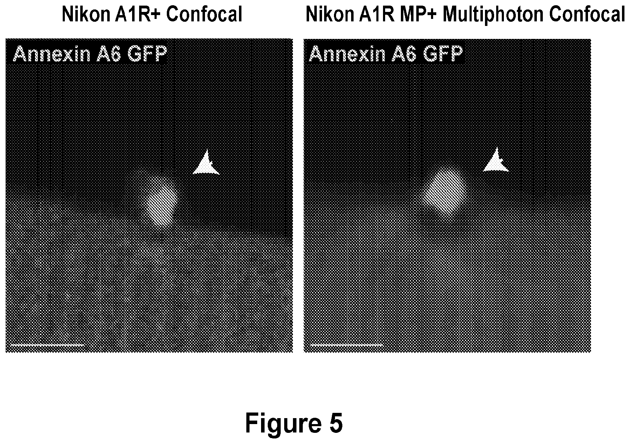

**Figure 5** shows myofiber laser injury on the Nikon A1R₊ GaSP confocal and the Nikon A1R MP+ multiphoton confocal induced comparable annexin A6-GFP repair caps (arrowhead) at the site of membrane injury. Scale 5µm. Myofibers from n≥ 3 mice per condition.

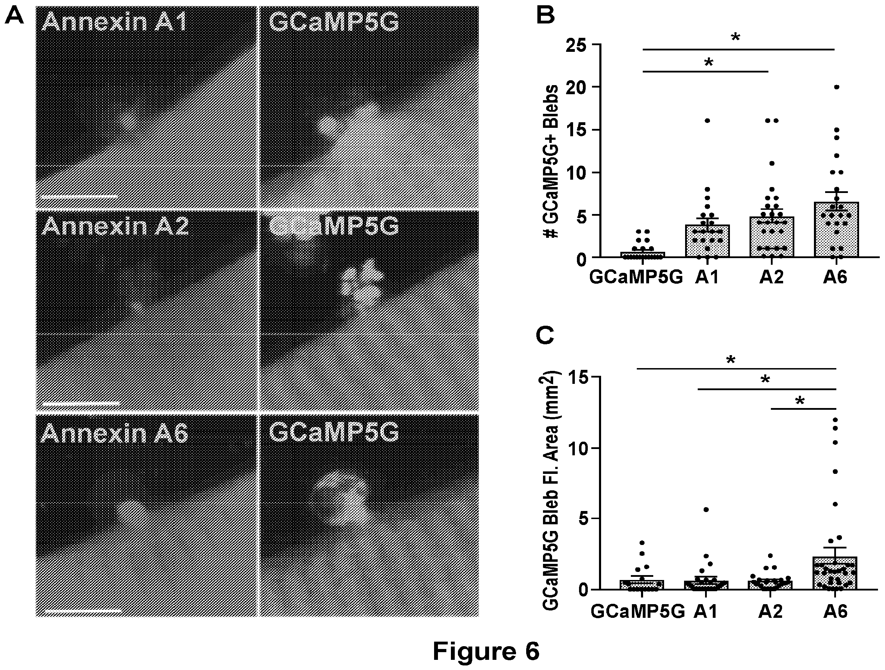

**Figure 6** shows that annexin expression promoted release of blebs from the site of myofiber repair. Myofibers were electroporated with the Ca²⁺ indicator GCaMP5G with or without tdTomato-labeled annexin A1, annexin A2, or annexin A6. Ca²⁺ area and fluorescence were assessed after membrane damage. A) High magnification z-projection images illustrate external blebs filled with the Ca²⁺ indicator emanating from the lesion when annexin A1, A2, or A6 was co-expressed and a corresponding reduction of Ca²⁺ indicator within the myofiber when compared to GCaMP5G alone. B) Expression of annexin A6 or A2 resulted in an increased number of GCaMP5G-positive blebs. C) Expression of annexin A6 resulted in the formation of the largest GCaMP5G-positive blebs. * p<0.05. (myofibers from n≥3 mice per condition).

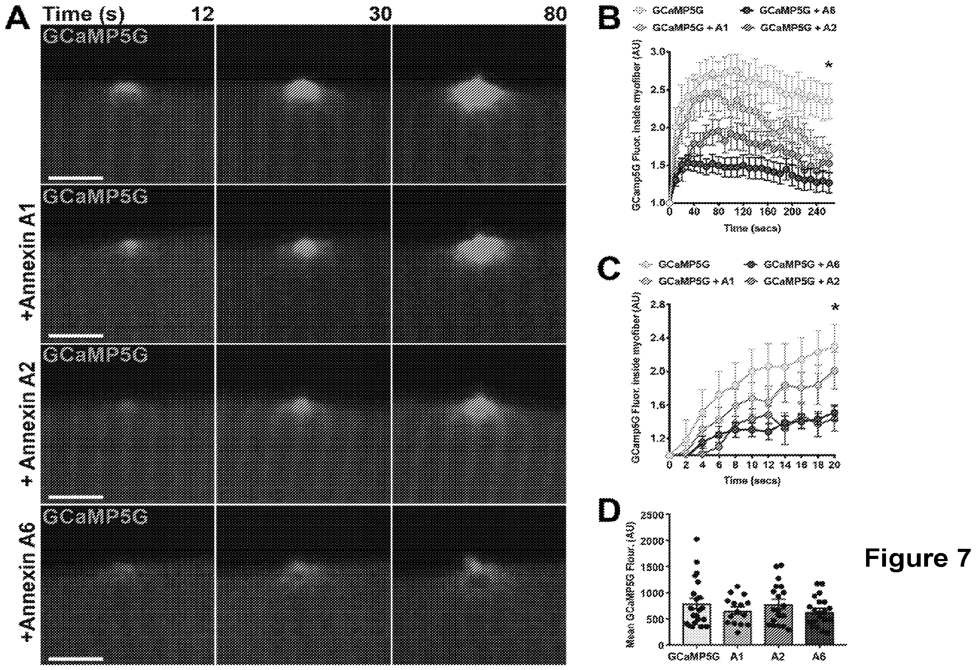

**Figure 7** shows that annexin expression reduced Ca²⁺ within the myofiber. Myofibers were electroporated with the Ca²⁺ indicator GCaMP5G with or without tdTomato-labeled annexin A1, annexin A2, or annexin A6 (annexin channel not shown). Ca²⁺ area and fluorescence were assessed after membrane damage. A) Time-lapse single slice images illustrate co-expression of either annexin A1, A2 or A6 resulted in a significant reduction in GCaMP5G fluorescence measured inside the myofiber at the site of injury over time. B) Expression of either annexin A1, A2 or A6 resulted in a significant reduction in GCaMP5G fluorescence measured inside the myofiber at the site of injury over 240 seconds of imaging, with annexin A6 inducing the greatest reduction in GCaMP5G fluorescence. C) Both annexin A2 and A6 contributed to the early reduction in GCaMP5G fluorescence as seen by imaging during the first 20 seconds after injury. D) Initial GCaMP5G mean fluorescence is not significantly different between groups. Scale bar 5µm. * p<0.05. Myofibers from n≥3 mice per condition. Data are expressed as mean ± SEM. Differences were tested by 2-way-ANOVA test with Bonferroni's multiple comparisons test (B, C) or one-way ANOVA test with Tukey's multiple comparisons test (D). * p<0.05, (n≥9 myofibers from n≥3 mice per condition).

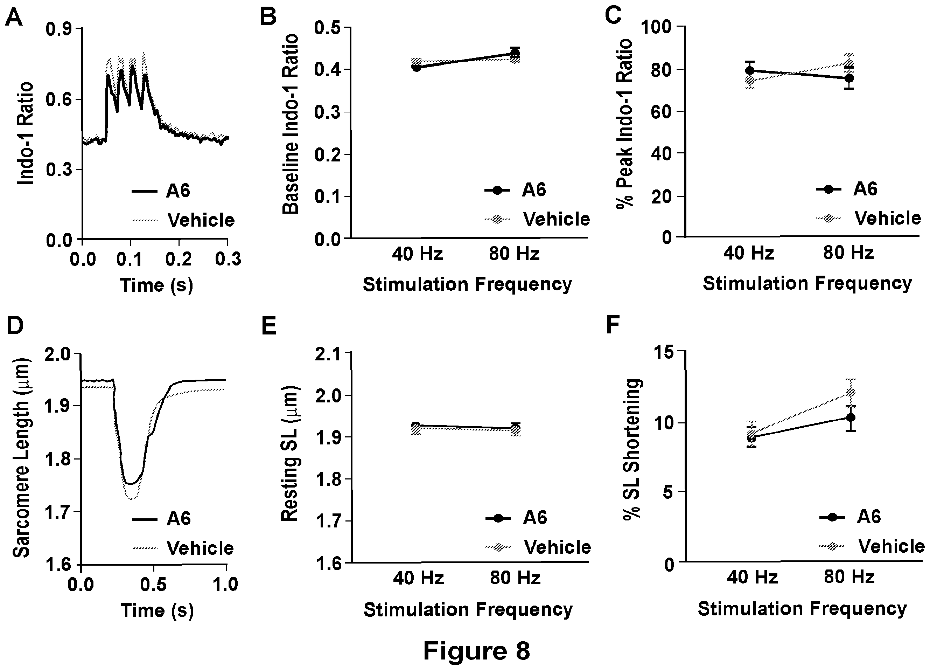

**Figure 8** shows that baseline Ca²⁺ is not changed in myofibers overexpressing annexin A6. Myofibers electroporated with annexin A6-GFP or vehicle control. Isolated myofibers were loaded with Indo-1 AM dye and stimulated to measure Ca²⁺ cycling and cell shortening. A) Representative Ca²⁺ transient at 80Hz. B & C) Resting Ca²⁺ levels and peak Ca²⁺ transient values were not changed between myofibers electroporated with annexin A6-GFP or vehicle control. D) Representative sarcomere length shortening traces. E & F) Resting sarcomere length and peak unloaded sarcomere length shortening did not differ between treatment groups. Myofibers from n≥3 mice per condition. Data are expressed as mean ± SEM. Differences were tested by 2-way ANOVA (A, D) or two-tailed t-test (B, C, E, F), (n≥30 myofibers from n≥3 mice per condition).

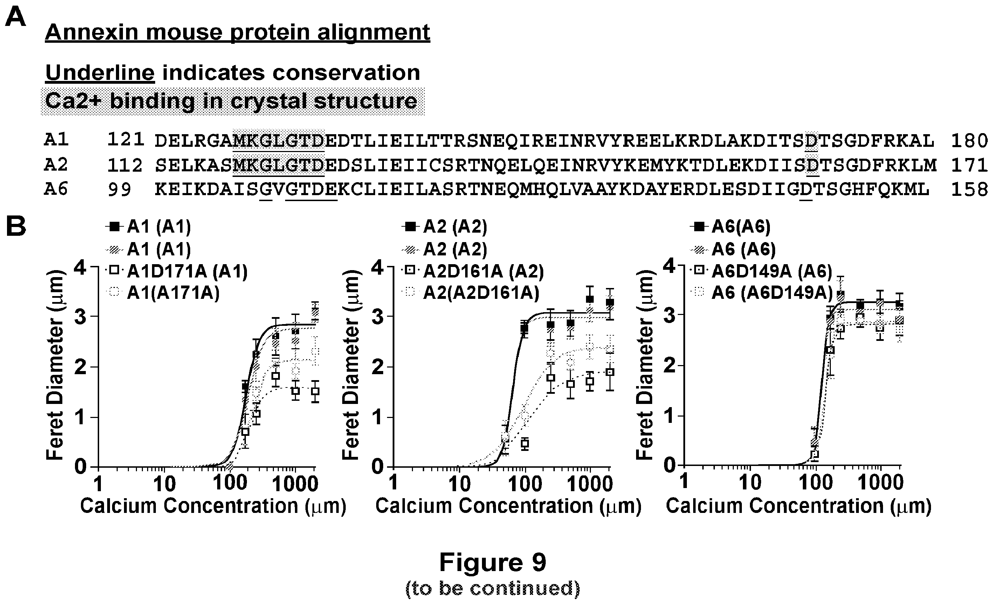

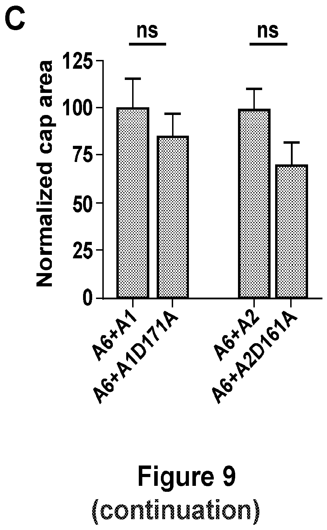

**Figure 9** shows A) The first type II Ca²⁺-binding site in annexin A1 and A2 is conserved, while it is not conserved in annexin A6. B) Myofibers were co-electroporated with wildtype+wildtype or wildtype+mutant annexin constructs and cap size was assessed after sarcolemmal injury. Parentheses indicate protein that is co-expressed in the myofiber, but not visualized within the channel, to determine the effect on the co-expressed annexin. Cap kinetics were plotted as cap feret diameter over a range of Ca²⁺ concentrations, from 0-2mM. Expression of mutant annexin A1D171A and A2D161A, but not A6D149A was sufficient to significantly reduce the co-expressed wildtype annexin cap maximum diameter (DMAX). C) Expression of annexin A1D171A and A2D161A was not sufficient to significantly reduce the cap area of co-expressed annexin A6, although annexin A2D161A results were trending. #, * p<0.05 ns= non-significant. Myofibers from n≥3 mice per condition. Data are expressed as mean ± SEM. Differences were tested by 2-way ANOVA with Bonferroni's multiple comparisons test (B) or two-tailed t-test (C). * p<0.05 #, ns = non-significant, (n≥5 myofibers from n≥3 mice per condition).

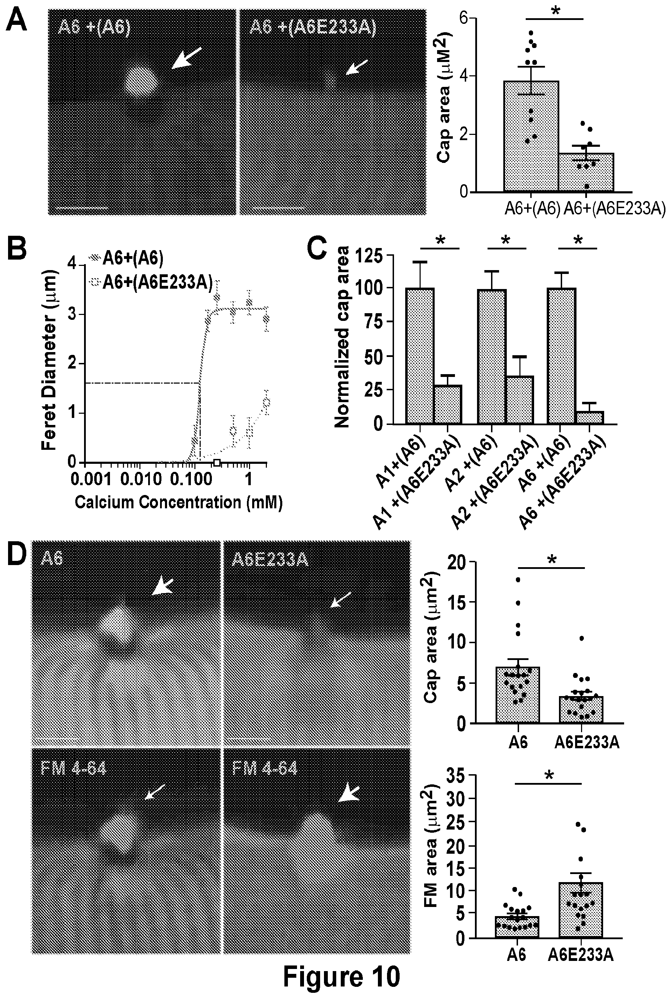

**Figure 10** shows annexin A6 Ca²⁺ binding mutant reduced annexin repair cap recruitment and decreased myofiber membrane repair capacity. A) Myofibers were co-electroporated with wildtype-tdTomato and either wildtype-GFP or mutant-GFP annexin constructs. Cap size was assessed after membrane damage and only the red channel is shown to demonstrate the effect on wildtype annexin. B) Co-expression of mutant annexin A6E233A was sufficient to reduce wildtype annexin A6 cap assembly. Cap kinetics were plotted as cap feret diameter over a range of Ca²⁺ concentrations, from 0-2mM. C) Co-expression of annexin A6E233A was sufficient to significantly reduce the cap area of co-expressed annexin A1, A2 and A6. * p<0.05 WT (WT) vs. WT (MUT). D) Myofibers were electroporated with annexin A6-GFP or mutant A6E233A-GFP. Annexin A6E233A cap area was significantly smaller compared (small arrow) to annexin A6 (large arrowhead), correlating with increased FM 4-64 fluorescence area (large arrowhead). Scale bar 5µm. * p<0.05. Myofibers from n≥3 mice per condition. Data are expressed as mean ± SEM. Differences were tested by two-tailed t-test (A, C, D) * p<0.05, (n = 4-18 myofibers from n≥3 mice per condition).

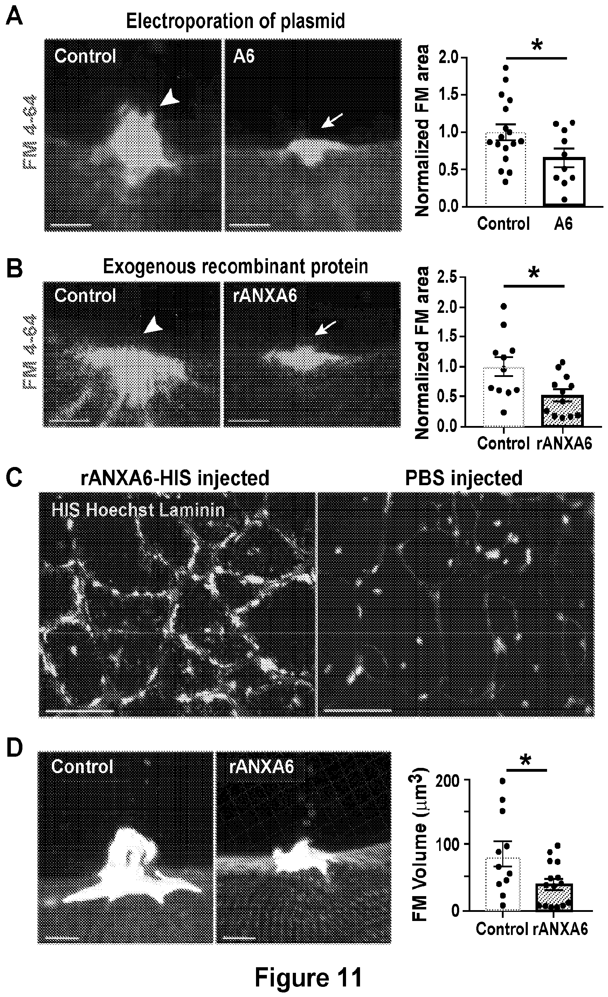

**Figure 11** shows annexin A6 enhanced myofiber membrane repair capacity. A) Overexpression of annexin A6 in wildtype myofibers reduced FM 4-64 dye uptake, a marker of membrane damage, after laser-induced injury as compared to control myofibers. B) Wildtype myofibers injured in the presence of extracellular recombinant annexin A6 (rANXA6) had significantly smaller FM 4-64 dye uptake than control myofibers. C) rANXA6 with a C-terminal HIS tag localized to the plasma membrane of mdx/hLTBP4 myofibers 6 hours post systemic injection, visualized by immunofluorescence microscopy. Anti-HIS staining was not visible in control muscle from mice injected with phosphate buffered saline (PBS). D) mdx/hLTBP4 myofibers injured in the presence of extracellular rANXA6 had significantly smaller FM 4-64 dye uptake (volume) than control myofibers depicted by the Imaris surface rendering of FM 4-64 fluorescence. Scale bar 5µm (A, B, D). Scale bar 1mm (C). * p<0.05. (myofibers from n≥3 mice per condition).

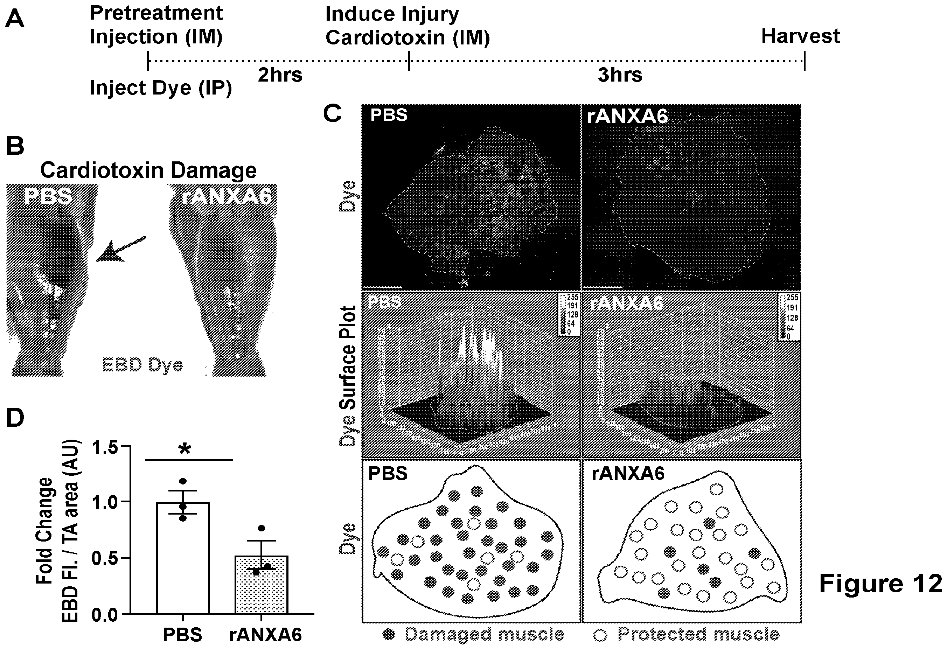

**Figure 12** shows that intramuscular injection of recombinant annexin A6 (rANXA6) protected against muscle damage *in vivo.* A) Tibialis anterior muscles of wildtype mice were pre-injected with rANXA6 or control solution and then damaged with cardiotoxin to induce muscle injury. B) Gross imaging revealed decreased dye uptake in rANXA6 pretreated muscle compared to the contralateral control muscle. C) Immunofluorescence imaging revealed decreased dye uptake in muscle pretreated with rANXA6. Surface plots of dye uptake depict reduced fluorescence in muscle pretreated with rANXA6. White dotted lines outline the muscle sections. D) Muscle pretreated with rANXA6 had a significant reduction, approximately 50%, of dye fluorescence over muscle area compared to control muscle. Scale 1mm. * p>0.05 (from n=3 mice per condition). Data are expressed as mean ± SEM. Differences were tested by two-tailed t-test * p<0.05, (n=3 mice per condition).

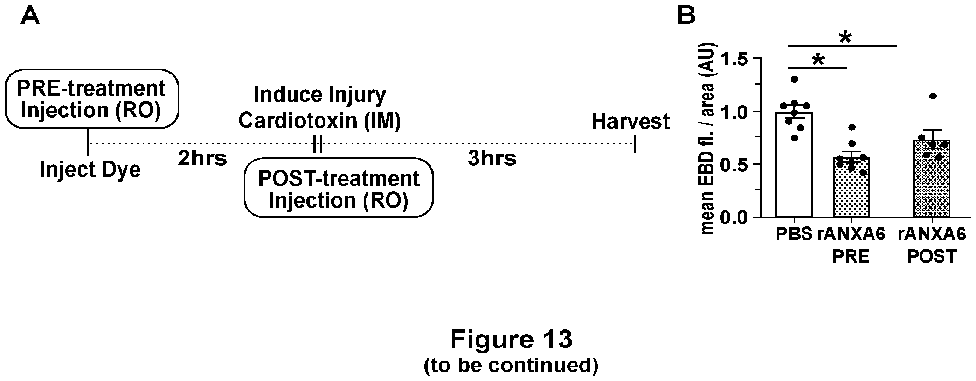

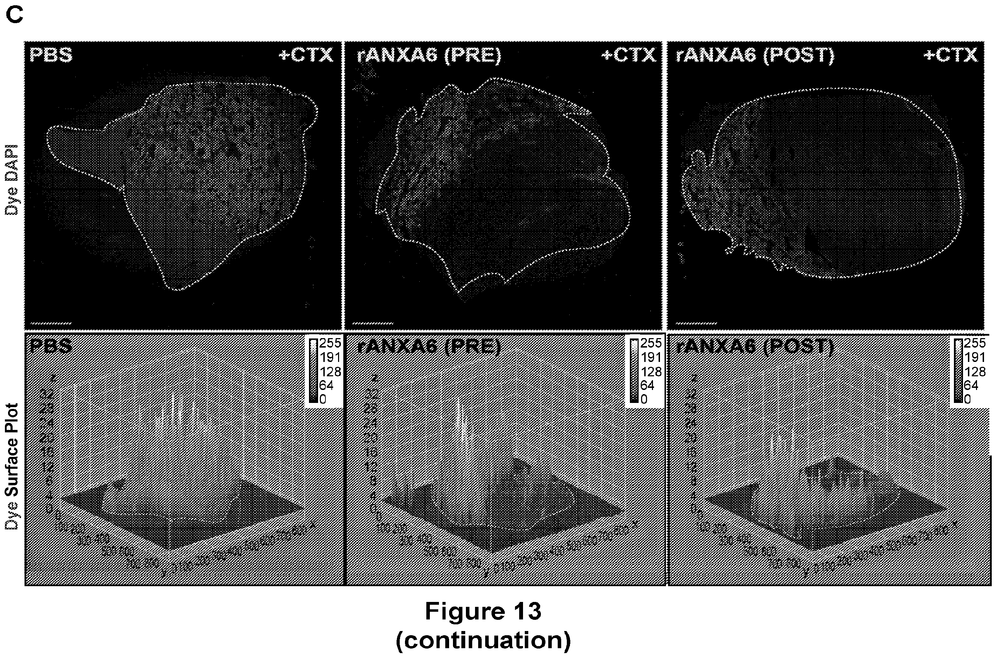

**Figure 13** shows that systemic injection of recombinant annexin A6 (rANXA6) protected against muscle damage *in vivo.* A) Wildtype mice were systemically injected with rANXA6 or control solution prior to cardiotoxin-induced muscle injury (pre-injected) or after cardiotoxin-induced muscle injury (post-injury). B-C) Immunofluorescence imaging revealed a significant decrease in dye uptake in Tibialis muscle pretreated or post-treated with rANXA6 compared to vehicle control. White dotted lines outline the muscle sections. Surface plots of dye uptake depict reduced fluorescence in muscle pretreated with rANXA6. Scale 1mm. * p>0.05 (from n ≥ 3 mice, n ≥ 6 legs per condition).

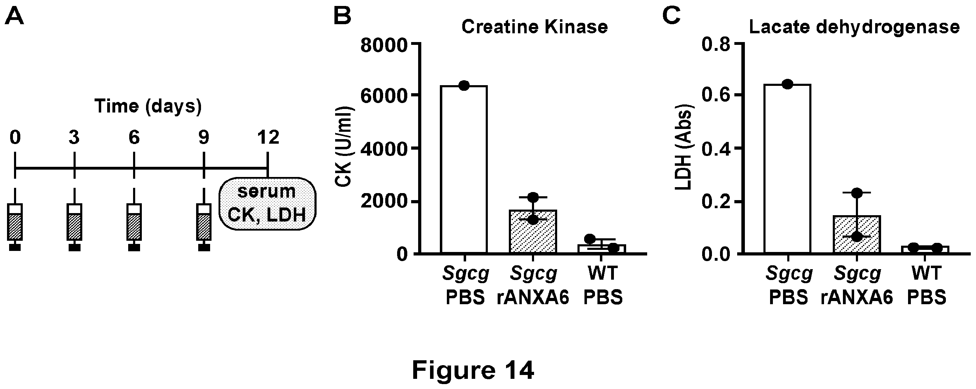

**Figure 14** shows that systemic injection of recombinant annexin A6 (rANXA6) protected against chronic muscle damage *in vivo.* A) Sgcg-null mice, a model of limb girdle muscular dystrophy 2C (LGMD2C), were injected with rANXA6 or control solution every 3 days for a total of 12 days. B-C) Creatine kinase (CK) and lactate dehydrogenase (LDH), clinically validated serum biomarkers of muscle injury, were then evaluated. Treatment with rANXA6 reduced serum CK and LDH compared to control, indicating enhanced repair of chronically injured muscle tissue.

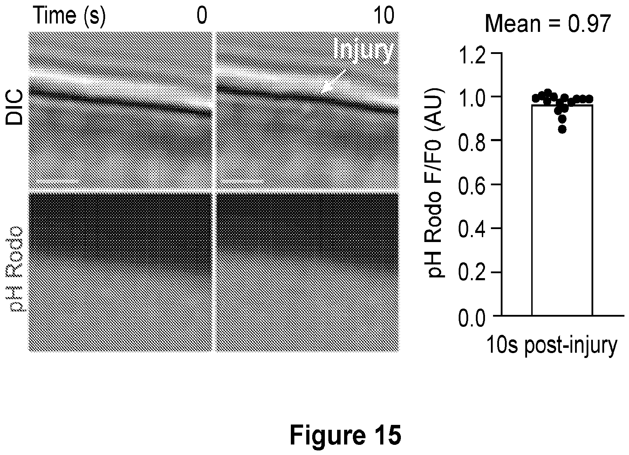

**Figure 15** shows the lack of visible pH change immediately after membrane injury. Myofibers were laser injured in the presence of the pH fluorescence indicator pH Rodo AM. The pH remained unchanged between preinjury (0s) and post-injury (10s) expressed as F/F0 mean = 0.97, where a value of 1 is identical. Scale 5µm.

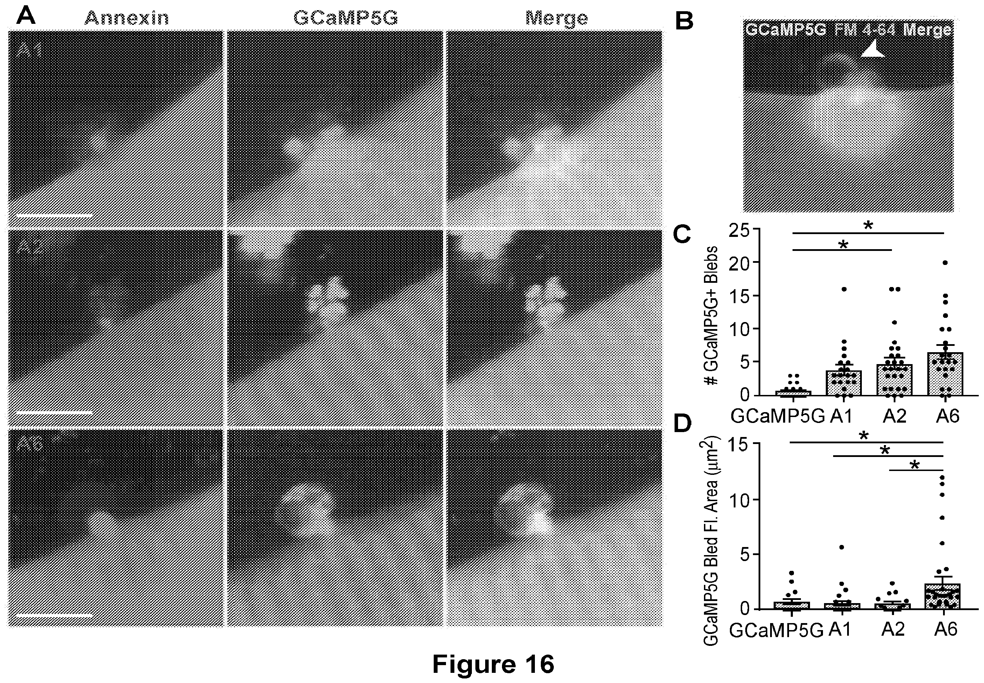

**Figure 16** shows annexin expression promoted release of blebs from the site if myofiber repair. Myofibers were electroporated with the Ca²⁺ indicator GCaMP5G (green) with or without tdTomato-labelled annexin A1, annexin A2, or annexin A6. Ca²⁺ area and fluorescence were assessed after membrane damage. (A) High magnification z-projection images illustrate external blebs filled with the Ca²⁺ indicator emanating from the lesion when annexin A1, A2, or A6 was co-expressed and a corresponding reduction of Ca²⁺ indicator within the myofiber when compared to GCaMP5G alone (B). (B) Membrane marked by FM 4-64 shows GCaMP5G-negative vesicles form in the absence of annexin overexpression. (C) Expression of annexin A6 or A2 resulted in an increased number of GCaMP5G-positive blebs. (D) Expression of annexin A6 resulted in the formation of the largest GCaMP5G-positive blebs. Data are expressed as mean ± SEM. Differences were tested by one-way-ANOVA test with Tukey's multiple comparisons test *p<0.05, (n≥16 myofibers from n≥3 mice per condition).

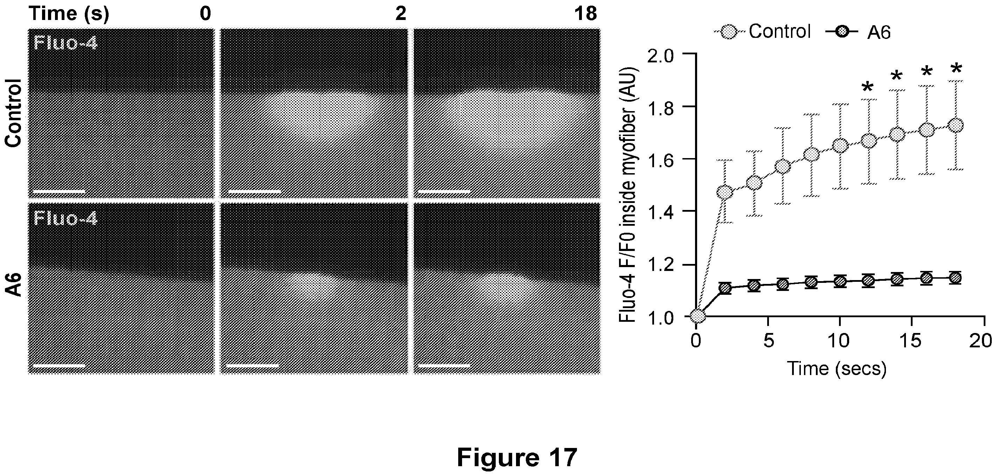

**Figure 17** shows decreased Fluo-4 Ca²⁺ levels at that site of injury with annexin A6 expression. Myofibers were preloaded with the fluorescent Ca²⁺ indicator dye, Fluo-4, and the sarcolemma subsequently injured with a confocal laser. Myofibers overexpressing annexin A6 had significantly decreased levels of internal Fluo-4 Ca²⁺ fluorescence at the site of membrane injury compared to control myofibers, similar to results obtained with the protein-based Ca²⁺ indicator GCaMP5G. Scale 5µm. Data are expressed as mean ± SEM. Differences were tested by 2-way ANOVA with Bonferroni's multiple comparisons test * p<0.05, (n=3 mice; n≥ 8 myofibers per condition).

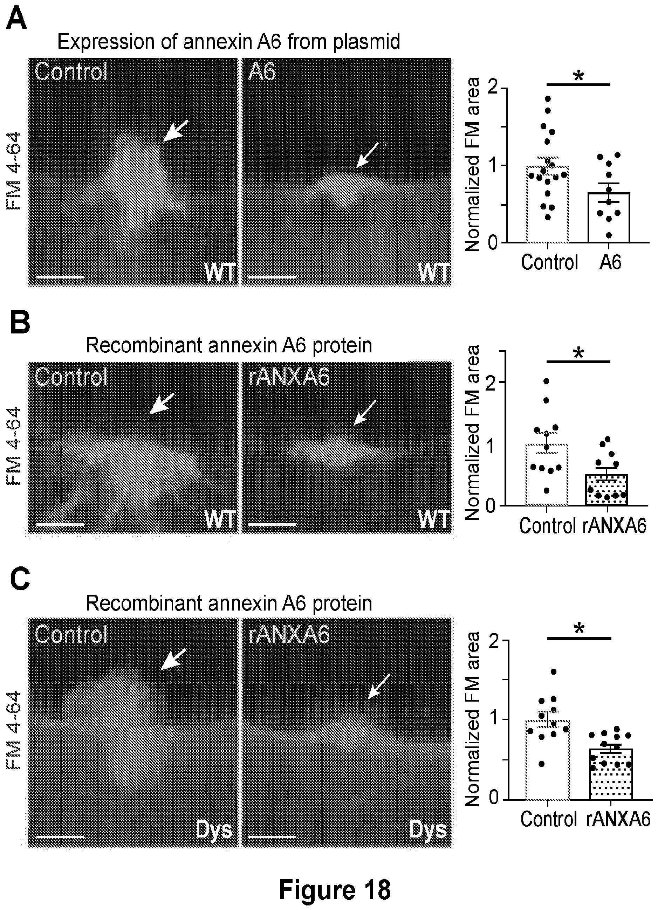

**Figure 18** shows that annexin A6 enhanced membrane repair capacity of healthy and dystrophic myofibers in vitro. (A) Plasmid expression of annexin A6 in wildtype (WT) myofibers reduced FM 4-64 dye uptake, a marker of membrane damage, after laser-induced injury as compared to control myofibers. (B) Wildtype myofibers injured in the presence of extracellular recombinant annexin A6 (rANXA6) had significantly less FM 4-64 dye uptake compared to control myofibers. (C) Dystrophic (Dys) myofibers injured in the presence of recombinant annexin A6 (rANXA6) had significantly less FM 4-64 dye uptake than control myofibers. Scale 5µm. Data are expressed as mean ± SEM. Differences were tested by two-tailed t-test * p<0.05, (n≥10 myofibers from n≥3 mice per condition).

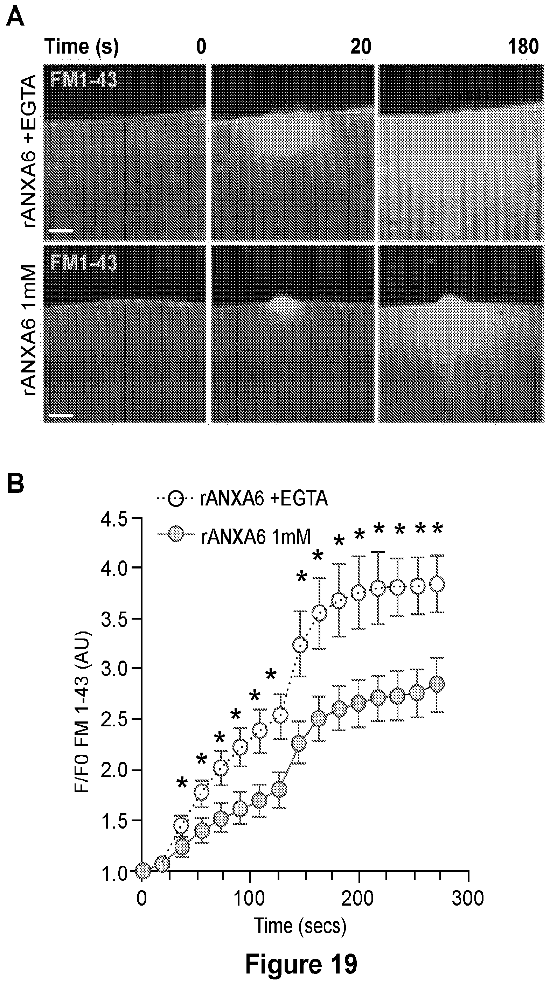

**Figure 19** shows Ca²⁺-dependency of the protective effects of recombinant annexin A6. A and B) Wildtype myofibers were isolated and loaded with the fluorescence marker of membrane damage, FM 1-43 (green). Myofibers were pretreated with recombinant annexin A6 (rANXA6) then damaged in 1mM Ca²⁺ solution or 0mM Ca²⁺ + EGTA, a calcium chelator. FM 1-43 fluorescence uptake over time was significantly reduced at 1mM Ca²⁺ compared to when EGTA was present. Scale 5µm. Data are expressed as mean ± SEM. Differences were tested by 2-way ANOVA with Bonferroni's multiple comparisons test * p<0.05, (n≥ 10 myofibers per condition; n=3 mice per condition).

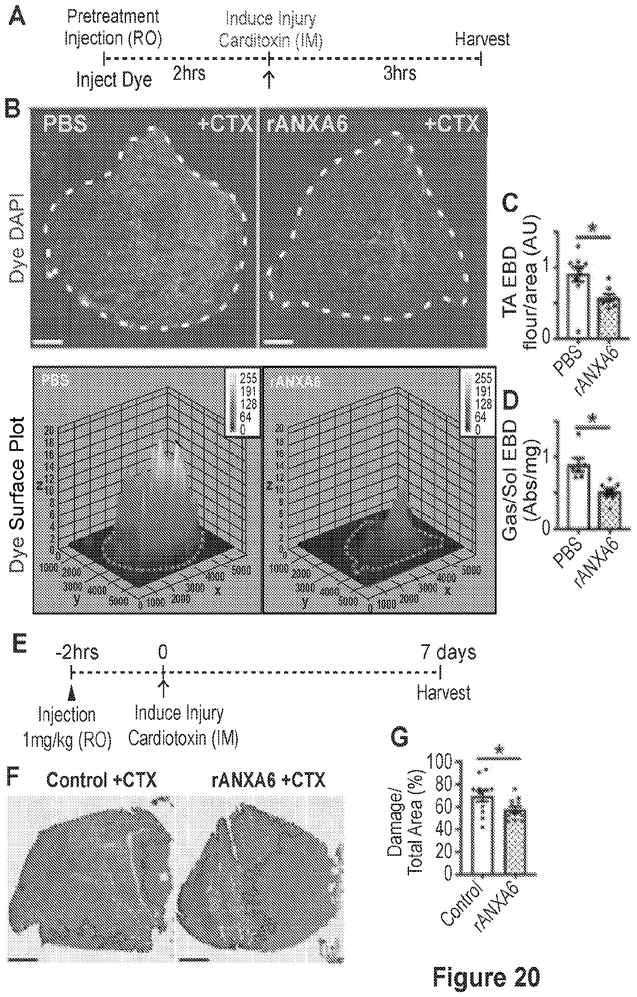

**Figure 20** shows systemic delivery using retro-orbital injection of recombinant annexin A6 protected against muscle damage in vivo. (A) Wildtype mice were injected intravenously with recombinant human annexin A6 (rANXA6) or control solution. Following this, muscles were damaged with cardiotoxin (CTX). (B-C) Immunofluorescence imaging revealed approximately 38% less dye uptake (red) in muscle pretreated with rANXA6. Dotted lines outline the tibialis anterior muscle sections (top panel). DAPI (blue) marks nuclei. Surface plots of dye uptake depict reduced fluorescence in muscle pretreated with rANXA6. (D) Whole tissue spectroscopic analysis of injured gastrocnemius/soleus muscles revealed a 58% reduction in dye uptake with rANXA6 pretreatment compared to control muscle. (E) Wildtype mice were injected intravenously with rANXA6 or control solution. Two hours later tibialis anterior muscles were damaged with cardiotoxin. Seven days post injury muscles were harvested. (F & G) Hematoxylin and eosin images were quantified and show a reduction in percent myofiber damage (dotted lines), in rANXA6 treated mice compared to controls. B, Scale 1mm. F, Scale 500µm. Data are expressed as mean ± SEM. Differences were tested by two-tailed t-test * p<0.05, (B, C, D n≥3 mice, n≥6 legs per condition; F, G n≥6 mice; n≥11 muscles per condition).

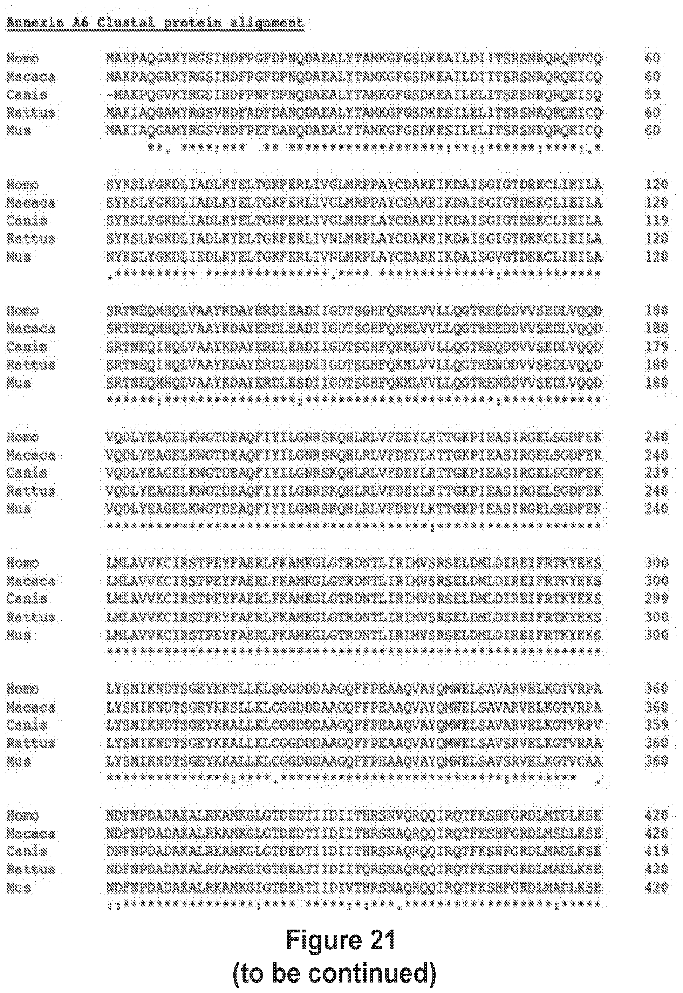

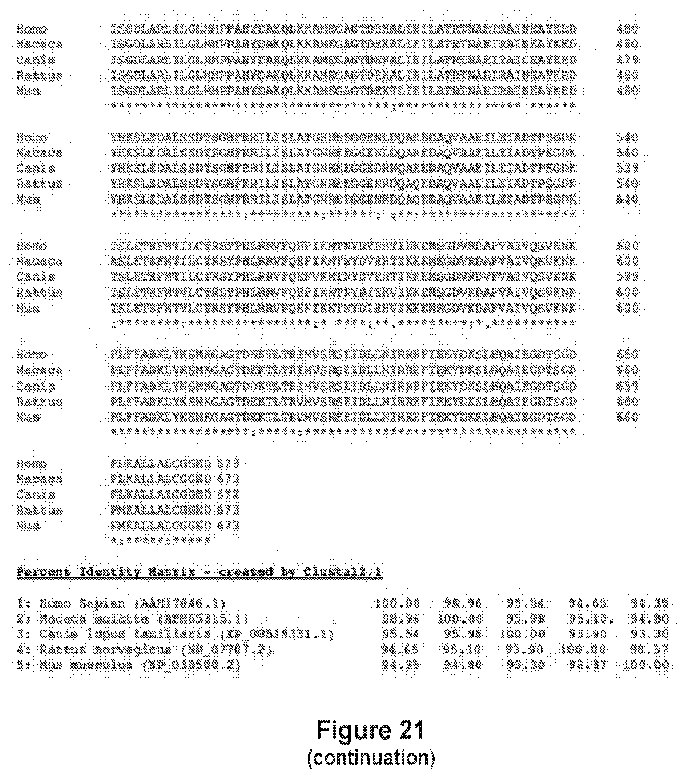

**Figure 21** depicts the amino acid conservation of annexin A6.

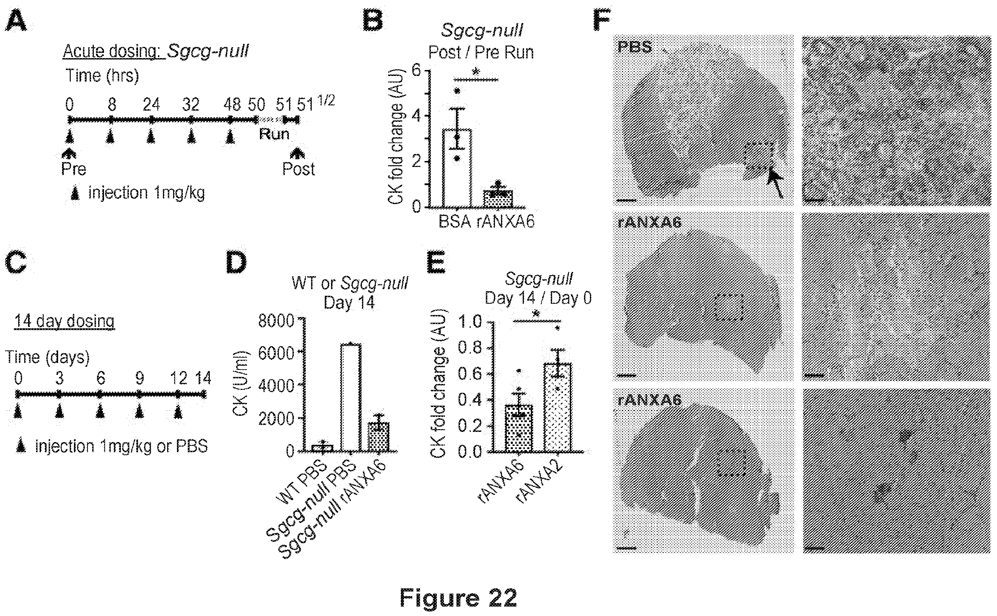

**Figure 22** shows that recombinant annexin A6 protected against muscle damage in a mouse model of muscular dystrophy *in vivo.* (A-B) Sgcg-null mice, a model of Limb Girdle Muscular Dystrophy 2C, were injected intravenously with recombinant human annexin A6 (rANXA6) or BSA control solution five times over 48 hours. Prior to injections, serum CK was measured. Two hours after the 5th injection, mice were subjected to 60 minutes of downhill running. Thirty minutes post exercise, serum CK was measured. The fold change in CK post/pre running was significantly reduced with rANXA6 administration compared to BSA-injected controls, consistent with a reduction in muscle injury from acute running. (C-D) Sgcg-null mice were injected intravenously every 3 days over 14 days with rANXA6 or control. On day 14, serum creatine kinase was evaluated. Serum CK in Sgcg-null mice treated with rANXA6 was lower than PBS control. (E) Sgcg-null mice were injected intravenously every 3 days for 14 days with rANXA6 or recombinant annexin A2. The serum CK fold change post / pretreatment (Day 14 / Day 0) was significantly reduced in Sgcg-null mice treated with recombinant annexin A6 compared to annexin A2. (F) Histological analysis of gastrocnemius/soleus muscles from Sgcg-null mice shown in part D injected with PBS or recombinant annexin A6. Low magnification on the left and high magnification on the right of boxed area. F, Scale 500µm (left), Scale 50µm (right). Data are expressed as mean ± SEM. Differences were tested by two-tailed t-test (B & E) * p<0.05, (n≥3 mice per condition, except part D).

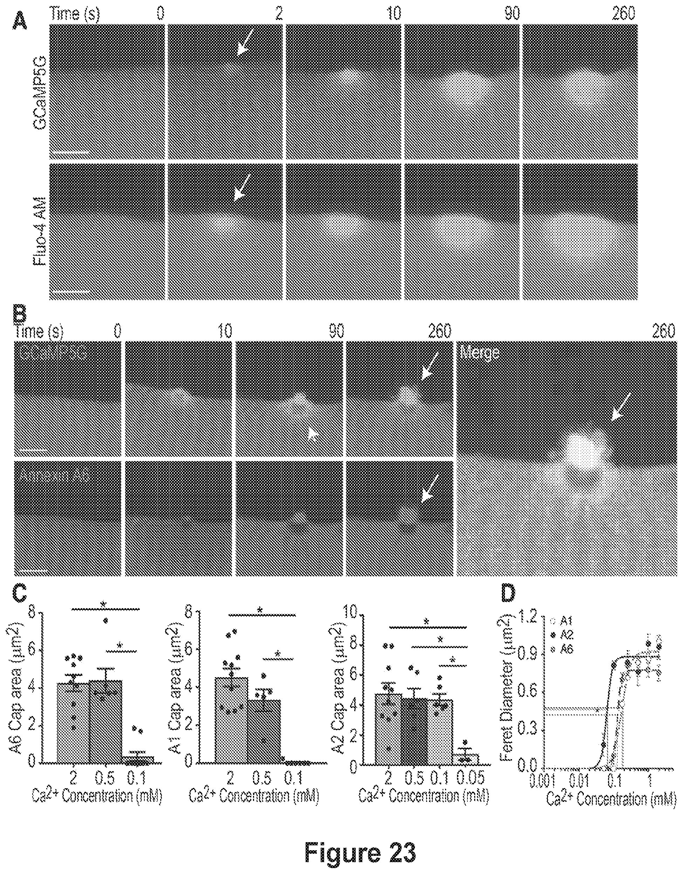

**Figure 23** shows Ca²⁺-dependent annexin repair cap recruitment at the site of injury. Myofibers were generated to express the Ca²⁺ indicator GCaMP5G (green), and time-lapse single slice images were assessed at time points after membrane disruption. (A) GCaMP5G fluorescence was present at the site of injury, at 2 seconds (arrow), indicating the presence of Ca²⁺ immediately after damage at the site of injury (top panel). These data were validated with a non-protein Ca²⁺ indicator, Fluo-4 AM (green, bottom panel). (B) Time-lapse images of myofibers co-electroporated with GCaMP5G and annexin A6-tdTomato (A6, red). GCaMP5G fluorescence was present at the site of injury localized around the annexin A6-free zone (arrowhead) and at the annexin A6 cap (arrow). GCaMP5G colocalized (merge, yellow, arrow) with the annexin A6 repair cap. Scale bar 5µm. (C) Myofibers expressing fluorescently tagged annexins A1, A2 or A6 were injured at multiple Ca²⁺ concentrations. Annexin A1 and A6 repair cap size was reduced at 0.1mM Ca²⁺ compared to 2mM and 0.5mM. Annexin A2 repair cap area was significantly reduced at 0.05mM Ca²⁺compared to 2mM, 0.5mM, and 0.1mM Ca²⁺. (D) Cap kinetics were plotted as cap feret diameter over a range of Ca²⁺ concentrations. Annexin A2 had a statistically significant leftward shift in Km_{1/2} followed by annexin A6 then A1. Scale 5µm. Data are expressed as mean ± SEM. Differences were tested by one-way-ANOVA test with Tukey's multiple comparisons test (C) *p<0.05, (n≥5 myofibers per condition).

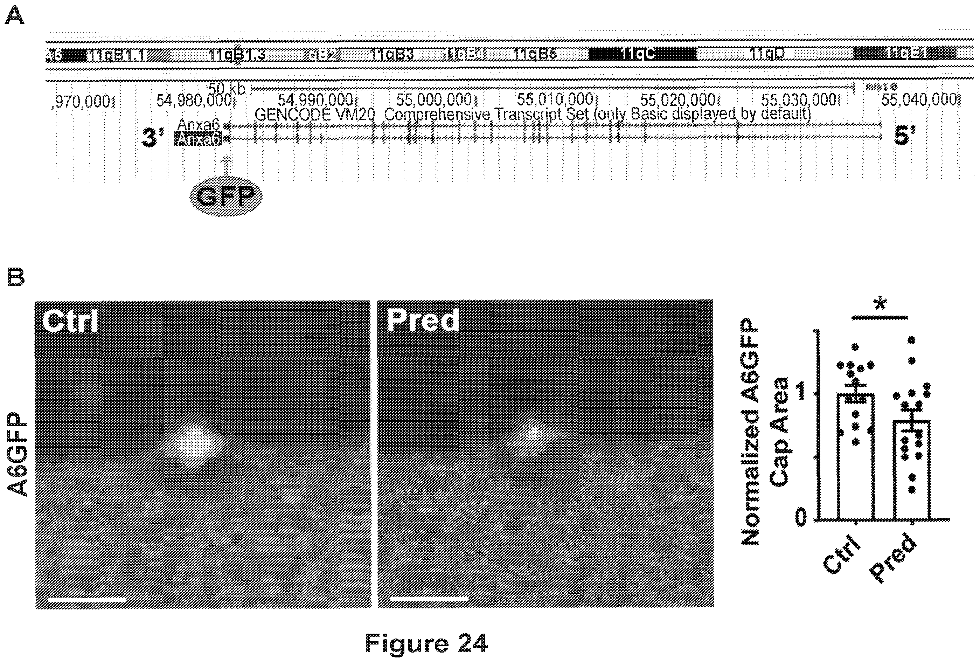

**Figure 24** shows genetically-encoded annexin A6GFP responds to prednisone exposure. A) Targeting strategy for generating genetically-encoded annexin A6GFP in mice. B) Mice were injected with the prednisone (pred) or control (Ctrl) into the intraperitoneal cavity. Twenty-four hours post injection, myofibers were isolated and subjected to laser injury. Annexin A6GFP repair cap size was reduced in response to prednisone administration, indicative of protection against injury. Scale 5µm. * p<0.05. t-test n≥3 mice per treatment.

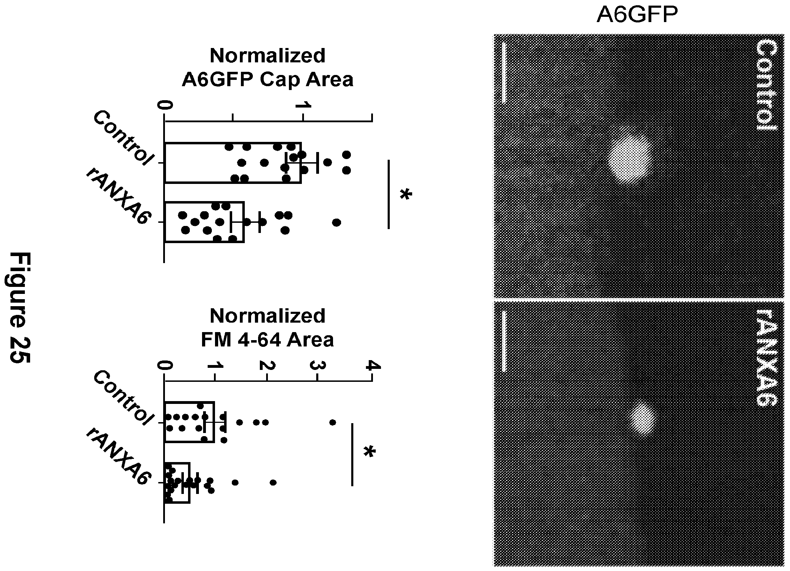

**Figure 25** shows recombinant annexin A6 pretreatment reduces genetically-encoded annexin A6GFP cap size and dye influx, indicating enhanced protection against injury. Myofibers were isolated, pretreated with recombinant annexin A6 (rANAXA6) and subsequently subjected to laser-induced membrane injury. Z-stack imaging revealed annexin A6GFP repair cap size was reduced in the presence of recombinant annexin A6, indicative of protection against injury. This correlated with a reduction in FM 4-64 uptake, a marker of membrane injury, in the presence of rANXA6. Scale 5µm. *p<0.05. t-test. n≥3 mice per treatment.

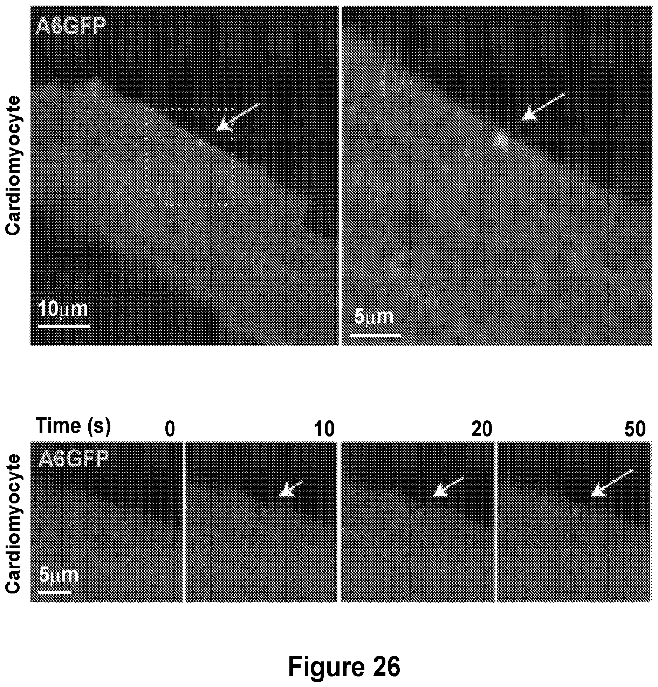

**Figure 26** shows genetically-encoded annexin A6GFP localizes at the site of cardiomyocyte membrane injury. Adult cardiomyocytes were isolated from *Anxa6^{em1(GFP)}* mice and subsequently laser-damaged. Annexin A6GFP (green) localizes in a repair cap (white arrow, left image) at the site of injury. Area within the dotted box is magnified on the right. Timelapse imaging shows that annexin A6GFP localizes in a repair cap (white arrow) at the site of injury over 50 seconds of imaging, in adult cardiomyocytes isolated from *Anxa6^{em1(GFP)}* mice. Scale 5µm or 10µm.

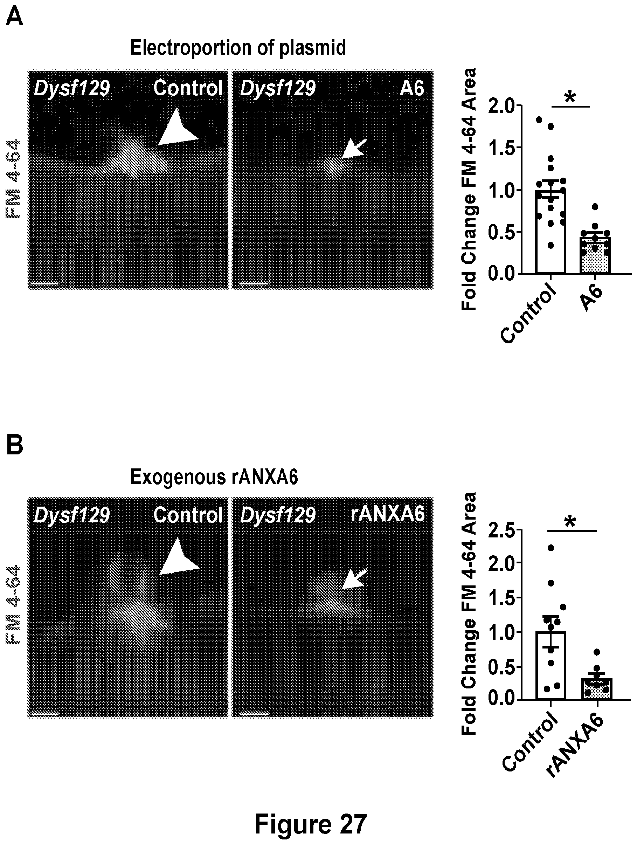

**Figure 27** shows annexin A6 enhanced membrane repair capacity of dystrophic, dysferlin-null myofibers *in vitro,* protecting against injury. A) Plasmid expression of annexin A6 in dysferlin-null (Dysf129) myofibers reduced FM 4-64 dye uptake (small arrow), a marker of membrane damage, after laser-induced injury as compared to control myofibers (arrowhead). B) Pretreatment with recombinant annexin A6 decreased FM 4-64 uptake (small arrow) compared to control (arrowhead) after laser injury in dysferlin-null (Dysf129) myofibers. Scale 5µm. Data are expressed as mean ± SEM. Differences were tested by two-tailed t-test * p<0.05, (n≥9 myofibers per condition).

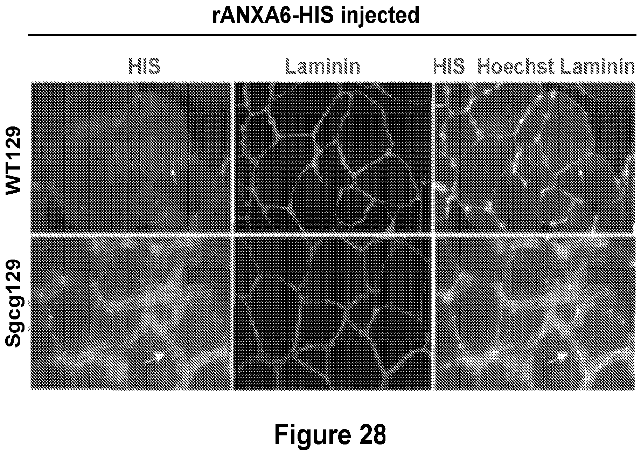

**Figure 28** shows recombinant Annexin A6 localizes to the sarcolemma of dystrophic, of Limb girdle muscular dystrophy 2C muscle. Recombinant annexin A6 (rANXA6) with a carboxy-terminal HIS tag was injected in healthy, wildtype mice or a mouse model of Limb girdle muscular dystrophy 2C, Sgcg-null, that undergoes chronic membrane disruption. Mice were on the 129Sv/EmsJ background. anti-HIS fluorescence intensity was increased at the plasma membrane of Sgcg-null muscle, visualized through anti-laminin staining, compared to wildtype controls. Hoechst labeled nuclei.

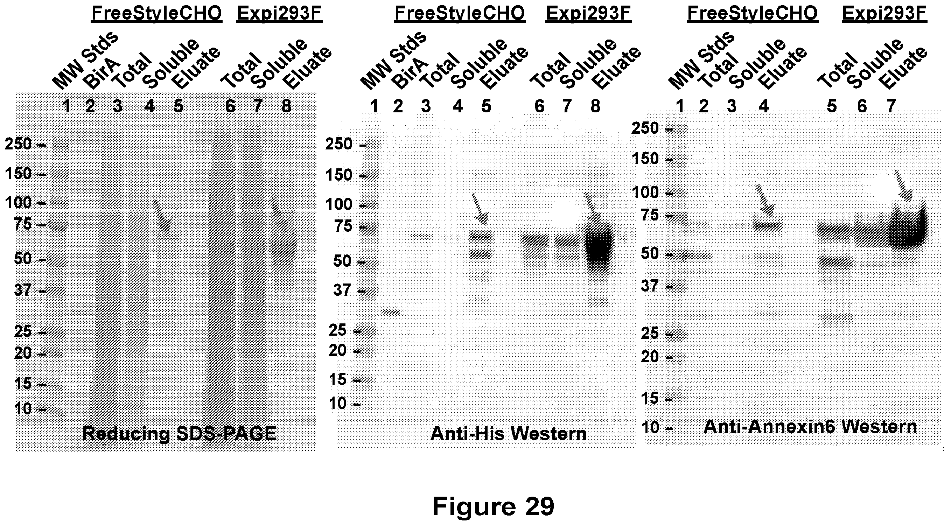

**Figure 29** shows results of SDS-PAGE (left panel) and Western blot analysis (middle and right panels) of recombinant annexin A6 protein expressed in mammalian cells. The middle panel is blotted anti-HIS as the recombinant annexin A6 was made with a C-terminal HIS epitope tag for detection and purification purposes. Right panel was blotted against A6 itself.

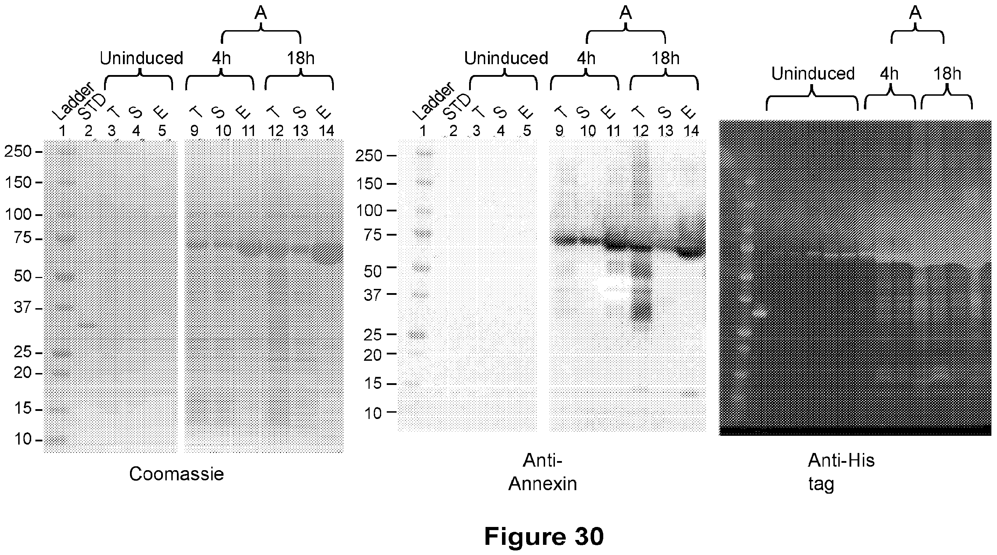

**Figure 30** shows results of SDS-PAGE (Coomassie stained) (left panel) and Western blot analysis (middle and right panels) of recombinant annexin A6 protein expressed in prokaryotic cells. Lanes in left and middle panels are labeled T (total), S (soluble), and E (eluate). The contents of the lanes in the right panel mirror those of the left and middle panels. The middle panel is blotted for annexin A6. The right panel is blotted for anti-HIS as the recombinant annexin A6 was made with a C-terminal HIS tag for purification purposes.

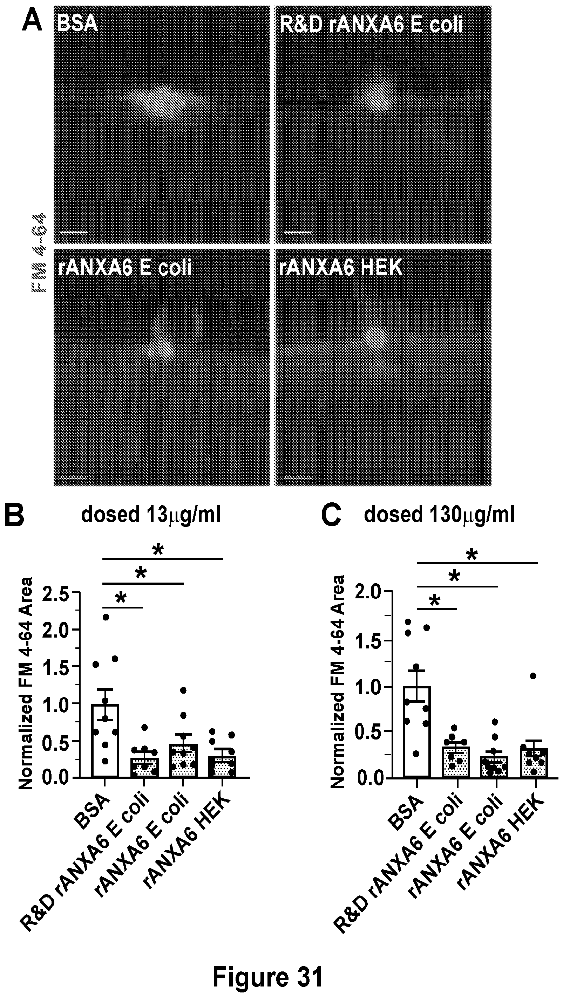

**Figure 31** shows in panels A, B, and C that Wildtype myofibers injured in the presence of extracellular recombinant annexin A6 (rANXA6), produced in *E coli* or mammalian HEK cells, had significantly less FM 4-64 dye uptake compared to BSA control treated myofibers, indicating protection against membrane injury and enhanced repair with rANXA6 treatment. (B & C) The beneficial effects of rANXA6 on membrane repair were not significantly different between *E coli* produced rANXA6, which was obtained commercially through R&D Systems or produced by a contract research organization (CRO; Evotec SE), compared to mammalian HEK cell produced rANXA6 protein at 13µg/ml and 130µg/ml. Scale = 5µm. Data are expressed as mean ± SEM. Differences were tested by two-tailed t-test * p<0.05, (n≥8 myofibers from n≥3 mice per condition).

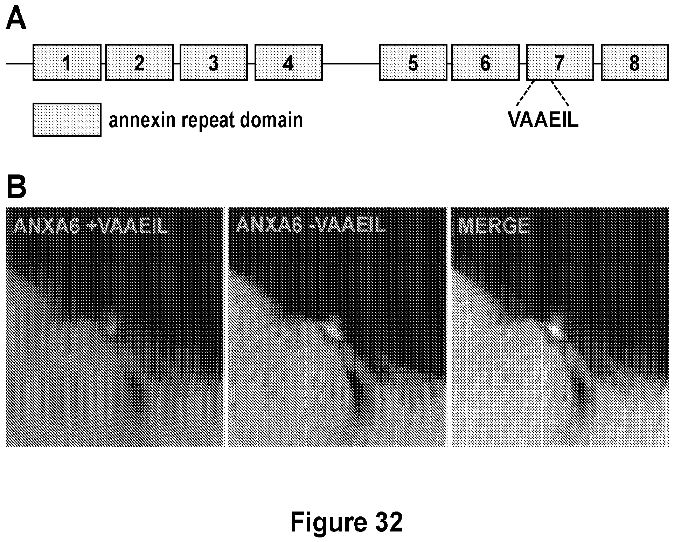

**Figure 32** demonstrates that annexin A6 lacking the amino acid sequence VAAEIL (SEQ ID NO: 47) localizes to the site of muscle membrane injury. A) Schematic showing the structure of annexin A6. The amino acid sequence, VAAEIL, is located in annexin repeat domain 7, exon 21. B) Myofibers were electroporated with GFP-labelled annexin A6 lacking VAAEIL (SEQ ID NO: 47) and tdTomato-labelled annexin A6. High magnification images show that annexin A6 lacking the VAAEIL (SEQ ID NO: 47) sequence partially colocalizes with annexin A6 in a repair cap at the site of muscle membrane injury.

### DETAILED DESCRIPTION

Membrane repair is essential to cell survival and requires a quick and efficient process to limit cellular injury. Muscle is prone to membrane disruption due to the elongated shape of muscle cells and the continuous stress brought on by muscle contractions. Mutations in genes encoding membrane-associated proteins produce fragile membranes, reduce membrane repair capacity and result in progressive muscular dystrophy. Therapeutics and methods disclosed herein to enhance membrane repair and reduce susceptibility to membrane injury are contemplated to benefit muscle in both acute and chronic injury settings.

As used herein, an agent that "increases the activity of an annexin protein" is one that increases a property of an annexin protein as a calcium-binding membrane associated repair protein that enhances restoration of membrane integrity. The enhancement to restoring membrane integrity may be through facilitating the formation of a macromolecular repair complex at the membrane lesion including proteins such as, without limitation, annexin A1 (SEQ ID NO: 1), annexin A2 (SEQ ID NO: 2 or SEQ ID NO: 3), EHD2, dysferlin, and MG53.

As used herein, the term "treating" or "treatment" refers to an intervention performed with the intention of preventing the further development of or altering the pathology of a disease or infection. Accordingly, "treatment" refers to both therapeutic treatment and prophylactic or preventative measures. Of course, when "treatment" is used in conjunction with a form of the separate term "prophylaxis," it is understood that "treatment" refers to the narrower meaning of altering the pathology of a disease or condition. "Preventing" refers to a preventative measure taken with a subject not having a condition or disease. A therapeutic agent may directly decrease the pathology of a disease, or render the disease more susceptible to treatment by another therapeutic agent(s) or, for example, the host's own cellular membrane repair system. Treatment of patients suffering from clinical, biochemical, or subjective symptoms of a disease may include alleviating one or more of such symptoms or reducing the predisposition to the disease. Improvement after treatment may be manifested as a decrease or elimination of one or more of such symptoms.

### ANNEXIN PROTEINS

The annexin protein family is characterized by the ability to bind phospholipids and actin in a Ca²⁺-dependent manner. Annexins preferentially bind phosphatidylserine, phosphatidylinositols, and cholesterol (Gerke *et al.,* 2005). In humans, dominant or recessive mutations in annexin genes have not been associated with muscle disease. However, annexin A5 genetic variants are associated with pregnancy loss (de Laat *et al.,* 2006). The annexin family is known to comprise over 160 distinct proteins that are present in more than 65 unique species (Gerke and Moss, 2002). Humans have 12 different annexin genes, characterized by distinct tissue expression and localization. Annexins are involved in a variety of cellular processes including membrane permeability, mobility, vesicle fusion, and membrane bending. These properties are Ca²⁺-dependent. Although annexins do not contain EF hand domains, calcium ions bind to the individual annexin repeat domains. Differential Ca²⁺ affinity allows each annexin protein to respond to changes in intracellular calcium levels under unique spatiotemporal conditions (Blackwood and Ernst, 1990).

Structurally, the annexin family of proteins contains a conserved carboxy-terminal core domain composed of multiple annexin repeats and a variable amino-terminal head. The amino-terminus differs in length and amino acid sequence amongst the annexin family members. Additionally, post-translational modifications alter protein function and protein localization (Goulet *et al.,* 1992; Kaetzel *et al.,* 2001). Annexin proteins have the potential to self-oligomerize and interact with membrane surfaces and actin in the presence of Ca²⁺ (Zaks and Creutz, 1991, Hayes *et al.,* 2006) , Jaiswal *et al.,* 2014)). The amino-terminal region is thought to bind actin or one lipid membrane in a Ca²⁺-dependent manner, while the annexin core region binds an additional lipid membrane.

Annexins do not contain a predicted hydrophobic signal sequence targeting the annexins for classical secretion through the endoplasmic reticulum, yet annexins are found both on the interior and exterior of the cell (Christmas *et al.,* 1991; Deora *et al.,* 2004; Wallner *et al.,* 1986). The process by which the annexins are externalized remains unknown. It is hypothesized that annexins may be released through exocytosis or cell lysis, although the method of externalization may vary by cell type. Functionally, localization both inside and outside the cell adds to the complexity of the roles annexins play within tissues and cell types. Annexin A5 is used commonly as a marker for apoptosis due to its high affinity to phosphatidylserine (PS). During cell death and injury, PS reverses membrane orientation from the inner to outer membrane, providing access for annexin binding from the cell exterior. Annexins have been shown to have anti-inflammatory, pro-fibrinolytic, and antithrombotic effects. The annexin A1-deleted mouse model exhibits an exacerbated inflammatory response when challenged and is resistant to the anti-inflammatory effects of glucocorticoids (Hannon *et al.,* 2003). The annexin A2 null-mouse develops fibrin accumulation in the microvasculature and is defective in clearance of arterial thrombi (Ling *et al.,* 2004). Although little is known about the precise function of extracellular annexins, the expression level of annexin proteins may function as a diagnostic marker for a number of diseases due to the strong correlation between high expression levels of annexins and the clinical severity of disease (Cagliani *et al.,* 2005).

### AGENTS

In some aspects, the disclosure provides methods of the disclosure contemplate treating a cellular membrane injury comprising administering to a patient in need thereof a therapeutically effective amount of a composition comprising an agent that increases the activity of an annexin protein. In further aspects, methods of delaying onset, preventing a cellular membrane injury, or enhancing recovery from cellular membrane injury are provided, comprising administering to a patient in need thereof a therapeutically effective amount of a composition comprising an agent that increases the activity of an annexin protein. "Increase the activity of an annexin protein" means that administration of the agent results in an overall increase in the activity (i.e., the increase in activity derived from administration of the agent plus any endogenous activity) of one or more annexin proteins as disclosed herein.

The term "agent" as used herein refers to a recombinant protein (e.g., a recombinant annexin protein), a steroid, an annexin peptide, and a polynucleotide capable of expressing an annexin protein.

### PROTEINS/RECOMBINANT PROTEINS

Methods of the disclosure include those in which a recombinant protein (e.g., one or more annexin proteins) is administered to a patient in need thereof in a therapeutically effective amount. Thus, in any of the aspects or embodiments of the disclosure, the agent that increases the activity of an annexin protein is a recombinant protein (e.g., an annexin protein). As used herein a "protein" refers to a polymer comprised of amino acid residues. "Annexin protein" as used herein includes without limitation a wild type annexin protein, a modified annexin protein, an annexin-like protein, or a fragment, analog, variant, fusion or mimetic, each as described herein. An "annexin peptide" is a shorter version (e.g., about 50 amino acids or less) of a wild type annexin protein, an annexin-like protein, or a fragment, analog, variant, fusion or mimetic that is sufficient to increase the overall activity of the annexin protein to which the annexin peptide is related.

Proteins of the present disclosure may be either naturally occurring or non-naturally occurring. Naturally occurring proteins include without limitation biologically active proteins that exist in nature or can be produced in a form that is found in nature by, for example, chemical synthesis or recombinant expression techniques. Naturally occurring proteins also include post-translationally modified proteins, such as, for example and without limitation, glycosylated proteins. Non-naturally occurring proteins contemplated by the present disclosure include but are not limited to synthetic proteins, as well as fragments, analogs and variants of naturally occurring or non-naturally occurring proteins as defined herein. Non-naturally occurring proteins also include proteins or protein substances that have D-amino acids, modified, derivatized, or non-naturally occurring amino acids in the D- or L- configuration and/or peptidomimetic units as part of their structure. The term "protein" typically refers to large polypeptides. The term "peptide" generally refers to short (e.g., about 50 amino acids or less) polypeptides.

Non-naturally occurring proteins are prepared, for example, using an automated protein synthesizer or, alternatively, using recombinant expression techniques using a modified oligonucleotide which encodes the desired protein.

As used herein a "fragment" of a protein is meant to refer to any portion of a protein smaller than the full-length protein expression product.

As used herein an "analog" refers to any of two or more proteins substantially similar in structure and having the same biological activity, but can have varying degrees of activity, to either the entire molecule, or to a fragment thereof. Analogs differ in the composition of their amino acid sequences based on one or more mutations involving substitution, deletion, insertion and/or addition of one or more amino acids for other amino acids. Substitutions can be conservative or non-conservative based on the physicochemical or functional relatedness of the amino acid that is being replaced and the amino acid replacing it.

As used herein a "variant" refers to a protein or analog thereof that is modified to comprise additional chemical moieties not normally a part of the molecule. Such moieties may modulate, for example and without limitation, the molecule's solubility, absorption, and/or biological half-life. Moieties capable of mediating such effects are disclosed in Remington's Pharmaceutical Sciences (1980). Procedures for coupling such moieties to a molecule are well known in the art. In various aspects, polypeptides are modified by biotinylation, glycosylation, PEGylation, and/or polysialylation.

Fusion proteins, including fusion proteins wherein one fusion component is a fragment or a mimetic, are also contemplated. A "mimetic" as used herein means a peptide or protein having a biological activity that is comparable to the protein of which it is a mimetic.

In any of the aspects or embodiments of the disclosure, the recombinant protein is an annexin protein (*e.g*., a recombinant wild type annexin protein, a modified annexin protein, an annexin-like protein, or a fragment of a wild type annexin protein or annexin-like protein that exhibits one or more biological activities of an annexin protein). By "annexin-like protein" is meant a protein having sufficient amino acid sequence identity to a referent wild type annexin protein to exhibit the activity of an annexin protein, for example and without limitation, activity as a calcium-binding membrane associated repair protein that enhances restoration of membrane integrity through facilitating the formation of a macromolecular repair complex at the membrane lesion including proteins such as annexin A1 (SEQ ID NO: 1), annexin A2 (SEQ ID NO: 2 or SEQ ID NO: 3), EHD2, dysferlin, and MG53. In some embodiments, the annexin-like protein is a protein having about or at least about 75% amino acid sequence identity with a referent wild type human annexin protein (*e.g*., annexin A1 (SEQ ID NO: 1), annexin A2 (SEQ ID NO: 2 or SEQ ID NO: 3), annexin A3 (SEQ ID NO: 4), annexin A4 (SEQ ID NO: 5), annexin A5 (SEQ ID NO: 6), annexin A6 (SEQ ID NO: 7, SEQ ID NO: 8, SEQ ID NO: 45, or a combination thereof), annexin A7 (SEQ ID NO: 9 or SEQ ID NO: 10), annexin A8 (SEQ ID NO: 11 or SEQ ID NO: 12), annexin A9 (SEQ ID NO: 13), annexin A10 (SEQ ID NO: 14), annexin A11 (SEQ ID NO: 15 or SEQ ID NO: 16), or annexin A13 (SEQ ID NO: 17 or SEQ ID NO: 18)). In further embodiments, the annexin-like protein is a protein having about or at least about 80%, about or at least about 85%, about or at least about 90%, about or at least about 95%, or about 99% amino acid sequence identity with a wild type human annexin protein.

In some embodiments, an agent of the disclosure is an annexin protein that comprises a post-translational modification. In various embodiments, the post-translational modification increases production of an annexin or annexin-like protein, increases solubility of an annexin or annexin-like protein, decreases aggregation of an annexin or annexin-like protein, increases the half-life of an annexin or annexin-like protein, increases the stability of an annexin or annexin-like protein, enhances target membrane engagement of an annexin or annexin-like protein, or is a codon-optimized version of an annexin or annexin-like protein.

### POLYNUCLEOTIDES

In some embodiments, an agent of the disclosure is a polynucleotide capable of expressing an annexin protein as described herein. The term "nucleotide" or its plural as used herein is interchangeable with modified forms as discussed herein and otherwise known in the art. In certain instances, the art uses the term "nucleobase" which embraces naturally-occurring nucleotide, and non-naturally-occurring nucleotides which include modified nucleotides. Thus, nucleotide or nucleobase means the naturally occurring nucleobases A, G, C, T, and U. Non-naturally occurring nucleobases include, for example and without limitations, xanthine, diaminopurine, 8-oxo-N6-methyladenine, 7-deazaxanthine, 7-deazaguanine, N4,N4-ethanocytosin, N',N'-ethano-2,6-diaminopurine, 5-methylcytosine (mC), 5-(C3-C6)-alkynyl-cytosine, 5-fluorouracil, 5-bromouracil, pseudoisocytosine, 2-hydroxy-5-methyl-4-tr- iazolopyridin, isocytosine, isoguanine, inosine and the "non-naturally occurring" nucleobases described in Benner et al., U.S. Patent No. 5,432,272 and Susan M. Freier and Karl-Heinz Altmann, 1997, Nucleic Acids Research, vol. 25: pp 4429-4443. The term "nucleobase" also includes not only the known purine and pyrimidine heterocycles, but also heterocyclic analogues and tautomers thereof. Further naturally and non-naturally occurring nucleobases include those disclosed in U.S. Patent No. 3,687,808 (Merigan, et al.), in Chapter 15 by Sanghvi, in Antisense Research and Application, Ed. S. T. Crooke and B. Lebleu, CRC Press, 1993, in Englisch et al., 1991, Angewandte Chemie, International Edition, 30: 613-722 (see especially pages 622 and 623, and in the Concise Encyclopedia of Polymer Science and Engineering, J. I. Kroschwitz Ed., John Wiley & Sons, 1990, pages 858-859, Cook, Anti-Cancer Drug Design 1991, 6, 585-607, each of which are hereby incorporated by reference in their entirety). In various aspects, polynucleotides also include one or more "nucleosidic bases" or "base units" which are a category of non-naturally-occurring nucleotides that include compounds such as heterocyclic compounds that can serve like nucleobases, including certain "universal bases" that are not nucleosidic bases in the most classical sense but serve as nucleosidic bases. Universal bases include 3-nitropyrrole, optionally substituted indoles (*e.g*., 5-nitroindole), and optionally substituted hypoxanthine. Other desirable universal bases include, pyrrole, diazole or triazole derivatives, including those universal bases known in the art.

Modified nucleotides are described in EP 1 072 679 and WO 97/12896, the disclosures of which are incorporated herein by reference. Modified nucleobases include without limitation, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified bases include tricyclic pyrimidines such as phenoxazine cytidine(1H-pyrimido[5 ,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (*e.g*. 9-(2-aminoethoxy)-H-pyrimido[5,4-b][1,4]benzox- azin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified bases may also include those in which the purine or pyrimidine base is replaced with other heterocycles, for example 7-deaza-adenine, 7-deazaguanosine, 2-aminopyridine and 2-pyridone. Additional nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, those disclosed by Englisch et al., 1991, Angewandte Chemie, International Edition, 30: 613, and those disclosed by Sanghvi, Y. S., Chapter 15, Antisense Research and Applications, pages 289-302, Crooke, S. T. and Lebleu, B., ed., CRC Press, 1993. Certain of these bases are useful for increasing binding affinity and include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2° C and are, in certain aspects combined with 2'-O-methoxyethyl sugar modifications. See, U.S. Patent Nos. 3,687,808, U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,750,692 and 5,681,941, the disclosures of which are incorporated herein by reference.

Methods of making polynucleotides of a predetermined sequence are well-known. See, *e.g.,* Sambrook et al., Molecular Cloning: A Laboratory Manual (2nd ed. 1989) and F. Eckstein (ed.) Oligonucleotides and Analogues, 1st Ed. (Oxford University Press, New York, 1991). Solid-phase synthesis methods are preferred for both polyribonucleotides and polydeoxyribonucleotides (the well-known methods of synthesizing DNA are also useful for synthesizing RNA). Polynucleotides and polyribonucleotides can also be prepared enzymatically via, *e.g*., polymerase chain reaction (PCR). Non-naturally occurring nucleobases can be incorporated into the polynucleotide, as well. See, *e.g.,* U.S. Patent No. 7,223,833; Katz, J. Am. Chem. Soc., 74:2238 (1951); Yamane, et al., J. Am. Chem. Soc., 83:2599 (1961); Kosturko, et al., Biochemistry, 13:3949 (1974); Thomas, J. Am. Chem. Soc., 76:6032 (1954); Zhang, et al., J. Am. Chem. Soc., 127:74-75 (2005); and Zimmermann, et al., J. Am. Chem. Soc., 124:13684-13685 (2002).

### STEROIDS

In some embodiments, the agent that increases the activity of an annexin protein is a steroid. In further embodiments, the steroid is a corticosteroid, a glucocorticoid, or a mineralocorticoid. In still further embodiments, the corticosteroid is Betamethasone, Budesonide, Cortisone, Dexamethasone, Hydrocortisone, Methylprednisolone, Prednisolone, or Prednisone. In some embodiments, the corticosteroid is salmeterol, fluticasone, or budesonide.

In some embodiments, the steroid is an anabolic steroid. In further embodiments anabolic steroids, include, but are not limited to, testosterone or related steroid compounds with muscle growth inducing properties, such as cyclostanazol or methadrostenol, prohomones or derivatives thereof, modulators of estrogen, and selective androgen receptor modulators (SARMS).

### VECTORS

An appropriate expression vector may be used to deliver exogenous nucleic acid to a recipient muscle cell in the methods of the disclosure. In order to achieve effective gene therapy, the expression vector must be designed for efficient cell uptake and gene product expression. In some embodiments, the vector is within a chloroplast. In some embodiments, the vector is a viral vector. In some embodiments, the viral vector is selected from the group consisting of a herpes virus vector, an adeno-associated virus (AAV) vector, an adeno virus vector, and a lentiviral vector.

Use of adenovirus or adeno-associated virus (AAV) based vectors for gene delivery have been described [Berkner, Current Topics in Microbiol. and Imunol. 158: 39-66 (1992); Stratford-Perricaudet et al., Hum. Gene Ther. 1: 241-256 (1990); Rosenfeld et al., Cell 8: 143-144 (1992); Stratford-Perricaudet et al., J. Clin. Invest. 90: 626-630 (1992)]. In various embodiments, the adeno-associated virus vector is AAV5, AAV6, AAV8, AAV9, or AAV74. In some embodiments, the adeno-associated virus vector is AAV9. In further embodiments, the adeno-associated virus vector is AAVrh74.

Specific methods for gene therapy useful in the context of the present disclosure depend largely upon the expression system employed; however, most methods involve insertion of coding sequence at an appropriate position within the expression vector, and subsequent delivery of the expression vector to the target muscle tissue for expression.

Additional delivery systems useful in the practice of the methods of the disclosure are discussed in U.S. Patent Publication Numbers 2012/0046345 and 2012/0039806, each of which is incorporated herein by reference in its entirety.

### DISORDERS/INJURIES

In various aspects, the disclosure provides compositions for treating, delaying onset, enhancing recovery from, or preventing a cellular membrane injury, comprising administering an agent and optionally an additional agent to a patient in need thereof.

Such a patient is one that is suffering from, for example, Duchenne Muscular Dystrophy, Limb Girdle Muscular Dystrophy, Becker Muscular Dystrophy, Emery-Dreifuss Muscular Dystrophy (EDMD), Myotonic Dystrophy, Fascioscapulohumeral Dystrophy (FSHD), Oculopharyngeal Muscular Dystrophy, Distal Muscular Dystrophy, cystic fibrosis, pulmonary fibrosis, muscle atrophy, cerebral palsy, an epithelial disorder, an epidermal disorder, a kidney disorder, a liver disorder, sarcopenia, cardiomyopathy, myopathy, cystic fibrosis, pulmonary fibrosis, cardiomyopathy (including hypertrophic, dilated, congenital, arrhythmogenic, restrictive, ischemic, or heart failure), acute lung injury, acute muscle injury, acute myocardial injury, radiation-induced injury, colon cancer, idiopathic pulmonary fibrosis, idiopathic interstitial pneumonia, autoimmune lung diseases, benign prostate hypertrophy, cerebral infarction, musculoskeletal fibrosis, post-surgical adhesions, liver cirrhosis, renal fibrotic disease, fibrotic vascular disease, neurofibromatosis, Alzheimer's disease, diabetic retinopathy, skin lesions, lymph node fibrosis associated with HIV, chronic obstructive pulmonary disease (COPD), inflammatory pulmonary fibrosis, rheumatoid arthritis; rheumatoid spondylitis; osteoarthritis; gout, other arthritic conditions; sepsis; septic shock; endotoxic shock; gram-negative sepsis; toxic shock syndrome; myofacial pain syndrome (MPS); Shigellosis; asthma; adult respiratory distress syndrome; inflammatory bowel disease; Crohn's disease; psoriasis; eczema; ulcerative colitis; glomerular nephritis; scleroderma; chronic thyroiditis; Grave's disease; Ormond's disease; autoimmune gastritis; myasthenia gravis; autoimmune hemolytic anemia; autoimmune neutropenia; thrombocytopenia; pancreatic fibrosis; chronic active hepatitis including hepatic fibrosis; renal fibrosis, irritable bowel syndrome; pyresis; restenosis; cerebral malaria; stroke and ischemic injury; neural trauma; Huntington's disease; Parkinson's disease; allergies, including allergic rhinitis and allergic conjunctivitis; cachexia; Reiter's syndrome; acute synoviitis; muscle degeneration, bursitis; tendonitis; tenosynoviitis; osteopetrosis; thrombosis; silicosis; pulmonary sarcosis; bone resorption diseases, such as osteoporosis or multiple myeloma-related bone disorders; cancer, including but not limited to metastatic breast carcinoma, colorectal carcinoma, malignant melanoma, gastric cancer, and non-small cell lung cancer; graft-versus-host reaction; and auto-immune diseases, such as multiple sclerosis, lupus and fibromyalgia; viral diseases such as Herpes Zoster, Herpes Simplex I or II, influenza virus, Severe Acute Respiratory Syndrome (SARS) and cytomegalovirus.

As used herein, "cardiomyopathy" refers to any disease or dysfunction of the myocardium (heart muscle) in which the heart is abnormally enlarged, thickened and/or stiffened. As a result, the heart muscle's ability to pump blood is usually weakened, often leading to congestive heart failure. The disease or disorder can be, for example, inflammatory, metabolic, toxic, infiltrative, fibrotic, hematological, genetic, or unknown in origin. Such cardiomyopathies may result from a lack of oxygen. Other diseases include those that result from myocardial injury which involves damage to the muscle or the myocardium in the wall of the heart as a result of disease or trauma. Myocardial injury can be attributed to many things such as, but not limited to, cardiomyopathy, myocardial infarction, or congenital heart disease. The cardiac disorder may be pediatric in origin. Cardiomyopathy includes, but is not limited to, cardiomyopathy (dilated, hypertrophic, restrictive, arrhythmogenic, genetic, idiopathic and unclassified cardiomyopathy), sporadic dilated cardiomyopathy, X-linked Dilated Cardiomyopathy (XLDC), acute and chronic heart failure, right heart failure, left heart failure, biventricular heart failure, congenital heart defects, myocardiac fibrosis, mitral valve stenosis, mitral valve insufficiency, aortic valve stenosis, aortic valve insufficiency, tricuspidal valve stenosis, tricuspidal valve insufficiency, pulmonal valve stenosis, pulmonal valve insufficiency, combined valve defects, myocarditis, acute myocarditis, chronic myocarditis, viral myocarditis, diastolic heart failure, systolic heart failure, diabetic heart failure and accumulation diseases.

### ADDITIONAL (SECOND) AGENTS

In various embodiments of the disclosure it is contemplated that a second agent is administered with the agent that increases the activity of an annexin protein. Nonlimiting examples of the second agent are mitsugumin 53 (MG53), micro-dystrophin, a modulator of latent TGF-β binding protein 4 (LTBP4), a modulator of transforming growth factor β(TGF-β) activity, a modulator of androgen response, a modulator of an inflammatory response, a promoter of muscle growth, a chemotherapeutic agent, a modulator of fibrosis, and a combination thereof. Further, the methods disclosed herein can, in various embodiments, encompass one or more of such agents, or one or more of such agents in composition with any other active agent(s).

### MODULATORS OF LTBP4

LTBP4 is located on human chromosome 19q13.1-q13.2, and is an extracellular matrix protein that binds and sequesters TGFβ. LTBP4 modifies murine muscular dystrophy through a polymorphism in the *Ltbp4* gene. *See* U.S. Patent No. 9,873,739, which is incorporated by reference herein in its entirety. There are two common variants of the *Ltbp4* gene in mice. Most strains of mice, including the *mdx* mouse, have the *Ltbp4* insertion allele (*Ltbp4*^{*I*/*I*})*.* Insertion of 36 base pairs (12 amino acids) into the proline-rich region of LTBP4 encoded by *Ltbp4*^{*I*/*I*} leads to milder disease. Deletion of 36 bp/12aa in the proline-rich region is associated with more severe disease (*Ltbp4*^{*D*/*D*}). It was found that the *Ltbp4* genotype correlated strongly with two different aspects of muscular dystrophy pathology, *i.e.,* membrane leakage and fibrosis, and these features define DMD pathology.

Modulators of LTBP4 are described in U.S. Patent No. 9,873,739, which is incorporated by reference herein in its entirety.

### MODULATORS OF TGF-β ACTIVITY

Transforming Growth Factor-β (TGF-β) superfamily is a family of secreted proteins that is comprised of over 30 members including activins, nodals, bone morphogenic proteins (BMPs) and growth and differentiation factors (GDFs). Superfamily members are generally ubiquitously expressed and regulate numerous cellular processes including growth, development, and regeneration. Mutations in TGF- β superfamily members result in a multitude of diseases including autoimmune disease, cardiac disease, fibrosis and cancer.

TGF- β ligand family includes TGF-β1, TGF-β2, and TGF-β3. TGF- β is secreted into the extracellular matrix in an inactive form bound to latency associated peptide (LAP). Latent TGF- β proteins (LTBPs) binding the TGF-β /LAP complex provide yet another level of regulation. Extracellular proteases cleave LTBP/LAP/TGF-β releasing TGF- β. As a result, TGF-β is free to bind its receptors TGFBRI or TGFBRII. TGF-β /receptor binding, activates downstream canonical and non-canonical SMAD pathways, including activation of SMAD factors, leading to gene transcription. TGF-β signaling has emerged as a prominent mediator of the fibrotic response and disease progression in muscle disease and its expression is upregulated in dystrophy in both mouse and human. Blockade of TGF-β signaling in mice through expression of a dominant negative receptor (TGFBRII) expression, improved the dystrophic pathology, enhanced regeneration, and reduced muscle injury of δ-sarcoglycan-null mice, a mouse model of muscular dystrophy (Accornero, McNally et al Hum Mol Genet 2014). Additionally, antibody-mediated blockade of TGF-β signaling with a pan anti-TGF-β antibody, 1d11 monocloncal antibody, improved respiratory outcome measures in a mouse model of Duchenne muscular dystrophy (Nelson, Wentworth et al Am J Pathol 2011). Thus, therapeutic approaches against TGF-β signaling are contemplated herein to improve repair and delay disease progression.

Therapeutics contemplated as effective against TGF-β signaling include galunisertib (LY2157299 monohydrate),TEW-7917, monoclonal antibodies against TGF-β ligands ( TGF-β 1, 2, 3 alone or pan 1,2,3), Fresolimemub (GC-1008), TGF-β peptide P144, LY2382770, small molecule, SB-525334, and GW788388.

### MODULATORS OF AN ANDROGEN RESPONSE

Selective androgen receptor modulators (SARMs) are a class of androgen receptor ligands that activate androgenic signaling and exist in nonsteroidal and steroidal forms. Studies have shown that SARMs have the potential to increase both muscle and bone mass. Testosterone is one of the most well-known SARMs, which promotes skeletal muscle growth in healthy and diseased tissue. Testosterone and dihydrotestosterone (DHT) promote myocyte differentiation and upregulate follistatin, while also downregulates TGF-β signaling, resulting in muscle growth (Singh et al 2003, Singh et al 2009, Gupta et al 2008). It is conceivable that SARM-mediated inhibition of TGF-β protects against muscle injury and improves repair. SARMS may include, testosterone, estrogen, dihydrotestosterone, estradiol, include dihydronandrolone, nandrolone, nandrolone decanoate, Ostarine, Ligandrol, LGD-3303, andarine, cardarine, 7-alpha methyl, 19-nortestosterone aryl-propionamide, bicyclic hydantoin, quinolinones, tetrahydroquinoline analog, benizimidazole, imidazolopyrazole, indole, and pyrazoline derivatives, azasteroidal derivatives, and aniline, diaryl aniline, and bezoxazepinones derivatives.

### MODULATORS OF AN INFLAMMATORY RESPONSE

A modulator of an inflammatory response includes the following agents. In one embodiment of the disclosure, the modulator of an inflammatory response is a beta2-adrenergic receptor agonist (*e.g*., albuterol). The term beta2-adrenergic receptor agonist is used herein to define a class of drugs which act on the β2-adrenergic receptor, thereby causing smooth muscle relaxation resulting in dilation of bronchial passages, vasodilation in muscle and liver, relaxation of uterine muscle and release of insulin. In one embodiment, the beta2-adrenergic receptor agonist for use according to the disclosure is albuterol, an immunosuppressant drug that is widely used in inhalant form for asthmatics. Albuterol is thought to slow disease progression by suppressing the infiltration of macrophages and other immune cells that contribute to inflammatory tissue loss. Albuterol also appears to have some anabolic effects and promotes the growth of muscle tissue. Albuterol may also suppress protein degradation (possibly via calpain inhibition).

In DMD, the loss of dystrophin leads to breaks in muscle cell membrane, and destabilizes neuronal nitric oxide synthase (nNOS), a protein that normally generates nitric oxide (NO). It is thought that at least part of the muscle degeneration observed in DMD patients may result from the reduced production of muscle membrane-associated neuronal nitric oxide synthase. This reduction may lead to impaired regulation of the vasoconstrictor response and eventual muscle damage.

In one embodiment, modulators of an inflammatory response suitable for use in compositions of the disclosure are Nuclear Factor Kappa-B (NF-κB) inhibitors. NF-κB is a major transcription factor modulating cellular immune, inflammatory and proliferative responses. NF-κB functions in activated macrophages to promote inflammation and muscle necrosis and in skeletal muscle fibers to limit regeneration through the inhibition of muscle progenitor cells. The activation of this factor in DMD contributes to diseases pathology. Thus, NF-κB plays an important role in the progression of muscular dystrophy and the IKK/NF-κB signaling pathway is a potential therapeutic target for the treatment of a TGFβ-related disease. Inhibitors of NF-κB (for example and without limitation, IRFI 042, a vitamin E analog) enhance muscle function, decrease serum creatine kinase (CK) level and muscle necrosis and enhance muscle regeneration. Edasalonexent is a small molecule inhibitor NF-κB. Edasalonexent administered orally as 100mg/kg delayed muscle disease progression in Duchenne muscular dystrophy boys. Furthermore, specific inhibition of NF-κB -mediated signaling by IKK has similar benefits.

In a further embodiment, the modulator of an inflammatory response is a tumor necrosis factor alpha antagonist. TNF-α is one of the key cytokines that triggers and sustains the inflammation response. In one specific embodiment of the disclosure, the modulator of an inflammatory response is the TNF-α antagonist infliximab.

TNF-α antagonists for use according to the disclosure include, in addition to infliximab (Remicade^{™}), a chimeric monoclonal antibody comprising murine VK and VH domains and human constant Fc domains. The drug blocks the action of TNF-α by binding to it and preventing it from signaling the receptors for TNF-α on the surface of cells. Another TNF-α antagonist for use according to the disclosure is adalimumab (Humira^{™}). Adalimumab is a fully human monoclonal antibody. Another TNF-α antagonist for use according to the disclosure is etanercept (Enbrel^{™}). Etanercept is a dimeric fusion protein comprising soluble human TNF receptor linked to an Fc portion of an IgG1. It is a large molecule that binds to TNF-α and thereby blocks its action. Etanercept mimics the inhibitory effects of naturally occurring soluble TNF receptors, but as a fusion protein it has a greatly extended half-life in the bloodstream and therefore a more profound and long-lasting inhibitory effect.

Another TNF-α antagonist for use according to the disclosure is pentoxifylline (Trental^{™}), chemical name 1-(5-oxohexyl)-3,7-dimethylxanthine. The usual dosage in controlled-release tablet form is one tablet (400 mg) three times a day with meals.

Dosing: Remicade is administered by intravenous infusion, typically at 2-month intervals. The recommended dose is 3 mg/kg given as an intravenous infusion followed with additional similar doses at 2 and 6 weeks after the first infusion, then every 8 weeks thereafter. For patients who have an incomplete response, consideration may be given to adjusting the dose up to 10 mg/kg or treating as often as every 4 weeks. Humira is marketed in both preloaded 0.8 ml (40 mg) syringes and also in preloaded pen devices, both injected subcutaneously, typically by the patient at home. Etanercept can be administered at a dose of 25 mg (twice weekly) or 50 mg (once weekly).

In another embodiment of the disclosure, the modulator of an inflammatory response is cyclosporin. Cyclosporin A, the main form of the drug, is a cyclic nonribosomal peptide of 11 amino acids produced by the fungus *Tolypocladium inflatum.* Cyclosporin is thought to bind to the cytosolic protein cyclophilin (immunophilin) of immunocompetent lymphocytes (especially T-lymphocytes). This complex of cyclosporin and cyclophylin inhibits calcineurin, which under normal circumstances is responsible for activating the transcription of interleukin-2. It also inhibits lymphokine production and interleukin release and therefore leads to a reduced function of effector T-cells. It does not affect cytostatic activity. It has also an effect on mitochondria, preventing the mitochondrial PT pore from opening, thus inhibiting cytochrome c release (a potent apoptotic stimulation factor). Cyclosporin may be administered at a dose of 1-10 mg/kg/day.

### PROMOTERS OF MUSCLE GROWTH

In some embodiments of the disclosure, a therapeutically effective amount of a promoter of muscle growth is administered to a patient. Promoters of muscle growth contemplated by the disclosure include, but are not limited to, insulin-like growth factor-1 (IGF-1), Akt/protein kinase B, clenbuterol, creatine, decorin (see U.S. Patent Publication Number 20120058955), a steroid (for example and without limitation, a corticosteroid or a glucocorticoid steroid), testosterone and a myostatin antagonist.

### Myostatin Antagonists

Another class of promoters of muscle growth suitable for use in the combinations of the disclosure is myostatin antagonists. Myostatin, also known as growth/differentiation factor 8 (GDF-8) is a transforming growth factor-β (TGFβ) superfamily member involved in the regulation of skeletal muscle mass. Most members of the TGF-β-GDF family are widely expressed and are pleiotropic; however, myostatin is primarily expressed in skeletal muscle tissue where it negatively controls skeletal muscle growth. Myostatin is synthesized as an inactive preproprotein which is activated by proteolyic cleavage. The precursor protein is cleaved to produce an approximately 109-amino-acid COOH-terminal protein which, in the form of a homodimer of about 25 kDa, is the mature, active form. The mature dimer appears to circulate in the blood as an inactive latent complex bound to the propeptide. As used herein the term "myostatin antagonist" defines a class of agents that inhibits or blocks at least one activity of myostatin, or alternatively, blocks or reduces the expression of myostatin or its receptor (for example, by interference with the binding of myostatin to its receptor and/or blocking signal transduction resulting from the binding of myostatin to its receptor). Such agents therefore include agents which bind to myostatin itself or to its receptor.

Myostatin antagonists for use according to the disclosure include antibodies to GDF-8; antibodies to GDF-8 receptors; soluble GDF-8 receptors and fragments thereof (e.g., the ActRIIB fusion polypeptides as described in U.S. Patent Publication Number 2004/0223966, which is incorporated herein by reference in its entirety, including soluble ActRIIB receptors in which ActRIIB is joined to the Fc portion of an immunoglobulin); GDF-8 propeptide and modified forms thereof (*e.g*., as described in WO 2002/068650 or U.S. Pat. No. 7,202,210, including forms in which GDF-8 propeptide is joined to the Fc portion of an immunoglobulin and/or form in which GDF-8 is mutated at an aspartate (asp) residue, *e.g*., asp-99 in murine GDF-8 propeptide and asp-100 in human GDF-8 propeptide); a small molecule inhibitor of GDF-8; follistatin (*e.g*., as described in U.S. Pat. No. 6,004,937, incorporated herein by reference) or follistatin-domain-containing proteins (*e.g*., GASP-1 or other proteins as described in U.S. Patent Number 7,192,717 and U.S. Patent No. 7,572,763, each incorporated herein by reference); and modulators of metalloprotease activity that affect GDF-8 activation, as described in U.S. Patent Publication Number 2004/0138118, incorporated herein by reference.

Additional myostatin antagonists include myostatin antibodies which bind to and inhibit or neutralize myostatin (including the myostatin proprotein and/or mature protein, in monomeric or dimeric form). Myostatin antibodies are mammalian or non-mammalian derived antibodies, for example an IgNAR antibody derived from sharks, or humanized antibodies, or comprise a functional fragment derived from antibodies. Such antibodies are described, for example, in WO 2005/094446 and WO 2006/116269, the content of which is incorporated herein by reference. Myostatin antibodies also include those antibodies that bind to the myostatin proprotein and prevent cleavage into the mature active form. Additional antibody antagonists include the antibodies described in U.S. Patent Number 6,096,506 and U.S. Patent Number 6,468,535 (each of which is incorporated herein by reference). In some embodiments, the GDF-8 inhibitor is a monoclonal antibody or a fragment thereof that blocks GDF-8 binding to its receptor. Further embodiments include murine monoclonal antibody JA-16 (as described in U.S. Patent Number 7,320,789 (ATCC Deposit No. PTA-4236); humanized derivatives thereof and fully human monoclonal anti-GDF-8 antibodies (*e.g*., Myo29, Myo28 and Myo22, ATCC Deposit Nos. PTA-4741, PTA-4740, and PTA-4739, respectively, or derivatives thereof) as described in U.S. Patent Number 7,261,893 and incorporated herein by reference.

In still further embodiments, myostatin antagonists include soluble receptors which bind to myostatin and inhibit at least one activity thereof. The term "soluble receptor" herein includes truncated versions or fragments of the myostatin receptor that specifically bind myostatin thereby blocking or inhibiting myostatin signal transduction. Truncated versions of the myostatin receptor, for example, include the naturally occurring soluble domains, as well as variations produced by proteolysis of the N- or C-termini. The soluble domain includes all or part of the extracellular domain of the receptor, either alone or attached to additional peptides or other moieties. Because myostatin binds activin receptors (including the activin type IEB receptor (ActRHB) and activin type HA receptor (ActRHA)), activin receptors can form the basis of soluble receptor antagonists. Soluble receptor fusion proteins can also be used, including soluble receptor Fc (see U.S. Patent Publication Number 2004/0223966 and WO 2006/012627, both of which are incorporated herein by reference in their entireties).

Other myostatin antagonists based on the myostatin receptors are ALK-5 and/or ALK-7 inhibitors (see for example WO 2006/025988 and WO 2005/084699, each incorporated herein by reference). As a TGF-β cytokine, myostatin signals through a family of single transmembrane serine/threonine kinase receptors. These receptors can be divided in two classes, the type I or activin-like kinase (ALK) receptors and type II receptors. The ALK receptors are distinguished from the Type II receptors in that the ALK receptors (a) lack the serine/threonine-rich intracellular tail, (b) possess serine/threonine kinase domains that are highly homologous among Type I receptors, and (c) share a common sequence motif called the GS domain, consisting of a region rich in glycine and serine residues. The GS domain is at the amino terminal end of the intracellular kinase domain and is believed to be critical for activation by the Type II receptor. Several studies have shown that TGF-β signaling requires both the ALK (Type I) and Type II receptors. Specifically, the Type II receptor phosphorylates the GS domain of the Type 1 receptor for TGFβ ALK5, in the presence of TGFβ. The ALK5, in turn, phosphorylates the cytoplasmic proteins smad2 and smad3 at two carboxy terminal serines. Generally, it is believed that in many species, the Type II receptors regulate cell proliferation and the Type I receptors regulate matrix production. Various ALK5 receptor inhibitors have been described (see, for example, U.S. Patent Number 6,465,493, U.S. Patent Number 6,906,089, U.S. Patent Publication Numbers 2003/0166633, 2004/0063745 and 2004/0039198, the disclosures of which are incorporated herein by reference). Thus, the myostatin antagonists for use according to the disclosure may comprise the myostatin binding domain of an ALK5 and/or ALK7 receptor.

Other myostatin antagonists include soluble ligand antagonists that compete with myostatin for binding to myostatin receptors. The term "soluble ligand antagonist" herein refers to soluble peptides, polypeptides or peptidomimetics capable of non-productively binding the myostatin receptor(s) (*e.g*., the activin type HB receptor (ActRHA)) and thereby competitively blocking myostatin-receptor signal transduction. Soluble ligand antagonists include variants of myostatin, also referred to as "myostatin analogs" that have homology to, but not the activity of, myostatin. Such analogs include truncates (such as N- or C-terminal truncations, substitutions, deletions, and other alterations in the amino acid sequence, such as variants having non-amino acid substitutions).

Additional myostatin antagonists contemplated by the disclosure include inhibitory nucleic acids as described herein. These antagonists include antisense or sense polynucleotides comprising a single-stranded polynucleotide sequence (either RNA or DNA) capable of binding to target mRNA (sense) or DNA (antisense) sequences. Thus, RNA interference (RNAi) produced by the introduction of specific small interfering RNA (siRNA), may also be used to inhibit or eliminate the activity of myostatin.

In specific embodiments, myostatin antagonists include, but are not limited to, follistatin, the myostatin prodomain, growth and differentiation factor 11 (GDF-11) prodomain, prodomain fusion proteins, antagonistic antibodies or antibody fragments that bind to myostatin, antagonistic antibodies or antibody fragments that bind to the activin type IEB receptor, soluble activin type IHB receptor, soluble activin type IEB receptor fusion proteins, soluble myostatin analogs (soluble ligands), polynucleotides, small molecules, peptidomimetics, and myostatin binding agents. Other antagonists include the peptide immunogens described in U.S. Patent Number 6,369,201 and WO 2001/05820 (each of which is incorporated herein by reference) and myostatin multimers and immunoconjugates capable of eliciting an immune response and thereby blocking myostatin activity. Other antagonists include the protein inhibitors of myostatin described in WO 2002/085306 (incorporated herein by reference), which include the truncated Activin type II receptor, the myostatin pro-domain, and follistatin. Other myostatin inhibitors include those released into culture from cells overexpressing myostatin (see WO 2000/43781), dominant negative myostatin proteins (see WO 2001/53350) including the protein encoded by the Piedmontese allele, and mature myostatin peptides having a C-terminal truncation at a position either at or between amino acid positions 335 to 375. The small peptides described in U.S. Patent Publication Number 2004/0181033 (incorporated herein by reference) that comprise the amino acid sequence WMCPP, are also suitable for use in the compositions of the disclosure.

### CHEMOTHERAPEUTIC AGENTS

Chemotherapeutic agents contemplated for use include, without limitation, alkylating agents including: nitrogen mustards, such as mechlor-ethamine, cyclophosphamide, ifosfamide, melphalan and chlorambucil; nitrosoureas, such as carmustine (BCNU), lomustine (CCNU), and semustine (methyl-CCNU); ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil, fluorodeoxyuridine, gemcitabine, cytosine arabinoside (AraC, cytarabine), 5-azacytidine, 2,2'-difluorodeoxycytidine, purine analogs such as 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; epipodophylotoxins such as etoposide and teniposide; antibiotics such as actimomycin D, daunomycin (rubidomycin), doxorubicin, mitoxantrone, idarubicin, bleomycins, plicamycin (mithramycin), mitomycin C, and actinomycin; enzymes such as L-asparaginase; biological response modifiers such as interferon-alpha, IL-2, G-CSF and GM-CSF; miscellaneous agents including platinum coordination complexes such as cisplatin and carboplatin, anthracenediones such as mitoxantrone, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

### MODULATORS OF FIBROSIS

A "modulator of fibrosis" as used herein is synonymous with antifibrotic agent. The term "antifibrotic agent" refers to a chemical compound that has antifibrotic activity (*i.e*., prevents or reduces fibrosis) in mammals. This takes into account the abnormal formation of fibrous connective tissue, which is typically comprised of collagen. These compounds may have different mechanisms of action, some reducing the formation of collagen or another protein, others enhancing the catabolism or removal of collagen in the affected area of the body. All such compounds having activity in the reduction of the presence of fibrotic tissue are included herein, without regard to the particular mechanism of action by which each such drug functions. Antifibrotic agents useful in the methods and compositions of the disclosure include those described in U.S. Patent Number 5,720,950, incorporated herein by reference. Additional antifibrotic agents contemplated by the disclosure include, but are not limited to, Type II interferon receptor agonists (*e.g*., interferon-gamma); pirfenidone and pirfenidone analogs; anti-angiogenic agents, such as VEGF antagonists, VEGF receptor antagonists, bFGF antagonists, bFGF receptor antagonists, TGFβ antagonists, TGFβ receptor antagonists; anti-inflammatory agents, IL-1 antagonists, such as IL-1Ra, angiotensin-converting-enzyme (ACE) inhibitors, angiotensin receptor blockers and aldosterone antagonists.

### GENE CORRECTION APPROACHES

Gene correction approaches are contemplated by the disclosure to be used in conjunction with the methods and compositions as described herein. As used herein, "gene correction" approaches include, without limitation, technologies related to gene editing (i.e., CRISPR technology), exon skipping, and other technologies known in the art for modifying mRNA). Thus, in some embodiments, methods are provided in which an agent of the disclosure is used to increase the activity of an annexin protein in an individual suffering from Becker muscular dystrophy (BMD), Duchenne muscular dystrophy (DMD), all Limb Girdle muscular dystrophy (LGMD) type 1 subtypes, all LGMD type 2 subtypes, congenital muscular dystrophy, Emery-Dreifuss muscular dystrophy (EDMD), myotonic dystrophy, Fascioscapulohumeral dystrophy (FSHD), Oculopharyngeal muscular dystrophy, and Distal muscular dystrophy, wherein the patient will be, is concurrently being, or has previously been, administered a composition that results in correction of a gene involved in any one of the foregoing disorders. In further embodiments, methods are provided in which an agent of the disclosure is used to increase the activity of an annexin protein in an individual suffering from Becker muscular dystrophy (BMD), Duchenne muscular dystrophy (DMD), all Limb Girdle muscular dystrophy (LGMD) type 1 subtypes, all LGMD type 2 subtypes, congenital muscular dystrophy, Emery-Dreifuss muscular dystrophy (EDMD), myotonic dystrophy, Fascioscapulohumeral dystrophy (FSHD), Oculopharyngeal muscular dystrophy, and Distal muscular dystrophy, wherein the patient will be, is concurrently being, or has previously been, administered a viral-based or non-viral-based composition that results in correction of a gene involved in any one of the foregoing disorders.

Gene correction approaches are known in the art (see, *e.g.,* U.S. Patent Application Publication No. 2016/0130608 and U.S. Patent No. 9,499,817, respectively, each incorporated by reference herein in their entirety). Further discussion of such methods can be found in Echigoya et al., J Pers Med 8, 2018; Li et al., Trends Pharmacol Sci 39: 982-994, 2018; Min et al., Annu Rev Med, 2018; and Zhang et al., Physiol Rev 98: 1205-1240, 2018.

### COMPOSITIONS

Any of the agents and/or additional agents described herein (or nucleic acids encoding any of the agents and/or additional agents described herein) also is provided in a composition. In this regard, the agent and/or additional agent is formulated with a physiologically-acceptable (*i.e*., pharmacologically acceptable) carrier, buffer, or diluent, as described further herein. Optionally, the protein/recombinant protein is in the form of a physiologically acceptable salt, which is encompassed by the disclosure. "Physiologically acceptable salts" means any salts that are pharmaceutically acceptable. Some examples of appropriate salts include acetate, trifluoroacetate, hydrochloride, hydrobromide, sulfate, citrate, tartrate, glycolate, and oxalate. Accordingly, in some aspects the disclosure provides pharmaceutical compositions comprising one or more annexin proteins and a pharmaceutically acceptable carrier, buffer, and/or diluent. In any of the aspects or embodiments of the disclosure, one or more (or all) annexin proteins in a composition is a modified annexin protein. In any of the aspects or embodiments of the disclosure, one or more (or all) annexin proteins in a composition is a naturally-occurring mammalian annexin protein. In some embodiments, the modified annexin protein is expressed in a prokaryotic cell (for example and without limitation, an *E. coli* cell)*.* In general, a modified protein is a protein that is altered relative to the version of the protein that normally exists in nature. In some embodiments, a modified protein is one in which at least one amino acid of the modified protein has an altered posttranslational modification relative to the naturally-occurring mammalian protein. By way of example, a naturally-occurring mammalian protein may comprise an amino acid that is phosphorylated while the same amino acid in the modified protein has either a different posttranslational modification or has no posttranslational modification. In some embodiments, the annexin protein is annexin A1 (SEQ ID NO: 1), annexin A2 (SEQ ID NO: 2 or SEQ ID NO: 3), annexin A3 (SEQ ID NO: 4), annexin A4 (SEQ ID NO: 5), annexin A5 (SEQ ID NO: 6), annexin A6 (SEQ ID NO: 7, SEQ ID NO: 8, SEQ ID NO: 45, or a combination thereof), annexin A7 (SEQ ID NO: 9 or SEQ ID NO: 10), annexin A8 (SEQ ID NO: 11 or SEQ ID NO: 12), annexin A9 (SEQ ID NO: 13), annexin A10 (SEQ ID NO: 14), annexin A11 (SEQ ID NO: 15 or SEQ ID NO: 16), annexin A13 (SEQ ID NO: 17 or SEQ ID NO: 18), or a combination thereof. In some embodiments, the annexin protein is annexin A6 (SEQ ID NO: 7, SEQ ID NO: 8, SEQ ID NO: 45, or a combination thereof). In some embodiments, and as described herein, the pharmaceutical composition comprises a combination of annexin proteins wherein one or more of the annexin proteins is a modified annexin protein. In some embodiments, the pharmaceutical composition comprises a combination of annexin proteins and each annexin protein is a naturally-occurring mammalian annexin protein. Pharmaceutical compositions of the disclosure comprising one or more annexin proteins are formulated such that the one or more annexin proteins are present in the composition at a high level of purity. By "purity" it is meant that a protein (*e.g*., an annexin protein) used in a pharmaceutical composition is largely composed of the full-length protein (*e.g*., annexin protein) that was expressed and is largely free of truncated or degraded protein products. In various embodiments, the one or more annexin proteins that is/are present in a pharmaceutical composition is/are at least 90%, at least 95%, or at least 99% pure as measured by standard release assay including but not limited to one or more of SDS-PAGE, SEC-HPLC, and immunoblot analysis. A pharmaceutical composition of the disclosure is also relatively free of endotoxin. In various embodiments, a pharmaceutical composition of the disclosure has an endotoxin level that is or is less than about 10, is or is less than about 5, is or is less than about 1, is or is less than about 0.50000, is or is less than about 0.40000, is or is less than about 0.30000 endotoxin units per milligram (EU/mg) A280 annexin protein as determined by standard methods.

As disclosed herein, the disclosure provides compositions comprising one or more agents and/or additional agents that increase the activity of an annexin protein. In various embodiments, the annexin protein is annexin A1 (SEQ ID NO: 1), annexin A2 (SEQ ID NO: 2 and/or SEQ ID NO: 3), annexin A3 (SEQ ID NO: 4), annexin A4 (SEQ ID NO: 5), annexin A5 (SEQ ID NO: 6), annexin A6 (SEQ ID NO: 7 and/or SEQ ID NO: 8), annexin A7 (SEQ ID NO: 9 and/or SEQ ID NO: 10), annexin A8 (SEQ ID NO: 11 and/or SEQ ID NO: 12), annexin A9 (SEQ ID NO: 13), annexin A10 (SEQ ID NO: 14), annexin A11 (SEQ ID NO: 15 and/or SEQ ID NO: 16), annexin A13 (SEQ ID NO: 17 and/or SEQ ID NO: 18), or a combination thereof. In some embodiments, the composition increases the activity of annexin A1 (SEQ ID NO: 1), annexin A2 (SEQ ID NO: 2 and/or SEQ ID NO: 3), and annexin A6 (SEQ ID NO: 7 and/or SEQ ID NO: 8). In further embodiments, the composition increases the activity of annexin A2 (SEQ ID NO: 2 and/or SEQ ID NO: 3) and annexin A6 (SEQ ID NO: 7 and/or SEQ ID NO: 8). In still further embodiments, the composition increases the activity of annexin A1 (SEQ ID NO: 1) and annexin A6 (SEQ ID NO: 7 and/or SEQ ID NO: 8).

The disclosure also contemplates, in various embodiments, compositions that increase the activity of annexin A1 (SEQ ID NO: 1), annexin A2 (SEQ ID NO: 2 and/or SEQ ID NO: 3), annexin A3 (SEQ ID NO: 4), annexin A4 (SEQ ID NO: 5), annexin A5 (SEQ ID NO: 6), annexin A6 (SEQ ID NO: 7 and/or SEQ ID NO: 8), annexin A7 (SEQ ID NO: 9 and/or SEQ ID NO: 10), annexin A8 (SEQ ID NO: 11 and/or SEQ ID NO: 12), annexin A9 (SEQ ID NO: 13), annexin A10 (SEQ ID NO: 14), annexin A11 (SEQ ID NO: 15 and/or SEQ ID NO: 16), and annexin A13 (SEQ ID NO: 17 and/or SEQ ID NO: 18) in any combination. Note that when more than one sequence identifier is used to identify an annexin protein herein (*e.g*., annexin A2 is identified herein by SEQ ID NO: 2 and/or SEQ ID NO: 3) it will be understood that the different sequence identifiers serve to identify isoforms of the particular annexin protein, and that the isoforms may be used interchangeably or in combination in methods and compositions of the disclosure.
Refseq Accession Number NP_000691.1 annexin A1 [Homo sapiens] (SEQ ID NO: 1): 
MAMVSEFLKQAWFIENEEQEYVQTVKSSKGGPGSAVSPYPTFNPSSDVAALHKAIMVKGV
DEATIIDILTKRNNAQRQQIKAAYLQETGKPLDETLKKALTGHLEEVVLALLKTPAQFDADELR
AAMKGLGTDEDTLIEILASRTNKEIRDINRVYREELKRDLAKDITSDTSGDFRNALLSLAKGD
RSEDFGVNEDLADSDARALYEAGERRKGTDVNVFNTILTTRSYPQLRRVFQKYTKYSKHD
MNKVLDLELKGDIEKCLTAIVKCATSKPAFFAEKLHQAMKGVGTRHKALIRIMVSRSEIDMND
IKAFYQKMYGISLCQAILDETKGDYEKILVALCGGN
Refseq Accession Number NP_001002858.1 annexin A2 isoform 1 [Homo sapiens] (SEQ ID NO: 2): 
MGRQLAGCGDAGKKASFKMSTVHEILCKLSLEGDHSTPPSAYGSVKAYTNFDAERDALNIE
TAIKTKGVDEVTIVNILTNRSNAQRQDIAFAYQRRTKKELASALKSALSGHLETVILGLLKTPA
QYDASELKASMKGLGTDEDSLIEIICSRTNQELQEINRVYKEMYKTDLEKDIISDTSGDFRKL
MVALAKGRRAEDGSVIDYELIDQDARDLYDAGVKRKGTDVPKWISIMTERSVPHLQKVFDR
YKSYSPYDMLESIRKEVKGDLENAFLNLVQCIQNKPLYFADRLYDSMKGKGTRDKVLIRIMV
SRSEVDMLKIRSEFKRKYGKSLYYYIQQDTKGDYQKALLYLCGGDD
Refseq Accession Number NP_001129487.1 annexin A2 isoform 2 [Homo sapiens] (SEQ ID NO: 3): 
MSTVHEILCKLSLEGDHSTPPSAYGSVKAYTNFDAERDALNIETAIKTKGVDEVTIVNILTNRS
NAQRQDIAFAYQRRTKKELASALKSALSGHLETVILGLLKTPAQYDASELKASMKGLGTDED
SLIEIICSRTNQELQEINRVYKEMYKTDLEKDIISDTSGDFRKLMVALAKGRRAEDGSVIDYELI
DQDARDLYDAGVKRKGTDVPKWISIMTERSVPHLQKVFDRYKSYSPYDMLESIRKEVKGDL
ENAFLNLVQCIQNKPLYFADRLYDSMKGKGTRDKVLIRIMVSRSEVDMLKIRSEFKRKYGKS
LYYYIQQDTKGDYQKALLYLCGGDD
Refseq Accession Number NP_005130.1 annexin A3 [Homo sapiens] (SEQ ID NO: 4): 
MASIWVGHRGTVRDYPDFSPSVDAEAIQKAIRGIGTDEKMLISILTERSNAQRQLIVKEYQAA
YGKELKDDLKGDLSGHFEHLMVALVTPPAVFDAKQLKKSMKGAGTNEDALIEILTTRTSRQ
MKDISQAYYTVYKKSLGDDISSETSGDFRKALLTLADGRRDESLKVDEHLAKQDAQILYKAG
 
ENRWGTDEDKFTEILCLRSFPQLKLTFDEYRNISQKDIVDSIKGELSGHFEDLLLAIVNCVRN
TPAFLAERLHRALKGIGTDEFTLNRIMVSRSEIDLLDIRTEFKKHYGYSLYSAIKSDTSGDYEI
TLLKICGGDD
Refseq Accession Number NP_001144.1 annexin A4 isoform a [Homo sapiens] (SEQ ID NO: 5): 
MAMATKGGTVKAASGFNAMEDAQTLRKAMKGLGTDEDAIISVLAYRNTAQRQEIRTAYKST
IGRDLIDDLKSELSGNFEQVIVGMMTPTVLYDVQELRRAMKGAGTDEGCLIEILASRTPEEIR
RISQTYQQQYGRSLEDDIRSDTSFMFQRVLVSLSAGGRDEGNYLDDALVRQDAQDLYEAG
EKKWGTDEVKFLTVLCSRNRNHLLHVFDEYKRISQKDIEQSIKSETSGSFEDALLAIVKCMR
NKSAYFAEKLYKSMKGLGTDDNTLIRVMVSRAEIDMLDIRAHFKRLYGKSLYSFIKGDTSGD
YRKVLLVLCGGDD
Refseq Accession Number NP_001145.1 annexin A5 [Homo sapiens] (SEQ ID NO: 6): 
MAQVLRGTVTDFPGFDERADAETLRKAMKGLGTDEESILTLLTSRSNAQRQEISAAFKTLFG
RDLLDDLKSELTGKFEKLIVALMKPSRLYDAYELKHALKGAGTNEKVLTEIIASRTPEELRAIK
QVYEEEYGSSLEDDVVGDTSGYYQRMLVVLLQANRDPDAGIDEAQVEQDAQALFQAGELK
WGTDEEKFITIFGTRSVSHLRKVFDKYMTISGFQIEETIDRETSGNLEQLLLAVVKSIRSIPAYL
AETLYYAMKGAGTDDHTLIRVMVSRSEIDLFNIRKEFRKNFATSLYSMIKGDTSGDYKKALLL
LCGEDD
Refseq Accession Number NP_001146.2 annexin A6 isoform 1 [Homo sapiens] (SEQ ID NO: 7): 
MAKPAQGAKYRGSIHDFPGFDPNQDAEALYTAMKGFGSDKEAILDIITSRSNRQRQEVCQS
YKSLYGKDLIADLKYELTGKFERLIVGLMRPPAYCDAKEIKDAISGIGTDEKCLIEILASRTNEQ
MHQLVAAYKDAYERDLEADIIGDTSGHFQKMLVVLLQGTREEDDVVSEDLVQQDVQDLYEA
GELKWGTDEAQFIYILGNRSKQHLRLVFDEYLKTTGKPIEASIRGELSGDFEKLMLAVVKCIR
STPEYFAERLFKAMKGLGTRDNTLIRIMVSRSELDMLDIREIFRTKYEKSLYSMIKNDTSGEY
KKTLLKLSGGDDDAAGQFFPEAAQVAYQMWELSAVARVELKGTVRPANDFNPDADAKALR
KAMKGLGTDEDTIIDIITHRSNVQRQQIRQTFKSHFGRDLMTDLKSEISGDLARLILGLMMPP
AHYDAKQLKKAMEGAGTDEKALIEILATRTNAEIRAINEAYKEDYHKSLEDALSSDTSGHFRR
ILISLATGHREEGGENLDQAREDAQVAAEILEIADTPSGDKTSLETRFMTILCTRSYPHLRRV
FQEFIKMTNYDVEHTIKKEMSGDVRDAFVAIVQSVKNKPLFFADKLYKSMKGAGTDEKTLTR
IMVSRSEIDLLNIRREFIEKYDKSLHQAIEGDTSGDFLKALLALCGGED
Refseq Accession Number NP_001180473.1 annexin A6 isoform 2 [Homo sapiens] (SEQ ID NO: 8): 
MKGFGSDKEAILDIITSRSNRQRQEVCQSYKSLYGKDLIADLKYELTGKFERLIVGLMRPPAY
CDAKEIKDAISGIGTDEKCLIEILASRTNEQMHQLVAAYKDAYERDLEADIIGDTSGHFQKML
 
VVLLQGTREEDDVVSEDLVQQDVQDLYEAGELKWGTDEAQFIYILGNRSKQHLRLVFDEYL
KTTGKPIEASIRGELSGDFEKLMLAVVKCIRSTPEYFAERLFKAMKGLGTRDNTLIRIMVSRS
ELDMLDIREIFRTKYEKSLYSMIKNDTSGEYKKTLLKLSGGDDDAAGQFFPEAAQVAYQMW
ELSAVARVELKGTVRPANDFNPDADAKALRKAMKGLGTDEDTIIDIITHRSNVQRQQIRQTF
KSHFGRDLMTDLKSEISGDLARLILGLMMPPAHYDAKQLKKAMEGAGTDEKALIEILATRTN
AEIRAINEAYKEDYHKSLEDALSSDTSGHFRRILISLATGHREEGGENLDQAREDAQVAAEIL
EIADTPSGDKTSLETRFMTILCTRSYPHLRRVFQEFIKMTNYDVEHTIKKEMSGDVRDAFVAI
VQSVKNKPLFFADKLYKSMKGAGTDEKTLTRIMVSRSEIDLLNIRREFIEKYDKSLHQAIEGD
TSGDFLKALLALCGGED
Refseq Accession Number NP_001147.1 annexin A7 isoform 1 [Homo sapiens] (SEQ ID NO: 9): 
MSYPGYPPTGYPPFPGYPPAGQESSFPPSGQYPYPSGFPPMGGGAYPQVPSSGYPGAG
GYPAPGGYPAPGGYPGAPQPGGAPSYPGVPPGQGFGVPPGGAGFSGYPQPPSQSYGG
GPAQVPLPGGFPGGQMPSQYPGGQPTYPSQPATVTQVTQGTIRPAANFDAIRDAEILRKA
MKGFGTDEQAIVDVVANRSNDQRQKIKAAFKTSYGKDLIKDLKSELSGNMEELILALFMPPT
YYDAWSLRKAMQGAGTQERVLIEILCTRTNQEIREIVRCYQSEFGRDLEKDIRSDTSGHFER
LLVSMCQGNRDENQSINHQMAQEDAQRLYQAGEGRLGTDESCFNMILATRSFPQLRATME
AYSRMANRDLLSSVSREFSGYVESGLKTILQCALNRPAFFAERLYYAMKGAGTDDSTLVRIV
VTRSEIDLVQIKQMFAQMYQKTLGTMIAGDTSGDYRRLLLAIVGQ
Refseq Accession Number NP_004025.1 annexin A7 isoform 2 [Homo sapiens] (SEQ ID NO: 10): 
MSYPGYPPTGYPPFPGYPPAGQESSFPPSGQYPYPSGFPPMGGGAYPQVPSSGYPGAG
GYPAPGGYPAPGGYPGAPQPGGAPSYPGVPPGQGFGVPPGGAGFSGYPQPPSQSYGG
GPAQVPLPGGFPGGQMPSQYPGGQPTYPSQINTDSFSSYPVFSPVSLDYSSEPATVTQVT
QGTIRPAANFDAIRDAEILRKAMKGFGTDEQAIVDVVANRSNDQRQKIKAAFKTSYGKDLIKD
LKSELSGNMEELILALFMPPTYYDAWSLRKAMQGAGTQERVLIEILCTRTNQEIREIVRCYQS
EFGRDLEKDIRSDTSGHFERLLVSMCQGNRDENQSINHQMAQEDAQRLYQAGEGRLGTD
ESCFNMILATRSFPQLRATMEAYSRMANRDLLSSVSREFSGYVESGLKTILQCALNRPAFFA
ERLYYAMKGAGTDDSTLVRIVVTRSEIDLVQIKQMFAQMYQKTLGTMIAGDTSGDYRRLLLA
IVGQ
Refseq Accession Number NP_001258631.1 annexin A8 isoform 1 [Homo sapiens] (SEQ ID NO: 11): 
MAWWKSWIEQEGVTVKSSSHFNPDPDAETLYKAMKGIGVGSQLLSHQAAAFAFPSSALTS
VSPWGQQGHLCCNPAGTNEQAIIDVLTKRSNTQRQQIAKSFKAQFGKDLTETLKSELSGKF
ERLIVALMYPPYRYEAKELHDAMKGLGTKEGVIIEILASRTKNQLREIMKAYEEDYGSSLEEDI
QADTSGYLERILVCLLQGSRDDVSSFVDPGLALQDAQDLYAAGEKIRGTDEMKFITILCTRS
 
ATHLLRVFEEYEKIANKSIEDSIKSETHGSLEEAMLTVVKCTQNLHSYFAERLYYAMKGAGT
RDGTLIRNIVSRSEIDLNLIKCHFKKMYGKTLSSMIMEDTSGDYKNALLSLVGSDP
Refseq Accession Number NP_001035173.1 annexin A8 isoform 2 [Homo sapiens] (SEQ ID NO: 12): 
MAWWKSWIEQEGVTVKSSSHFNPDPDAETLYKAMKGIGTNEQAIIDVLTKRSNTQRQQIAK
SFKAQFGKDLTETLKSELSGKFERLIVALMYPPYRYEAKELHDAMKGLGTKEGVIIEILASRT
KNQLREIMKAYEEDYGSSLEEDIQADTSGYLERILVCLLQGSRDDVSSFVDPGLALQDAQDL
YAAGEKIRGTDEMKFITILCTRSATHLLRVFEEYEKIANKSIEDSIKSETHGSLEEAMLTVVKC
TQNLHSYFAERLYYAMKGAGTRDGTLIRNIVSRSEIDLNLIKCHFKKMYGKTLSSMIMEDTS
GDYKNALLSLVGSDP
Refseq Accession Number NP_003559.2 annexin A9 [Homo sapiens] (SEQ ID NO: 13): 
MSVTGGKMAPSLTQEILSHLGLASKTAAWGTLGTLRTFLNFSVDKDAQRLLRAITGQGVDR
SAIVDVLTNRSREQRQLISRNFQERTQQDLMKSLQAALSGNLERIVMALLQPTAQFDAQEL
RTALKASDSAVDVAIEILATRTPPQLQECLAVYKHNFQVEAVDDITSETSGILQDLLLALAKG
GRDSYSGIIDYNLAEQDVQALQRAEGPSREETWVPVFTQRNPEHLIRVFDQYQRSTGQELE
EAVQNRFHGDAQVALLGLASVIKNTPLYFADKLHQALQETEPNYQVLIRILISRCETDLLSIRA
EFRKKFGKSLYSSLQDAVKGDCQSALLALCRAEDM
Refseq Accession Number NP_009124.2 annexin A10 [Homo sapiens] (SEQ ID NO: 14): 
MFCGDYVQGTIFPAPNFNPIMDAQMLGGALQGFDCDKDMLINILTQRCNAQRMMIAEAYQS
MYGRDLIGDMREQLSDHFKDVMAGLMYPPPLYDAHELWHAMKGVGTDENCLIEILASRTN
GEIFQMREAYCLQYSNNLQEDIYSETSGHFRDTLMNLVQGTREEGYTDPAMAAQDAMVLW
EACQQKTGEHKTMLQMILCNKSYQQLRLVFQEFQNISGQDMVDAINECYDGYFQELLVAIV
LCVRDKPAYFAYRLYSAIHDFGFHNKTVIRILIARSEIDLLTIRKRYKERYGKSLFHDIRNFASG
HYKKALLAICAGDAEDY
Refseq Accession Number NP_665875.1 annexin A11 isoform 1 [Homo sapiens] (SEQ ID NO: 15):
Refseq Accession Number NP_001265338.1 annexin A11 isoform 2 [Homo sapiens] (SEQ ID NO: 16): 
MPPIGLDNVATYAGQFNQDYLSGMAANMSGTFGGANMPNLYPGAPGAGYPPVPPGGFG
QPPSAQQPVPPYGMYPPPGGNPPSRMPSYPPYPGAPVPGQPMPPPGQQPPGAYPGQPP
VTYPGQPPVPLPGQQQPVPSYPGYPGSGTVTPAVPPTQFGSRGTITDAPGFDPLRDAEVL
RKAMKGFGTDEQAIIDCLGSRSNKQRQQILLSFKTAYGKDLIKDLKSELSGNFEKTILALMKT
PVLFDIYEIKEAIKGVGTDEACLIEILASRSNEHIRELNRAYKAEFKKTLEEAIRSDTSGHFQRL
LISLSQGNRDESTNVDMSLAQRDAQELYAAGENRLGTDESKFNAVLCSRSRAHLVAVFNEY
QRMTGRDIEKSICREMSGDLEEGMLAVVKCLKNTPAFFAERLNKAMRGAGTKDRTLIRIMV
SRSETDLLDIRSEYKRMYGKSLYHDISGDTSGDYRKILLKICGGND
Refseq Accession Number NP_004297.2 annexin A13 isoform a [Homo sapiens] (SEQ ID NO: 17): 
MGNRHAKASSPQGFDVDRDAKKLNKACKGMGTNEAAIIEILSGRTSDERQQIKQKYKATYG
KELEEVLKSELSGNFEKTALALLDRPSEYAARQLQKAMKGLGTDESVLIEVLCTRTNKEIIAIK
EAYQRLFDRSLESDVKGDTSGNLKKILVSLLQANRNEGDDVDKDLAGQDAKDLYDAGEGR
WGTDELAFNEVLAKRSYKQLRATFQAYQILIGKDIEEAIEEETSGDLQKAYLTLVRCAQDCE
DYFAERLYKSMKGAGTDEETLIRIVVTRAEVDLQGIKAKFQEKYQKSLSDMVRSDTSGDFR
KLLVALLH
Refseq Accession Number NP_001003954.1 annexin A13 isoform b [Homo sapiens] (SEQ ID NO: 18): 
MGNRHSQSYTLSEGSQQLPKGDSQPSTVVQPLSHPSRNGEPEAPQPAKASSPQGFDVDR
DAKKLNKACKGMGTNEAAIIEILSGRTSDERQQIKQKYKATYGKELEEVLKSELSGNFEKTAL
ALLDRPSEYAARQLQKAMKGLGTDESVLIEVLCTRTNKEIIAIKEAYQRLFDRSLESDVKGDT
SGNLKKILVSLLQANRNEGDDVDKDLAGQDAKDLYDAGEGRWGTDELAFNEVLAKRSYKQ
LRATFQAYQILIGKDIEEAIEEETSGDLQKAYLTLVRCAQDCEDYFAERLYKSMKGAGTDEET
LIRIVVTRAEVDLQGIKAKFQEKYQKSLSDMVRSDTSGDFRKLLVALLH
Refseq Accession Number NP_001350043.1 annexin A6 isoform 3 [Homo sapiens] (SEQ ID NO: 45):

MAKPAQGAKYRGSIHDFPGFDPNQDAEALYTAMKGFGSDKEAILDIITSRSNRQRQEVCQS
YKSLYGKDLIADLKYELTGKFERLIVGLMRPPAYCDAKEIKDAISGIGTDEKCLIEILASRTNEQ
MHQLVAAYKDAYERDLEADIIGDTSGHFQKMLVVLLQGTREEDDVVSEDLVQQDVQDLYEA
GELKWGTDEAQFIYILGNRSKQHLRLVFDEYLKTTGKPIEASIRGELSGDFEKLMLAVVKCIR
STPEYFAERLFKAMKGLGTRDNTLIRIMVSRSELDMLDIREIFRTKYEKSLYSMIKNDTSGEY
KKTLLKLSGGDDDAAGQFFPEAAQVAYQMWELSAVARVELKGTVRPANDFNPDADAKALR

KAMKGLGTDEDTIIDIITHRSNVQRQQIRQTFKSHFGRDLMTDLKSEISGDLARLILGLMMPP
AHYDAKQLKKAMEGAGTDEKALIEILATRTNAEIRAINEAYKEDYHKSLEDALSSDTSGHFRR
ILISLATGHREEGGENLDQAREDAQEIADTPSGDKTSLETRFMTILCTRSYPHLRRVFQEFIK
MTNYDVEHTIKKEMSGDVRDAFVAIVQSVKNKPLFFADKLYKSMKGAGTDEKTLTRIMVSR
SEIDLLNIRREFIEKYDKSLHQAIEGDTSGDFLKALLALCGGED

The disclosure also contemplates corresponding polynucleotides that encode each of the foregoing annexin proteins. The following polynucleotides are contemplated for use according to the disclosure. Specifically, the following polynucleotides are messenger RNA (mRNA) sequences contemplated for use with a vector of the disclosure to increase activity of an annexin protein. As discussed above, when more than one sequence identifier is used to identify an mRNA sequence in relation to the same annexin species herein (*e.g*., mRNA sequences relating to annexin A2 are identified herein by SEQ ID NO: 20 and SEQ ID NO: 21) it will be understood that the different sequence identifiers serve to identify transcript variants that may be utilized with a vector of the disclosure to be translated into the particular annexin protein, and that the transcript variants may be used interchangeably or in combination in the methods and compositions of the disclosure.
NM_000700.3 Homo sapiens annexin A1 (ANXA1), mRNA (SEQ ID NO: 19) 
AGTGTGAAATCTTCAGAGAAGAATTTCTCTTTAGTTCTTTGCAAGAAGGTAGA
GATAAAGACACTTTTTCAAAAATGGCAATGGTATCAGAATTCCTCAAGCAGGCCTGGTTT
ATTGAAAATGAAGAGCAGGAATATGTTCAAACTGTGAAGTCATCCAAAGGTGGTCCCGG
ATCAGCGGTGAGCCCCTATCCTACCTTCAATCCATCCTCGGATGTCGCTGCCTTGCATA
AGGCCATAATGGTTAAAGGTGTGGATGAAGCAACCATCATTGACATTCTAACTAAGCGA
AACAATGCACAGCGTCAACAGATCAAAGCAGCATATCTCCAGGAAACAGGAAAGCCCC
TGGATGAAACACTGAAGAAAGCCCTTACAGGTCACCTTGAGGAGGTTGTTTTAGCTCTG
CTAAAAACTCCAGCGCAATTTGATGCTGATGAACTTCGTGCTGCCATGAAGGGCCTTGG
AACTGATGAAGATACTCTAATTGAGATTTTGGCATCAAGAACTAACAAAGAAATCAGAGA
CATTAACAGGGTCTACAGAGAGGAACTGAAGAGAGATCTGGCCAAAGACATAACCTCA
GACACATCTGGAGATTTTCGGAACGCTTTGCTTTCTCTTGCTAAGGGTGACCGATCTGA
GGACTTTGGTGTGAATGAAGACTTGGCTGATTCAGATGCCAGGGCCTTGTATGAAGCA
GGAGAAAGGAGAAAGGGGACAGACGTAAACGTGTTCAATACCATCCTTACCACCAGAA
GCTATCCACAACTTCGCAGAGTGTTTCAGAAATACACCAAGTACAGTAAGCATGACATG
AACAAAGTTCTGGACCTGGAGTTGAAAGGTGACATTGAGAAATGCCTCACAGCTATCGT
GAAGTGCGCCACAAGCAAACCAGCTTTCTTTGCAGAGAAGCTTCATCAAGCCATGAAAG
GTGTTGGAACTCGCCATAAGGCATTGATCAGGATTATGGTTTCCCGTTCTGAAATTGAC
ATGAATGATATCAAAGCATTCTATCAGAAGATGTATGGTATCTCCCTTTGCCAAGCCATC
CTGGATGAAACCAAAGGAGATTATGAGAAAATCCTGGTGGCTCTTTGTGGAGGAAACTA
 
AACATTCCCTTGATGGTCTCAAGCTATGATCAGAAGACTTTAATTATATATTTTCATCCTA
TAAGCTTAAATAGGAAAGTTTCTTCAACAGGATTACAGTGTAGCTACCTACATGCTGAAA
AATATAGCCTTTAAATCATTTTTATATTATAACTCTGTATAATAGAGATAAGTCCATTTTTT
AAAAATGTTTTCCCCAAACCATAAAACCCTATACAAGTTGTTCTAGTAACAATACATGAG
AAAGATGTCTATGTAGCTGAAAATAAAATGACGTCACAAGACAA
NM_001002858.2 Homo sapiens annexin A2 (ANXA2), transcript variant 1, mRNA (SEQ ID NO: 20) 
GCTCAGCATTTGGGGACGCTCTCAGCTCTCGGCGCACGGCCCAGGTAAGCG
GGGCGCGCCCTGCCCGCCCGCGATGGGCCGCCAGCTAGCGGGGTGTGGAGACGCTG
GGAAGAAGGCTTCCTTCAAAATGTCTACTGTTCACGAAATCCTGTGCAAGCTCAGCTTG
GAGGGTGATCACTCTACACCCCCAAGTGCATATGGGTCTGTCAAAGCCTATACTAACTT
TGATGCTGAGCGGGATGCTTTGAACATTGAAACAGCCATCAAGACCAAAGGTGTGGAT
GAGGTCACCATTGTCAACATTTTGACCAACCGCAGCAATGCACAGAGACAGGATATTGC
CTTCGCCTACCAGAGAAGGACCAAAAAGGAACTTGCATCAGCACTGAAGTCAGCCTTAT
CTGGCCACCTGGAGACGGTGATTTTGGGCCTATTGAAGACACCTGCTCAGTATGACGC
TTCTGAGCTAAAAGCTTCCATGAAGGGGCTGGGAACCGACGAGGACTCTCTCATTGAG
ATCATCTGCTCCAGAACCAACCAGGAGCTGCAGGAAATTAACAGAGTCTACAAGGAAAT
GTACAAGACTGATCTGGAGAAGGACATTATTTCGGACACATCTGGTGACTTCCGCAAGC
TGATGGTTGCCCTGGCAAAGGGTAGAAGAGCAGAGGATGGCTCTGTCATTGATTATGA
ACTGATTGACCAAGATGCTCGGGATCTCTATGACGCTGGAGTGAAGAGGAAAGGAACT
GATGTTCCCAAGTGGATCAGCATCATGACCGAGCGGAGCGTGCCCCACCTCCAGAAAG
TATTTGATAGGTACAAGAGTTACAGCCCTTATGACATGTTGGAAAGCATCAGGAAAGAG
GTTAAAGGAGACCTGGAAAATGCTTTCCTGAACCTGGTTCAGTGCATTCAGAACAAGCC
CCTGTATTTTGCTGATCGGCTGTATGACTCCATGAAGGGCAAGGGGACGCGAGATAAG
GTCCTGATCAGAATCATGGTCTCCCGCAGTGAAGTGGACATGTTGAAAATTAGGTCTGA
ATTCAAGAGAAAGTACGGCAAGTCCCTGTACTATTATATCCAGCAAGACACTAAGGGCG
ACTACCAGAAAGCGCTGCTGTACCTGTGTGGTGGAGATGACTGAAGCCCGACACGGCC
TGAGCGTCCAGAAATGGTGCTCACCATGCTTCCAGCTAACAGGTCTAGAAAACCAGCTT
GCGAATAACAGTCCCCGTGGCCATCCCTGTGAGGGTGACGTTAGCATTACCCCCAACC
TCATTTTAGTTGCCTAAGCATTGCCTGGCCTTCCTGTCTAGTCTCTCCTGTAAGCCAAAG
AAATGAACATTCCAAGGAGTTGGAAGTGAAGTCTATGATGTGAAACACTTTGCCTCCTG
TGTACTGTGTCATAAACAGATGAATAAACTGAATTTGTACTTTAGAAACACGTACTTTGT
GGCCCTGCTTTCAACTGAATTGTTTGAAAATTAAACGTGCTTGGGGTTCAGCTGGTGAG
GCTGTCCCTGTAGGAAGAAAGCTCTGGGACTGAGCTGTACAGTATGGTTGCCCCTATC
CAAGTGTCGCTATTTAAGTTAAATTTAAATGAAATAAAATAAAATAAAATCAAAAAAA
NM_001136015.2 Homo sapiens annexin A2 (ANXA2), transcript variant 4, mRNA (SEQ ID NO: 21) 
GCTCAGCATTTGGGGACGCTCTCAGCTCTCGGCGCACGGCCCAGGGTGAAA
ATGTTTGCCATTAAACTCACATGAAGTAGGAAATATTTATATGGATACAAAAGGCACCTG
CATGGGATAATGTCAAATTTCATAGATACTGCTTTGTGCTTCCTTCAAAATGTCTACTGT
TCACGAAATCCTGTGCAAGCTCAGCTTGGAGGGTGATCACTCTACACCCCCAAGTGCA
TATGGGTCTGTCAAAGCCTATACTAACTTTGATGCTGAGCGGGATGCTTTGAACATTGA
AACAGCCATCAAGACCAAAGGTGTGGATGAGGTCACCATTGTCAACATTTTGACCAACC
GCAGCAATGCACAGAGACAGGATATTGCCTTCGCCTACCAGAGAAGGACCAAAAAGGA
ACTTGCATCAGCACTGAAGTCAGCCTTATCTGGCCACCTGGAGACGGTGATTTTGGGC
CTATTGAAGACACCTGCTCAGTATGACGCTTCTGAGCTAAAAGCTTCCATGAAGGGGCT
GGGAACCGACGAGGACTCTCTCATTGAGATCATCTGCTCCAGAACCAACCAGGAGCTG
CAGGAAATTAACAGAGTCTACAAGGAAATGTACAAGACTGATCTGGAGAAGGACATTAT
TTCGGACACATCTGGTGACTTCCGCAAGCTGATGGTTGCCCTGGCAAAGGGTAGAAGA
GCAGAGGATGGCTCTGTCATTGATTATGAACTGATTGACCAAGATGCTCGGGATCTCTA
TGACGCTGGAGTGAAGAGGAAAGGAACTGATGTTCCCAAGTGGATCAGCATCATGACC
GAGCGGAGCGTGCCCCACCTCCAGAAAGTATTTGATAGGTACAAGAGTTACAGCCCTT
ATGACATGTTGGAAAGCATCAGGAAAGAGGTTAAAGGAGACCTGGAAAATGCTTTCCTG
AACCTGGTTCAGTGCATTCAGAACAAGCCCCTGTATTTTGCTGATCGGCTGTATGACTC
CATGAAGGGCAAGGGGACGCGAGATAAGGTCCTGATCAGAATCATGGTCTCCCGCAGT
GAAGTGGACATGTTGAAAATTAGGTCTGAATTCAAGAGAAAGTACGGCAAGTCCCTGTA
CTATTATATCCAGCAAGACACTAAGGGCGACTACCAGAAAGCGCTGCTGTACCTGTGTG
GTGGAGATGACTGAAGCCCGACACGGCCTGAGCGTCCAGAAATGGTGCTCACCATGCT
TCCAGCTAACAGGTCTAGAAAACCAGCTTGCGAATAACAGTCCCCGTGGCCATCCCTG
TGAGGGTGACGTTAGCATTACCCCCAACCTCATTTTAGTTGCCTAAGCATTGCCTGGCC
TTCCTGTCTAGTCTCTCCTGTAAGCCAAAGAAATGAACATTCCAAGGAGTTGGAAGTGA
AGTCTATGATGTGAAACACTTTGCCTCCTGTGTACTGTGTCATAAACAGATGAATAAACT
GAATTTGTACTTTAGAAACACGTACTTTGTGGCCCTGCTTTCAACTGAATTGTTTGAAAA
TTAAACGTGCTTGGGGTTCAGCTGGTGAGGCTGTCCCTGTAGGAAGAAAGCTCTGGGA
CTGAGCTGTACAGTATGGTTGCCCCTATCCAAGTGTCGCTATTTAAGTTAAATTTAAATG
AAATAAAATAAAATAAAATCAAAAAAA
NM_005139.3 Homo sapiens annexin A3 (ANXA3), mRNA (SEQ ID NO: 22) 
AGCGCGGAGCACCTGCGCCCGCGGCTGACACCTTCGCTCGCAGTTTGTTCG
CAGTTTACTCGCACACCAGTTTCCCCCACCGCGCTTTGGATTAGTGTGATCTCAGCTCA
AGGCAAAGGTGGGATATCATGGCATCTATCTGGGTTGGACACCGAGGAACAGTAAGAG
ATTATCCAGACTTTAGCCCATCAGTGGATGCTGAAGCTATTCAGAAAGCAATCAGAGGA
 
ATTGGAACTGATGAGAAAATGCTCATCAGCATTCTGACTGAGAGGTCAAATGCACAGCG
GCAGCTGATTGTTAAGGAATATCAAGCAGCATATGGAAAGGAGCTGAAAGATGACTTGA
AGGGTGATCTCTCTGGCCACTTTGAGCATCTCATGGTGGCCCTAGTGACTCCACCAGC
AGTCTTTGATGCAAAGCAGCTAAAGAAATCCATGAAGGGCGCGGGAACAAACGAAGAT
GCCTTGATTGAAATCTTAACTACCAGGACAAGCAGGCAAATGAAGGATATCTCTCAAGC
CTATTATACAGTATACAAGAAGAGTCTTGGAGATGACATTAGTTCCGAAACATCTGGTGA
CTTCCGGAAAGCTCTGTTGACTTTGGCAGATGGCAGAAGAGATGAAAGTCTGAAAGTG
GATGAGCATCTGGCCAAACAAGATGCCCAGATTCTCTATAAAGCTGGTGAGAACAGATG
GGGCACGGATGAAGACAAATTCACTGAGATCCTGTGTTTAAGGAGCTTTCCTCAATTAA
AACTAACATTTGATGAATACAGAAATATCAGCCAAAAGGACATTGTGGACAGCATAAAA
GGAGAATTATCTGGGCATTTTGAAGACTTACTGTTGGCCATAGTTAATTGTGTGAGGAA
CACGCCGGCCTTTTTAGCCGAAAGACTGCATCGAGCCTTGAAGGGTATTGGAACTGAT
GAGTTTACTCTGAACCGAATAATGGTGTCCAGATCAGAAATTGACCTTTTGGACATTCG
AACAGAGTTCAAGAAGCATTATGGCTATTCCCTATATTCAGCAATTAAATCGGATACTTC
TGGAGACTATGAAATCACACTCTTAAAAATCTGTGGTGGAGATGACTGAACCAAGAAGA
TAATCTCCAAAGGTCCACGATGGGCTTTCCCAACAGCTCCACCTTACTTCTTCTCATACT
ATTTAAGAGAACAAGCAAATATAAACAGCAACTTGTGTTCCTAACAGGAATTTTCATTGT
TCTATAACAACAACAACAAAAGCGATTATTATTTTAGAGCATCTCATTTATAATGTAGCAG
CTCATAAATGAAATTGAAAATGGTATTAAAGATCTGCAACTACTATCCAACTTATATTTCT
GCTTTCAAAGTTAAGAATCTTTATAGTTCTACTCCATTAAATATAAAGCAAGATAATAAAA
ATTGTTGCTTTTGTTAAAA
NM_001153.5 Homo sapiens annexin A4 (ANXA4), transcript variant 2, mRNA (SEQ ID NO: 23) 
GTGACCTCCGCAGCCGCAGAGGAGGAGCGCAGCCCGGCCTCGAAGAACTTC
TGCTTGGGTGGCTGAACTCTGATCTTGACCTAGAGTCATGGCCATGGCAACCAAAGGA
GGTACTGTCAAAGCTGCTTCAGGATTCAATGCCATGGAAGATGCCCAGACCCTGAGGA
AGGCCATGAAAGGGCTCGGCACCGATGAAGACGCCATTATTAGCGTCCTTGCCTACCG
CAACACCGCCCAGCGCCAGGAGATCAGGACAGCCTACAAGAGCACCATCGGCAGGGA
CTTGATAGACGACCTGAAGTCAGAACTGAGTGGCAACTTCGAGCAGGTGATTGTGGGG
ATGATGACGCCCACGGTGCTGTATGACGTGCAAGAGCTGCGAAGGGCCATGAAGGGA
GCCGGCACTGATGAGGGCTGCCTAATTGAGATCCTGGCCTCCCGGACCCCTGAGGAG
ATCCGGCGCATAAGCCAAACCTACCAGCAGCAATATGGACGGAGCCTTGAAGATGACA
TTCGCTCTGACACATCGTTCATGTTCCAGCGAGTGCTGGTGTCTCTGTCAGCTGGTGG
GAGGGATGAAGGAAATTATCTGGACGATGCTCTCGTGAGACAGGATGCCCAGGACCTG
TATGAGGCTGGAGAGAAGAAATGGGGGACAGATGAGGTGAAATTTCTAACTGTTCTCT
GTTCCCGGAACCGAAATCACCTGTTGCATGTGTTTGATGAATACAAAAGGATATCACAG
 
AAGGATATTGAACAGAGTATTAAATCTGAAACATCTGGTAGCTTTGAAGATGCTCTGCTG
GCTATAGTAAAGTGCATGAGGAACAAATCTGCATATTTTGCTGAAAAGCTCTATAAATCG
ATGAAGGGCTTGGGCACCGATGATAACACCCTCATCAGAGTGATGGTTTCTCGAGCAG
AAATTGACATGTTGGATATCCGGGCACACTTCAAGAGACTCTATGGAAAGTCTCTGTAC
TCGTTCATCAAGGGTGACACATCTGGAGACTACAGGAAAGTACTGCTTGTTCTCTGTGG
AGGAGATGATTAAAATAAAAATCCCAGAAGGACAGGAGGATTCTCAACACTTTGAATTTT
TTTAACTTCATTTTTCTACACTGCTATTATCATTATCTCAGAATGCTTATTTCCAATTAAAA
CGCCTACAGCTGCCTCCTAGAATATAGACTGTCTGTATTATTATTCACCTATAATTAGTC
ATTATGATGCTTTAAAGCTGTACTTGCATTTCAAAGCTTATAAGATATAAATGGAGATTTT
AAAGTAGAAATAAATATGTATTCCATGTTTTTAAAAGATTACTTTCTACTTTGTGTTTCAC
AGACATTGAATATATTAAATTATTCCATATTTTCTTTTCAGTGAAAAATTTTTTAAATGGAA
GACTGTTCTAAAATCACTTTTTTCCCTAATCCAATTTTTAGAGTGGCTAGTAGTTTCTTCA
TTTGAAATTGTAAGCATCCGGTCAGTAAGAATGCCCATCCAGTTTTCTATATTTCATAGT
CAAAGCCTTGAAAGCATCTACAAATCTCTTTTTTTAGGTTTTGTCCATAGCATCAGTTGA
TCCTTACTAAGTTTTTCATGGGAGACTTCCTTCATCACATCTTATGTTGAAATCACTTTCT
GTAGTCAAAGTATACCAAAACCAATTTATCTGAACTAAATTCTAAAGTATGGTTATACAAA
CCATATACATCTGGTTACCAAACATAAATGCTGAACATTCCATATTATTATAGTTAATGTC
TTAATCCAGCTTGCAAGTGAATGGAAAAAAAAATAAGCTTCAAACTAGGTATTCTGGGAA
TGATGTAATGCTCTGAATTTAGTATGATATAAAGAAAACTTTTTTGTGCTAAAAATACTTT
TTAAAATCAATTTTGTTGATTGTAGTAATTTCTATTTGCACTGTGCCTTTCAACTCCAGAA
ACATTCTGAAGATGTACTTGGATTTAATTAAAAAGTTCACTTTGTAAGAACGTGGAAAAA
TAATTTTAATTTAAAAATGGTGTTTTTAGGCCGGGGGCGGGGGCTCACGCCAGTAATCC
CAACACTTTGGGAGGCCAAGGCGGGTGGATCACCTAAGGTCAGGAGTTCAAGACTAGC
CTGGCCAACATGGAGAAACTGCATCTCTACTAAAAATATAAAAATTAGCCGGGTGTGGT
GGCTGGTGCCTGTAATCCCAGCCACTCGGAGGCTGAGTCAGGGAGAACTGCTTGAAC
CCAGGAGGCAGGAGGCAAAGGTTGCAGTGAGCCGAGATCACGCCAGCCTGGGCGACA
GAGCGAGAATCCATCTAAAAAAAAAAAAAAAAAAAGTGTCTTTAAAGTGAGGTATAGTCT
TTCTCTGATCCACTTTTCACCTTCTGAGGTTTTTCATCTTGGCCCCTGAAAGGAGCTATT
TTTGAAGGACTTGTGTTACTCAGTTTCTACAGGAATTACAAGATAAGAAAAAAAAAATCA
TATTTAGTCTTATGCGTGCCTACTGGCTAATGTTCACATATGCCAAACACTACTCAATAA
CATAAAATAATGTATGAACTTATTCTCTGGAAATGAGTGATGCCCTCTGCTCTAAGTAGA
CCATTTATATTAAATATCATAAATGTATAAAGGACATTCATATTCTTA
NM_001154.4 Homo sapiens annexin A5 (ANXA5), mRNA (SEQ ID NO: 24) 
AGTCTAGGTGCAGCTGCCGGATCCTTCAGCGTCTGCATCTCGGCGTCGCCCC
GCGTACCGTCGCCCGGCTCTCCGCCGCTCTCCCGGGGTTTCGGGGCACTTGGGTCCC
ACAGTCTGGTCCTGCTTCACCTTCCCCTGACCTGAGTAGTCGCCATGGCACAGGTTCT
 
CAGAGGCACTGTGACTGACTTCCCTGGATTTGATGAGCGGGCTGATGCAGAAACTCTT
CGGAAGGCTATGAAAGGCTTGGGCACAGATGAGGAGAGCATCCTGACTCTGTTGACAT
CCCGAAGTAATGCTCAGCGCCAGGAAATCTCTGCAGCTTTTAAGACTCTGTTTGGCAGG
GATCTTCTGGATGACCTGAAATCAGAACTAACTGGAAAATTTGAAAAATTAATTGTGGCT
CTGATGAAACCCTCTCGGCTTTATGATGCTTATGAACTGAAACATGCCTTGAAGGGAGC
TGGAACAAATGAAAAAGTACTGACAGAAATTATTGCTTCAAGGACACCTGAAGAACTGA
GAGCCATCAAACAAGTTTATGAAGAAGAATATGGCTCAAGCCTGGAAGATGACGTGGT
GGGGGACACTTCAGGGTACTACCAGCGGATGTTGGTGGTTCTCCTTCAGGCTAACAGA
GACCCTGATGCTGGAATTGATGAAGCTCAAGTTGAACAAGATGCTCAGGCTTTATTTCA
GGCTGGAGAACTTAAATGGGGGACAGATGAAGAAAAGTTTATCACCATCTTTGGAACAC
GAAGTGTGTCTCATTTGAGAAAGGTGTTTGACAAGTACATGACTATATCAGGATTTCAAA
TTGAGGAAACCATTGACCGCGAGACTTCTGGCAATTTAGAGCAACTACTCCTTGCTGTT
GTGAAATCTATTCGAAGTATACCTGCCTACCTTGCAGAGACCCTCTATTATGCTATGAAG
GGAGCTGGGACAGATGATCATACCCTCATCAGAGTCATGGTTTCCAGGAGTGAGATTG
ATCTGTTTAACATCAGGAAGGAGTTTAGGAAGAATTTTGCCACCTCTCTTTATTCCATGA
TTAAGGGAGATACATCTGGGGACTATAAGAAAGCTCTTCTGCTGCTCTGTGGAGAAGAT
GACTAACGTGTCACGGGGAAGAGCTCCCTGCTGTGTGCCTGCACCACCCCACTGCCTT
CCTTCAGCACCTTTAGCTGCATTTGTATGCCAGTGCTTAACACATTGCCTTATTCATACT
AGCATGCTCATGACCAACACATACACGTCATAGAAGAAAATAGTGGTGCTTCTTTCTGA
TCTCTAGTGGAGATCTCTTTGACTGCTGTAGTACTAAAGTGTACTTAATGTTACTAAGTT
TAATGCCTGGCCATTTTCCATTTATATATATTTTTTAAGAGGCTAGAGTGCTTTTAGCCTT
TTTTAAAAACTCCATTTATATTACATTTGTAACCATGATACTTTAATCAGAAGCTTAGCCT
TGAAATTGTGAACTCTTGGAAATGTTATTAGTGAAGTTCGCAACTAAACTAAACCTGTAA
AATTATGATGATTGTATTCAAAAGATTAATGAAAAATAAACATTTCTGTCCCCCTGAATTA
TGTGTACATGTGTGTTTAGATTTATTATTAAATTTATTTAACAATGTT
NM_001155.5 Homo sapiens annexin A6 (ANXA6), transcript variant 1, mRNA (SEQ ID NO: 25) 
GCGGTTGCTGCTGGGCTAACGGGCTCCGATCCAGCGAGCGCTGCGTCCTCG
AGTCCCTGCGCCCGTGCGTCCGTCTGCGACCCGAGGCCTCCGCTGCGCGTGGATTCT
GCTGCGAACCGGAGACCATGGCCAAACCAGCACAGGGTGCCAAGTACCGGGGCTCCA
TCCATGACTTCCCAGGCTTTGACCCCAACCAGGATGCCGAGGCTCTGTACACTGCCAT
GAAGGGCTTTGGCAGTGACAAGGAGGCCATACTGGACATAATCACCTCACGGAGCAAC
AGGCAGAGGCAGGAGGTCTGCCAGAGCTACAAGTCCCTCTACGGCAAGGACCTCATT
GCTGATTTAAAGTATGAATTGACGGGCAAGTTTGAACGGTTGATTGTGGGCCTGATGAG
GCCACCTGCCTATTGTGATGCCAAAGAAATTAAAGATGCCATCTCGGGCATTGGCACTG
ATGAGAAGTGCCTCATTGAGATCTTGGCTTCCCGGACCAATGAGCAGATGCACCAGCT
 
GGTGGCAGCATACAAAGATGCCTACGAGCGGGACCTGGAGGCTGACATCATCGGCGA
CACCTCTGGCCACTTCCAGAAGATGCTTGTGGTCCTGCTCCAGGGAACCAGGGAGGAG
GATGACGTAGTGAGCGAGGACCTGGTACAACAGGATGTCCAGGACCTATACGAGGCA
GGGGAACTGAAATGGGGAACAGATGAAGCCCAGTTCATTTACATCTTGGGAAATCGCA
GCAAGCAGCATCTTCGGTTGGTGTTCGATGAGTATCTGAAGACCACAGGGAAGCCGAT
TGAAGCCAGCATCCGAGGGGAGCTGTCTGGGGACTTTGAGAAGCTAATGCTGGCCGTA
GTGAAGTGTATCCGGAGCACCCCGGAATATTTTGCTGAAAGGCTCTTCAAGGCTATGAA
GGGCCTGGGGACTCGGGACAACACCCTGATCCGCATCATGGTCTCCCGTAGTGAGTT
GGACATGCTCGACATTCGGGAGATCTTCCGGACCAAGTATGAGAAGTCCCTCTACAGC
ATGATCAAGAATGACACCTCTGGCGAGTACAAGAAGACTCTGCTGAAGCTGTCTGGGG
GAGATGATGATGCTGCTGGCCAGTTCTTCCCGGAGGCAGCGCAGGTGGCCTATCAGAT
GTGGGAACTTAGTGCAGTGGCCCGAGTAGAGCTGAAGGGAACTGTGCGCCCAGCCAA
TGACTTCAACCCTGACGCAGATGCCAAAGCGCTGCGGAAAGCCATGAAGGGACTCGG
GACTGACGAAGACACAATCATCGATATCATCACGCACCGCAGCAATGTCCAGCGGCAG
CAGATCCGGCAGACCTTCAAGTCTCACTTTGGCCGGGACTTAATGACTGACCTGAAGT
CTGAGATCTCTGGAGACCTGGCAAGGCTGATTCTGGGGCTCATGATGCCACCGGCCCA
TTACGATGCCAAGCAGTTGAAGAAGGCCATGGAGGGAGCCGGCACAGATGAAAAGGC
TCTTATTGAAATCCTGGCCACTCGGACCAATGCTGAAATCCGGGCCATCAATGAGGCCT
ATAAGGAGGACTATCACAAGTCCCTGGAGGATGCTCTGAGCTCAGACACATCTGGCCA
CTTCAGGAGGATCCTCATTTCTCTGGCCACGGGGCATCGTGAGGAGGGAGGAGAAAAC
CTGGACCAGGCACGGGAAGATGCCCAGGTGGCTGCTGAGATCTTGGAAATAGCAGAC
ACACCTAGTGGAGACAAAACTTCCTTGGAGACACGTTTCATGACGATCCTGTGTACCCG
GAGCTATCCGCACCTCCGGAGAGTCTTCCAGGAGTTCATCAAGATGACCAACTATGAC
GTGGAGCACACCATCAAGAAGGAGATGTCTGGGGATGTCAGGGATGCATTTGTGGCCA
TTGTTCAAAGTGTCAAGAACAAGCCTCTCTTCTTTGCCGACAAACTTTACAAATCCATGA
AGGGTGCTGGCACAGATGAGAAGACTCTGACCAGGATCATGGTATCCCGCAGTGAGAT
TGACCTGCTCAACATCCGGAGGGAATTCATTGAGAAATATGACAAGTCTCTCCACCAAG
CCATTGAGGGTGACACCTCCGGAGACTTCCTGAAGGCCTTGCTGGCTCTCTGTGGTGG
TGAGGACTAGGGCCACAGCTTTGGCGGGCACTTCTGCCAAGAAATGGTTATCAGCACC
AGCCGCCATGGCCAAGCCTGATTGTTCCAGCTCCAGAGACTAAGGAAGGGGCAGGGG
TGGGGGGAGGGGTTGGGTTGGGCTCTTATCTTCAGTGGAGCTTAGGAAACGCTCCCAC
TCCCACGGGCCATCGAGGGCCCAGCACGGCTGAGCGGCTGAAAAACCGTAGCCATAG
ATCCTGTCCACCTCCACTCCCCTCTGACCCTCAGGCTTTCCCAGCTTCCTCCCCTTGCT
ACAGCCTCTGCCCTGGTTTGGGCTATGTCAGATCCAAAAACATCCTGAACCTCTGTCTG
TAAAATGAGTAGTGTCTGTACTTTGAATGAGGGGGTTGGTGGCAGGGGCCAGTTGAAT
GTGCTGGGCGGGGTGGTGGGAAGGATAGTAAATGTGCTGGGGCAAACTGACAAATCTT
CCCATCCATTTCACCACCCATCTCCATCCAGGCCGCGCTAGAGTACTGGACCAGGAAT
 
TTGGATGCCTGGGTTCAAATCTGCATCTGCCATGCACTTGTTTCTGACCTTAGGCCAGC
CCCTTTCCCTCCCTGAGTCTCTATTTTCTTATCTACAATGAGACAGTTGGACAAAAAAAT
CTTGGCTTCCCTTCTAACATTAACTTCCTAAAGTATGCCTCCGATTCATTCCCTTGACAC
TTTTTATTTCTAAGGAAGAAATAAAAAGAGATACACAAACACATAAACACA
NM_001193544.1 Homo sapiens annexin A6 (ANXA6), transcript variant 2, mRNA (SEQ ID NO: 26) 
AGAGACCAGAGAGCATCCAGAGGCCTGGCCGGGGTCCTGCAGTGCAGACGT
TGGGAGGCACGGAGACGGGGAGAGGGGGAGGCGGTCCAGGACTCACTCTGCTCCAC
CTCTGACTCCTTGAAGGGTGCCAAGTACCGGGGCTCCATCCATGACTTCCCAGGCTTT
GACCCCAACCAGGATGCCGAGGCTCTGTACACTGCCATGAAGGGCTTTGGCAGTGACA
AGGAGGCCATACTGGACATAATCACCTCACGGAGCAACAGGCAGAGGCAGGAGGTCT
GCCAGAGCTACAAGTCCCTCTACGGCAAGGACCTCATTGCTGATTTAAAGTATGAATTG
ACGGGCAAGTTTGAACGGTTGATTGTGGGCCTGATGAGGCCACCTGCCTATTGTGATG
CCAAAGAAATTAAAGATGCCATCTCGGGCATTGGCACTGATGAGAAGTGCCTCATTGAG
ATCTTGGCTTCCCGGACCAATGAGCAGATGCACCAGCTGGTGGCAGCATACAAAGATG
CCTACGAGCGGGACCTGGAGGCTGACATCATCGGCGACACCTCTGGCCACTTCCAGA
AGATGCTTGTGGTCCTGCTCCAGGGAACCAGGGAGGAGGATGACGTAGTGAGCGAGG
ACCTGGTACAACAGGATGTCCAGGACCTATACGAGGCAGGGGAACTGAAATGGGGAAC
AGATGAAGCCCAGTTCATTTACATCTTGGGAAATCGCAGCAAGCAGCATCTTCGGTTGG
TGTTCGATGAGTATCTGAAGACCACAGGGAAGCCGATTGAAGCCAGCATCCGAGGGGA
GCTGTCTGGGGACTTTGAGAAGCTAATGCTGGCCGTAGTGAAGTGTATCCGGAGCACC
CCGGAATATTTTGCTGAAAGGCTCTTCAAGGCTATGAAGGGCCTGGGGACTCGGGACA
ACACCCTGATCCGCATCATGGTCTCCCGTAGTGAGTTGGACATGCTCGACATTCGGGA
GATCTTCCGGACCAAGTATGAGAAGTCCCTCTACAGCATGATCAAGAATGACACCTCTG
GCGAGTACAAGAAGACTCTGCTGAAGCTGTCTGGGGGAGATGATGATGCTGCTGGCCA
GTTCTTCCCGGAGGCAGCGCAGGTGGCCTATCAGATGTGGGAACTTAGTGCAGTGGC
CCGAGTAGAGCTGAAGGGAACTGTGCGCCCAGCCAATGACTTCAACCCTGACGCAGAT
GCCAAAGCGCTGCGGAAAGCCATGAAGGGACTCGGGACTGACGAAGACACAATCATC
GATATCATCACGCACCGCAGCAATGTCCAGCGGCAGCAGATCCGGCAGACCTTCAAGT
CTCACTTTGGCCGGGACTTAATGACTGACCTGAAGTCTGAGATCTCTGGAGACCTGGC
AAGGCTGATTCTGGGGCTCATGATGCCACCGGCCCATTACGATGCCAAGCAGTTGAAG
AAGGCCATGGAGGGAGCCGGCACAGATGAAAAGGCTCTTATTGAAATCCTGGCCACTC
GGACCAATGCTGAAATCCGGGCCATCAATGAGGCCTATAAGGAGGACTATCACAAGTC
CCTGGAGGATGCTCTGAGCTCAGACACATCTGGCCACTTCAGGAGGATCCTCATTTCT
CTGGCCACGGGGCATCGTGAGGAGGGAGGAGAAAACCTGGACCAGGCACGGGAAGA
TGCCCAGGTGGCTGCTGAGATCTTGGAAATAGCAGACACACCTAGTGGAGACAAAACT
 
TCCTTGGAGACACGTTTCATGACGATCCTGTGTACCCGGAGCTATCCGCACCTCCGGA
GAGTCTTCCAGGAGTTCATCAAGATGACCAACTATGACGTGGAGCACACCATCAAGAA
GGAGATGTCTGGGGATGTCAGGGATGCATTTGTGGCCATTGTTCAAAGTGTCAAGAAC
AAGCCTCTCTTCTTTGCCGACAAACTTTACAAATCCATGAAGGGTGCTGGCACAGATGA
GAAGACTCTGACCAGGATCATGGTATCCCGCAGTGAGATTGACCTGCTCAACATCCGG
AGGGAATTCATTGAGAAATATGACAAGTCTCTCCACCAAGCCATTGAGGGTGACACCTC
CGGAGACTTCCTGAAGGCCTTGCTGGCTCTCTGTGGTGGTGAGGACTAGGGCCACAG
CTTTGGCGGGCACTTCTGCCAAGAAATGGTTATCAGCACCAGCCGCCATGGCCAAGCC
TGATTGTTCCAGCTCCAGAGACTAAGGAAGGGGCAGGGGTGGGGGGAGGGGTTGGGT
TGGGCTCTTATCTTCAGTGGAGCTTAGGAAACGCTCCCACTCCCACGGGCCATCGAGG
GCCCAGCACGGCTGAGCGGCTGAAAAACCGTAGCCATAGATCCTGTCCACCTCCACTC
CCCTCTGACCCTCAGGCTTTCCCAGCTTCCTCCCCTTGCTACAGCCTCTGCCCTGGTTT
GGGCTATGTCAGATCCAAAAACATCCTGAACCTCTGTCTGTAAAATGAGTAGTGTCTGT
ACTTTGAATGAGGGGGTTGGTGGCAGGGGCCAGTTGAATGTGCTGGGCGGGGTGGTG
GGAAGGATAGTAAATGTGCTGGGGCAAACTGACAAATCTTCCCATCCATTTCACCACCC
ATCTCCATCCAGGCCGCGCTAGAGTACTGGACCAGGAATTTGGATGCCTGGGTTCAAA
TCTGCATCTGCCATGCACTTGTTTCTGACCTTAGGCCAGCCCCTTTCCCTCCCTGAGTC
TCTATTTTCTTATCTACAATGAGACAGTTGGACAAAAAAATCTTGGCTTCCCTTCTAACAT
TAACTTCCTAAAGTATGCCTCCGATTCATTCCCTTGACACTTTTTATTTCTAAGGAAGAAA
TAAAAAGAGATACACAAACACATAAACACAAAAAAAAAAA
NM_001156.5 Homo sapiens annexin A7 (ANXA7), transcript variant 1, mRNA (SEQ ID NO: 27) 
ATCTTGCGGGAGACCGGGTTGGGCTGTGACGCTGCTGCTGGGGTCAGAATG
TCATACCCAGGCTATCCCCCAACAGGCTACCCACCTTTCCCTGGATATCCTCCTGCAGG
TCAGGAGTCATCTTTTCCCCCTTCTGGTCAGTATCCTTATCCTAGTGGCTTTCCTCCAAT
GGGAGGAGGTGCCTACCCACAAGTGCCAAGTAGTGGCTACCCAGGAGCTGGAGGCTA
CCCTGCGCCTGGAGGTTATCCAGCCCCTGGAGGCTATCCTGGTGCCCCACAGCCAGG
GGGAGCTCCATCCTATCCCGGAGTTCCTCCAGGCCAAGGATTTGGAGTCCCACCAGGT
GGAGCAGGCTTTTCTGGGTATCCACAGCCACCTTCACAGTCTTATGGAGGTGGTCCAG
CACAGGTTCCACTACCTGGTGGCTTTCCTGGAGGACAGATGCCTTCTCAGTATCCTGG
AGGACAACCTACTTACCCTAGTCAGCCTGCCACAGTGACTCAGGTCACTCAAGGAACTA
TCCGACCAGCTGCCAACTTCGATGCTATAAGAGATGCAGAAATTCTTCGTAAGGCAATG
AAGGGTTTTGGGACAGATGAGCAGGCAATTGTGGATGTGGTGGCCAACCGTTCCAATG
ATCAGAGGCAAAAAATTAAAGCAGCATTTAAGACCTCCTATGGCAAGGATTTAATCAAA
GATCTCAAATCAGAGTTAAGTGGAAATATGGAAGAACTGATCCTGGCCCTCTTCATGCC
TCCTACGTATTACGATGCCTGGAGCTTACGGAAAGCAATGCAGGGAGCAGGAACTCAG
 
GAACGTGTATTGATTGAGATTTTGTGCACAAGAACAAATCAGGAAATCCGAGAAATTGT
CAGATGTTATCAGTCAGAATTTGGACGAGACCTTGAAAAGGACATTAGGTCAGATACAT
CAGGACATTTTGAACGTTTACTTGTGTCCATGTGCCAGGGAAATCGTGATGAGAACCAG
AGTATAAACCACCAAATGGCTCAGGAAGATGCTCAGCGTCTCTATCAAGCTGGTGAGG
GGAGACTAGGGACCGATGAATCTTGCTTTAACATGATCCTTGCCACAAGAAGCTTTCCT
CAGCTGAGAGCTACCATGGAGGCTTATTCTAGGATGGCTAATCGAGACTTGTTAAGCAG
TGTGAGCCGTGAGTTTTCCGGATATGTAGAAAGTGGTTTGAAGACCATCTTGCAGTGTG
CCCTGAACCGCCCTGCCTTCTTTGCTGAGAGGCTCTACTATGCTATGAAAGGTGCTGG
CACAGATGACTCCACCCTGGTCCGGATTGTGGTCACTCGAAGTGAGATTGACCTTGTA
CAAATAAAACAGATGTTCGCTCAGATGTATCAGAAGACTCTGGGCACAATGATTGCAGG
TGACACGAGTGGAGATTACCGAAGACTTCTTCTGGCTATTGTGGGCCAGTAGGAGGGA
TTTTTTTTTTTTTAATGAAAAAAAATTTCTATTCATAGCTTATCCTTCAGAGCAATGACCTG
CATGCAGCAATATCAAACATCAGCTAACCGAAAGAGCTTTCTGTCAAGGACCGTATCAG
GGTAATGTGCTTGGTTTGCACATGTTGTTATTGCCTTAATTCTAATTTTATTTTGTTCTCT
ACATACAATCAATGTAAAGCCATATCACAATGATACAGTAATATTGCAATGTTTGTAAAC
CTTCATTCTTACTAGTTTCATTCTAATCAAGATGTCAAATTGAATAAAAATCACAGCAATC
TCTGATTCTGTGTAATAATATTGAATAATTTTTTAGAAGGTTACTGAAAGCTCTGCCTTCC
GGAATCCCTCTAAGTCTGCTTGATAGAGTGGATAGTGTGTTAAAACTGTGTACTTTAAAA
AAAAATTCAACCTTTACATCTAGAATAATTTGCATCTCATTTTGCCTAAATTGGTTCTGTA
TTCATAAACACTTTCCACATAGAAAATAGATTAGTATTACCTGTGGCACCTTTTAAGAAA
GGGTCAAATGTTTATATGCTTAAGATACATAGCCTACTTTTTTTTCGCAGTTGTTTTCTTT
TTTTAAATTGAGTTATGACAAATAAAAAATTGCATATATTTAAGGTGTACAATATGGTGTT
TTGATATCAGCATTCCTTGTGTAATGATTCCACAATTAAGGTCAGGCTAATTACGTATCT
GTCACCTTGACATAGTTACCATTTTTTCATGTGTGGTGAAAACACTTAAGATCTACTACC
TTAGCAAATTTTAAGTGTTCAGTACATTATTAACTATAGATACTGTGCTCTACATTAAACC
TCTAGCATTTATTCGTTTTATAACTGAAAGTTTATACCCTTTGACCAACATCTCCCCATTT
TCCCCACCTCTCACCTGGACAACCACCACTGTGTTTAAGTTCAGCTATTTTAGATTCCAC
GTATAAATGGTATACAATA
NM_004034.3 Homo sapiens annexin A7 (ANXA7), transcript variant 2, mRNA (SEQ ID NO: 28) 
GCCCACCCTGGGCCCGCCCCCGGCTCCATCTTGCGGGAGACCGGGTTGGG
CTGTGACGCTGCTGCTGGGGTCAGAATGTCATACCCAGGCTATCCCCCAACAGGCTAC
CCACCTTTCCCTGGATATCCTCCTGCAGGTCAGGAGTCATCTTTTCCCCCTTCTGGTCA
GTATCCTTATCCTAGTGGCTTTCCTCCAATGGGAGGAGGTGCCTACCCACAAGTGCCAA
GTAGTGGCTACCCAGGAGCTGGAGGCTACCCTGCGCCTGGAGGTTATCCAGCCCCTG
GAGGCTATCCTGGTGCCCCACAGCCAGGGGGAGCTCCATCCTATCCCGGAGTTCCTC
 
CAGGCCAAGGATTTGGAGTCCCACCAGGTGGAGCAGGCTTTTCTGGGTATCCACAGCC
ACCTTCACAGTCTTATGGAGGTGGTCCAGCACAGGTTCCACTACCTGGTGGCTTTCCTG
GAGGACAGATGCCTTCTCAGTATCCTGGAGGACAACCTACTTACCCTAGTCAGATCAAT
ACAGATTCTTTTTCTTCCTATCCTGTTTTCTCTCCTGTTTCTTTGGATTATAGCAGTGAAC
CTGCCACAGTGACTCAGGTCACTCAAGGAACTATCCGACCAGCTGCCAACTTCGATGC
TATAAGAGATGCAGAAATTCTTCGTAAGGCAATGAAGGGTTTTGGGACAGATGAGCAGG
CAATTGTGGATGTGGTGGCCAACCGTTCCAATGATCAGAGGCAAAAAATTAAAGCAGCA
TTTAAGACCTCCTATGGCAAGGATTTAATCAAAGATCTCAAATCAGAGTTAAGTGGAAAT
ATGGAAGAACTGATCCTGGCCCTCTTCATGCCTCCTACGTATTACGATGCCTGGAGCTT
ACGGAAAGCAATGCAGGGAGCAGGAACTCAGGAACGTGTATTGATTGAGATTTTGTGC
ACAAGAACAAATCAGGAAATCCGAGAAATTGTCAGATGTTATCAGTCAGAATTTGGACG
AGACCTTGAAAAGGACATTAGGTCAGATACATCAGGACATTTTGAACGTTTACTTGTGTC
CATGTGCCAGGGAAATCGTGATGAGAACCAGAGTATAAACCACCAAATGGCTCAGGAA
GATGCTCAGCGTCTCTATCAAGCTGGTGAGGGGAGACTAGGGACCGATGAATCTTGCT
TTAACATGATCCTTGCCACAAGAAGCTTTCCTCAGCTGAGAGCTACCATGGAGGCTTAT
TCTAGGATGGCTAATCGAGACTTGTTAAGCAGTGTGAGCCGTGAGTTTTCCGGATATGT
AGAAAGTGGTTTGAAGACCATCTTGCAGTGTGCCCTGAACCGCCCTGCCTTCTTTGCTG
AGAGGCTCTACTATGCTATGAAAGGTGCTGGCACAGATGACTCCACCCTGGTCCGGAT
TGTGGTCACTCGAAGTGAGATTGACCTTGTACAAATAAAACAGATGTTCGCTCAGATGT
ATCAGAAGACTCTGGGCACAATGATTGCAGGTGACACGAGTGGAGATTACCGAAGACT
TCTTCTGGCTATTGTGGGCCAGTAGGAGGGATTTTTTTTTTTTTAATGAAAAAAAATTTCT
ATTCATAGCTTATCCTTCAGAGCAATGACCTGCATGCAGCAATATCAAACATCAGCTAAC
CGAAAGAGCTTTCTGTCAAGGACCGTATCAGGGTAATGTGCTTGGTTTGCACATGTTGT
TATTGCCTTAATTCTAATTTTATTTTGTTCTCTACATACAATCAATGTAAAGCCATATCACA
ATGATACAGTAATATTGCAATGTTTGTAAACCTTCATTCTTACTAGTTTCATTCTAATCAA
GATGTCAAATTGAATAAAAATCACAGCAATCTCTGATTCTGTGTAATAATATTGAATAATT
TTTTAGAAGGTTACTGAAAGCTCTGCCTTCCGGAATCCCTCTAAGTCTGCTTGATAGAG
TGGATAGTGTGTTAAAACTGTGTACTTTAAAAAAAAATTCAACCTTTACATCTAGAATAAT
TTGCATCTCATTTTGCCTAAATTGGTTCTGTATTCATAAACACTTTCCACATAGAAAATAG
ATTAGTATTACCTGTGGCACCTTTTAAGAAAGGGTCAAATGTTTATATGCTTAAGATACA
TAGCCTACTTTTTTTTCGCAGTTGTTTTCTTTTTTTAAATTGAGTTATGACAAATAAAAAAT
TGCATATATTTAAGGTGTACAATATGGTGTTTTGATATCAGCATTCCTTGTGTAATGATTC
CACAATTAAGGTCAGGCTAATTACGTATCTGTCACCTTGACATAGTTACCATTTTTTCAT
GTGTGGTGAAAACACTTAAGATCTACTACCTTAGCAAATTTTAAGTGTTCAGTACATTAT
TAACTATAGATACTGTGCTCTACATTAAACCTCTAGCATTTATTCGTTTTATAACTGAAAG
TTTATACCCTTTGACCAACATCTCCCCATTTTCCCCACCTCTCACCTGGACAACCACCAC
 
TGTGTTTAAGTTCAGCTATTTTAGATTCCACGTATAAATGGTATACAATAAAAAAAAAAAA
AAA
NM_001271702.1 Homo sapiens annexin A8 (ANXA8), transcript variant 1, mRNA (SEQ ID NO: 29) 
CTGGGTGGGGCCTGGGAGCCACAGGAGATGCCCAAAGCCAGGCAGAGCCC
GGGGGCGAGGGGACGGCAGGCAGGTGTGGCGCTGCCCTGGGCGGGCTTGCACCCC
CACACCCAAGTGAGCGGCCTGCTCACTCCTCAGCTGCAGGAGCCAGACGTGTGGAGT
CCCAGCAGAGGCCAACCTGTGTCTCTTCATCTCCCTGGGAAAGGTGCCCCCGAGGTGA
AAGAGATGGCCTGGTGGAAATCCTGGATTGAACAGGAGGGTGTCACAGTGAAGAGCAG
CTCCCACTTCAACCCAGACCCTGATGCAGAGACCCTCTACAAAGCCATGAAGGGGATC
GGTGTCGGGTCCCAACTGCTCAGCCACCAAGCAGCTGCCTTCGCCTTCCCCTCCTCCG
CCCTCACCAGTGTGTCACCCTGGGGGCAGCAGGGTCACTTGTGCTGTAACCCTGCAG
GGACCAACGAGCAGGCTATCATCGATGTGCTCACCAAGAGAAGCAACACGCAGCGGC
AGCAGATCGCCAAGTCCTTCAAGGCTCAGTTCGGCAAGGACCTCACTGAGACCTTGAA
GTCTGAGCTCAGTGGCAAGTTTGAGAGGCTCATTGTGGCCCTTATGTACCCGCCATAC
AGATACGAAGCCAAGGAGCTGCATGACGCCATGAAGGGCTTAGGAACCAAGGAGGGT
GTCATCATTGAGATCCTGGCCTCTCGGACCAAGAACCAGCTGCGGGAGATAATGAAGG
CGTATGAGGAAGACTATGGGTCCAGCCTGGAGGAGGACATCCAAGCAGACACAAGTG
GCTACCTGGAGAGGATCCTGGTGTGCCTCCTGCAGGGCAGCAGGGATGATGTGAGCA
GCTTTGTGGACCCAGGACTGGCCCTCCAAGACGCACAGGATCTGTATGCGGCAGGCG
AGAAGATTCGTGGGACTGATGAGATGAAATTCATCACCATCCTGTGCACGCGCAGTGC
CACTCACCTGCTGAGAGTGTTTGAAGAGTATGAGAAAATTGCCAACAAGAGCATTGAGG
ACAGCATCAAGAGTGAGACCCATGGCTCACTGGAGGAGGCCATGCTCACTGTGGTGAA
ATGCACCCAAAACCTCCACAGCTACTTTGCAGAGAGACTCTACTATGCCATGAAGGGAG
CAGGGACGCGTGATGGGACCCTGATAAGAAACATCGTTTCAAGGAGCGAGATTGACTT
AAATCTTATCAAATGTCACTTCAAGAAGATGTACGGCAAGACCCTCAGCAGCATGATCA
TGGAAGACACCAGCGGTGACTACAAGAACGCCCTGCTGAGCCTGGTGGGCAGCGACC
CCTGAGGCACAGAAGAACAAGAGCAAAGACCATGAAGCCAGAGTCTCCAGGACTCCTC
ACTCAACCTCGGCCATGGACGCAGGTTGGGTGTGAGGGGGGTCCCAGCCTTTCGGTC
TTCTATTTCCCTATTTCCAGTGCTTTCCAGCCGGGTTTCTGACCCAGAGGGTGGAACCG
GCCTGGACTCCTCTTCCCAACTTCCTCCAGGTCATTTCCCAGTGTGAGCACAATGCCAA
CCTTAGTGTTTCTCCAGCCAGACAGATGCCTCAGCATGAAGGGCTTGGGGACTTGTGG
ATCATTCCTTCCTCCCTGCAGGAGCTTCCCAAGCTGGTCACAGAGTCTCCTGGGCACA
GGTTATACAGACCCCAGCCCCATTCCCATCTACTGAAACAGGGTCTCCACAAGAGGGG
CCAGGGAATATGGGTTTTTAACAAGCGTCTTACAAAACACTTCTCTATCATGCAGCCGG
AGAGCTGGCTGGGAGCCCTTTTGTTTTAGAACACACATCCTTCAGCAGCTGAGAAACGA
 
ACACGAATCCATCCCAACCGAGATGCCATTAACATTCATCTAAAAATGTTAGGCTCTAAA
TGGACGAAAAATTCTCTCGCCATCTTAATAACAAAATAAACTACAAATTCCTGACCCAAG
GACACTGTGTTATAAGAGGCGTGGGCTCCCCTGGTGGCTGACCAGGTCAGCTGCCCT
GGCCTTGCACCCCTCTGCATGCAGCACAGAAGGGTGTGACCATGCCCTCAGCACCACT
CTTGTCCCCACTGAACGGCAACTGAGACTGGGTACCTGGAGATTCTGAAGTGCCTTTG
CTGTGGTTTTCAAAATAATAAAGATTTGTATTCAACTCAAAAAAAAAAAAAAAAAAAAA
AAAAAAAA
NM_001040084.2 Homo sapiens annexin A8 (ANXA8), transcript variant 2, mRNA (SEQ ID NO: 30) 
CTGGGTGGGGCCTGGGAGCCACAGGAGATGCCCAAAGCCAGGCAGAGCCC
GGGGGCGAGGGGACGGCAGGCAGGTGTGGCGCTGCCCTGGGCGGGCTTGCACCCC
CACACCCAAGTGAGCGGCCTGCTCACTCCTCAGCTGCAGGAGCCAGACGTGTGGAGT
CCCAGCAGAGGCCAACCTGTGTCTCTTCATCTCCCTGGGAAAGGTGCCCCCGAGGTGA
AAGAGATGGCCTGGTGGAAATCCTGGATTGAACAGGAGGGTGTCACAGTGAAGAGCAG
CTCCCACTTCAACCCAGACCCTGATGCAGAGACCCTCTACAAAGCCATGAAGGGGATC
GGGACCAACGAGCAGGCTATCATCGATGTGCTCACCAAGAGAAGCAACACGCAGCGG
CAGCAGATCGCCAAGTCCTTCAAGGCTCAGTTCGGCAAGGACCTCACTGAGACCTTGA
AGTCTGAGCTCAGTGGCAAGTTTGAGAGGCTCATTGTGGCCCTTATGTACCCGCCATA
CAGATACGAAGCCAAGGAGCTGCATGACGCCATGAAGGGCTTAGGAACCAAGGAGGG
TGTCATCATTGAGATCCTGGCCTCTCGGACCAAGAACCAGCTGCGGGAGATAATGAAG
GCGTATGAGGAAGACTATGGGTCCAGCCTGGAGGAGGACATCCAAGCAGACACAAGT
GGCTACCTGGAGAGGATCCTGGTGTGCCTCCTGCAGGGCAGCAGGGATGATGTGAGC
AGCTTTGTGGACCCAGGACTGGCCCTCCAAGACGCACAGGATCTGTATGCGGCAGGC
GAGAAGATTCGTGGGACTGATGAGATGAAATTCATCACCATCCTGTGCACGCGCAGTG
CCACTCACCTGCTGAGAGTGTTTGAAGAGTATGAGAAAATTGCCAACAAGAGCATTGAG
GACAGCATCAAGAGTGAGACCCATGGCTCACTGGAGGAGGCCATGCTCACTGTGGTGA
AATGCACCCAAAACCTCCACAGCTACTTTGCAGAGAGACTCTACTATGCCATGAAGGGA
GCAGGGACGCGTGATGGGACCCTGATAAGAAACATCGTTTCAAGGAGCGAGATTGACT
TAAATCTTATCAAATGTCACTTCAAGAAGATGTACGGCAAGACCCTCAGCAGCATGATC
ATGGAAGACACCAGCGGTGACTACAAGAACGCCCTGCTGAGCCTGGTGGGCAGCGAC
CCCTGAGGCACAGAAGAACAAGAGCAAAGACCATGAAGCCAGAGTCTCCAGGACTCCT
CACTCAACCTCGGCCATGGACGCAGGTTGGGTGTGAGGGGGGTCCCAGCCTTTCGGT
CTTCTATTTCCCTATTTCCAGTGCTTTCCAGCCGGGTTTCTGACCCAGAGGGTGGAACC
GGCCTGGACTCCTCTTCCCAACTTCCTCCAGGTCATTTCCCAGTGTGAGCACAATGCCA
ACCTTAGTGTTTCTCCAGCCAGACAGATGCCTCAGCATGAAGGGCTTGGGGACTTGTG
GATCATTCCTTCCTCCCTGCAGGAGCTTCCCAAGCTGGTCACAGAGTCTCCTGGGCAC
 
AGGTTATACAGACCCCAGCCCCATTCCCATCTACTGAAACAGGGTCTCCACAAGAGGG
GCCAGGGAATATGGGTTTTTAACAAGCGTCTTACAAAACACTTCTCTATCATGCAGCCG
GAGAGCTGGCTGGGAGCCCTTTTGTTTTAGAACACACATCCTTCAGCAGCTGAGAAAC
GAACACGAATCCATCCCAACCGAGATGCCATTAACATTCATCTAAAAATGTTAGGCTCTA
AATGGACGAAAAATTCTCTCGCCATCTTAATAACAAAATAAACTACAAATTCCTGACCCA
AGGACACTGTGTTATAAGAGGCGTGGGCTCCCCTGGTGGCTGACCAGGTCAGCTGCC
CTGGCCTTGCACCCCTCTGCATGCAGCACAGAAGGGTGTGACCATGCCCTCAGCACCA
CTCTTGTCCCCACTGAACGGCAACTGAGACTGGGTACCTGGAGATTCTGAAGTGCCTTT
GCTGTGGTTTTCAAAATAATAAAGATTTGTATTCAACTCAAAAAAAAAA
NM_003568.3 Homo sapiens annexin A9 (ANXA9), mRNA (SEQ ID NO: 31) 
CTCTACCAGGCCACACCGGAGGCAGTGCTCACACAGGCAAGCTACCAGGCC
ACAACAACGACACCCACCTCACCTCTGGCACCTCTGAGCATCCACGTACTTGCAAGAA
CTCTTGCTCACATCAGCTAAGAGATTGCACCTGCTGACCTAGAGATTCCGGCCTGTGCT
CCTGTGCTGCTGAGCAGGGCAACCAGTAGCACCATGTCTGTGACTGGCGGGAAGATG
GCACCGTCCCTCACCCAGGAGATCCTCAGCCACCTGGGCCTGGCCAGCAAGACTGCA
GCGTGGGGGACCCTGGGCACCCTCAGGACCTTCTTGAACTTCAGCGTGGACAAGGAT
GCGCAGAGGCTACTGAGGGCCATTACTGGCCAAGGCGTGGACCGCAGTGCCATTGTG
GACGTGCTGACCAACCGGAGCAGAGAGCAAAGGCAGCTCATCTCACGAAACTTCCAGG
AGCGCACCCAACAGGACCTGATGAAGTCTCTACAGGCAGCACTTTCCGGCAACCTGGA
GAGGATTGTGATGGCTCTGCTGCAGCCCACAGCCCAGTTTGACGCCCAGGAATTGAGG
ACAGCTCTGAAGGCCTCAGATTCTGCTGTGGACGTGGCCATTGAAATTCTTGCCACTCG
AACCCCACCCCAGCTGCAGGAGTGCCTGGCAGTCTACAAACACAATTTCCAGGTGGAG
GCTGTGGATGACATCACATCTGAGACCAGTGGCATCTTGCAGGACCTGCTGTTGGCCC
TGGCCAAGGGGGGCCGTGACAGCTACTCTGGAATCATTGACTATAATCTGGCAGAACA
AGATGTCCAGGCACTGCAGCGGGCAGAAGGACCTAGCAGAGAGGAAACATGGGTCCC
AGTCTTCACCCAGCGAAATCCTGAACACCTCATCCGAGTGTTTGATCAGTACCAGCGGA
GCACTGGGCAAGAGCTGGAGGAGGCTGTCCAGAACCGTTTCCATGGAGATGCTCAGG
TGGCTCTGCTCGGCCTAGCTTCGGTGATCAAGAACACACCGCTGTACTTTGCTGACAAA
CTTCATCAAGCCCTCCAGGAAACTGAGCCCAATTACCAAGTCCTGATTCGCATCCTTAT
CTCTCGATGTGAGACTGACCTTCTGAGTATCAGAGCTGAGTTCAGGAAGAAATTTGGGA
AGTCCCTCTACTCTTCTCTCCAGGATGCAGTGAAAGGGGATTGCCAGTCAGCCCTCCT
GGCCTTGTGCAGGGCTGAAGACATGTGAGACTTCCCTGCCCCACCCCACATGACATCC
GAGGATCTGAGATTTCCGTGTTTGGCTGAACCTGGGAGACCAGCTGGGCCTCCAAGTA
GGATAACCCCTCACTGAGCACCACATTCTCTAGCTTCTTGTTGAGGCTGGAACTGTTTC
TTTAAAATCCCTTAATTTTCCCATCTCAAAATTATATCTGTACCTGGGTCATCCAGCTCCT
 
TCTTGGGTGTGGGGAAATGAGTTTTCTTTGATAGTTTCTGCCTCACTCATCCCTCCTGTA
CCCTGGCCAGAACATCTCACTGATACTCGAATTCTTTTGGCAAA
NM_007193.4 Homo sapiens annexin A10 (ANXA10), mRNA (SEQ ID NO: 32) 
ATCCAGATTTGCTTTTACATTTTCTTGCCTGAGTCTGAGGTGAACAGTGAACAT
ATTTACATTTGATTTAACAGTGAACCTTAATTCTTTCTGGCTTCACAGTGAAACAAGTTTA
TGCAATCGATCAAATATTTTCATCCCTGAGGTTAACAATTACCATCAAAATGTTTTGTGG
AGACTATGTGCAAGGAACCATCTTCCCAGCTCCCAATTTCAATCCCATAATGGATGCCC
AAATGCTAGGAGGAGCACTCCAAGGATTTGACTGTGACAAAGACATGCTGATCAACATT
CTGACTCAGCGCTGCAATGCACAAAGGATGATGATTGCAGAGGCATACCAGAGCATGT
ATGGCCGGGACCTGATTGGGGATATGAGGGAGCAGCTTTCGGATCACTTCAAAGATGT
GATGGCTGGCCTCATGTACCCACCACCACTGTATGATGCTCATGAGCTCTGGCATGCC
ATGAAGGGAGTAGGCACTGATGAGAATTGCCTCATTGAAATACTAGCTTCAAGAACAAA
TGGAGAAATTTTCCAGATGCGAGAAGCCTACTGCTTGCAATACAGCAATAACCTCCAAG
AGGACATTTATTCAGAGACCTCAGGACACTTCAGAGATACTCTCATGAACTTGGTCCAG
GGGACCAGAGAGGAAGGATATACAGACCCTGCGATGGCTGCTCAGGATGCAATGGTC
CTATGGGAAGCCTGTCAGCAGAAGACGGGGGAGCACAAAACCATGCTGCAAATGATCC
TGTGCAACAAGAGCTACCAGCAGCTGCGGCTGGTTTTCCAGGAATTTCAAAATATTTCT
GGGCAAGATATGGTAGATGCCATTAATGAATGTTATGATGGATACTTTCAGGAGCTGCT
GGTTGCAATTGTTCTCTGTGTTCGAGACAAACCAGCCTATTTTGCTTATAGATTATATAG
TGCAATTCATGACTTTGGTTTCCATAATAAAACTGTAATCAGGATTCTCATTGCCAGAAG
TGAAATAGACCTGCTGACCATAAGGAAACGATACAAAGAGCGATATGGAAAATCCCTAT
TTCATGATATCAGAAATTTTGCTTCAGGGCATTATAAGAAAGCACTGCTTGCCATCTGTG
CTGGTGATGCTGAGGACTACTAAAATGAAGAGGACTTGGAGTACTGTGCACTCCTCTTT
CTAGACACTTCCAAATAGAGATTTTCTCACAAATTTGTACTGTTCATGGCACTATTAACA
AAACTATACAATCATATTTTCTCTTCTATCTTTGAAATTATTCTAAGCCAAAGAAAACTAT
GAATGAAAGTATATGATACTGAATTTGCCTACTATCCTGAATTTGCCTACTATCTAATCA
GCAATTAAATAAATTGTGCATGATGGAATAATAGAAAAATTGCATTGGAATAGATTTTATT
TAAATGTGAACCATCAACAACCTACAACAA
NM_145868.2 Homo sapiens annexin A11 (ANXA11), transcript variant b, mRNA (SEQ ID NO: 33) 
GGAGTTTTCCGCCCGGCGCTGACGGCTGCTGCGCCCGCGGCTCCCCAGTGC
CCCGAGTGCCCCGCGGGCCCCGCGAGCGGGAGTGGGACCCAGCCCCTAGGCAGAAC
CCAGGCGCCGCGCCCGGGACGCCCGCGGAGAGAGCCACTCCCGCCCACGTCCCATT
TCGCCCCTCGCGTCCGGAGTCCCCGTGGCCAGGTGTGTGTCTGGGGAAGAGACTTAC
AGAAGTGGAGTTGCTGAGTCAAAGATCTAACCATGAGCTACCCTGGCTATCCCCCGCC
CCCAGGTGGCTACCCACCAGCTGCACCAGGTGGTGGTCCCTGGGGAGGTGCTGCCTA
 
CCCTCCTCCGCCCAGCATGCCCCCCATCGGGCTGGATAACGTGGCCACCTATGCGGG
GCAGTTCAACCAGGACTATCTCTCGGGAATGGCGGCCAACATGTCTGGGACATTTGGA
GGAGCCAACATGCCCAACCTGTACCCTGGGGCCCCTGGGGCTGGCTACCCACCAGTG
CCCCCTGGCGGCTTTGGGCAGCCCCCCTCTGCCCAGCAGCCTGTTCCTCCCTATGGG
ATGTATCCACCCCCAGGAGGAAACCCACCCTCCAGGATGCCCTCATATCCGCCATACC
CAGGGGCCCCTGTGCCGGGCCAGCCCATGCCACCCCCCGGACAGCAGCCCCCAGGG
GCCTACCCTGGGCAGCCACCAGTGACCTACCCTGGTCAGCCTCCAGTGCCACTCCCTG
GGCAGCAGCAGCCAGTGCCGAGCTACCCAGGATACCCGGGGTCTGGGACTGTCACCC
CCGCTGTGCCCCCAACCCAGTTTGGAAGCCGAGGCACCATCACTGATGCTCCCGGCTT
TGACCCCCTGCGAGATGCCGAGGTCCTGCGGAAGGCCATGAAAGGCTTCGGGACGGA
TGAGCAGGCCATCATTGACTGCCTGGGGAGTCGCTCCAACAAGCAGCGGCAGCAGAT
CCTACTTTCCTTCAAGACGGCTTACGGCAAGGATTTGATCAAAGATCTGAAATCTGAAC
TGTCAGGAAACTTTGAGAAGACAATCTTGGCTCTGATGAAGACCCCAGTCCTCTTTGAC
ATTTATGAGATAAAGGAAGCCATCAAGGGGGTTGGCACTGATGAAGCCTGCCTGATTG
AGATCCTCGCTTCCCGCAGCAATGAGCACATCCGAGAATTAAACAGAGCCTACAAAGC
AGAATTCAAAAAGACCCTGGAAGAGGCCATTCGAAGCGACACATCAGGGCACTTCCAG
CGGCTCCTCATCTCTCTCTCTCAGGGAAACCGTGATGAAAGCACAAACGTGGACATGT
CACTCGCCCAGAGAGATGCCCAGGAGCTGTATGCGGCCGGGGAGAACCGCCTGGGAA
CAGACGAGTCCAAGTTCAATGCGGTTCTGTGCTCCCGGAGCCGGGCCCACCTGGTAG
CAGTTTTCAATGAGTACCAGAGAATGACAGGCCGGGACATTGAGAAGAGCATCTGCCG
GGAGATGTCCGGGGACCTGGAGGAGGGCATGCTGGCCGTGGTGAAATGTCTCAAGAA
TACCCCAGCCTTCTTTGCGGAGAGGCTCAACAAGGCCATGAGGGGGGCAGGAACAAA
GGACCGGACCCTGATTCGCATCATGGTGTCTCGCAGCGAGACCGACCTCCTGGACATC
AGATCAGAGTATAAGCGGATGTACGGCAAGTCGCTGTACCACGACATCTCGGGAGATA
CTTCAGGGGATTACCGGAAGATTCTGCTGAAGATCTGTGGTGGCAATGACTGAACAGT
GACTGGTGGCTCACTTCTGCCCACCTGCCGGCAACACCAGTGCCAGGAAAAGGCCAAA
AGAATGTCTGTTTCTAACAAATCCACAAATAGCCCCGAGATTCACCGTCCTAGAGCTTA
GGCCTGTCTTCCACCCCTCCTGACCCGTATAGTGTGCCACAGGACCTGGGTCGGTCTA
GAACTCTCTCAGGATGCCTTTTCTACCCCATCCCTCACAGCCTCTTGCTGCTAAAATAG
ATGTTTCATTTTTCTGACTCATGCAATCATTCCCCTTTGCCTGTGGCTAAGACTTGGCTT
CATTTCGTCATGTAATTGTATATTTTTATTTGGAGGCATATTTTCTTTTCTTACAGTCATTG
CCAGACAGAGGCATACAAGTCTGTTTGCTGCATACACATTTCTGGTGAGGGCGACTGG
GTGGGTGAAGCACCGTGTCCTCGCTGAGGAGAGAAAGGGAGGCGTGCCTGAGAAGGT
AGCCTGTGCATCTGGTGAGTGTGTCACGAGCTTTGTTACTGCCAAACTCACTCCTTTTT
AGAAAAAACAAAAAAAAAGGGCCAGAAAGTCATTCCTTCCATCTTCCTTGCAGAAACCA
CGAGAACAAAGCCAGTTCCCTGTCAGTGACAGGGCTTCTTGTAATTTGTGGTATGTGCC
TTAAACCTGAATGTCTGTAGCCAAAACTTGTTTCCACATTAAGAGTCAGCCAGCTCTGG
 
AATGGTCTGGAAATGTCTTCCTGGTACCAACTTGTTTTCTTCTGCTTGATTCTGCCCTGT
GGCTCAGAGGTCTGGCCTTATCAGCCAGTGAAAGTTCATGTAACCTTACGTAGAGATTT
GTGTGCAGGAAACCCTGAGCATACACTAGTTTGCAGGGACTCGTAAGGACATGGGAAG
GGAGGTTCCCGAAATCCAGGCAGGAGGCCCAGACACCTGAAAGGCAAAGGGATCTTG
GTTGGTTGCAGGTGCAGTGAAGTCCACTGAAGGTGTGGTGCGAAGAATGCAGTCCTTC
ACCCAGGTCCCAGGAGGGAAGAAGGGTGTGTGCTAATTCCTGGTGCCCCTCGGCGGG
GGCCAGAGAGAAGGATGGGGACAACCCAGAGAGTCACAAGACCAGTGCCTCCCCTCA
GGGTGCCTCCAGGCTGAAAGGGGCTCCTGGCTCTGGTCTCTGGGGACCCTGTGCCCG
TTGGTTGGTGGTGTGAGGGAAGAGAATCCATAAGAGAGTTTCTGAGAATTATGGTGTCA
TGTCCAGAAGCTAGAGCTTACCTTGCATCAGGGGTCTCCACCCACTCCTTTTCCAACCT
CCTGCGTTGAGGTTTAGAAAAGAGAGAATCGACTAGGCACTATGGCTCACGCCTGTAAT
CCAAGGACTTTGGGAAGCTGAGGTGAGAGGATCACTTGAGCTCAGGAGTTCAAGACTA
GCCTAGCCAACAGCGAGACCCCTGTCTCTACTAAAAAATTTGGCCAGGCGTGGTGGCT
CACGGCTGTAATCCCAGCACTTTGGGAGGTGAGGCGGGCAGATCACCTGAGGTCAGG
AGTTCGAGACCCAGCCTGGCCAACATGGTGAAACCCCATCTCTACTAAAAATACAAAAA
TTAGCCAGGCATGGTGGCACATTCCTGTAATCCCAGCTACACAGGATGCTGAGGCAGG
AGAATCACTTGAACCCAGGAGGCAGAGGTTGTAGTGAGCTGAGATCACACCATTGCAC
TTCAACCTGGGTGGACAGAGTGAGACTCTGTCTCAAAAAAAAAAAAAAATTTACCTGGC
ATTGTAGTGCATTCCCTATAGTCGGCTACTCTGGAGGCTGAGGCAGGAAGATCCTTAGA
GCCCAAGAAATTGAGGCCGTAGTAAGCTGTGATTACACCACTGCACTCCAGCCTGGAC
AACAGAGCGAGACCTTGTCTCAAATGAGAAAAAAACAAAAAGAAATGGGAGAATCCAGA
GAGACTAGGCTAGATCAAGCCTGCTGGGTCCTGGCAGGAGCCCCAGGGAGTAGCTCA
TCTGCAGACATTTGCTTGAGGACTACCCCCTAAACATAAAGGAAGAATGACATCCGAAG
GGTGTGGAGCAGCCATGAGCTGAGAACTAGCCTGGTCTACCTGAGATTGATGGCAGGT
CCTGGTCAACACGTCAGCTCTGCGTCAGAGTCCATGCCTCAAGCCCAAGCTGAAGCCC
CATCCCTGCTGCTCTCCCAAGAACTCCTCTGCTAGGGCAGGCCCCTTGCCCTTGGGTG
CCAGGTGGGACCTGCCTGATGGGATGGGGTGCTTGGCATATACAACTTGCCATGAACT
CAAGGTGACCCTGGGGGCCTCCTGAATTGTGATGGGGCCTAGAACCAATGTGCTCTGA
TGTGACCATATTCTGTGACATTACCTTGCCCTGTTTACTCCAAAGTTCCCAGCCTGGTG
CCCAGCAGGCAATATTGCACCTACAGACACATTTACTTTGGTTTCCAAAGTGTTTTTAGA
CATTTGAATTTGTTGCCAACATTTAAACATTGAGAGATTTCATATTTTTAAAAATCTGGAA
TTCTGGCTTCTCTTGAAAACTCAGAAATTCTGGCACTATGGGGCTTGCATTCCTGCATG
GCTGGAGCTGAGTTGCAGCTGCCCCTTTAGGCCTGTACTCCTTATTTGCTATAGGCTCC
GTCTTGTATTACACTAAGCCCATGTCACCCATTTGGCTCCTGCAGGCCTTTGGGTTTGA
GACCCTGGTCTACACACTTGGAGACCACCTGTTGTAAAGTACATGGATGTGCTTTGGTC
AAGGAATAGACCAAGGTGGATATCCAGGCCAGAGTGACTCAGCGAGTTTAGGTCACAG
GCGTATACTCCACTTGTTATATAACCTGCTTGTGTAAGTTCATACTTGGCTCAAAGCCAC
 
TATTGTTTGGAAAAGGTATAACTGCCCTGCTGACGCTGTACAGATGTTCTTGGGCTCGG
ATGGGCATGGCTCCACGTGGTGTGCACTAGCACCCAGAGAGAGTGAAGCTATTGACCC
CTGTAAGGGAGAGTGACCATCTGGCAGATAGATAGAGGGGAGCCAGGACATGGCTCA
GCTTGTGCCCAGAGGGAGAGTTAAGCCGCTGACCCTGTAGCCAGGGAGTGCACCTGC
AAGCATGGGGGTGGCAGGAGCCACAGAGCTGGCTGCTGAGAGGAGCTGCAGATCTGG
AGAAGACAGCCTAGGTAAAGGTGGACAGTGTGAGAGCTGCTGATGAGATAGCTGCTGA
ATAAAACTACATTTTACCTGCCTATGGCCCGCCAGGTTTTCTTTCAGCTATCGCCCATCC
ACCCAGTCCCCTCGAACCTCAGCATGGGCTGGAACCTGACCCTGGGCATGACATTTGG
CATAGTTGTGGACCTGACACCTGTGTTTGTCCTAGTCCTGTTTCTCCCTGCCTTCCTGTT
CCTCTCGCTGCCCTCATGGTCACTCCCAAGAGATCCAACCCATGTTAAGTATGGGCTG
GAGGACTGCATGAATGCCTCATGATCTTCCCAGAGGCAAAGGCACCTACTGCCTTCCA
AGGTCAGTGGGAGGTTGGGATCAACACTGTTTATTATGCTTAGGACAAAAAAGATAGGG
AGAAAGATGTGCAACCTTACAGGTCATCTTTCTGGGATAGAACACAATGGGTCTTCTCC
TGCCTCCTGGATATGTTAGTCAAGGCCAGTCCATGCTACACATCTAGTCTGACTTCTAA
AATAGAAGCACCAGATGAATTCAGCCCTGAGAGAATTTTCAGCAGCTGTGGGGGCGCT
GGAGGAAACACTATTAAATAGTTTTGCACCTGAGACAGATAGCCTCACTCGCCTCACCC
TAGTCCTGGTGGCATTTGTCTCAGGTGCAAAATTTAAGAAAGAAACCTTGGAGTGCTCA
CCCTGTGGCTGGGTAGATGGTCCTAAAGTGGTGGTTTTCAAGCCTGAGTGTGTATCAG
GATCATCAGGGGAGCTTGCTAAAGAGCAGTTCCTGCGGTCAGACCCTCATGCATTTTG
AGCAGGTGTGGGGACTGGGAAACTGCATCTGTAACCTGCTGTAATCTAACGCTTATCTA
AATACTACTGTGCTCACACAGAGAACACCGCAAAAGTAGAGGTGTTCCTCCAGAGGGC
AGGTGAGCAGATGGCACAGTCTGCTTGGAATTCAGTCAGGTGATGAGAGATGAGATGA
GGCACTCCTAGCTTTGGGAAGAGGGAGCTGAAAGATGAACCTTTGCAGGTGCCCACGG
TCAAAGTGGTGGTTTAATGCCATGCCATGCCCATTTTCTGTTGGCCTTGGCAGGGAGTT
ACAGCCCTACCTTAGGACCTGGCTCCTTATTTCTGCTGTAGGCTCTTTCCTGCCCTGGC
CGAGATGGAGTGGAATGAGACCTAGAAACATCAAGCTAAATACATGTCCTCAGAAAGAT
AAAGGTTTACATTTTCACCCCCATCAAATCTGAAAGCTCTCTGCCTGTGTTTTTCTAAGG
GATAGGGACATCATTACTCAGTCCACAACCTGGACTCATGTAGGGTCCCCTGTCAGTAA
AGGAGTCAGTCAAGCCCACCAGGTATACCAAGGACTCTTACCCTCAGCCCCTACTCCTT
GGAAAGCTGCCCCTTGGCCTAATATTGGTGTTTAGCTTGAGCCTGACTCCTTCTCAACA
CTAAGAGCTGATGAAGTCCTGAAGCAGAAAGAGCTCTGACCTGAGAGTCAAACATCCTT
ATTCTGATCTCAGCTCAGCCCCTGATTTGTTGTGTGACCCTGGATATGTCACTTCCTGT
CTTTTTGACTTTTTAAAATGAAGGGTAGACTAGAGGAGAGCTTCTAAAACTTTAATGTGG
TCAACGAAATGGAATAGGAAATTCCACAAGTCTGTCCTTCCACAAAAGCAGCAAATAAG
GTGGCAAAAACTCAAATTTATGGGAACTCTGGAAACGAATTGAAAGTTTACAGCAATCA
GGTGAATACCTAAGAATAAAAGCTGGATTTAGTAAGA
NM_001278409.1 Homo sapiens annexin A11 (ANXA1 1), transcript variant f, mRNA (SEQ ID NO: 34) 
GCACTGCCTCTGGCACCTGGGGCAGCCGCGCCCGCGGAGTTTTCCGCCCGG
CGCTGACGGCTGCTGCGCCCGCGGCTCCCCAGTGCCCCGAGTGCCCCGCGGGCCCC
GCGAGCGGGAGTGGGACCCAGCCCCTAGGCAGAACCCAGGCGCCGCGCCCGGGACG
CCCGCGGAGAGAGCCACTCCCGCCCACGTCCCATTTCGCCCCTCGCGTCCGGAGTCC
CCGTGGCCAGGTGTGTGTCTGGGGAAGAGACTTACAGAAGTGGAGTTGCTGAGTCAAA
GATCTAACCATGAGCTACCCTGGCTATCCCCCGCCCCCAGGTGGCTACCCACCAGCTG
CACCAGGTTGGCTGGCACTGGCCTGGGTTCTCTCTCTATAGTAGAAATCCTGCCATCCA
GATCCTGCCACTGCCACCTTTGCTAGCACAGCTGAGCAGCCTCTGAGCAGCAAGAGAG
GAGGAGGCAGGAAATTTAGGGAAGGTTCTTCCTGGAGGGTCTGGAGCCCTGGAGATG
AAGAGCCGATCCGAAGCTGCCATGTAGAGGAAAGCATCTAACAGGCCAGAGGCCCCAT
GATGATGTCGAATGCCCATCGGGCACCCAGCTGAGCCCTGCAGGTGGTGGTCCCTGG
GGAGGTGCTGCCTACCCTCCTCCGCCCAGCATGCCCCCCATCGGGCTGGATAACGTG
GCCACCTATGCGGGGCAGTTCAACCAGGACTATCTCTCGGGAATGGCGGCCAACATGT
CTGGGACATTTGGAGGAGCCAACATGCCCAACCTGTACCCTGGGGCCCCTGGGGCTG
GCTACCCACCAGTGCCCCCTGGCGGCTTTGGGCAGCCCCCCTCTGCCCAGCAGCCTG
TTCCTCCCTATGGGATGTATCCACCCCCAGGAGGAAACCCACCCTCCAGGATGCCCTC
ATATCCGCCATACCCAGGGGCCCCTGTGCCGGGCCAGCCCATGCCACCCCCCGGACA
GCAGCCCCCAGGGGCCTACCCTGGGCAGCCACCAGTGACCTACCCTGGTCAGCCTCC
AGTGCCACTCCCTGGGCAGCAGCAGCCAGTGCCGAGCTACCCAGGATACCCGGGGTC
TGGGACTGTCACCCCCGCTGTGCCCCCAACCCAGTTTGGAAGCCGAGGCACCATCACT
GATGCTCCCGGCTTTGACCCCCTGCGAGATGCCGAGGTCCTGCGGAAGGCCATGAAA
GGCTTCGGGACGGATGAGCAGGCCATCATTGACTGCCTGGGGAGTCGCTCCAACAAG
CAGCGGCAGCAGATCCTACTTTCCTTCAAGACGGCTTACGGCAAGGATTTGATCAAAGA
TCTGAAATCTGAACTGTCAGGAAACTTTGAGAAGACAATCTTGGCTCTGATGAAGACCC
CAGTCCTCTTTGACATTTATGAGATAAAGGAAGCCATCAAGGGGGTTGGCACTGATGAA
GCCTGCCTGATTGAGATCCTCGCTTCCCGCAGCAATGAGCACATCCGAGAATTAAACA
GAGCCTACAAAGCAGAATTCAAAAAGACCCTGGAAGAGGCCATTCGAAGCGACACATC
AGGGCACTTCCAGCGGCTCCTCATCTCTCTCTCTCAGGGAAACCGTGATGAAAGCACA
AACGTGGACATGTCACTCGCCCAGAGAGATGCCCAGGAGCTGTATGCGGCCGGGGAG
AACCGCCTGGGAACAGACGAGTCCAAGTTCAATGCGGTTCTGTGCTCCCGGAGCCGG
GCCCACCTGGTAGCAGTTTTCAATGAGTACCAGAGAATGACAGGCCGGGACATTGAGA
AGAGCATCTGCCGGGAGATGTCCGGGGACCTGGAGGAGGGCATGCTGGCCGTGGTG
AAATGTCTCAAGAATACCCCAGCCTTCTTTGCGGAGAGGCTCAACAAGGCCATGAGGG
GGGCAGGAACAAAGGACCGGACCCTGATTCGCATCATGGTGTCTCGCAGCGAGACCG
ACCTCCTGGACATCAGATCAGAGTATAAGCGGATGTACGGCAAGTCGCTGTACCACGA
 
CATCTCGGGAGATACTTCAGGGGATTACCGGAAGATTCTGCTGAAGATCTGTGGTGGC
AATGACTGAACAGTGACTGGTGGCTCACTTCTGCCCACCTGCCGGCAACACCAGTGCC
AGGAAAAGGCCAAAAGAATGTCTGTTTCTAACAAATCCACAAATAGCCCCGAGATTCAC
CGTCCTAGAGCTTAGGCCTGTCTTCCACCCCTCCTGACCCGTATAGTGTGCCACAGGA
CCTGGGTCGGTCTAGAACTCTCTCAGGATGCCTTTTCTACCCCATCCCTCACAGCCTCT
TGCTGCTAAAATAGATGTTTCATTTTTCTGACTCATGCAATCATTCCCCTTTGCCTGTGG
CTAAGACTTGGCTTCATTTCGTCATGTAATTGTATATTTTTATTTGGAGGCATATTTTCTT
TTCTTACAGTCATTGCCAGACAGAGGCATACAAGTCTGTTTGCTGCATACACATTTCTG
GTGAGGGCGACTGGGTGGGTGAAGCACCGTGTCCTCGCTGAGGAGAGAAAGGGAGG
CGTGCCTGAGAAGGTAGCCTGTGCATCTGGTGAGTGTGTCACGAGCTTTGTTACTGCC
AAACTCACTCCTTTTTAGAAAAAACAAAAAAAAAGGGCCAGAAAGTCATTCCTTCCATCT
TCCTTGCAGAAACCACGAGAACAAAGCCAGTTCCCTGTCAGTGACAGGGCTTCTTGTAA
TTTGTGGTATGTGCCTTAAACCTGAATGTCTGTAGCCAAAACTTGTTTCCACATTAAGAG
TCAGCCAGCTCTGGAATGGTCTGGAAATGTCA
NM_004306.4 Homo sapiens annexin A13 (ANXA13), transcript variant 1, mRNA (SEQ ID NO: 35) 
GCCTGTAGGAGGACTGATCTCTTGATGAAATACAGAAAAACCATCTCAGAAAA
AGGAAAATGGGCAATCGTCATGCTAAAGCGAGCAGTCCTCAGGGTTTTGATGTGGATC
GAGATGCCAAAAAGCTGAACAAAGCCTGCAAAGGAATGGGGACCAATGAAGCAGCCAT
CATTGAAATCTTATCGGGCAGGACATCAGATGAGAGGCAACAAATCAAGCAAAAGTACA
AGGCAACGTACGGCAAGGAGCTGGAGGAAGTACTCAAGAGTGAGCTGAGTGGAAACTT
CGAGAAGACAGCGTTGGCCCTTCTGGACCGTCCCAGCGAGTACGCCGCCCGGCAGCT
GCAGAAGGCTATGAAGGGTCTGGGCACAGATGAGTCCGTCCTCATTGAGGTCCTGTGC
ACGAGGACCAATAAGGAAATCATCGCCATTAAAGAGGCCTACCAAAGGCTATTTGATAG
GAGCCTCGAATCAGATGTCAAAGGTGATACAAGTGGAAACCTAAAAAAAATCCTGGTGT
CTCTGCTGCAGGCTAATCGCAATGAAGGAGATGACGTGGACAAAGATCTAGCTGGTCA
GGATGCCAAAGATCTGTATGATGCAGGGGAAGGCCGCTGGGGCACTGATGAGCTTGC
GTTCAATGAAGTCCTGGCCAAGAGGAGCTACAAGCAGTTACGAGCCACCTTTCAAGCC
TATCAAATTCTCATTGGCAAAGACATAGAAGAAGCCATTGAAGAAACATCAGGCGA
CTTGCAGAAGGCCTATTTAACTCTCGTGAGATGTGCCCAGGATTGTGAGGACTATTTTG
CTGAACGTCTGTACAAGTCGATGAAGGGTGCGGGGACCGATGAGGAGACGTTGATTCG
CATAGTCGTGACCAGGGCCGAGGTGGACCTTCAGGGGATCAAAGCAAAGTTCCAAGAG
AAGTATCAGAAGTCTCTCTCTGACATGGTTCGCTCAGATACCTCCGGGGACTTCCGGAA
ACTGCTAGTAGCCCTCTTGCACTGAGCCAAGCCAGGGCAATAGGAACACAGGGTGGAA
CCGCCTTTGTCAAGAGCACATTCCAAATCAAACTTGCAAATGAGACTCCCGCACGAAAA
CCCTTAAGAGTCCCGGATTACTTTCTTGGCAGCTTAAGTGGCGCAGCCAGGCCAAGCT
 
GTGTAAGTTAAGGGCAGTAACGTTAAGATGCGTGGGCAGGGCACCTTGAACTCTGGCT
TAGCAAGCATCTAGGCTGCCTCTTCACTTTCTTTTAGCATGGTAACTGGATGTTTTCTAA
ACACTAATGAAATCAGCAGTTGATGAAAAAACTATGCATTTGTAATGGCACATTTAGAAG
GATATGCATCACACAAGTAAGGTACAGGAAAGACAAAATTAAACAATTTATTAATTTTCC
TTCTGTGTGTTCAATTTGAAAGCCTCATTGTTAATTAAAGTTGTGGATTATGCCTCTA
NM_001003954.2 Homo sapiens annexin A13 (ANXA13), transcript variant 2, mRNA (SEQ ID NO: 36) 
ATTATGTCCGGGGGGAAAACTGTTGTAAACTTTGCCTGTAGGAGGACTGATCT
CTTAATGAAATACAGAAAAACCATCTCAGAAAAAGGAAAATGGGCAATCGTCATAGCCA
GTCGTACACCCTCTCAGAAGGCAGTCAACAGTTGCCTAAAGGGGACTCCCAACCCTCG
ACAGTCGTGCAGCCTCTCAGCCACCCATCACGGAATGGAGAGCCAGAGGCCCCACAG
CCTGCTAAAGCGAGCAGTCCTCAGGGTTTTGATGTGGATCGAGATGCCAAAAAGCTGA
ACAAAGCCTGCAAAGGAATGGGGACCAATGAAGCAGCCATCATTGAAATCTTATCGGG
CAGGACATCAGATGAGAGGCAACAAATCAAGCAAAAGTACAAGGCAACGTACGGCAAG
GAGCTGGAGGAAGTACTCAAGAGTGAGCTGAGTGGAAACTTCGAGAAGACAGCGTTGG
CCCTTCTGGACCGTCCCAGCGAGTACGCCGCCCGGCAGCTGCAGAAGGCTATGAAGG
GTCTGGGCACAGATGAGTCCGTCCTCATTGAGGTCCTGTGCACGAGGACCAATAAGGA
AATCATCGCCATTAAAGAGGCCTACCAAAGGCTATTTGATAGGAGCCTCGAATCAGATG
TCAAAGGTGATACAAGTGGAAACCTAAAAAAAATCCTGGTGTCTCTGCTGCAGGCTAAT
CGCAATGAAGGAGATGACGTGGACAAAGATCTAGCTGGTCAGGATGCCAAAGATCTGT
ATGATGCAGGGGAAGGCCGCTGGGGCACTGATGAGCTTGCGTTCAATGAAGTCCTGG
CCAAGAGGAGCTACAAGCAGTTACGAGCCACCTTTCAAGCCTATCAAATTCTCATTGGC
AAAGACATAGAAGAAGCCATTGAAGAAACATCAGGCGACTTGCAGAAGGCCTATTT
AACTCTCGTGAGATGTGCCCAGGATTGTGAGGACTATTTTGCTGAACGTCTGTACAAGT
CGATGAAGGGTGCGGGGACCGATGAGGAGACGTTGATTCGCATAGTCGTGACCAGGG
CCGAGGTGGACCTTCAGGGGATCAAAGCAAAGTTCCAAGAGAAGTATCAGAAGTCTCT
CTCTGACATGGTTCGCTCAGATACCTCCGGGGACTTCCGGAAACTGCTAGTAGCCCTC
TTGCACTGAGCCAAGCCAGGGCAATAGGAACACAGGGTGGAACCGCCTTTGTCAAGAG
CACATTCCAAATCAAACTTGCAAATGAGACTCCCGCACGAAAACCCTTAAGAGTCCCGG
ATTACTTTCTTGGCAGCTTAAGTGGCGCAGCCAGGCCAAGCTGTGTAAGTTAAGGGCA
GTAACGTTAAGATGCGTGGGCAGGGCACCTTGAACTCTGGCTTAGCAAGCATCTAGGC
TGCCTCTTCACTTTCTTTTAGCATGGTAACTGGATGTTTTCTAAACACTAATGAAATCAG
CAGTTGATGAAAAAACTATGCATTTGTAATGGCACATTTAGAAGGATATGCATCACACAA
GTAAGGTACAGGAAAGACAAAATTAAACAATTTATTAATTTTCCTTCTGTGTGTTCAATTT
GAAAGCCTCATTGTTAATTAAAGTTGTGGATTATGCCTCTAAAAAAAAAAAAAAAAAA
AA
NM_001363114.2 Homo sapiens annexin A6 (ANXA6), transcript variant 3, mRNA (SEQ ID NO: 46):

GCGGTTGCTGCTGGGCTAACGGGCTCCGATCCAGCGAGCGCTGCGTCCTCGAGTCCC
TGCGCCCGTGCGTCCGTCTGCGACCCGAGGCCTCCGCTGCGCGTGGATTCTGCTGCG
AACCGGAGACCATGGCCAAACCAGCACAGGGTGCCAAGTACCGGGGCTCCATCCATG
ACTTCCCAGGCTTTGACCCCAACCAGGATGCCGAGGCTCTGTACACTGCCATGAAGGG
CTTTGGCAGTGACAAGGAGGCCATACTGGACATAATCACCTCACGGAGCAACAGGCAG
AGGCAGGAGGTCTGCCAGAGCTACAAGTCCCTCTACGGCAAGGACCTCATTGCTGATT
TAAAGTATGAATTGACGGGCAAGTTTGAACGGTTGATTGTGGGCCTGATGAGGCCACC
TGCCTATTGTGATGCCAAAGAAATTAAAGATGCCATCTCGGGCATTGGCACTGATGAGA
AGTGCCTCATTGAGATCTTGGCTTCCCGGACCAATGAGCAGATGCACCAGCTGGTGGC
AGCATACAAAGATGCCTACGAGCGGGACCTGGAGGCTGACATCATCGGCGACACCTCT
GGCCACTTCCAGAAGATGCTTGTGGTCCTGCTCCAGGGAACCAGGGAGGAGGATGAC
GTAGTGAGCGAGGACCTGGTACAACAGGATGTCCAGGACCTATACGAGGCAGGGGAA
CTGAAATGGGGAACAGATGAAGCCCAGTTCATTTACATCTTGGGAAATCGCAGCAAGCA
GCATCTTCGGTTGGTGTTCGATGAGTATCTGAAGACCACAGGGAAGCCGATTGAAGCC
AGCATCCGAGGGGAGCTGTCTGGGGACTTTGAGAAGCTAATGCTGGCCGTAGTGAAGT
GTATCCGGAGCACCCCGGAATATTTTGCTGAAAGGCTCTTCAAGGCTATGAAGGGCCT
GGGGACTCGGGACAACACCCTGATCCGCATCATGGTCTCCCGTAGTGAGTTGGACATG
CTCGACATTCGGGAGATCTTCCGGACCAAGTATGAGAAGTCCCTCTACAGCATGATCAA
GAATGACACCTCTGGCGAGTACAAGAAGACTCTGCTGAAGCTGTCTGGGGGAGATGAT
GATGCTGCTGGCCAGTTCTTCCCGGAGGCAGCGCAGGTGGCCTATCAGATGTGGGAA
CTTAGTGCAGTGGCCCGAGTAGAGCTGAAGGGAACTGTGCGCCCAGCCAATGACTTCA
ACCCTGACGCAGATGCCAAAGCGCTGCGGAAAGCCATGAAGGGACTCGGGACTGACG
AAGACACAATCATCGATATCATCACGCACCGCAGCAATGTCCAGCGGCAGCAGATCCG
GCAGACCTTCAAGTCTCACTTTGGCCGGGACTTAATGACTGACCTGAAGTCTGAGATCT
CTGGAGACCTGGCAAGGCTGATTCTGGGGCTCATGATGCCACCGGCCCATTACGATGC
CAAGCAGTTGAAGAAGGCCATGGAGGGAGCCGGCACAGATGAAAAGGCTCTTATTGAA
ATCCTGGCCACTCGGACCAATGCTGAAATCCGGGCCATCAATGAGGCCTATAAGGAGG
ACTATCACAAGTCCCTGGAGGATGCTCTGAGCTCAGACACATCTGGCCACTTCAGGAG
GATCCTCATTTCTCTGGCCACGGGGCATCGTGAGGAGGGAGGAGAAAACCTGGACCA
GGCACGGGAAGATGCCCAGGAAATAGCAGACACACCTAGTGGAGACAAAACTTCCTTG
GAGACACGTTTCATGACGATCCTGTGTACCCGGAGCTATCCGCACCTCCGGAGAGTCT
TCCAGGAGTTCATCAAGATGACCAACTATGACGTGGAGCACACCATCAAGAAGGAGAT
GTCTGGGGATGTCAGGGATGCATTTGTGGCCATTGTTCAAAGTGTCAAGAACAAGCCT
CTCTTCTTTGCCGACAAACTTTACAAATCCATGAAGGGTGCTGGCACAGATGAGAAGAC
TCTGACCAGGATCATGGTATCCCGCAGTGAGATTGACCTGCTCAACATCCGGAGGGAA

TTCATTGAGAAATATGACAAGTCTCTCCACCAAGCCATTGAGGGTGACACCTCCGGAGA
CTTCCTGAAGGCCTTGCTGGCTCTCTGTGGTGGTGAGGACTAGGGCCACAGCTTTGGC
GGGCACTTCTGCCAAGAAATGGTTATCAGCACCAGCCGCCATGGCCAAGCCTGATTGT
TCCAGCTCCAGAGACTAAGGAAGGGGCAGGGGTGGGGGGAGGGGTTGGGTTGGGCT
CTTATCTTCAGTGGAGCTTAGGAAACGCTCCCACTCCCACGGGCCATCGAGGGCCCAG
CACGGCTGAGCGGCTGAAAAACCGTAGCCATAGATCCTGTCCACCTCCACTCCCCTCT
GACCCTCAGGCTTTCCCAGCTTCCTCCCCTTGCTACAGCCTCTGCCCTGGTTTGGGCT
ATGTCAGATCCAAAAACATCCTGAACCTCTGTCTGTAAAATGAGTAGTGTCTGTACTTTG
AATGAGGGGGTTGGTGGCAGGGGCCAGTTGAATGTGCTGGGCGGGGTGGTGGGAAG
GATAGTAAATGTGCTGGGGCAAACTGACAAATCTTCCCATCCATTTCACCACCCATCTC
CATCCAGGCCGCGCTAGAGTACTGGACCAGGAATTTGGATGCCTGGGTTCAAATCTGC
ATCTGCCATGCACTTGTTTCTGACCTTAGGCCAGCCCCTTTCCCTCCCTGAGTCTCTAT
TTTCTTATCTACAATGAGACAGTTGGACAAAAAAATCTTGGCTTCCCTTCTAACATTAACT
TCCTAAAGTATGCCTCCGATTCATTCCCTTGACACTTTTTATTTCTAAGGAAGAAATAAAA
AGAGATACACAAACACATAAACACA

### THERAPEUTIC ENDPOINTS

In various aspects of the disclosure, use of the agent(s) and optional additional agent(s) as described herein provide one or more benefits related to specific therapeutic endpoints relative to a patient not receiving the agent(s) and/or additional agent(s).

Creatine kinase (CK) is a clinically validated serum biomarker of skeletal muscle, cardiac, kidney, and brain injury. Lactate dehydrogenase (LDH) is a clinically validated serum biomarker of skeletal muscle, cardiac, kidney, liver, lung, and brain injury. Creatine kinase and lactate dehydrogenase levels in serum are elevated with both acute and chronic tissue injury. In theoretical or verified conditions of comparable muscle mass levels, a reduction in creatine kinase and/or lactate dehydrogenase may be indicative of enhanced repair or protection against injury. Aspartate aminotransferase (AST) is yet another clinically validated serum biomarker of skeletal muscle, cardiac, kidney, liver, and brain injury. Additionally, increased serum troponin is indicative of cardiac injury, while elevated alanine transaminase (ALT) is a biomarker of liver injury. Reduction in AST, ALT, or troponin in the acute period following injury may indicate enhanced repair or protection against injury. Evan's blue due is a vital dye that binds serum albumin and is normally excluded from healthy, intact muscle. Membrane disruption due to acute or chronic injury promotes the influx of dye into the damaged cell. Evan's blue dye is commonly used to quantify cellular damage in experimental settings, measuring inherent dye fluorescence and/or through measuring radiolabeled-dye uptake. Reduction in dye uptake after acute injury or in models of chronic damage would indicate protection against injury and/or enhanced repair. Indocyanine green (ICG) is a near-infared dye that binds plasma proteins and is used clinically to evaluate blood flow and tissue damage (ischemia; necrosis) in organs including heart, liver, kidney, skin, vasculature, lung, muscle and eye. Improved blood flow and reduction in ischemic areas indicate protection from injury and/ or improved repair.

It is contemplated that increasing membrane integrity and repair results in enhanced function measured through multiple modalities including plethysmography, echocardiography, muscle force, 6-min walk test. Additionally, histological benefits may be noted, including decreased necrosis, decreased inflammation, reduced fibrosis, reduced fatty infiltrate and reduced edema. These beneficial effects may also be visible through MR and PET imaging.

### DOSING/ADMINISTRATION/KITS

A particular administration regimen for a particular subject will depend, in part, upon the agent and optional additional agent used, the amount of the agent and optional additional agent administered, the route of administration, the particular ailment being treated, and the cause and extent of any side effects. The amount of agent and optional additional agent administered to a subject (*e.g.*, a mammal, such as a human) is sufficient to effect the desired response. Dosage typically depends upon a variety of factors, including the particular agent and/or additional agent employed, the age and body weight of the subject, as well as the existence and severity of any disease or disorder in the subject. The size of the dose also will be determined by the route, timing, and frequency of administration. Accordingly, the clinician may titer the dosage and modify the route of administration to obtain optimal therapeutic effect, and conventional range-finding techniques are known to those of ordinary skill in the art. Purely by way of illustration, in some embodiments, the method comprises administering an agent (*e.g.,* a protein), *e.g.,* from about 0.1 µg/kg up to about 100 mg/kg or more, depending on the factors mentioned above. In other embodiments, the dosage may range from 1 µg/kg up to about 75 mg/kg; or 5 µg/kg up to about 50 mg/kg; or 10 µg/kg up to about 20 mg/kg. In certain embodiments, the dose comprises about 0.5 mg/kg to about 20 mg/kg (e.g., about 1 mg/kg, 1.5 mg/kg, 2 mg/kg, 2.3 mg/kg, 2.5 mg/kg, 3 mg/kg, 3.5 mg/kg, 4 mg/kg, 4.5 mg/kg, 5 mg/kg, 5.5 mg/kg, 6 mg/kg, 6.5 mg/kg, 7 mg/kg, 8 mg/kg, 9 mg/kg, or 10 mg/kg) of agent and optional additional agent. In embodiments in which an agent and additional agent are administered, the above dosages are contemplated to represent the amount of each agent administered, or in further embodiments the dosage represents the total dosage administered. In some embodiments wherein a chronic condition is treated, it is envisioned that a subject will receive the agent and/or additional agent over a treatment course lasting weeks, months, or years, and may require one or more doses daily or weekly. In any of the aspects or embodiments of the disclosure, the amount of an annexin protein in a pharmaceutical composition is from about 0.1 µg/kg up to about 100 mg/kg or more, depending on the factors mentioned above. In other embodiments, the dosage may range from 1 µg/kg up to about 75 mg/kg; or 5 µg/kg up to about 50 mg/kg; or 10 µg/kg up to about 20 mg/kg. In some embodiments, the dose comprises about 0.5 mg/kg to about 20 mg/kg (e.g., about 1 mg/kg, 1.5 mg/kg, 2 mg/kg, 2.3 mg/kg, 2.5 mg/kg, 3 mg/kg, 3.5 mg/kg, 4 mg/kg, 4.5 mg/kg, 5 mg/kg, 5.5 mg/kg, 6 mg/kg, 6.5 mg/kg, 7 mg/kg, 8 mg/kg, 9 mg/kg, or 10 mg/kg) of annexin protein. Dosages are also contemplated for once daily, twice daily (BID) or three times daily (TID) dosing. A unit dose may be formulated in either capsule or tablet form. In other embodiments, the agent and optional additional agent is administered to treat an acute condition (e.g., acute muscle injury or acute myocardial injury) for a relatively short treatment period, *e.g.*, one to 14 days.

Suitable methods of administering a physiologically-acceptable composition, such as a pharmaceutical composition comprising an agent (*e.g.*, a recombinant protein) and optional additional agent described herein, are well known in the art. Although more than one route can be used to administer an agent and/or additional agent, a particular route can provide a more immediate and more effective avenue than another route. Depending on the circumstances, a pharmaceutical composition is applied or instilled into body cavities, absorbed through the skin or mucous membranes, ingested, inhaled, and/or introduced into circulation. In some embodiments, a composition comprising an agent and/or additional agent is administered intravenously, intraarterially, or intraperitoneally to introduce an agent and optional additional agent into circulation. Non-intravenous administration also is appropriate, particularly with respect to low molecular weight therapeutics. In certain circumstances, it is desirable to deliver a pharmaceutical composition comprising the agent and/or additional agent orally, topically, sublingually, vaginally, rectally; through injection by intracerebral (intra-parenchymal), intracerebroventricular, intramuscular, intra-ocular, intraportal, intralesional, intramedullary, intrathecal, intraventricular, transdermal, subcutaneous, intranasal, urethral, or enteral means; by sustained release systems; or by implantation devices. If desired, the agent and/or additional agent is administered regionally via intraarterial or intravenous administration to a region of interest, *e.g.*, via the femoral artery for delivery to the leg. In one embodiment, the composition is administered via implantation of a membrane, sponge, or another appropriate material within or upon which the desired agent and optional additional agent has been absorbed or encapsulated. Where an implantation device is used, the device in one aspect is implanted into any suitable tissue, and delivery of the desired agent and/or additional agent is, in various embodiments, effected via diffusion, time-release bolus, or continuous administration. In other embodiments, the agent and optional additional agent is administered directly to exposed tissue during surgical procedures or treatment of injury, or is administered via transfusion of blood products. Therapeutic delivery approaches are well known to the skilled artisan, some of which are further described, for example, in U.S. Patent No. 5,399,363.

In some embodiments facilitating administration, the agent and optional additional agent in one embodiment is formulated into a physiologically acceptable composition comprising a carrier (*i.e.*, vehicle, adjuvant, buffer, or diluent). The particular carrier employed is limited only by chemico-physical considerations, such as solubility and lack of reactivity with the agent and/or additional agent, by the route of administration, and by the requirement of compatibility with the recipient organism. Physiologically acceptable carriers are well known in the art. Illustrative pharmaceutical forms suitable for injectable use include, without limitation, sterile aqueous solutions or dispersions and sterile powders for the extemporaneous preparation of sterile injectable solutions or dispersions (for example, see U.S. Patent No. 5,466,468). Injectable formulations are further described in, *e.g.*, Pharmaceutics and Pharmacy Practice, J. B. Lippincott Co., Philadelphia. Pa., Banker and Chalmers. eds., pages 238-250 (1982), and ASHP Handbook on Injectable Drugs, Toissel, 4th ed., pages 622-630 (1986), incorporated herein by reference).

A pharmaceutical composition comprising an agent (*e.g.*, a recombinant protein) and optional additional agent as provided herein is optionally placed within containers/kits, along with packaging material that provides instructions regarding the use of such pharmaceutical compositions. Generally, such instructions include a tangible expression describing the reagent concentration, as well as, in certain embodiments, relative amounts of excipient ingredients or diluents that may be necessary to reconstitute the pharmaceutical composition.

The disclosure thus includes administering to a subject one or more agent(s), in combination with one or more additional agent(s), each being administered according to a regimen suitable for that medicament. In some embodiments, the agent is a recombinant protein such as an annexin protein (*e.g.,* annexin A6). Administration strategies include concurrent administration (*i.e.,* substantially simultaneous administration) and non-concurrent administration (*i.e.,* administration at different times, in any order, whether overlapping or not) of the agent and one or more additional agents(s). It will be appreciated that different components are optionally administered in the same or in separate compositions, and by the same or different routes of administration

### EXAMPLES

The following examples show results from experiments in which muscle membrane repair was monitored with real-time high-resolution imaging to visualize annexin cap formation and Ca²⁺ dynamics after laser-induced injury. It is demonstrated herein that overexpression of annexin proteins induced extracellular bleb formation at the site of membrane injury, which correlated with decreased intracellular Ca²⁺ accumulation. Pretreatment with recombinant annexin A6 prevented muscle injury *in vitro* and *in vivo.* Post-treatment with recombinant annexin A6 enhanced muscle repair *in vivo.* Overexpression of annexin A6 enhanced myofiber membrane repair, while overexpression of a dominant-negative annexin A6 mutant protein decreased membrane repair capacity. In addition, treatment with extracellular recombinant annexin A6 protein reduced laser-induced muscle damage of both wildtype and dystrophic myofibers. Moreover, administration of recombinant annexin A6 protected against toxin-induced muscle injury *in vivo* using multiple routes of administration. Furthermore, in dystrophic mice administration of recombinant annexin A6 reduced circulating levels of creatine kinase and lactate dehydrogenase, indicating enhanced membrane repair. The data provided herein show that annexin proteins are important agonists of membrane repair and that recombinant annexin A6 is a therapeutic target to treat conditions resulting from membrane fragility like muscle injury and muscular dystrophies.

A polymorphism in *Anxa6,* the gene encoding annexin A6, was previously identified in several commonly used experimental mouse strains that correlated with impaired muscle repair (Demonbreun *et al.,* 2016a; Quattrocelli *et al.,* 2017b; Swaggart *et al.,* 2014). This polymorphism produced a truncated annexin A6 protein that interferes with sarcolemmal repair. Additional studies in mice have shown that loss of annexin A2 results in poor myofiber repair (Defour *et al.,* 2017).
Annexin Ca²⁺ binding during muscle membrane repair was investigated and the kinetics of annexin A1, A2, and A6 repair cap formation after injury was assessed. Mutations in Ca²⁺-coordinating residues in annexin A1, A2, and A6 interfered with normal annexin repair cap formation. Annexin overexpression promoted the formation of external blebs at the site of membrane injury, which were released from the repair cap. Overexpression of annexin A6 resulted in the formation of larger blebs being released from the repair cap. These vesicles were enriched in Ca²⁺-binding protein GCaMP5G, which correlated with a reduction of intracellular Ca²⁺ fluorescence at the site of injury. Overexpression of annexin A6 reduced membrane injury, while mutation of E233, a critical Ca²⁺-coordinating residue in annexin A6 interfered with annexin repair complex formation, and correlated with decreased repair capacity. Local and systemic treatment with recombinant annexin A6 reduced muscle damage and promoted membrane repair *in vivo.* The data provided herein identify a new role for annexins in bleb release from muscle membrane lesions during membrane repair and identifies annexin A6 as a therapeutic target to enhance membrane repair capacity.

### Example 1

### Materials and Methods

**Animals.** Wildtype mice from the 129T2/SvEmsJ background were bred and housed in a specific pathogen free facility on a 12-hour light/dark cycle and fed ad libitum in accordance with the Northwestern University's Institutional Animal Care and Use Committee regulations. 129T2/SvEmsJ (129T2) mice were purchased from the Jackson Laboratory (Ben Harbor, ME; Stock # 002065). mdx/hLTBP4 mice were generated as described in (Ceco *et al.,* 2014; Quattrocelli *et al.,* 2017b). *sgcg-*null mice were generated as described (Hack et al 1998). Two to three-month-old male and female were used for all experiments.

**Plasmids.** Plasmids encoding annexin A1, A2 and A6 with a carboxyl-terminal turboGFP tag were obtained from Origene (Rockville, MD). Subcloning of annexin A1, A2, and A6 to replace the GFP tag with tdTomato (Addgene) was performed by Mutagenix (Suwanee, GA). Site directed mutagenesis was performed by Mutagenix on annexin A1-GFP, A2-GFP and A6-GFP to create the Ca²⁺-binding mutants A1-D171A-GFP, A2-D161A-GFP, A6-D149A-GFP, and A6-E233A-GFP. Constructs were sequenced to verify mutagenesis. Plasmid DNA was isolated using the Qiagen endo-free Maxi prep kit (Qiagen #12362). The Ca²⁺ sensor GCaMP5G was purchased from (Addgene #31788).

**Sequence comparison and protein schematics.** Protein ribbon diagrams were generated using Swiss-PdbViewer using solved crystal structures of annexin A1 (1MCX), A2 (2HYW), and annexin A6 (1AVC) available on www.rcsb.org. Clustal Omega from the European Bioinformatics Institute (EMBL-EBI) was used to align annexin mouse sequences from www.ncbi.nlm.nih.gov annexin A1 (NM_010730; SEQ ID NO: 37), A2 (NM_007585; SEQ ID NO: 38) and A6 (NM_013472:SEQ ID NO:39).).

**Electroporation, myofiber isolation, laser injury, cap and vesicle measurement.** Flexor digitorum brevis (FDB) fibers were transfected with endo-free plasmid DNA by in vivo electroporation. Methods were described previously in (Demonbreun and McNally, 2015; Demonbreun *et al.,* 2016b; DiFranco *et al.,* 2009). Z-stack projections were acquired from consecutive acquisitions after the final time-lapse frame, approximately 4 minutes post damage, with a 0.125µM step size between slices. Z-stack renderings were constructed in FIJI. Measurement of the cap area and feret diameter were conducted from a single slice near the middle of the z-stack using FIJI imaging tools. Fibers expressing similar levels of tagged or GCaMP5G protein were compared. GCaMP5G Ca²⁺ fluorescence was measured from the acquired timelapse images, using a standard rectangular ROI, placed inside the myofiber below the site of damage using FIJI. Fluorescence was expressed as F/F0. External vesicle number and GCaMP5G area were measured from endpoint z-stacks and max projection images using FIJI. Vesicles were considered external if they were found outside the sarcolemma assessed in brightfield and fluorescent channels. All measurements were acquired from myofibers isolated from at least n≥3 mice, n≥3 myofibers per mouse.

For recombinant myofibers studies, myofibers were isolated from mdx/ hLTBP4 mice as described above. Myofibers were incubated in Ringer's media with or without 25µg/ml recombinant annexin A6 (5186-A6-050, R&D systems). FM 4-64 (2.5µm) was added to the myofibers just prior to imaging. Images were acquired and quantitated as described above. FM 4-64 area was measured using FIJI at imaging endpoint from a single slice near the middle of the z-stack. Z-stack step size (0.125µm) was acquired from cap end to end.

Myofiber quality control was based on a number of characteristics including using adherent myofibers with intact sarcomere structure detected through brightfield imaging. Myofibers appeared devoid of tears or ruptures induced during the isolation protocol. The region of the myofiber selected for damage was linear and not located on a nucleus or neuromuscular junction. Additionally, fluorescence intensity within both the red and green channels suggested similar expression levels prior to damage.

**Multiphoton laser injury and imaging.** Fibers were subjected to laser-induced damage at room temperature using the Nikon A1R-MP multiphoton microscope. Imaging was performed using a 25x1.1NA objective directed by the NIS-Elements AR imaging software. Green fluorescence protein (GFP) and FM 4-64 were excited using a 920nm wavelength laser and emission wavelengths of 575nm and 629nm were collected respectively. To induce laser damage on isolated myofibers, a diffraction limited spot (diameter approximately 410nm) was created on the lateral membrane of the myofiber using a 920nm wavelength laser at 10-15% laser power for 1 second. Time lapse images were collected as follows: one image was collected prior to damage, one image upon damage, then every 8 seconds for 80 seconds (10 images) followed by every 30 seconds for 5 minutes (10 images). At the end of the time lapsed image series, z-stack images were collected at 250 nm intervals through the damaged site on the myofiber directed by the NIS-Elements AR imaging software.

**Cardiotoxin Injury and analysis.** Tibialis anterior muscle of wildtype mice were injected with 25µg/ml recombinant annexin A6 (5186-A6-050, R&D systems) or Ringers in sedated mice (3% isoflurane, 0.8 l/min O₂). Additionally, mice were injected intraperitoneally with Evans' blue dye at 5µl/g body weight (E-2129; Sigma-Aldrich, St. Louis, MO) dissolved in phosphate-buffered saline at 10 mg/mL. For systemic administration, wildtype mice were injected with 1mg/kg recombinant annexin A6 (5186-A6-050, R&D systems) or PBS diluted in EBD (5µl/g body weight) into the retro-orbital cavity of sedated mice (3% isoflurane, 0.8 I/min O₂). Cardiotoxin injury was performed injecting 20µl of a 10µM cardiotoxin (discontinued, Sigma-Aldrich) solution in PBS in tibialis muscles in sedated animals (3% isoflurane, 0.8 l/min O₂) 2 hours post pretreatment. Cardiotoxin was released down the midline of the muscle to induce a homogenous area of injury at the center of the muscle. 3 hours post cardiotoxin injection muscle was harvested and frozen in liquid nitrogen.

**Chronic Injury and biomarker analysis.** For systemic administration, sgcg-null mice or wildtype mice were injected with 1mg/kg recombinant annexin A6 (5186-A6-050, R&D systems) or PBS into the retro-orbital cavity of sedated mice (3% isoflurane, 0.8 l/min O₂). Mice were injected once every 3 days for a period of 12 days. Serum was acquired as previously described (Demonbreun et al 2016a). Serum CK was analyzed for each mouse using the EnzyChrom Creatine Kinase Assay (catalog ECPK-100, BioAssay Systems) following the manufacturer's instructions. Lactate dehydrogenase was analyzed for each mouse using (LDH cytotoxicity kit MK401, Takara) following the manufacturer's instructions. Results were acquired with the Synergy HTX multi-mode plate reader (BioTeK).

**Immunofluorescence microscopy.** Sections (10µm thick) from the center of frozen-embedded muscles were collected on the cryostat (chamber, -20°C; sample, -15°C; catalog number CM1950; Leica, Wetzlar, Germany) for immunostaining. Tissues were fixed with 4% paraformaldehyde for 10 minutes on ice. Block and permeabilization were with 0.1% Triton (catalog number X-100; Sigma-Aldrich), 10% fetal bovine serum, and PBS for 60 minutes. For dystrophin detection, anti-dystrophin (ab15277; Abeam, Cambridge, MA) was used at a dilution of 1:100 overnight at 4°C. Sections were PBS rinsed, incubated with secondary antibody goat anti rabbit (A11008, Invitrogen) for 1 hour, PBS rinsed, and mounted in vectashield with DAPI (H-1200, Vector Laboratories). Imaging was performed using a Zeiss Axio Observer A1 microscope (Zeiss, Oberkochen, Germany), using a 10× objective. ZEN software (Zeiss, Jena, Germany) was used for acquiring images. Fluorescence quantitation and muscle area were performed using FIJI (NIH). Surface plots were created in FIJI (NIH). Fluorescence volume was quantified using Imaris Software v9.1.2.

**Calcium kinetics.** FDB muscle was electroporated and isolated as described above. Myofibers were damaged in Ringers solution with Ca²⁺ concentrations of 2mM, 1mM, 0.5mM, 0.25mM, 0.175mM, 0.1mM, 0.050mM, and 0mM. EDTA was added as a Ca²⁺ chelating agent in only in the 0mM Ca²⁺ Ringers. Myofibers were isolated directly into 2mM, 1mM and 0.5mM Ringers for those experiments respectively. For experiments using less than 0.5mM Ca²⁺ myofibers were isolated in 0.5mM Ca²⁺ Ringers and then diluted with 0mM EDTA-free Ca²⁺ Ringers. For 0mM experiments, myofibers were isolated in 0.5mM Ca²⁺Ringers and then replaced with 0mM Ca²⁺+EDTA Ringers just prior to imaging. Co-electroporation of wildtype annexin + wildtype annexin constructs was performed in one mouse foot, while the contralateral foot was co-electroporated with wildtype annexin + mutant annexin. All measurements were acquired from myofibers isolated from at least n≥2 mice, n≥3 myofibers per mouse at each Ca²⁺ concentration. Area-Ca²⁺ curves were fitted with a Hill Curve at Ca²⁺ concentrations ranging from 0-2mM. Kinetic parameters were calculated using Prism Graphpad.

**Single cell Ca²⁺ and shortening measurements.** Isolated FDB fibers were plated on laminin coated glass-bottomed 35 mm dishes for one hour and then cultured overnight in DMEM with 10% FBS and 1% penicillin/streptomycin at 37°C in a 10% CO₂ incubator. One hour prior to data acquisition, the medium was removed and cells were incubated in Tyrode buffer (119 mM NaCl, 5 mM KCI, 25 mM HEPES, 2 mM CaCl₂, 2 mM MgCl₂) with 10 µM Indo-1 AM (TefLabs) for 1 hour at 37°C in a 10% CO₂ incubator. Dishes were then filled with Tyrode buffer, mounted on a custom stage and platinum pacing electrodes were inserted into the dish. Stimulation was elicited using a 701C high-powered stimulator controlled by the 950A software (Aurora Scientific). Stimulation was performed at 40 and 80 Hz, 5 ms pulse width, 100 ms duration. Ratiometric Ca²⁺ signals were collected with two photomultiplier tubes and a FluoroDaq controller. Video sarcomere length was recorded with a high-speed camera and fast Fourier transform using the Aurora Scientific 900B-VSL system (Aurora, Ontario). Ten transients were collected over 20 seconds and averaged together per cell per frequency.

**Statistical analysis.** Statistical analyses were performed with Prism (Graphpad, La Jolla, CA). Comparisons relied on ANOVA ((1way ANOVA for 1 variable, 2way ANOVA for two variables (typically area and Ca²⁺ concentration)). Otherwise, unpaired t-tests were performed. Error bars represent +/- standard error of the mean (SEM).

### Example 2

### Ca²⁺ localizes to the repair cap upon membrane damage.

Activation of muscle membrane repair requires the presence of external Ca²⁺ (Bansal *et al.,* 2003). It was previously shown that annexin proteins aggregate into repair caps at the site of injury bordered by annexin-free zone within the cytoplasm under the repair cap (Demonbreun *et al.,* 2016a; Demonbreun *et al.,* 2016b; Quattrocelli *et al.,* 2017a; Swaggart *et al.,* 2014). To visualize Ca²⁺ dynamics at the site of injury in real-time, an *in vivo* fluorescent Ca²⁺ indicator protein, GCaMP5G, was utilized. GCaMP5G is a fusion protein composed of green fluorescent protein (GFP), the calcium-binding protein calmodulin, and the calmodulin M13 binding peptide. GCaMP5G has minimal fluorescence when not bound to Ca²⁺, and Ca²⁺ binding results in a conformational change within the protein increasing the fluorescence intensity of GFP (Akerboom *et al.,* 2012). Wildtype flexor digitorum brevis (FDB) muscle was electroporated with the GCaMP5G plasmid and then injured the plasma membrane using laser ablation (Demonbreun and McNally, 2015; Demonbreun *etal.,* 2016b). Within two seconds of membrane injury, GCaMP5G fluorescence accumulated in the cytoplasm at the site of injury. GCaMP5G fluorescence intensity progressively increased through 90 seconds of imaging (Figure 1A, arrow). In myofibers co-electroporated with plasmids expressing GCaMP5G and annexin A6 with a carboxyl-terminal tdTomato fluorescent tag, GCaMP5G fluorescence localized in a ring around the annexin A6-free zone (Figure 1B, arrowhead) and co-localized with annexin A6 at the repair cap (Figure 1B, arrow, merge). This temporal sequence is consistent with Ca²⁺ increasing at the site of injury likely facilitating annexin translocation and assembly into repair caps.

### Annexin repair caps exhibit differential Ca²⁺ sensitivity during repair cap recruitment.

Annexin proteins are Ca²⁺-dependent phospholipid and actin binding proteins that contain four annexin repeat domains or eight in the case of annexin A6 (Figure 2). Annexin repeat domains bind Ca²⁺, but are distinct from the Ca²⁺ binding of C2 domains and EF-hands seen in other classes of repair proteins (Gerke and Moss, 2002). Annexins coordinate Ca²⁺ and bind membranes from their convex face (Figure 2), and both type II and type III Ca²⁺ binding sites have been described in annexin proteins. To further define the Ca²⁺ requirements in annexin-mediated sarcolemmal repair in myofibers, annexins A1, A2, or A6 repair cap formation were examined over a range of Ca²⁺ concentrations. Cap size was measured from the center of a z-stack, and the type of fluorescent tag, turboGFP or tdTomato, did not alter assessed parameters (Figure 3A and 3B). Annexin A1 and A6 repair cap size was Ca²⁺-dependent, with the largest repair caps forming at 2mM and smaller repair caps forming at 0.1mM, while annexin A2 repair caps were not significantly reduced until 0.05mM Ca²⁺ (Figure 1C and 1D). Repair cap area was plotted as a function of Ca²⁺ concentration using a modified Hill equation. Annexin A2 formed a repair cap at the lowest concentration of Ca²⁺, 0.05mM, while annexins A1 and A6 did not form a discernable cap at Ca²⁺ concentrations lower than 0.1mM Ca²⁺, seen as the significant leftward annexin A2 curve with a K_{m1/2} of 0.067mM compared to A6 and A1, which showed K_{m1/2} of 0.12mM and 0.17mM, respectively (Figure 1D). Annexin A1 and A6 repair cap size and rate was highly dependent on Ca²⁺ concentration (Figure 4). The rate of annexin A2 cap formation and cap size was similar at 2 mM, 0.5 mM and 0.1mM Ca²⁺, while annexin A1 and A6 rates decreased with lower Ca²⁺ concentrations, suggesting a high Ca²⁺ affinity for annexin A2 (Figure 4). To ensure that repair cap formation was not artifact due to the type of laser injury, we induced laser injury on both the Nikon A1R GaSP confocal and the Nikon A1R MP+ multiphoton confocal. Injury induced by a multiphoton laser is more focused theoretically producing less collateral membrane damage. Annexin A6 repair caps appeared comparable with both types of lasers (Figure 5). These data indicate that annexin A1, A2, and A6 repair cap formation is influenced by the level of Ca²⁺ present during myofiber repair with annexin A2 being the most Ca²⁺ sensitive of the three annexins studied.

### Annexin overexpression promotes bleb formation at the site of membrane injury.

Membrane repair assays in *Lytechinus pictus* and *Xenopus* oocytes suggested that membranous structures merge and erupt at the site of membrane repair (Bi *et al.,* 1995; Davenport *et al.,* 2016). Additionally, extracellular recombinant annexins promoted membrane folding and bleb formation of artificial membrane patches at sites of membrane imperfection in a Ca²⁺ dependent manner (Boye *et al.,* 2018; Boye *et al.,* 2017). It was next investigated whether similar findings could be observed at the site of muscle membrane injury, which are sites of membrane imperfection, in live skeletal myofibers. GCaMP5G was expressed alone or in combination with annexin A1, A2 or A6 in skeletal myofibers. Overexpression of annexins was found to promote the formation of extracellular blebs emanating from annexin repair caps at the membrane lesion (Figure 6A). These blebs appeared after the formation of annexin repair caps and were seen at the extracellular tip of the repair cap (Figure 6A). Overexpression of annexin A6 and annexin A2 induced significantly more blebs than were observed after annexin A1 overexpression or GCaMP5G alone (Figure 6A and 6B). Furthermore, annexin-induced blebs contained significant GCaMP5G signal, and annexin A6 induced the formation of significantly larger GCaMP5G-containing blebs as compared to annexin A1, A2, or GCaMP5G alone (Figure 6A and 6C). These data indicated that annexins not only form a repair cap at the site of membrane disruption, but that these caps serve as sites for excretion of extracellular blebs enriched for Ca²⁺-binding proteins.

### Decreased intracellular Ca²⁺ fluorescence at the site of injury with annexin overexpression.

Time lapse imaging of the Ca²⁺ indicator GCaMP5G after laser injury suggested intracellular Ca²⁺ was decreasing concomitant with extracellular bleb formation suggesting that these blebs serve to reduce intracellular Ca²⁺ accumulation and/or excretion of cytoplasmic protein content emanating from the site of injury. The annexin-induced reduction in intracellular Ca²⁺ fluorescence was seen for all three annexins A1, A2 and A6, but was most evident for annexin A2 and A6 (Figure 7A). Over the 240 seconds of imaging, overexpression of annexin A6 induced the most significant reduction in intracellular Ca²⁺, visualized as internal GCaMP5G Ca²⁺ fluorescence (Figure7B). Detailed analysis of the first 20 seconds post injury showed a significant reduction in internal GCaMP5G Ca²⁺ fluorescence with annexin A2 and A6, but not annexin A1, when compared to GCaMP5G alone (Figure 7C). Baseline GCaMP5G fluorescence intensity prior to injury was not significantly different between groups (Figure 7D). Thus, annexin expression induces a reduction of Ca²⁺ signal within the injured myofiber concomitant with enhanced egress of Ca²⁺-binding protein-filled blebs. Moreover, annexin A6 was the most effective of the three annexins tested at sustaining this response.

Next, the Ca²⁺ handling and contractile properties of isolated myofibers overexpressing annexin A6 compared to controls was evaluated. Isolated myofibers expressing annexin A6 were loaded with the ratiometric Ca²⁺ indicator dye Indo-1, and no differences in Ca²⁺ cycling at 40 or 80 Hz stimulation frequencies between annexin A6 or control fibers (Figure 8A, 8B, and 8C) were observed. Unloaded cell shortening was also unaffected by the presence of overexpressed annexin A6 (Figure 8D, 8E, and 8F). These results indicated that annexin A6 overexpression was well-tolerated by myofibers.

### Annexin A6 Ca²⁺ binding is required for repair cap formation and myofiber repair.

Mutation of annexin A1D171 and A2D161 were previously shown to inhibit annexin membrane translocation in HEK cells (Jost *et al.,* 1992; McNeil *et al.,* 2006). It was queried whether these mutations would inhibit translocation and formation of the macromolecular annexin repair cap formed after muscle membrane injury. Alignment of annexins A1, A2 and A6 protein sequences identified conserved residues within the consensus sequence of type II Ca²⁺ binding sites across all three annexin proteins (Figure 2). In order to disrupt Ca²⁺ binding in annexin A1, A2, and A6, site-directed mutagenesis was performed to convert the aspartic acid residue in the first type II Ca²⁺ binding site into an alanine residue (A1D171A, A2D161A, A6D149A, respectively) (Figure 9A). E233A was also generated in annexin A6 to create a similar change in the Ca²⁺ binding site in the second annexin repeat domain of annexin A6. Each construct also contained turboGFP or tdTomato at the C-terminus. To assess the effect of homotypic annexin interactions during repair cap formation, myofibers were co-electroporated with wildtype+wildtype (A6+A6) or wildtype+mutant (A6+A6E233A) annexin combinations. Mutation of E233 in annexin A6 acted in a dominant-negative fashion, significantly decreasing cap size of the co-expressed wildtype annexin A6 protein (Figure 10A). Prior structural studies suggested that D149 in the first annexin repeat domain of annexin A6 does not bind Ca²⁺ (Avila-Sakar *et al.,* 1998), and consistent with this, the D149A mutant in annexin A6 had little effect on cap size (Figure 9B, right panel). The repair cap feret diameter was plotted as a function of Ca²⁺ concentration using a modified Hill equation. Expression of mutant annexin A6E233A was sufficient to significantly reduce the cap diameter (DMAX) of the co-expressed wildtype annexin A6 protein (Figure 10B). To assess the effect of heterotypic annexin interactions on repair cap formation, myofibers were co-electroporated with various combinations of wildtype and mutant annexin constructs. Co-expression of mutant annexin A6E233A resulted in a significant reduction in annexin A1, A2, and A6 cap size compared to A1+A6, A2+A6, A6+A6 controls, respectively (Figure 10C). Together, these data showed that annexin proteins interact in a homotypic and heterotypic fashion influencing annexin repair complex-assembly and that the mutant annexin A6 protein is sufficient to negatively modulate annexin complex assembly during repair.

Ca²⁺-binding of both annexin A1 and A2 was also required for repair cap formation. A1D171A and A2D161A mutant cap size was reduced compared to wildtype annexin A1 and A2 controls, respectively. Expression of mutant annexin A1D171A and A2D161A was sufficient to significantly reduce the repair cap diameter (DMAX) of the respective co-expressed wildtype annexin protein (Figure 9B, left and middle panels). Despite the ability of mutant annexin A1 D171A and A2D161A to significantly decrease co-expressed wildtype annexin A1 and A2 cap size, respectively, A1D171A or A2D171A had minimal effect of wildtype annexin A6 cap size (Figure 9C). These data showed that annexin A1 and A2 interact in a homotypic fashion influencing self-cap assembly, while A6 localization to the repair cap is minimally modulated by annexin A1 and A2 localization.

To determine the effect of dominant negative annexin A6 on the assembly of annexins A1, A2, and A6 at the repair cap and membrane repair capacity, laser injury was similarly performed on isolated myofibers in the presence of FM 4-64. FM 4-64 is a membrane impermeable dye that is non-fluorescent in aqueous solution and increases fluorescence intensity as it binds membrane phospholipids exposed during injury, and is commonly used as a marker of membrane injury (Bansal *et al.,* 2003; Cai *et al.,* 2009; Demonbreun and McNally, 2015; Yeung *et al.,* 2009; Zweifach, 2000). Myofibers expressing annexin A6E233A-GFP had increased FM 4-64 fluorescence area after laser injury compared to control myofibers expressing wildtype annexin A6-GFP (Figure 10D). These results indicated that a functional annexin repair complex is required for proper membrane repair and annexin A6 participates in orchestrating complex formation.

### Annexin A6 protected against laser-induced myofiber injury in vitro.

Since annexin A6 facilitates the formation of the macromolecular repair cap complex and was the most efficient at forming large, Ca²⁺-filled blebs at the site of membrane injury, whether overexpression of annexin A6 would reduce membrane injury in wildtype myofibers was assessed. Wildtype myofibers were electroporated with annexin A6-GFP or mock electroporated and then laser damaged in the presence of FM 4-64, to mark the injury area. Wildtype myofibers overexpressing annexin A6 had decreased FM 4-64 dye uptake after laser-induced membrane injury compared to control myofibers (Figure 11A). These results suggested that overexpression of annexin A6 is effective at improving membrane repair in isolated myofibers.

Next, it was tested whether extracellular recombinant annexin A6 could also protect against membrane injury in wildtype myofibers. Wildtype myofibers were isolated and incubated with recombinant annexin A6 (rANXA6) or vehicle control. Laser injury was conducted in the presence of FM 4-64. Pretreatment with extracellular recombinant annexin A6 reduced FM 4-64 fluorescence area compared to vehicle control treated myofibers (Figure 11B). These data demonstrated that recombinant annexin A6 protects against membrane injury through extracellular exposure.

### Recombinant annexin A6 protected against myofiber injury in a model of chronic muscle disease.

Muscular dystrophy is a progressive muscle wasting disease, due to loss-of-function mutations in critical cytoskeletal or membrane associated proteins, that results in fragile membranes. *mdx* mice lack the integral membrane protein dystrophin and are a model of Duchenne muscular dystrophy (DMD) (Hoffman *et al.,* 1987; Petrof *et al.,* 1993). *mdx* mice expressing human Latent TGFβ binding protein 4 (mdx/hLTBP4) have a more severe form of muscular dystrophy, similar to what is seen in humans with DMD (Ceco *et al.,* 2014; Flanigan *et al.,* 2013; Quattrocelli *et al.,* 2017b). Since recombinant annexin A6 protected against laser-induced membrane injury in wildtype muscle, it was next assessed whether exposure to recombinant annexin A6 would protect against membrane injury in the context of chronic muscle disease. anti-HIS (green) immunostaining revealed the presence of recombinant annexin A6-HIS at the sarcolemma of *mdx*/hLTBP4 dystrophic muscle after systemic injection, while vehicle control muscle lacked anti-HIS staining (Figure 11C). Similar to isolated wildtype myofibers, pretreatment with extracellular recombinant annexin A6 reduced FM 4-64 fluorescence after laser-induced injury in mdx/hLTBP4 myofibers as compared to vehicle control treated myofibers (Figure 11D). These combined data indicated that extracellular annexin A6 targets injured membrane, promotes more efficient repair and protects against injury of healthy and dystrophic myofibers.

### Recombinant annexin A6 protected against myofiber injury in vivo.

It was next assessed whether recombinant annexin A6 could protect against muscle injury in vivo. Recombinant annexin A6 or vehicle control was injected intramuscularly into the tibialis anterior (TA) muscles of wildtype mice. Mice were also injected intraperitoneally with Evan's blue dye, a vital tracer that is excluded by intact healthy myofibers but is readily taken up in injured permeable myofibers (Jennische and Hansson, 1986). Two hours post injection of recombinant annexin A6, the TA muscle was injured with cardiotoxin. Muscle was harvested 3 hours post-cardiotoxin injury and evaluated for Evan's blue dye uptake (Figure 12A). Gross imaging showed that pretreatment with recombinant annexin A6 reduced cardiotoxin-induced muscle damage *in vivo,* as seen by reduced dye uptake compared to controls (Figure 12B). Fluorescence imaging showed a 50% decrease in dye uptake with recombinant annexin A6 pretreatment compared to control muscle (Figure 12C and 12D). Surface plot profiles illustrate reduced dye fluorescence in tibialis anterior muscle pretreated with intramuscular recombinant annexin A6 (Figure 12C). These results indicated that intramuscular recombinant annexin A6 can reduce membrane injury and promote membrane repair *in vivo.*

Although intramuscular injection of annexin A6 was effective at reducing injury, this route of application is not optimal for large muscle groups, internal tissues, or treatment of chronic diseases. Therefore, the efficacy of recombinant annexin A6 administered via systemic retro-orbital (RO) injection was examined. Recombinant annexin A6 or control solution was injected 2 hours prior to cardiotoxin-induced tibialis anterior muscle injury (pretreatment). Alternatively, recombinant annexin A6 was administered immediately after tibialis anterior (TA) muscles were injured with cardiotoxin (post-treatment). Additionally, Evan's blue dye, was injected prior to injury. Muscle was harvested 3 hours post cardiotoxin injury and evaluated for dye uptake (Figure 13A). Fluorescence imaging showed a significant decrease in dye uptake with recombinant annexin A6 pretreatment and post-treatment compared to vehicle control (Figure 13B and 13C). Surface plot profiles illustrate reduced dye fluorescence in tibialis anterior muscle pretreated and post-treated with systemic recombinant annexin A6 (Figure 13C). These results demonstrated that recombinant annexin A6 reduces membrane injury and promotes membrane repair through intravenous systemic administration *in vivo* administered both before and after muscle injury.

### Recombinant annexin A6 protected against chronic muscle injury in vivo.

Since recombinant annexin A6 protected against acute membrane injury in wildtype muscle in vivo, it was next assessed whether exposure to recombinant annexin A6 would protect against membrane injury in the context of chronic muscle disease. Mutations in the gene γ-sarcoglycan (SGCG) cause Limb-Girdle muscular dystrophy 2C (LGMD2C) in both mice and humans. To determine the if recombinant annexin A6 could facilitate repair in a chronic injury setting, sgcg-null mice were injected once every 3 days for 12 days with recombinant protein or control solution and then serum biomarkers of muscle damage, creatine kinase (CK) and lactate dehydrogenase (LDH), were quantified (Figure 14A). Systemic treatment with recombinant annexin A6 reduced both serum CK and LDH levels in sgcg-null mice (Figure 14B and 14C). These results demonstrated that systemic recombinant annexin A6 promotes membrane repair in a model of chronic muscle disease.

### Discussion

**Annexins promote bleb formation at the site of membrane injury.** It was found that increased expression of annexins in muscle fibers decreased injury-associated Ca²⁺ fluorescence accumulation within myofibers. This reduction correlated with extracellular bleb formation arising at the site of annexin repair caps. We found that annexin A2 and A6 induced the formation of membranous blebs containing the Ca²⁺-binding protein GCaMP5G emanating from the repair cap. Furthermore, overexpression of annexins A1, A2, and A6 each reduced endpoint Ca²⁺ fluorescence accumulation within the myofiber after injury. Annexin A6 overexpression resulted in the most sustained effect on reducing injury-associated Ca²⁺ accumulation inducing the formation of large GCaMP5G-containing blebs. A model is contemplated in which annexin A6 facilitates Ca²⁺ and protein excretion into blebs whose formation was further induced by annexin A1 and annexin A2. In artificial membrane patches, the presence of annexin A1 or annexin A2 induced bleb formation at sites of membrane imperfection (Boye *et al.,* 2018). In contrast, the presence of annexin A6 induced contraction of artificial membrane into large folds, in a Ca²⁺-dependent manner (Boye *et al.,* 2018). The difference between annexin A6 inducing blebs in live myofibers or folds in artificial membrane is likely do to the presence of endogenously expressed annexin A1 and A2 in isolated myofibers compared with exposure to single recombinant annexin protein in the artificial membrane studies. Without being bound by theory, it is hypothesized that within the macromolecular repair complex, annexins actively participate in bleb formation which acts to remove large membrane lesions facilitating wound closure, excision of damaged membrane, and reduction of Ca²⁺ at the injury site.

Similar to the data described herein, others have shown that in damaged *Xenopus* oocytes, GCaMP5G fluorescence quickly localized in a ring around the site of membrane disruption, fading by approximately 5 minutes post injury, as healing progressed (Davenport *et al.,* 2016). Overexpression of annexin A1-GFP in injured *Xenopus* oocytes resulted in annexin A1 positive blebs originating from the site of damage (Davenport *et al.,* 2016). However, the effect of annexin overexpression on GCaMP5G fluorescence was not assessed. These data combined suggest that bleb formation, as a mechanism of membrane repair, is conserved across species and tissue types, and is facilitated by the presence of annexin proteins.

**Annexin A6 protects against muscle membrane injury and enhances membrane repair.** As shown herein, annexin proteins, including annexin A1, A2, and A6, localize to the site of membrane injury facilitating membrane repair cap and bleb formation. Mutation of annexin A6 abrogated repair cap formation, decreasing repair capacity, resulting in increased dye uptake. On the other hand, pretreatment with recombinant annexin A6 reduced dye uptake after laser-induced muscle injury and after toxin-induced muscle injury *in vivo.* This data, however, does not distinguish between annexin A6 enhancing membrane repair, reducing membrane injury, or a combination of both mechanisms. As a therapeutic tool, enhancing the cells' ability to repair and/or reduce injury through stabilizing the cell membrane are both beneficial avenues that can lead to improved cell survival. Previous studies have shown that annexin A6 is upregulated in muscle from models of chronic muscular dystrophy (Demonbreun *et al.,* 2016a; Demonbreun *et al.,* 2014; Swaggart *et al.,* 2014). Additional proteomic approaches in the *mdx* mouse model have shown that annexin A1, A2, and A6 are enriched in mdx muscle membrane, again, suggesting a role for annexins at the membrane of injured cells (Murphy *et al.,* 2018). Annexins bind membrane phospholipids, including phosphatidylserine, which is exposed during membrane disruption. Phosphatidylserine rearrangement upon injury provides a likely binding target for extracellular annexins to facilitate membrane folding, blebbing, and rolling at sites of membrane damage and imperfection (Boye *et al.,* 2018). Based on the data herein, upregulation of annexin A6 is a compensatory mechanism to facilitate excision of defective membrane in fibers undergoing chronic damage. It is further contemplated that recombinant annexin A6 can facilitate membrane repair and reduce the susceptibility to injury long-term in chronic models of disease and in tissues beyond skeletal muscle.

**Combinatorial approaches to improve membrane repair.** As shown herein, recombinant annexin A6 protected normal and dystrophic muscle from laser-induced membrane injury. In addition, both intramuscular and systemic administration of recombinant annexin A6 protected against toxin-induced muscle membrane injury *in vivo.* Intriguingly, glucocorticoid administration increased annexin expression in muscle and this correlated with enhanced muscle repair in multiple mouse models of muscular dystrophy including *mdx* (DMD), dysferlin-null (LGMD2B), and γ-sarcoglycan-null (LGMD2C) mice (Quattrocelli *et al.,* 2017a; Quattrocelli *et al.,* 2017c). Importantly, glucocorticoid treatment also increased the expression of mitsugumin 53 (also known as MG53 or Trim 72), a repair protein that localizes to the site of membrane injury and is considered a "molecular band-aid" improving cellular wound healing. Similar to the annexins, MG53 is upregulated in chronic muscle injury and enhances repair in dystrophic muscles, as well as other tissues like heart, lung, kidney (Waddell *et al.,* 2011). (Duann *et al.,* 2015; He *et al.,* 2012; Jia *et al.,* 2014; Liu *et al.,* 2015; Weisleder *et al.,* 2012). MG53 is a component of the annexin-mediated repair complex, localizing juxtaposed to the annexin repair cap (Demonbreun *et al.,* 2016b). It is contemplated that co-administration of recombinant annexin A6 with glucocorticoids and/or MG53 will further strengthen clinical relevance of these therapeutics for conditions resulting from membrane lesions.

In summary, the data provided herein demonstrate that annexins promote the formation of blebs released at the site of muscle membrane insult, with annexin A6 being the most effective at facilitating this process. Furthermore, recombinant annexin A6 protects against membrane injury of normal and dystrophic muscle. These data identify annexin A6 as a therapeutic target that enhances membrane repair capacity in healthy and diseased muscle.

### Example 3

This example details the results of additional and updated experiments that were performed.

Membrane repair is essential to cell survival. In skeletal muscle, injury often associates with plasma membrane disruption. Additionally, muscular dystrophy is linked to mutations in genes that produce fragile membranes or reduce membrane repair. Methods to enhance repair and reduce susceptibility to injury could benefit muscle in both acute and chronic injury settings. Annexins are a family of membrane-associated Ca²⁺-binding proteins implicated in repair, and annexin A6 was previously identified as a genetic modifier of muscle injury and disease. Annexin A6 forms the repair cap over the site of membrane disruption. To elucidate how annexins facilitate repair, annexin cap formation was visualized during injury. Annexin cap size was found to be positively correlated with increasing Ca²⁺ concentrations. It was also found that annexin overexpression promoted external blebs enriched in Ca²⁺ fluorescence and correlated with a reduction of intracellular Ca²⁺ fluorescence at the injury site. Annexin A6 overexpression reduced membrane injury, consistent with enhanced repair. Treatment with recombinant annexin A6 protected against acute muscle injury *in vitro* and *in vivo.* Moreover, administration of recombinant annexin A6 in a model of muscular dystrophy reduced serum creatinine kinase, a biomarker of disease. These data identified annexins as mediators of membrane-associated Ca²⁺ release during membrane repair and annexin A6 as a therapeutic target to enhance membrane repair capacity.

Annexin A6 forms a repair cap at the site of muscle membrane disruption. The data herein demonstrated that adding exogenous recombinant annexin A6 (rANXA6) promotes membrane resealing and recovery from injury.

### Introduction

Plasma membrane repair occurs after membrane disruption and is a highly conserved process. The active process required for resealing membrane disruptions is thought to rely on Ca²⁺-dependent vesicle fusion and local cytoskeletal remodeling (McNeil PL, and Khakee R. Disruptions of muscle fiber plasma membranes. Role in exercise-induced damage. Am J Pathol. 1992;140(5):1097-109; McNeil PL, and Kirchhausen T. An emergency response team for membrane repair. Nat Rev Mol Cell Biol. 2005;6(6):499-505). Other models suggest that membrane repair is mediated through the fusion of lysosomal vesicles, lateral diffusion of membrane to the site of injury, and the extrusion of membranous blebs (Rodriguez A, Webster P, Ortego J, and Andrews NW. Lysosomes behave as Ca2+-regulated exocytic vesicles in fibroblasts and epithelial cells. J Cell Biol. 1997;137(1):93-104; Reddy A, Caler EV, and Andrews NW. Plasma membrane repair is mediated by Ca(2+)-regulated exocytosis of lysosomes. Cell. 2001;106(2):157-69; Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18; McDade JR, Archambeau A, and Michele DE. Rapid actin-cytoskeleton-dependent recruitment of plasma membrane-derived dysferlin at wounds is critical for muscle membrane repair. FASEB J. 2014;28(8):3660-70; Babiychuk EB, Monastyrskaya K, Potez S, and Draeger A. Blebbing confers resistance against cell lysis. Cell Death Differ. 2011;18(1):80-9). These models are not mutually exclusive and may depend on the type and extent of damage. Skeletal muscle is highly dependent on plasma membrane repair as mutation in genes encoding repair proteins lead to muscle disease (Bansal D, Miyake K, Vogel SS, Groh S, Chen CC, Williamson R, et al. Defective membrane repair in dysferlin-deficient muscular dystrophy. Nature. 2003;423(6936):168-72; Bashir R, Britton S, Strachan T, Keers S, Vafiadaki E, Lako M, et al. A gene related to Caenorhabditis elegans spermatogenesis factor fer-1 is mutated in limb-girdle muscular dystrophy type 2B. Nat Genet. 1998;20(1):37-42; Demonbreun AR, Swanson KE, Rossi AE, Deveaux HK, Earley JU, Allen MV, et al. Eps 15 Homology Domain (EHD)-1 Remodels Transverse Tubules in Skeletal Muscle. PLoS One. 2015;10(9):e0136679; Demonbreun AR, and McNally EM. Plasma Membrane Repair in Health and Disease. Curr Top Membr. 2016;77:67-96; Defour A, Medikayala S, Van der Meulen JH, Hogarth MW, Holdreith N, Malatras A, et al. Annexin A2 links poor myofiber repair with inflammation and adipogenic replacement of the injured muscle. Human molecular genetics. 2017;26(11):1979-91; Cai C, Masumiya H, Weisleder N, Matsuda N, Nishi M, Hwang M, et al. MG53 nucleates assembly of cell membrane repair machinery. Nat Cell Biol. 2009;11(1):56-64).

The annexins are a family of Ca²⁺-binding proteins that regulate lipid binding, cytoskeletal reorganization, and membrane folding, steps necessary for membrane repair (Jimenez AJ, and Perez F. Plasma membrane repair: the adaptable cell life-insurance. Curr Opin Cell Biol. 2017;47:99-107; Lauritzen SP, Boye TL, and Nylandsted J. Annexins are instrumental for efficient plasma membrane repair in cancer cells. Semin Cell Dev Biol. 2015;45:32-8; Bizzarro V, Petrella A, and Parente L. Annexin A1: novel roles in skeletal muscle biology. J Cell Physiol. 2012;227(8):3007-15; Grewal T, Hoque M, Conway JRW, Reverter M, Wahba M, Beevi SS, et al. Annexin A6-A multifunctional scaffold in cell motility. Cell Adh Migr. 2017;11(3):288-304; Boye TL, Jeppesen JC, Maeda K, Pezeshkian W, Solovyeva V, Nylandsted J, et al. Annexins induce curvature on free-edge membranes displaying distinct morphologies. Sci Rep. 2018;8(1):10309; Boye TL, Maeda K, Pezeshkian W, Sonder SL, Haeger SC, Gerke V, et al. Annexin A4 and A6 induce membrane curvature and constriction during cell membrane repair. Nat Commun. 2017;8(1):1623). Individual annexin repeat domains coordinate Ca²⁺ binding with unique annexin-specific type II or type III binding sites. Differential Ca²⁺ affinity of the type II and type III binding sites provides each annexin a unique ability to respond to a range of intracellular Ca²⁺ levels and phospholipid binding (Blackwood RA, and Ernst JD. Characterization of Ca2(+)-dependent phospholipid binding, vesicle aggregation and membrane fusion by annexins. The Biochemical journal. 1990;266(1):195-200). Annexins have the ability to self- and hetero-oligomerize (Zaks WJ, and Creutz CE. Ca(2+)-dependent annexin self-association on membrane surfaces. Biochemistry. 1991;30(40):9607-15). Typical annexins like A1 and A2 contain one annexin core composed of four annexin repeat domains. In contrast, annexin A6 contains two annexin cores and thus eight annexin repeat domains (Benz J, Bergner A, Hofmann A, Demange P, Gottig P, Liemann S, et al. The structure of recombinant human annexin VI in crystals and membrane-bound. J Mol Biol. 1996;260(5):638-43). Annexin A6's duplicated structure makes it possible for the amino- and carboxyl-terminal annexin core domains to bind one or two distinct membranes making annexin A6 a prime target for facilitating membrane coalescence and folding required during membrane repair (Boye TL, Jeppesen JC, Maeda K, Pezeshkian W, Solovyeva V, Nylandsted J, et al. Annexins induce curvature on free-edge membranes displaying distinct morphologies. Sci Rep. 2018;8(1):10309; Boye TL, Maeda K, Pezeshkian W, Sonder SL, Haeger SC, Gerke V, et al. Annexin A4 and A6 induce membrane curvature and constriction during cell membrane repair. Nat Commun. 2017;8(1):1623; Buzhynskyy N, Golczak M, Lai-Kee-Him J, Lambert O, Tessier B, Gounou C, et al. Annexin-A6 presents two modes of association with phospholipid membranes. A combined QCM-D, AFM and cryo-TEM study. Journal of structural biology. 2009;168(1):107-16).

Annexins have a high affinity for phosphatidylserine, phosphatidylinositol, and cholesterol, which are highly enriched in the sarcolemma (Gerke V, Creutz CE, and Moss SE. Annexins: linking Ca2+ signalling to membrane dynamics. Nat Rev Mol Cell Biol. 2005;6(6):449-61; Fiehn W, Peter JB, Mead JF, and Gan-Elepano M. Lipids and fatty acids of sarcolemma, sarcoplasmic reticulum, and mitochondria from rat skeletal muscle. The Journal of biological chemistry. 1971;246(18):5617-20). Multiple annexins, including annexins A1, A2, and A6, have been implicated in membrane repair in skeletal muscle, as well as Xenopus oocytes, human trophoblasts, and HeLa cancer cells, suggesting a conserved mechanism (Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18; Babbin BA, Laukoetter MG, Nava P, Koch S, Lee WY, Capaldo CT, et al. Annexin A1 regulates intestinal mucosal injury, inflammation, and repair. J Immunol. 2008;181(7):5035-44; Lennon NJ, Kho A, Bacskai BJ, Perlmutter SL, Hyman BT, and Brown RH, Jr. Dysferlin interacts with annexins A1 and A2 and mediates sarcolemmal wound-healing. The Journal of biological chemistry. 2003;278(50):50466-73; McNeil AK, Rescher U, Gerke V, and McNeil PL. Requirement for annexin A1 in plasma membrane repair. The Journal of biological chemistry. 2006;281(46):35202-7; Roostalu U, and Strahle U. In vivo imaging of molecular interactions at damaged sarcolemma. Dev Cell. 2012;22(3):515-29; Davenport NR, Sonnemann KJ, Eliceiri KW, and Bement WM. Membrane dynamics during cellular wound repair. Mol Biol Cell. 2016;27(14):2272-85; Carmeille R, Degrelle SA, Plawinski L, Bouvet F, Gounou C, Evain-Brion D, et al. Annexin-A5 promotes membrane resealing in human trophoblasts. Biochimica et biophysica acta. 2015;1853(9):2033-44; Bement WM, Mandate CA, and Kirsch MN. Wound-induced assembly and closure of an actomyosin purse string in Xenopus oocytes. Curr Biol. 1999;9(11):579-87). Annexins are recruited to the injured membrane in a sequential manner forming a macromolecular repair complex at the membrane lesion referred to as a repair cap (Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18; Boye TL, Maeda K, Pezeshkian W, Sonder SL, Haeger SC, Gerke V, et al. Annexin A4 and A6 induce membrane curvature and constriction during cell membrane repair. Nat Commun. 2017;8(1):1623; Roostalu U, and Strahle U. In vivo imaging of molecular interactions at damaged sarcolemma. Dev Cell. 2012;22(3):515-29). A polymorphism in Anxa6, the gene encoding annexin A6, was previously identified in several commonly used experimental mouse strains that correlated with impaired muscle repair (Demonbreun AR, Allen MV, Warner JL, Barefield DY, Krishnan S, Swanson KE, et al. Enhanced Muscular Dystrophy from Loss of Dysferlin Is Accompanied by Impaired Annexin A6 Translocation after Sarcolemmal Disruption. Am J Pathol. 2016;186(6):1610-22; Quattrocelli M, Capote J, Ohiri JC, Warner JL, Vo AH, Earley JU, et al. Genetic modifiers of muscular dystrophy act on sarcolemmal resealing and recovery from injury. PLoS Genet. 2017;13(10):e1007070; Swaggart KA, Demonbreun AR, Vo AH, Swanson KE, Kim EY, Fahrenbach JP, et al. Annexin A6 modifies muscular dystrophy by mediating sarcolemmal repair. Proceedings of the National Academy of Sciences of the United States of America. 2014;111(16):6004-9). This polymorphism produces a truncated annexin A6 protein that acts in a dominant-negative manner to reduce repair cap formation and interferes with sarcolemmal repair. Additional studies in mice have shown that loss of annexin A2 results in poor myofiber repair (Defour A, Medikayala S, Van der Meulen JH, Hogarth MW, Holdreith N, Malatras A, et al. Annexin A2 links poor myofiber repair with inflammation and adipogenic replacement of the injured muscle. Human molecular genetics. 2017;26(11):1979-91). These data suggest that there is a coordinated recruitment of annexin proteins to the repair cap facilitated by dynamic protein-protein interactions.

Herein, the kinetics of annexin A1, A2, and A6 were assessed in repair cap formation after membrane injury at multiple Ca²⁺ concentrations. The repair cap formed by annexins A1, A2, and A6 increased with increasing Ca²⁺ concentrations, while mutations in Ca²⁺-coordinating residues interfered with normal annexin repair cap formation. Annexin overexpression promoted the formation of external blebs at the site of membrane injury that were released from the repair cap. Overexpression of annexin A6 resulted in the formation of larger blebs being released from the repair cap. These vesicles were enriched in Ca²⁺-binding marker protein GCaMP5G, and this enrichment of Ca²⁺ correlated with a reduction of intracellular Ca²⁺ fluorescence near the injury site. Annexin A6 overexpression promoted membrane repair, while mutation of residue E233, a critical Ca²⁺-coordinating residue in annexin A6, interfered with annexin repair complex formation and decreased repair capacity. Local and systemic administration of recombinant annexin A6 reduced muscle damage *in vivo.* These data identified a new role for annexins in bleb release from muscle membrane lesions during membrane repair and identify annexin A6 as a therapeutic target to protect against muscle injury.

### Methods

**Animals.** Wildtype mice from the 129T2/SvEmsJ background were bred and housed in a specific pathogen free facility on a 12-hour light/dark cycle and fed ad libitum in accordance with the Northwestern University's Institutional Animal Care and Use Committee regulations. 129T2/SvEmsJ (129T2) mice were purchased from the Jackson Laboratory (Ben Harbor, ME; Stock # 002065). mdx/hLTBP4 mice were generated as described in (Quattrocelli M, Capote J, Ohiri JC, Warner JL, Vo AH, Earley JU, et al. Genetic modifiers of muscular dystrophy act on sarcolemmal resealing and recovery from injury. PLoS Genet. 2017;13(10):e1007070; Ceco E, Bogdanovich S, Gardner B, Miller T, DeJesus A, Earley JU, et al. Targeting latent TGFbeta release in muscular dystrophy. Science translational medicine. 2014;6(259):259ra144). *Sgcg-null* mice were generated as described in (Hack AA, Cordier L, Shoturma DI, Lam MY, Sweeney HL, and McNally EM. Muscle degeneration without mechanical injury in sarcoglycan deficiency. Proceedings of the National Academy of Sciences of the United States of America. 1999;96(19):10723-8). Two to three-month-old male and female were used for all wildtype mouse experiments. *Sgcg-null*cohorts were age and sex-matched with mice between 2-5 months old.

**Plasmids.** Plasmids encoding annexin A1, A2 and A6 with a carboxyl-terminal turboGFP tag were obtained from Origene (Rockville, MD). Subcloning of annexin A1, A2, and A6 to replace the GFP tag with tdTomato (Addgene) was performed by Mutagenix (Suwanee, GA). Site directed mutagenesis was performed by Mutagenix on annexin A1-GFP, A2-GFP and A6-GFP to create the Ca²⁺-binding mutants A1-D171A-GFP, A2-D161A-GFP, A6-D149A-GFP, and A6-E233A-GFP. Constructs were sequenced to verify mutagenesis. Plasmid DNA was isolated using the Qiagen endo-free Maxi prep kit (Qiagen #12362). The Ca²⁺ sensor GCaMP5G was purchased from (Addgene #31788).

**Sequence comparison and protein schematics** Protein ribbon diagrams were generated using Swiss-PdbViewer using solved crystal structures of annexin A1 (1MCX), annexin A2 (2HYW), and annexin A6 (1AVC) available on www.rcsb.org. Clustal Omega from the European Bioinformatics Institute (EMBL-EBI) was used to align annexin sequences from www.ncbi.nlm.nih.gov annexin A1 (NM_010730; SEQ ID NO: 37), annexin A2 (NM_007585; SEQ ID NO: 38), annexin A6 (NM_013472; SEQ ID NO: 39), and annexin A6-encoding sequencing from multiple species (homo sapiens (AAH17046.1; SEQ ID NO: 40, macaca (AFE65315.1; SEQ ID NO: 41), canis (XP_005619331.1; SEQ ID NO: 42), rattus (NP_077070.2; SEQ ID NO: 43) and mus (NP_038500.2; SEQ ID NO: 44).

**Electroporation, myofiber isolation, laser injury, cap and vesicle measurement.** Flexor digitorum brevis (FDB) fibers were transfected with endo-free plasmid DNA by in vivo electroporation. Methods were described previously in (Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18; Demonbreun AR, and McNally EM. DNA Electroporation, Isolation and Imaging of Myofibers. Journal of visualized experiments : JoVE. 2015;106(106):e53551; DiFranco M, Quinonez M, Capote J, and Vergara J. DNA transfection of mammalian skeletal muscles using in vivo electroporation. Journal of visualized experiments : JoVE. 2009;32(32)). Z-stack projections were acquired from consecutive acquisitions after the final time-lapse frame, approximately 4 minutes post damage, with a 0.125µM step size between slices. Z-stack renderings were constructed in FIJI. Measurement of the cap area and feret diameter were conducted from a single slice near the middle of the z-stack using FIJI imaging tools. Fibers expressing similar levels of tagged or GCaMP5G protein were compared. GCaMP5G Ca²⁺ fluorescence was measured from the acquired timelapse images, using a standard rectangular ROI, placed inside the myofiber below the site of damage using FIJI. Fluorescence was expressed as F/F0. External vesicle number and GCaMP5G area were measured from endpoint z-stacks and max projection images using FIJI. Vesicles were considered external if they were found outside the sarcolemma assessed in brightfield and fluorescent channels. All measurements were acquired from myofibers isolated from at least n≥3 mice, n≥3 myofibers per mouse.

For recombinant myofibers studies, myofibers were isolated from mdx/ hLTBP4 mice as described above. Myofibers were incubated in Ringer's media with or without 25µg/ml recombinant annexin A6 (5186-A6-050, R&D systems). FM 4-64 (2.5µm) was added to the myofibers just prior to imaging. Images were acquired and quantitated as described above. FM 4-64 area was measured using FIJI at imaging endpoint from a single slice near the middle of the z-stack. Z-stack step size (0.125µm) was acquired from cap end to end.

Myofiber quality control was based on a number of characteristics including using adherent myofibers with intact sarcomere structure detected through brightfield imaging. Myofibers appeared devoid of tears or ruptures induced during the isolation protocol. The region of the myofiber selected for damage was linear and not located on a nucleus or neuromuscular junction. Additionally, fluorescence intensity within both the red and green channels suggested similar expression levels prior to damage.

**Multiphoton laser injury and imaging.** Fibers were subjected to laser-induced damage at room temperature using the Nikon A1R-MP multiphoton microscope. Imaging was performed using a 25x1.1NA objective directed by the NIS-Elements AR imaging software. Green fluorescence protein (GFP) and FM 4-64 were excited using a 920nm wavelength laser and emission wavelengths of 575nm and 629nm were collected respectively. To induce laser damage on isolated myofibers, a diffraction limited spot (diameter approximately 410nm) was created on the lateral membrane of the myofiber using a 920nm wavelength laser at 10-15% laser power for 1s. Time lapse images were collected as follows: one image was collected prior to damage, one image upon damage, then every 8 seconds for 80 seconds (10 images) followed by every 30 seconds for 5 minutes (10 images). At the end of the time lapsed image series, z-stack images were collected at 250 nm intervals through the damaged site on the myofiber directed by the NIS-Elements AR imaging software. The multiphoton was used to acquire data presented in Figure 5, Figure 10D and Figure 19.

For recombinant protein calcium studies, myofibers were isolated from wildtype mice as described above. Myofibers were incubated in 20µg/ml recombinant annexin A6 (5186-A6-050, R&D systems) in 1mM Ca²⁺ Ringers or 0mM Ca²⁺ +EGTA. FM 1-43 (2.5µm) was added to the myofibers just prior to imaging. Images were acquired and quantitated as described above. FM 1-43 fluorescence over time was measured using FIJI and plotted overtime as F/F0.

**Cardiotoxin Injury and analysis.** Tibialis anterior muscle of wildtype mice were injected with 25µg/ml recombinant annexin A6 (5186-A6-050, R&D systems) or Ringers in sedated mice (3% isoflurane, 0.8 l/min O₂). For systemic administration, wildtype mice were injected with 1mg/kg recombinant annexin A6 (5186-A6-050, R&D systems) or PBS diluted in EBD (5µl/g body weight) into the retro-orbital cavity of sedated mice (3% isoflurane, 0.8 l/min O₂). Additionally, mice were injected with Evans' blue dye at 5µl/g body weight (E-2129; Sigma-Aldrich, St. Louis, MO) dissolved in phosphate-buffered saline at 10 mg/mL cardiotoxin injury was performed injecting 20µl of a 10µM cardiotoxin solution in PBS in tibialis anterior or gastroc/soleus muscles in sedated animals (3% isoflurane, 0.8 l/min O₂) 2 hours post pretreatment. Cardiotoxin was released down the midline of the muscle to induce a homogenous area of injury at the center of the muscle. 3 hours post cardiotoxin injection muscle was harvested.

**Evans blue dye uptake.** Sections (10µm thick) from the center of frozen-embedded muscles were collected on the cryostat (chamber, -20°C; sample, -15°C; catalog number CM1950; Leica, Wetzlar, Germany). Tissue sections were fixed with methanol for 2 minutes, rinsed and mounted with vectashield with DAPI (H-1200, Vector Laboratories). Imaging was performed using a Zeiss Axio Observer A1 microscope (Zeiss, Oberkochen, Germany), using a 10× objective. ZEN software (Zeiss, Jena, Germany) was used for acquiring images. Fluorescence quantitation, surface plots, and muscle area were performed using FIJI (NIH). For whole tissue dye quantification, whole tissue was dissected, finely minced, weighed, and incubated at 55°C in 1mL of formamide for 2 hours. Spectrophotometric absorbance was measured at 620 nm.

**Serum collection and CK analysis.** Mice were sedated (3% isoflurane, 0.8 l/min O₂) and blood was collected by means of retro-orbital puncture with heparinized capillary tubes (20-362-566; Fisher Scientific, Waltham, MA) into Microtainer^{™} Gold Top Serum Separator (365967 Becton Dickinson, Franklin Lakes, NJ) and centrifuged at 8,000 × g for 10 minutes. The plasma fractions were frozen and stored at -80°C. Serum creatine kinase (CK) was analyzed in duplicate for each mouse using the EnzyChrom Creatine Kinase Assay (ECPK-100; BioAssay Systems, Hayward, CA) following manufacturer's instructions as described (Demonbreun AR, Allen MV, Warner JL, Barefield DY, Krishnan S, Swanson KE, et al. Enhanced Muscular Dystrophy from Loss of Dysferlin Is Accompanied by Impaired Annexin A6 Translocation after Sarcolemmal Disruption. Am J Pathol. 2016;186(6):1610-22). Results were acquired with the Synergy HTX multi-mode plate reader (BioTek^{®}, Winooski, VT).

**Short-term chronic dosing regimen.** *Sgcg-null* mice were sedated (3% isoflurane, 0.8 l/min O₂) and blood collected as described above. While sedated, mice were injected with 1mg/kg recombinant annexin A6 (5186-A6-050, R&D systems) or PBS into the right retro-orbital cavity once every 3 days for a total of 5 injections and then blood drawn on day 14 post initial injection. An additional cohort of *Sgcg-null* mice had blood drawn as described above immediately prior to the first protein injection. Then, 1mg/kg recombinant annexin A6 or annexin A2 (both produced by Northwestern's Protein Production Core) was injected into the right retro-orbital cavity of sedated mice, once every 3 days for a total of 5 injections and then blood drawn on day 14 post initial injection.

**Exercise Injury.** *Sgcg-null* mice were sedated (3% isoflurane, 0.8 l/min O₂) and pre-exercise blood was collected by means of left retro-orbital puncture as described above. While sedated, mice were then injected 1mg/kg recombinant annexin A6 (5186-A6-050, R&D systems) or PBS + 1mg/kg BSA into the right retro-orbital cavity at 9am and 5pm for 5 consecutive injection over 48 hours. Two hours post the 5th RO-injection, mice were subjected to 60 mins of treadmill running at 10m/min at a 15° decline. Thirty minutes post exercise, blood was collected from the left retro-orbital cavity. Serum creatine kinase (CK) was analyzed as described above. Injections, exercise, and blood draws were performed blinded to treatment group.

**Protein production.** Recombinant mouse annexin A6 and mouse annexin A2 were produced and purified by the Northwestern's recombinant protein production core. Briefly, mouse annexin A6 (MG222645, Origene, Rockville, MD) and annexin A2 (MG205064, Origene, Rockville, MD) were subcloned into the pCMV6-AC-HIS backbone (PS100002, Origene, Rockville, MD). Plasmid was transfected and expressed with ExpiCHO expression system (A29133, ThermoFisher Scientific, Waltham, MA). Carboxy-terminally tagged recombinant protein was purified with Ni-charged MagBeads (L00295; GenScript, Piscataway, NJ) and purity evaluated through SDS-Page. Protein purity was additionally validated through immunoblot using anti-HIS (MAB050; R&D Systems, Minneapolis, MN) and anti-annexin A6 (ab31026, Abeam) or anti-annexin A2 (ab154113, Abcam) antibodies. Protein was diluted in phosphate buffered saline and stored at -80°C.

**Calcium kinetics.** FDB muscle was electroporated and isolated as described above. Myofibers were damaged in Ringers solution with Ca²⁺ concentrations of 2mM, 1mM, 0.5mM, 0.25mM, 0.175mM, 0.1mM, 0.050mM, and 0mM. EDTA was added as a Ca²⁺ chelating agent in only in the 0mM Ca²⁺ Ringers. Myofibers were isolated directly into 2mM, 1mM and 0.5mM Ringers for those experiments respectively. For experiments using less than 0.5mM Ca²⁺ myofibers were isolated in 0.5mM Ca²⁺ Ringers and then diluted with 0mM EDTA-free Ca²⁺ Ringers. For 0 mM experiments, myofibers were isolated in 0.5mM Ca²⁺Ringers and then replaced with 0mM Ca²⁺+EDTA Ringers just prior to imaging. Co-electroporation of wildtype annexin + wildtype annexin constructs was performed in one mouse foot, while the contralateral foot was co-electroporated with wildtype annexin + mutant annexin. All measurements were acquired from myofibers isolated from at least n≥2 mice, n≥3 myofibers per mouse at each Ca²⁺ concentration. Area-Ca²⁺ curves were fitted with a Hill Curve at Ca2+ concentrations ranging from 0-2mM. Kinetic parameters were calculated using Prism Graphpad.

**Calcium and pH indicator dye measurements.** Wildtype FDBs were isolated and plated in Ringers on Matek glass-bottom dishes as described above. Twenty minutes prior to imaging, myofibers were loaded with Fluo-4 AM at 37°C (F10489, ThermoFisher Scientific, Waltham, MA) or pHrodo AM (P35373, ThermoFisher Scientific, Waltham, MA) as described in the instruction manual. Fibers were rinsed once with Ringers then subsequently damaged and imaged on the Nikon A1R GaSP, as described above. Fluorescence intensity was measured using FIJI (NIH). phRodo change in fluorescence intensity was calculated as F/F0. Data was acquired from n=3 mice per experiment from multiple myofibers per mouse. Additionally, wildtype myofibers were incubated in 0mM Ca²⁺ for 1 hour., preloaded with Fluo-4 AM for 20 minutes prior to imaging, rinsed and then damaged on the MP+ in the presence of 0mM external Ca²⁺ with or without EGTA in the external Ringers Solution.

**Cardiotoxin injection and histology.** Wildtype mice were sedated (3% isoflurane, 0.8 l/min O₂) and then injected 1mg/kg recombinant annexin A6 (5186-A6-050, R&D systems) or PBS + 1mg/kg BSA into the right retro-orbital cavity and allowed to recover. Two hours post injection mice were sedated for a second time. While sedated the tibialis anterior muscles were injected along the midline with 10µm cardiotoxin in 20ul of PBS as described above. Seven days post injection, the muscle was isolated and frozen. Muscle sections were acquired every 100µm from muscle tendon into the mid belly, fixed and stained with hematoxylin and eosin. Imaging was performed using a Zeiss Axio Observer A1 microscope (Zeiss, Oberkochen, Germany), using a 10× objective. ZEN software (Zeiss, Jena, Germany) was used for acquiring tiled images. Percent injury area was calculated as the average injured area (containing internal myonuclei) divided by total muscle area of 3 sections per muscle. One muscle from each group was excluded due to technical error in tissue processing. Damage area was measured using FIJI (NIH).

**Single cell Ca²⁺ and shortening measurements.** Isolated FDB fibers were plated on laminin coated glass-bottomed 35 mm dishes for one hour and then cultured overnight in DMEM with 10% FBS and 1% penicillin/streptomycin at 37°C in a 10% CO₂ incubator. One hour prior to data acquisition, the medium was removed and cells were incubated in Tyrode buffer (119 mM NaCl, 5 mM KCI, 25 mM HEPES, 2 mM CaCl₂, 2 mM MgCl₂) with 10 µM Indo-1 AM (TefLabs) for 1 hour at 37°C in a 10% CO₂ incubator. Dishes were then filled with Tyrode buffer, mounted on a custom stage and platinum pacing electrodes were inserted into the dish. Stimulation was elicited using a 701C high-powered stimulator controlled by the 950A software (Aurora Scientific). Stimulation was performed at 40 and 80 Hz, 5 ms pulse width, 100 ms duration. Ratiometric Ca²⁺ signals were collected with two photomultiplier tubes and a FluoroDaq controller. Video sarcomere length was recorded with a high-speed camera and fast Fourier transform using the Aurora Scientific 900B-VSL system (Aurora, Ontario). Ten transients were collected over 20 seconds and averaged together per cell per frequency.

**Statistical analysis.** Statistical analyses were performed with Prism (Graphpad, La Jolla, CA). Comparisons relied on ANOVA ((1way ANOVA for 1 variable, 2way ANOVA for two variables (typically area and Ca²⁺ concentration)). Otherwise, unpaired two-tailed t-tests were performed. P-values of less than or equal to 0.05 were considered significant. Error bars represent +/- standard error of the mean (SEM).

**Study approval.** The study was conducted with the approval of Northwestern University's Institutional Animal Care and Use Committee (Chicago, IL).

### Results

**Ca²⁺ localizes to the repair cap upon membrane damage.** Activation of muscle membrane repair requires the presence of external Ca²⁺ (Bansal D, Miyake K, Vogel SS, Groh S, Chen CC, Williamson R, et al. Defective membrane repair in dysferlin-deficient muscular dystrophy. Nature. 2003;423(6936):168-72). Annexin proteins have previously been shown to aggregate into repair caps at the site of injury bordered by annexin-free zone within the cytoplasm under the repair cap (Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18; Demonbreun AR, Allen MV, Warner JL, Barefield DY, Krishnan S, Swanson KE, et al. Enhanced Muscular Dystrophy from Loss of Dysferlin Is Accompanied by Impaired Annexin A6 Translocation after Sarcolemmal Disruption. Am J Pathol. 2016;186(6):1610-22; Swaggart KA, Demonbreun AR, Vo AH, Swanson KE, Kim EY, Fahrenbach JP, et al. Annexin A6 modifies muscular dystrophy by mediating sarcolemmal repair. Proceedings of the National Academy of Sciences of the United States of America. 2014;111(16):6004-9; Quattrocelli M, Barefield DY, Warner JL, Vo AH, Hadhazy M, Earley JU, et al. Intermittent glucocorticoid steroid dosing enhances muscle repair without eliciting muscle atrophy. J Clin Invest. 2017;127(6):2418-32). To visualize Ca²⁺ dynamics at the site of injury in real-time, an *in vivo* fluorescent Ca²⁺ indicator protein, GCaMP5G, was utilized. GCaMP5G is a fusion protein composed of green fluorescent protein (GFP), the calcium-binding protein calmodulin, and the calmodulin M13 binding peptide. GCaMP5G has minimal fluorescence when not bound to Ca²⁺, and Ca²⁺ binding results in a conformational change within the protein increasing the fluorescence intensity of GFP (Akerboom J, Chen TW, Wardill TJ, Tian L, Marvin JS, Mutlu S, et al. Optimization of a GCaMP calcium indicator for neural activity imaging. J Neurosci. 2012;32(40):13819-40). Wildtype flexor digitorum brevis (FDB) muscle was electroporated with the GCaMP5G plasmid and then injured the plasma membrane using laser ablation (Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18; Demonbreun AR, and McNally EM. DNA Electroporation, Isolation and Imaging of Myofibers. Journal of visualized experiments : JoVE. 2015;106(106):e53551). Within two seconds of membrane injury (arrow), GCaMP5G fluorescence accumulated in the cytoplasm at the site of injury. GCaMP5G fluorescence intensity progressively increased through 260 seconds of imaging (Figure 23A, top panel).

To ensure these results were not a reflection of protein aggregation of the GCaMP5G sensor, wildtype myofibers were injured in the presence of Fluo-4 AM. Fluo-4 AM is a non-protein, Ca²⁺ indicator dye that increases fluorescence intensity upon binding Ca²⁺ and is routinely used to measure Ca²⁺ dynamics. Similar to GCaMP5G fluorescence, Fluo-4 AM fluorescence intensity increased at the site of laser-induced membrane injury 2 seconds post damage (arrow) and continued to increase intensity through the 260 seconds of imaging (Figure 23A, bottom panel). In myofibers co-electroporated with plasmids expressing GCaMP5G and annexin A6 with a carboxyl-terminal tdTomato fluorescent tag, GCaMP5G fluorescence localized in a ring around the annexin A6-free zone (Figure 23B, arrowhead) and co-localized with annexin A6 at the repair cap (Figure 23B, arrow, merge). It was also evaluated whether pH changed with injury using pHrodo fluorescence, a non-protein pH indicator dye that changes fluorescence with different pH levels. No change from the preinjury state (0s) compared to 10s post injury was noted, when Ca²⁺ indicator fluorescence is already increased at the site of injury (Figure 15). This temporal sequence is consistent with Ca²⁺ accumulation at the site of injury facilitating annexin translocation and assembly into repair caps.

**Annexin repair caps exhibit differential Ca²⁺ sensitivity during repair cap recruitment.** Annexin proteins are Ca²⁺-dependent phospholipid and actin binding proteins that contain four annexin repeat domains or eight in the case of annexin A6 (Figure 2). Annexin repeat domains bind Ca²⁺, but are distinct from the Ca²⁺ binding of C2 domains and EF-hands seen in other classes of repair proteins (Gerke V, and Moss SE. Annexins: from structure to function. Physiol Rev. 2002;82(2):331-71). Annexins coordinate Ca²⁺ and bind membranes from their convex face (Figure 2), and both type II and type III Ca²⁺ binding sites have been described in annexin proteins. To further define the Ca²⁺ requirements in annexin-mediated sarcolemmal repair in myofibers, annexin A1, A2, or A6 repair cap formation was examined at multiple Ca²⁺ concentrations. Cap size was measured from the center of a z-stack, and the type of fluorescent tag, turboGFP or tdTomato, did not alter assessed parameters (Figure 3A and 3B). Annexin A1 and A6 repair cap size was Ca²⁺-dependent, with the largest repair caps forming at 2mM and smaller repair caps forming at 0.1mM, while annexin A2 repair caps were not significantly reduced until 0.05mM Ca²⁺ (Figure 23C and 23D). Repair cap area was plotted as a function of Ca²⁺ concentration using a modified Hill equation. Annexin A2 formed a repair cap at the lowest concentration of Ca²⁺, 0.05mM, while annexins A1 and A6 did not form a discernable cap at Ca²⁺ concentrations lower than 0.1mM Ca²⁺, seen as the significant leftward annexin A2 curve with a Km_{1/2} of 0.067mM compared to A6 and A1, which showed Km_{1/2} of 0.12mM and 0.17mM, respectively (Figure 23D). Annexin A1 and A6 repair cap size and rate was highly dependent on Ca²⁺ concentration (Figure 4). The rate of annexin A2 cap formation and cap size was similar at 2mM, 0.5mM and 0.1mM Ca²⁺, while annexin A1 and A6 rates decreased with lower Ca²⁺ concentrations, suggesting a high Ca²⁺ affinity for annexin A2 (Figure 4). To ensure that repair cap formation was not artifact due to the type of laser injury, we induced laser injury on both the Nikon A1R GaSP confocal and the Nikon A1R MP+ multiphoton confocal. Injury induced by a multiphoton laser is more focused producing less collateral damage. Annexin A6 repair caps appeared comparable with both types of lasers (Figure 5). These data indicate that annexin A1, A2, and A6 repair cap formation is influenced by the level of Ca²⁺ present during myofiber repair with annexin A2 being the most Ca²⁺ sensitive of the three annexins studied.

**Annexin overexpression promotes bleb formation at the site of membrane injury.** Membrane repair studies in *Lytechinus pictus* and *Xenopus* oocytes have observed membranous structures emerging and erupting from the site of membrane repair (Davenport NR, Sonnemann KJ, Eliceiri KW, and Bement WM. Membrane dynamics during cellular wound repair. Mol Biol Cell. 2016;27(14):2272-85; Bi GQ, Alderton JM, and Steinhardt RA. Calcium-regulated exocytosis is required for cell membrane resealing. The Journal of cell biology. 1995;131(6 Pt 2):1747-58). Additionally, in artificial membrane preparations, the addition of recombinant annexins induced membrane folding or blebbing in a Ca²⁺ - dependent manner at sites of membrane imperfection (Boye TL, Jeppesen JC, Maeda K, Pezeshkian W, Solovyeva V, Nylandsted J, et al. Annexins induce curvature on free-edge membranes displaying distinct morphologies. Sci Rep. 2018;8(1):10309; Boye TL, Maeda K, Pezeshkian W, Sonder SL, Haeger SC, Gerke V, et al. Annexin A4 and A6 induce membrane curvature and constriction during cell membrane repair. Nat Commun. 2017;8(1):1623). It was investigated whether similar findings could be observed at the site of muscle membrane injury in live skeletal myofibers. GCaMP5G was expressed alone or in combination with annexin A1, A2 or A6 in skeletal myofibers. It was found that overexpression of annexins promoted the formation of extracellular blebs emanating from annexin repair caps at the membrane lesion (Figure 16A, red channel). These blebs appeared after the formation of repair caps and were seen at the extracellular tip of the repair cap, coincident with FM 4-64 fluorescence (Figure 16A and 16B). FM 4-64 is a membrane impermeable dye that is non-fluorescent in aqueous solution and increases fluorescence intensity as it binds membrane phospholipids exposed during injury; FM 4-64 is commonly used as a marker of membrane injury (Bansal D, Miyake K, Vogel SS, Groh S, Chen CC, Williamson R, et al. Defective membrane repair in dysferlin-deficient muscular dystrophy. Nature. 2003;423(6936):168-72; Cai C, Masumiya H, Weisleder N, Matsuda N, Nishi M, Hwang M, et al. MG53 nucleates assembly of cell membrane repair machinery. Nat Cell Biol. 2009;11(1):56-64; Demonbreun AR, and McNally EM. DNA Electroporation, Isolation and Imaging of Myofibers. Journal of visualized experiments : JoVE. 2015;106(106):e53551; Yeung T, Heit B, Dubuisson JF, Fairn GD, Chiu B, Inman R, et al. Contribution of phosphatidylserine to membrane surface charge and protein targeting during phagosome maturation. The Journal of cell biology. 2009;185(5):917-28; Zweifach A. FM1-43 reports plasma membrane phospholipid scrambling in T-lymphocytes. The Biochemical journal. 2000;349(Pt 1):255-60). Overexpression of annexin A6 and annexin A2 induced significantly more blebs than were observed after annexin A1 overexpression or GCaMP5G alone (Figure 16C and 16D). Furthermore, annexin-induced blebs were enriched for GCaMP5G, and annexin A6 induced the formation of significantly larger GCaMP5G-containing blebs as compared to annexin A1, A2, or GCaMP5G alone (Figure 16A, green channel, and 16D). A z-stack compilation demonstrated large annexin A6-induced GCaMP5G-positive blebs emanating from the site of injury. In contrast, annexin A2 resulted in smaller blebs extruding from the repair cap. These data indicate that annexins not only form a repair cap at the site of membrane disruption, but that these caps serve as sites for excretion of extracellular components enriched for Ca²⁺-binding proteins.

**Decreased intracellular Ca²⁺ fluorescence at the site of injury with annexin overexpression.** Time lapse imaging of the Ca²⁺ indicator GCaMP5G after laser injury suggested intracellular Ca²⁺ was decreasing concomitant with extracellular bleb formation suggesting that these blebs serve to reduce intracellular Ca²⁺ accumulation through excretion. The annexin-induced reduction in intracellular Ca²⁺ fluorescence could be seen for all three annexins A1, A2 and A6, but was most evident for annexin A2 and A6 (Figure 7A). Over the 240 seconds of imaging, overexpression of annexin A6 induced the most significant reduction in intracellular Ca²⁺, visualized as internal GCaMP5G Ca²⁺ fluorescence (Figure 7B). Detailed analysis of the first 20 seconds post injury showed a significant reduction in internal GCaMP5G Ca²⁺ fluorescence with annexin A2 and A6, but not annexin A1, when compared to GCaMP5G alone (Figure 7C). Baseline GCaMP5G fluorescence intensity prior to injury was not significantly different between groups (Figure 7D). Reduction in internal Ca²⁺ fluorescence at the lesion with annexin A6 expression was confirmed using Fluo-4 AM (Figure 17). Thus, annexin expression induced a reduction of Ca²⁺ signal within the injured myofiber concomitant with enhanced egress of Ca²⁺-binding protein-filled blebs. Moreover, annexin A6 was the most effective of the three annexins tested at sustaining this response.

Overexpression of Ca²⁺-binding proteins like annexins may have unexpected effects on intracellular Ca²⁺ signaling and cellular function. Therefore, the Ca²⁺ handling and contractile properties of isolated myofibers overexpressing annexin A6 was evaluated compared to controls. Isolated myofibers expressing annexin A6 were loaded with the ratiometric Ca²⁺ indicator dye Indo-1, and we observed no differences in Ca²⁺ cycling at 40 or 80 Hz stimulation frequencies between annexin A6 or control fibers (Figure 8A, 8B, and 8C). Unloaded cell shortening was also unaffected by the presence of overexpressed annexin A6 (Figure 8D, 8E, and 8F). These results demonstrated that annexin A6 overexpression was well-tolerated by myofibers.

**Annexin A6 Ca²⁺ binding is required for repair cap formation and myofiber repair.** Mutation of annexin A1 residue D171 and annexin A2 residue D161 were previously shown to inhibit annexin membrane translocation in HEK cells (McNeil AK, Rescher U, Gerke V, and McNeil PL. Requirement for annexin A1 in plasma membrane repair. The Journal of biological chemistry. 2006;281(46):35202-7; Jost M, Thiel C, Weber K, and Gerke V. Mapping of three unique Ca(2+)-binding sites in human annexin II. Eur J Biochem. 1992;207(3):923-30). It was queried whether these mutations would inhibit translocation and formation of the macromolecular annexin repair cap formed after muscle membrane injury in live myofibers. Alignment of annexins A1, A2 and A6 protein sequences was used to identify the conserved residues within the consensus sequence of type II Ca²⁺ binding sites across all three annexin proteins (Figure 2). In order to disrupt Ca²⁺ binding in annexin A1, A2, and A6, site-directed mutagenesis was performed to convert the aspartic acid residue in the first type II Ca²⁺ binding site into an alanine residue (A1D171A, A2D161A, A6D149A, respectively) (Figure 9A). E233A was also generated in annexin A6, to create a similar change in the Ca²⁺ binding site in the second annexin repeat domain of annexin A6. Each construct also contained turboGFP or tdTomato at the C-terminus. To assess the effect of homotypic annexin interactions during repair cap formation, myofibers were co-electroporated with wildtype+wildtype (A6+A6) or wildtype+mutant (A6+A6E233A) annexin combinations. Mutation of E233 in annexin A6 acted in a dominant-negative fashion significantly decreasing cap size of the co-expressed wildtype annexin A6 protein (Figure 10A). Prior structural studies suggested that D149 in the first annexin repeat domain of annexin A6 did not bind Ca²⁺ (Avila-Sakar AJ, Creutz CE, and Kretsinger RH. Crystal structure of bovine annexin VI in a calcium-bound state. Biochimica et biophysica acta. 1998;1387(1-2):103-16), and consistent with this, the D149A mutant in annexin A6 had little effect on cap size (Figure 9B, right panel). The repair cap feret diameter was plotted as a function of Ca²⁺ concentration using a modified Hill equation. Expression of mutant annexin A6E233A significantly reduced the cap diameter (DMAX) of the co-expressed wildtype annexin A6 protein (Figure 10B). To assess the effect of heterotypic annexin interactions on repair cap formation, myofibers were co-electroporated with various combinations of wildtype and mutant annexin constructs. Co-expression of mutant annexin A6E233A resulted in a significant reduction in annexin A1, A2, and A6 cap size compared to A1+A6, A2+A6, A6+A6 controls, respectively (Figure 10C). Together, these data showed that annexin proteins interact in a homotypic and heterotypic fashion influencing annexin repair complex-assembly and that the mutant annexin A6 protein is sufficient to negatively modulate annexin complex assembly during repair.

Ca²⁺-binding of both annexin A1 and A2 was also required for repair cap formation. A1D171 A and A2D161A mutant cap size was reduced compared to wildtype annexin A1 and A2 controls, respectively. Expression of mutant annexin A1D171A and A2D161A significantly reduced the repair cap diameter (DMAX) of the respective co-expressed wildtype annexin protein (Figure 9B, left and middle panels). Despite the ability of mutant annexin A1D171A and A2D161A to significantly decrease co-expressed wildtype annexin A1 and A2 cap size, respectively, A1D171A or A2D171A had minimal effect of wildtype annexin A6 cap size (Figure 9C). These data showed that annexin A1 and A2 interact in a homotypic fashion influencing self-cap assembly, while A6 localization to the repair cap is minimally modulated by annexin A1 and A2 localization.

To determine the effect of dominant negative annexin A6 on the assembly of annexins A1, A2, and A6 at the repair cap and membrane repair capacity, laser injury was similarly performed on isolated myofibers in the presence of FM 4-64. Myofibers expressing annexin A6E233A-GFP had increased FM 4-64 fluorescence area after laser injury compared to control myofibers expressing wildtype annexin A6-GFP (Figure 10D). These results indicate that a functional annexin repair complex is required for proper membrane repair and annexin A6 participates in orchestrating complex formation.

**Annexin A6 protected against laser-induced myofiber injury *in vitro* in a Ca²⁺**-**dependent manner.** Since annexin A6 facilitates the formation of the macromolecular repair cap complex and was the most efficient at forming large, Ca²⁺-filled blebs at the site of membrane injury, it was assessed whether expression of annexin A6 would reduce membrane injury in wildtype myofibers. Wildtype myofibers were electroporated with annexin A6-GFP or mock electroporated and then laser damaged in the presence of FM 4-64 to mark the injury area. Wildtype myofibers overexpressing annexin A6 had decreased FM 4-64 dye uptake after laser-induced membrane injury compared to control myofibers (Figure 18A). These results indicate that intracellular overexpression of annexin A6 is effective at improving membrane repair and/or protecting against laser-induced membrane injury in isolated myofibers.

Since intracellular annexin A6 targets phospholipids exposed at the site of membrane injury and enhances membrane repair capacity, we hypothesized that extracellular recombinant annexin A6 (rANXA6) would also localize to the site of injury and protect against membrane injury. Muscular dystrophy is a progressive muscle wasting disease, arising from loss-of-function mutations in critical cytoskeletal or membrane-associated proteins, often resulting in fragile plasma membranes. To determine if recombinant annexin A6 could protect against membrane insult in both healthy and dystrophic muscle, wildtype and dystrophic myofibers from a model representing Duchenne Muscular Dystrophy were isolated and incubated with recombinant annexin A6 or vehicle control. Laser injury was conducted in the presence of FM 4-64 to visualize the extent of injury. Treatment with extracellular recombinant annexin A6 reduced FM 4-64 fluorescence area compared to vehicle control-treated myofibers, indicating enhanced repair in both healthy and dystrophic myofibers (Figure 18B and 18C).

To assess whether recombinant annexin A6's protective effects required external Ca²⁺, wildtype myofibers were pretreated with recombinant annexin A6, loaded with FM 1-43, a fluorescence marker of membrane damage similar to FM4-64. and subsequently damaged in solution containing 1mM Ca²⁺ or 0mM Ca²⁺+EGTA, a Ca²⁺ chelator. FM 1-43 fluorescence accumulation at the lesion over time (F/F0) was significantly increased in the absence of Ca²⁺ compared to in the presence of 1mM Ca²⁺ (Figure 19A and 19B). These data demonstrated that extracellular recombinant annexin A6 protects against membrane injury and/or enhances repair through extracellular exposure in a Ca²⁺-dependent manner.

**Recombinant annexin A6 protected against acute muscle injury *in vivo.*** To determine the therapeutic potential of recombinant annexin A6 to protect against muscle injury *in vivo,* recombinant annexin A6 was utilized as a tool compound. Recombinant annexin A6 or vehicle control was injected intramuscularly into the tibialis anterior (TA) muscles of wildtype mice two hours prior to toxin-induced injury. Mice were injected intraperitoneally with Evan's blue dye, a vital tracer that is excluded by intact healthy myofibers but is readily taken up in injured permeable myofibers (Jennische E, and Hansson HA. Postischemic skeletal muscle injury: patterns of injury in relation to adequacy of reperfusion. Exp Mol Pathol. 1986;44(3):272-80). Three hours post-cardiotoxin injury muscle was evaluated for Evan's blue dye uptake (Figure 12A). Gross imaging showed that pretreatment with recombinant annexin A6 reduced cardiotoxin-induced muscle damage *in vivo,* as seen as less dye (blue) uptake compared to controls (Figure 12B). Fluorescence imaging showed a 50% decrease in dye (red) uptake with recombinant annexin A6 pretreatment compared to control muscle (Figure 12C). Surface plot profiles illustrate reduced dye fluorescence in tibialis anterior muscle pretreated with intramuscular recombinant annexin A6 (Figure 12D).

Although intramuscular injection of annexin A6 was effective at reducing acute injury, this route of application is not optimal for large muscle groups, internal tissues, or treatment of chronic diseases. Therefore, the efficacy of recombinant annexin A6 administered systemically via retro-orbital (RO) injection in the protection against acute muscle injury was examined. Recombinant annexin A6 or vehicle was injected into the retro-orbital cavity two hours prior to toxin-induced injury. Mice were simultaneously injected with Evan's blue dye. Three hours post-cardiotoxin injury muscle was evaluated for Evan's blue dye uptake (Figure 20A). Fluorescence imaging showed a 38% decrease in dye (red) uptake with recombinant annexin A6 pretreatment compared to vehicle control (Figure 20B and 20C). Surface plot profiles illustrate reduced dye fluorescence in muscle pretreated with systemic recombinant annexin A6 (Figure 20C). In addition, whole tissue spectroscopic analysis of injured gastrocnemius/soleus muscles revealed a 58% reduction in dye uptake with rANXA6 pretreatment compared to vehicle treated (Figure 20D). These results demonstrated that local and systemic administration of recombinant annexin A6 protects against acute muscle injury *in vivo.*

To determine if recombinant annexin A6 administration enhanced myocyte survival and/or recovery from injury, mice were administered recombinant annexin A6 or BSA control, at 1mg/kg, through retro-orbital systemic injection. Two hours post protein administration, cardiotoxin was injected into the tibialis anterior muscles to induce focal muscle injury. Muscle was harvested 7 days post injury and histology evaluated for injury area (Figure 20E). Pretreament with recombinant annexin A6 reduced the percentage of injured muscle, marked by internal myonuclei (black dotted outline), at 7 days post insult (Figure 20F and 20G). These data illustrated that systemic administration of recombinant annexin A6 protects against acute muscle injury enhancing myocyte survival /recovery from injury.

**Recombinant annexin A6 protected against chronic muscle injury** ***in vivo**.* The ability of recombinant annexin A6, administered systemically, to protect against muscle damage in the Sgcg-null mouse model of Limb Girdle Muscular Dystrophy type 2C (LGMD2C) (Hack AA, Cordier L, Shoturma DI, Lam MY, Sweeney HL, and McNally EM. Muscle degeneration without mechanical injury in sarcoglycan deficiency. Proceedings of the National Academy of Sciences of the United States of America. 1999;96(19):10723-8) was assessed next. *Sgcg-null* mice lack γ-sarcogiycan, an integral membrane component of the dystrophin glycoprotein complex required for membrane stability and function. Humans and mice lacking γ-sarcogiycan develop progressive muscle disease, reduced muscle function and elevated serum creatine kinase (CK), a serum biomarker of muscle injury and membrane leak. To determine if recombinant annexin A6 protected in dystrophic muscle, *Sgcg-null* mice were treated systemically with recombinant annexin A6 or BSA control over 48 hours (Figure 22A). Mice were then subjected to 60 minutes of treadmill running to induce physiological muscle damage and CK release. Recombinant annexin A6 reduced the fold change of CK kinase post exercise to pretreatment, consistent with improved membrane resealing (Figure 22A and 22B). Recombinant annexin A6 was also injected over a two-week interval in *Sgcg-null* mice. 1mg/kg of recombinant annexin A6 was injected once every three days for 14 days (Figure 22C). Administration of recombinant annexin A6 significantly decreased levels of serum creatine kinase (CK) compared to control treatment at day 14 (Figure 22D). Annexin A6 was more effective than annexin A2 at reducing intracellular Ca²⁺ after injury (Figure 16D). Using the same 14-day dosing regimen in *Sgcg-null* mice, short-term systemic administration of recombinant annexin A6 significantly reduced serum CK levels compared to recombinant annexin A2 (Figure 22E). The gastrocnemius/soleus muscle from recombinant annexin treated mice showed qualitatively less injury (Figure 22F). These data showed that extracellular recombinant annexin A6 protects against injury or enhances repair in chronically injured, dystrophic mouse muscle, *in vivo.*

### Discussion

**Annexins promote calcium-filled bleb formation at the site of membrane injury.** Plasma membrane instability is inherent to many forms of muscular dystrophy and thought to contribute to dysregulated Ca²⁺ homoeostasis and disease pathogenesis. Molkentin and colleagues showed that transgenic overexpression of TRPC3 was sufficient to increase myofiber Ca²⁺ influx and result in a dystrophy-like phenotype (Millay DP, Goonasekera SA, Sargent MA, Maillet M, Aronow BJ, and Molkentin JD. Calcium influx is sufficient to induce muscular dystrophy through a TRPC-dependent mechanism. Proceedings of the National Academy of Sciences of the United States of America. 2009;106(45):19023-8). Correspondingly, transgenic overexpression of SERCA1 reduced cytosolic Ca²⁺ levels and mitigated dystrophic muscle pathology implicating Ca²⁺ in disease progression (Goonasekera SA, Lam CK, Millay DP, Sargent MA, Hajjar RJ, Kranias EG, et al. Mitigation of muscular dystrophy in mice by SERCA overexpression in skeletal muscle. J Clin Invest. 2011;121(3):1044-52). As shown and described herein, increased expression of annexins in muscle fibers decreased injury-associated Ca²⁺ fluorescence within myofibers. This reduction of Ca²⁺-associated fluorescence was at the injury site and correlated with extracellular bleb formation emanating from annexin repair caps. Both annexin A2 or A6 could induce the formation of membranous blebs containing the Ca²⁺-binding protein GCaMP5G. Furthermore, overexpression of annexins A1, A2, and A6 each reduced endpoint Ca²⁺ fluorescence accumulation within the myofiber after injury. Of the three annexins tested, annexin A6 overexpression resulted in the most sustained effect on reducing injury-associated Ca²⁺ accumulation inducing the formation of large GCaMP5G-containing blebs. In HEK293 cells damaged with streptolysin O (SLO), the presence of extracellular membranous blebs correlated with increased cell survival and reduction in cytoplasmic Ca²⁺ levels, a process facilitated by annexin A1 (Babiychuk EB, Monastyrskaya K, Potez S, and Draeger A. Blebbing confers resistance against cell lysis. Cell Death Differ. 2011;18(1):80-9). Davenport and colleagues showed overexpression of annexin A1-GFP in injured Xenopus oocytes resulted in annexin A1 positive blebs originating from the site of damage (Davenport NR, Sonnemann KJ, Eliceiri KW, and Bement WM. Membrane dynamics during cellular wound repair. Mol Biol Cell. 2016;27(14):2272-85). However, the effects of annexins A2 or A6 overexpression were not assessed in either study. These data combined suggest bleb formation as a mechanism of membrane repair is conserved across species and tissue types and is facilitated by the presence of annexin proteins.

Without being bound by theory, it is contemplated that annexin A6 facilitates cytoplasmic Ca²⁺ and protein excretion into extracellular blebs whose formation is further induced by annexin A1 and annexin A2. In artificial membrane patches, the presence of annexin A1 or annexin A2 induced bleb formation at sites of membrane imperfection (Boye TL, Jeppesen JC, Maeda K, Pezeshkian W, Solovyeva V, Nylandsted J, et al. Annexins induce curvature on free-edge membranes displaying distinct morphologies. Sci Rep. 2018;8(1):10309). In contrast, the presence of annexin A6 induced Ca²⁺-dependent contraction of artificial membrane into large folds (Boye TL, Jeppesen JC, Maeda K, Pezeshkian W, Solovyeva V, Nylandsted J, et al. Annexins induce curvature on free-edge membranes displaying distinct morphologies. Sci Rep. 2018;8(1):10309). The difference between annexin A6 inducing blebs in live myofibers or folds in artificial membrane may reflect the presence of endogenously expressed annexin A1 and A2 in isolated myofibers compared with exposure to single recombinant annexin protein in the artificial membrane studies. Without being bound by theory, it is contemplated that within the macromolecular repair complex, multiple annexins actively participate in bleb formation, which acts to remove large membrane lesions facilitating wound closure, excision of damaged membrane, and reduction of Ca²⁺ at the injury site.

**Annexin A6 protects against muscle membrane injury and enhances membrane repair.** We showed that annexin proteins, including annexin A1, A2, and A6, localize to the site of membrane injury facilitating membrane repair cap and bleb formation. Mutation of annexin A6 abrogated repair cap formation, decreasing repair capacity, resulting in increased dye uptake. On the other hand, pretreatment with recombinant annexin A6 reduced dye uptake after laser-induced muscle injury and after toxin-induced muscle injury *in vivo.* As a therapeutic tool, enhancing the cells' ability to repair and/or reduce injury through stabilizing the cell membrane are both beneficial avenues that can lead to improved cell survival. Previous studies have shown that annexin A6 is upregulated in muscle from models of chronic muscular dystrophy (Demonbreun AR, Allen MV, Warner JL, Barefield DY, Krishnan S, Swanson KE, et al. Enhanced Muscular Dystrophy from Loss of Dysferlin Is Accompanied by Impaired Annexin A6 Translocation after Sarcolemmal Disruption. Am J Pathol. 2016;186(6):1610-22; Swaggart KA, Demonbreun AR, Vo AH, Swanson KE, Kim EY, Fahrenbach JP, et al. Annexin A6 modifies muscular dystrophy by mediating sarcolemmal repair. Proceedings of the National Academy of Sciences of the United States of America. 2014;111(16):6004-9; Demonbreun AR, Rossi AE, Alvarez MG, Swanson KE, Deveaux HK, Earley JU, et al. Dysferlin and myoferlin regulate transverse tubule formation and glycerol sensitivity. Am J Pathol. 2014;184(1):248-59). Proteomic profiling of mdx mouse muscle showed that annexins A1 and A2 are enriched in mdx muscle membrane, consistent with a role for annexins at the membrane of injured muscle cells (Murphy S, Zweyer M, Henry M, Meleady P, Mundegar RR, Swandulla D, et al. Proteomic analysis of the sarcolemma-enriched fraction from dystrophic mdx-4cv skeletal muscle. J Proteomics. 2018). Annexins bind membrane phospholipids, including phosphatidylserine, which is exposed during membrane disruption. Phosphatidylserine rearrangement after injury provides an optimal binding target for extracellular annexins to facilitate membrane folding, blebbing, and rolling at sites of membrane damage and imperfection (Boye TL, Jeppesen JC, Maeda K, Pezeshkian W, Solovyeva V, Nylandsted J, et al. Annexins induce curvature on free-edge membranes displaying distinct morphologies. Sci Rep. 2018;8(1):10309). Upregulation of annexins is contemplated to be a compensatory mechanism to facilitate excision of defective membrane in fibers undergoing chronic damage.

Additional studies of cardiac muscle injury further suggest a role for annexin proteins in modulating the repair response. Administration of recombinant annexin A1 or the N-terminal annexin A1 peptide (AC2-26) elicited a cardioprotective response in a rat model of myocardial ischemia-reperfusion-induced injury (La M, D'Amico M, Bandiera S, Di Filippo C, Oliani SM, Gavins FN, et al. Annexin 1 peptides protect against experimental myocardial ischemia-reperfusion: analysis of their mechanism of action. FASEB J. 2001;15(12):2247-56). Meng et al demonstrated that downregulation of annexin A3 resulted in cardioprotection, decreasing rat myocardial infarct size through activation of AKT signaling (Meng H, Zhang Y, An ST, and Chen Y. Annexin A3 gene silencing promotes myocardial cell repair through activation of the PI3K/Akt signaling pathway in rats with acute myocardial infarction. J Cell Physiol. 2019;234(7):10535-46). In addition to membrane reorganization, annexins act as scaffolds regulating multiple downstream intracellular signaling cascades important for orchestrating repair from injury. Both intra- and extra-cellular functions of annexin proteins should be considered when evaluating the therapeutic potential of annexin proteins.

The studies presented herein indicate that human recombinant annexin A6 protein is a suitable biologic to protect against acute muscle injury. It is shown herein that human recombinant annexin A6 was capable of resealing injured membrane in mouse models, confirming functional activity of the human recombinant protein in a mouse preclinical model. Human and mouse annexin A6 proteins are 94.35% identical at the amino acid level with increasing percent amino acid conservation between humans and rat (94.65%), dog (95.54%), and monkey (98.96%), and this high degree of similarly is consistent with human recombinant protein having efficacy in a mouse model (Figure 21). The current studies are limited by available recombinant protein, and further studies are needed to determine if recombinant annexin A6 can facilitate membrane repair and reduce the susceptibility to injury long-term in chronic models of muscle disease and in tissues beyond skeletal muscle. Annexin A6 was originally identified in a mouse model of muscular dystrophy as a genetic modifier of muscle membrane leak, and annexin A6 was subsequently shown to modify injury response in healthy mouse muscle (Swaggart KA, Demonbreun AR, Vo AH, Swanson KE, Kim EY, Fahrenbach JP, et al. Annexin A6 modifies muscular dystrophy by mediating sarcolemmal repair. Proceedings of the National Academy of Sciences of the United States of America. 2014;111(16):6004-9). It is contemplated herein that enhancing repair through administration of recombinant annexin A6 protein will provide similar protection in dystrophic muscle but would require long term intermittent dosing. Future studies will require optimizing mammalian recombinant annexin A6 protein production to generate sufficient quantities of purified protein.

**Combinatorial approaches to improve membrane repair.** Recombinant annexin A6 and its ability to protect normal and dystrophic muscle from laser-induced membrane injury is described and shown herein. In addition, both intramuscular and systemic administration of recombinant annexin A6 protected against toxin-induced muscle membrane injury *in vivo.* It was previously found that glucocorticoid administration increased annexin expression in muscle, and this correlated with enhanced muscle repair in multiple mouse models of muscular dystrophy including mdx (DMD), dysferlin-null (Limb Girdle Muscular Dystrophy 2B), and γ-sarcoglycan-null (Limb Girdle Muscular Dystrophy 2C) mice (Quattrocelli M, Barefield DY, Warner JL, Vo AH, Hadhazy M, Earley JU, et al. Intermittent glucocorticoid steroid dosing enhances muscle repair without eliciting muscle atrophy. J Clin Invest. 2017;127(6):2418-32; Quattrocelli M, Salamone IM, Page PG, Warner JL, Demonbreun AR, and McNally EM. Intermittent Glucocorticoid Dosing Improves Muscle Repair and Function in Mice with Limb-Girdle Muscular Dystrophy. Am J Pathol. 2017;187(11):2520-35). Glucocorticoid treatment also increased the expression of the Trim72 gene that encodes mitsugumin 53 (known as MG53), a repair protein that localizes to the site of membrane injury and considered a "molecular band-aid" for improving cellular wound healing. Similar to the annexins, MG53 is upregulated in chronic muscle injury and enhances repair in dystrophic muscles, as well as other tissues like heart, lung, kidney (Waddell LB, Lemckert FA, Zheng XF, Tran J, Evesson FJ, Hawkes JM, et al. Dysferlin, annexin A1, and mitsugumin 53 are upregulated in muscular dystrophy and localize to longitudinal tubules of the T-system with stretch. J Neuropathol Exp Neurol. 2011;70(4):302-13; Duann P, Li H, Lin P, Tan T, Wang Z, Chen K, et al. MG53-mediated cell membrane repair protects against acute kidney injury. Science translational medicine. 2015;7(279):279ra36; He B, Tang RH, Weisleder N, Xiao B, Yuan Z, Cai C, et al. Enhancing muscle membrane repair by gene delivery of MG53 ameliorates muscular dystrophy and heart failure in delta-Sarcoglycan-deficient hamsters. Molecular therapy : the journal of the American Society of Gene Therapy. 2012;20(4):727-35; Jia Y, Chen K, Lin P, Lieber G, Nishi M, Yan R, et al. Treatment of acute lung injury by targeting MG53-mediated cell membrane repair. Nat Commun. 2014;5:4387; Liu J, Zhu H, Zheng Y, Xu Z, Li L, Tan T, et al. Cardioprotection of recombinant human MG53 protein in a porcine model of ischemia and reperfusion injury. Journal of molecular and cellular cardiology. 2015;80:10-9; Weisleder N, Takizawa N, Lin P, Wang X, Cao C, Zhang Y, et al. Recombinant MG53 protein modulates therapeutic cell membrane repair in treatment of muscular dystrophy. Science translational medicine. 2012;4(139):139ra85). MG53 is a component of the annexin-mediated repair complex, localizing adjacent to the annexin repair cap (Demonbreun AR, Quattrocelli M, Barefield DY, Allen MV, Swanson KE, and McNally EM. An actin-dependent annexin complex mediates plasma membrane repair in muscle. The Journal of cell biology. 2016;213(6):705-18).

### Example 4

Additional experiments were designed and conducted to evaluate the role of endogenous annexin A6, annexin A6 in heart, and annexin A6 in additional models of muscular dystrophy.

### Methods

**Animals.** Genetically-encoded annexin A6GFP mice were generated and backcrossed onto the 129T2/SvEmsJ background. Dysferlin-null mice on the 129T2/SvEmsJ background were previously generated (Demonbreun et al. HMG 2011). *Sgcg-null* mice were generated as described in (Hack et al PNAS 1999). Mice were bred and housed in a specific pathogen free facility on a 12-hour light/dark cycle and fed ad libitum in accordance with the Northwestern University's Institutional Animal Care and Use Committee regulations. 129T2/SvEmsJ (129T2) mice were originally purchased from the Jackson Laboratory (Ben Harbor, ME; Stock # 002065). Two to three-month-old male and female were used for all experiments. All animal experiments were approved in accordance with the Northwestern University's Institutional Animal Care and Use Committee regulations.

**Genomic structure schematic.** Annexin A6 genomic structure was visualized using UCSC genome browser.

**Myofiber isolation.** Flexor digitorum brevis (FDB) fibers were isolated with methods that were described previously in (Demonbreun and McNally, 2015; Demonbreun et al., 2016b; DiFranco et al., 2009). Briefly, fibers were dissociated in 0.2% BSA plus collagenase type II (Cat # 17101, Thermo Fisher Scientific, Waltham, MA) for 60 minutes at 37 degrees in 10% CO2. Fibers were then moved to Ringers solution and placed on MatTek confocal microscopy dishes (Cat # P35G-1.5-14-C, MatTek, Ashland MA).

**Immunofluorescence microscopy.** Mice were systemically injected with recombinant annexin A6 with a C-terminal HIS tag (R&D). Muscle was harvested and flash frozen. Muscle sections (10µm thick) from the center of frozen-embedded muscles were collected on the cryostat (chamber, -20°C; sample, -15°C; catalog number CM1950; Leica, Wetzlar, Germany) for immunostaining. Tissues were fixed with 4% paraformaldehyde for 10 minutes on ice. Block and permeabilization were with 0.1% Triton (catalog number X-100; Sigma-Aldrich), 10% fetal bovine serum, and PBS for 60 minutes. For laminin detection, anti-laminin antibody was used at a dilution of 1:100 and for HIS detection, anti-HIS antibody was used at 1:100 overnight at 4°C. Hoechst labelled nuclei. Sections were PBS rinsed, incubated with secondary antibody for 1 hour, PBS rinsed, and mounted. Imaging was performed using a Zeiss Axio Observer A1 microscope (Zeiss, Oberkochen, Germany).

**Cardiomyocyte isolation.** Mice were treated with 50 U heparin intraperitoneally 20 minutes before sacrifice. Mice were anesthetized under 5% vaporized isoflurane mixed with 100% oxygen. A thoracotomy was performed and the heart and lungs rapidly excised and submerged into ice-cold Tyrode solution without calcium (143-mM NaCl, 2.5-mM KCI, 16-mM MgCl₂, 11-mM glucose, 25-mM NaHCO₃, pH adjusted to 7.4). The ascending aorta was dissected out of the surrounding tissue and cannulated with an animal feeding needle (7900, Cadence Science, Staunton, Virginia) and secured with a 6-0 silk suture. The heart was initially perfused with 1 ml of ice-cold calcium-free Tyrode solution before being transferred to a Langendorff apparatus (Radnoti, Covina, California). Hearts were perfused with 37°C calcium-free Tyrode solution using a constant pressure (65-cm vertical distance between the buffer reservoir and cannula tip) for 1 to 2 minutes before perfusion for 5.5 minutes with digestion solution (0.15% collagenase type 2 [Worthington Biochemical, Lakewood, New Jersey], 0.1% 2,3-butanedione monoxime, 0.1% glucose, 100-U/ml penicillin/streptomycin, 112-mM NaCl, 4.7-mM KCI, 0.6-mM KH2PO₄, 40-µM CaCl₂, 0.6-mM Na2HPO4, 1.2-mM MgSO4, 30-µM phenol red, 21.4-mM NaHCO₃, 10-mM HEPES, and 30-mM taurine; pH adjusted to 7.4). The heart was removed from the cannula, triturated with a transfer pipette, and filtered through a 100-µm cell strainer. Cardiomyocytes were allowed to pellet by gravity for 7 minutes, followed by aspiration of digestion media and washing with stop buffer (formulated identically to digestion solution except with no collagenase and with 1% bovine serum albumin). Cells were again allowed to gravity pellet followed by a wash in stop buffer without bovine serum albumin. Cardiomyocytes were tolerated to calcium by adding Tyrode buffer with 0.3-mM CaCl₂ dropwise. Cell culture dishes were coated with 20 µg/ml laminin (23017-015; Gibco, Thermo Fisher Scientific, Waltham, Massachusetts) for 1 hour at room temperature. Laminin solution was aspirated followed by plating of cardiomyocytes for 1 hour to allow cell adhesion before experimentation.

**Multiphoton laser injury and imaging.** Isolated fibers were subjected to laser-induced damage at room temperature using the Nikon A1R-MP multiphoton microscope. Imaging was performed using a 25×1.1NA objective directed by the NIS-Elements AR imaging software. Green fluorescence protein (GFP) and FM 4-64 were excited using a 920nm wavelength laser and emission wavelengths of 575nm and 629nm were collected respectively. To induce laser damage on isolated myofibers, a diffraction limited spot (diameter approximately 410nm) was created on the lateral membrane of the myofiber using a 920nm wavelength laser at 10-15% laser power for 1 second. Time lapse images were collected as follows: one image was collected prior to damage, one image upon damage, then every 8 s for 80s (10 images) followed by every 30 seconds for 5 min (10 images). At the end of the time lapsed image series, z-stack images were collected at 250nm intervals through the damaged site on the myofiber directed by the NIS-Elements AR imaging software. Fluorescence intensity and cap area were measured using Fiji (NIH).

For recombinant protein studies, myofibers were isolated from mice as described above. Myofibers were incubated in 10-40µg/ml recombinant annexin A6 (5186-A6-050, R&D systems) in 1mM Ca²⁺ Ringers or BSA control. Cap size was assessed from acquired images in FIJI. FM 4-64 (2.5µm) was added to the myofibers just prior to imaging. Images were acquired and quantitated as described above. FM 4-64 fluorescence at endpoint was measured using FIJI.

For prednisone studies, prednisone (catalog P6254) was resuspended in DMSO (catalog D2650, Sigma-Aldrich) at 5 mg/ml. Dosing was based on pretreatment weights (1 mg/kg body weight) in 30 µl total PBS. Mice were injected at 7 am on the day prior to imaging. On injection days, stock solutions stored at -20°C were diluted into sterile Eppendorf tubes containing sterile PBS (catalog 14190, Life Technologies). Sterile BD Micro-Fine IV Insulin Syringes (catalog 14-829-1A, Fisher Scientific) were used to inject the I.P. cavity of non-sedated animals. 24 hours post injection myofibers were harvest and then imaged the same day.

**Statistical analysis.** Statistical analyses were performed with Prism (Graphpad, La Jolla, CA). Comparisons relied on ANOVA (1way ANOVA for 1 variable). Otherwise, unpaired two-tailed t-tests were performed. P value less than or equal to 0.05 was considered significant. Data were presented as single values were appropriate. Error bars represent +/- standard error of the mean (SEM).

Results of the experiments are shown in Figures 24-28.

**Glucocorticoid steroids modify endogenous annexin A6GFP localization, protecting against insult.** Gene editing was used to introduce a GFP tag at the carboxyl terminus of the Anxa6 locus in mice to create *Anxa6^{em1(GFP)}* mice. These mice offer the advantage of examining genomically-encoded annexin A6 under the control of the Anxa6 promoter and regulatory sequences. Because *Anxa6^{em1(GFP)}* mice express annexin A6 protein under the control of the Anxa6 gene, expression levels are at normal to lower levels than plasmid encoded annexin A6-GFP. This animal model allows for the examination of a range of annexin A6 protein expression in real time. Glucocorticoid steroids, including prednisone, have been shown to upregulate annexin A1 and A6 expression. Furthermore, prednisone administration protected against membrane injury, reducing plasmid overexpressed annexin GFP cap formation after laser injury. To determine if prednisone modulated genomically-encoded annexin A6GFP cap kinetics, *Anxa6^{em1(GFP)}* mice were injected with 1mg/kg prednisone or control DMSO into the intraperitoneal cavity. Twenty-four hours post injection, myofibers were isolated and subjected to laser injury. Annexin A6GFP cap size was reduced in myofibers treated with prednisone compared to controls (Figure 24), indicating responsiveness of annexin A6GFP protein to steroid administration. This illustrated the potential of external agents to modify endogenous annexin A6 expression to elicit protection against injury.

**Recombinant annexin A6 promotes skeletal muscle repair.** It was shown that recombinant annexin A6 protected against laser-induced injury by reducing FM dye uptake in healthy and dystrophic myofibers. To further elucidate the mechanism by which recombinant annexin A6 confers protection, *Anxa6^{em1(GFP)}* myofibers were pretreated with recombinant annexin A6 protein or control. Subsequently, myofibers were incubated in FM 4-64 dye and subjected to laser injury. Myofibers pretreated with recombinant annexin A6 had smaller annexin A6GFP repair caps and a concomitant reduction in FM dye uptake, compared to control myofibers (Figure 25). These data indicated that recombinant annexin A6 participates in orchestrating the endogenous repair complex, enhancing membrane protection and repair, reducing membrane leak.

**Annexin A6GFP is expressed in the heart and forms repair caps in injured cardiomyocytes.** Annexin A6 was identified as a modifier of membrane leak in the heart, however, methods to evaluate cardiomyocyte repair were previously lacking. It was hypothesized that if endogenous annexin A6GFP was expressed in the heart that this new *Anxa6^{em1(GFP)}* mouse model could be used as a tool to evaluate endogenous cardiomyocyte membrane repair. To determine if endogenous annexin A6GFP played a role in membrane repair in tissues beyond skeletal muscle, *Anxa6^{em1(GFP)}* cardiomyocytes were isolated and subjected to laser injury. Endogenous annexin A6GFP localized in a sarcomeric pattern in live, isolated cardiomyocytes, similar to skeletal myofibers (Figure 26). Within seconds of laser-induced damage, annexin A6GFP localized to the membrane lesion organizing into a repair cap in the cardiomyocyte (Figure 26, white arrow). A magnified image of the white dotted box depicting a bright annexin A6GFP repair cap is shown in the image on the right (Figure 26). Timelapse images illustrating the progression of annexin A6GFP localization into the repair cap (arrow) in an isolated cardiomyocyte is shown up to 50 seconds post injury (Figure 26). These data showed that endogenous annexin A6GFP localizes to the site of membrane injury forming a repair cap at the membrane lesion in live, adult cardiomyocytes, suggesting a broad role for annexin A6 in membrane repair.

**Annexin A6 enhances repair in a model of Limb Girdle Muscular Dystrophy 2B (LGMD2B).** Dysferlin is a Ca2+-binding, membrane-associated protein implicated in membrane repair. Loss-of-function mutations is dysferlin reduce membrane repair capacity resulting in Limb Girdle Muscular Dystrophy 2B (LGMD2B). It was hypothesized that agents that enhance repair or reduce injury susceptibility could provide therapeutic benefit for treating LGMD2B. To test this hypothesis, mice lacking dysferlin on the 129 background (Dysf129) were electroporated with annexin A6 plasmid and then were subjected to laser injury in the presence of FM 4-64. Myofibers overexpressing annexin A6 had a significant reduction in FM 4-64 dye uptake after injury (Figure 27A). Since plasmid overexpression protected dysferlin-null myofibers from injury, we next tested the effectiveness of recombinant annexin A6. To determine if recombinant annexin A6 could protect against membrane insult in dystrophic, dysferlin-null muscle, myofibers from Dysf129 mice were isolated and incubated with recombinant annexin A6 or vehicle control. Laser injury was conducted in the presence of FM 4-64 to visualize the extent of injury. Treatment with extracellular recombinant annexin A6 reduced FM 4-64 fluorescence area compared to vehicle control-treated myofibers (Figure 27B). These data indicated that annexin A6 enhances repair and protects against injury of dystrophic, LGMD2B myofibers.

**Recombinant annexin A6 localizes to the sarcolemma in a model of Limb Girdle Muscular Dystrophy 2C (LGMD2C).** Since recombinant annexin A6 protected against membrane injury, the localization ability of recombinant annexin A6, administered systemically, was assessed in the *Sgcg-null* mouse model of LGMD2C compared to wildtype controls. *Sgcg-null* mice lack γ-sarcoglycan, an integral membrane component of the dystrophin glycoprotein complex required for membrane stability and function. Recombinant annexin A6 localized to the muscle membrane of chronically injured, *Sgcg-null* mice, visualized by strong anti-HIS immunofluorescence signal colocalizing with anti-laminin membrane staining (Figure 28). In comparison, minimal sarcolemma anti-HIS fluorescence was present in uninjured, wildtype muscle membrane (Figure 28). These data illustrated that recombinant annexin A6 localizes to the membrane of chronically injured muscle.

### Example 5

This example details the production of recombinant annexin A6 protein (rANXA6) expressed from plasmids in both mammalian cells and prokaryotic cells.

### Production of rANXA6protein in mammalian cells

rANXA6 protein production was carried out in a total volume of 4 liters using two mammalian cell lines (Expi293F^{™} Cells and Freestyle^{™} CHO-S Cells, ThermoFisher Scientific). The estimated yield (as determined by Coomassie staining (Figure 29)) was 15 mg/L from the Expi293F^{™} Cells and 2.3 mg/L from the Freestyle^{™} CHO-S Cells. The recombinant protein was also evaluated by immunoblot analysis to detect annexin A6 and an anti-HIS antibody to detect the C-terminal HIS tag found on the recombinantly-produced rANXA6 protein (Figure 29).

The buffers and Immobilized metal affinity chromatography (IMAC) protocols are indicated below in Table 1.

**Table 1. Pellet processing buffers**

| **Buffers - Lysis and IMAC** | |
|---|---|
| **Pellet re-suspension buffer** (5 ml cell pellet re-suspended in 1 ml lysis buffer) | 50 mM Tris-HCl pH 7.5 |
| | 150 mM NaCl |
| | 5mM Imidazole |
| | 1%TX-100 |
| | 1 mM DTT |
| | 1mM EGTA |
| | 1 mM PMSF |
| | 1 Roche Protease tablet per |
| | 50 ml buffer |
| **IMAC pulldown Buffer A** (IMAC wash & equilibration buffer) | 25 mM Tris-HCl pH 7.5 |
| | 150 mM NaCl |
| | 5 mM Imidazole |
| | 1 mM DTT |
| | 1 mM PMSF |
| | 1 Roche Protease tablet per |
| | 50 ml buffer |
| **IMAC pulldown Buffer B** (elution buffer) | 25 mM Tris-HCl pH 7.5, 150 mM NaCl |
| | 500 mM Imidazole, 1 mM DTT |
| | 1 mM PMSF |
| | 1 Roche Protease tablet per |
| | 50 ml buffer |
| **Column type** | PhyTip^{™} Micro-Scale Affinity |
| | Column (10µl resin) |

**Purification of Annexin 6 (ANXA6) from Expi293F^{™} Cells.** The protein lysate was loaded on to an IMAC column and eluted by 500 mM imidazole. The yield at this stage was determined to be approximately 270 mg. The relevant fractions were pooled and loaded on to an Ion Exchange Q (IExQ) column, and the indicated fractions were pooled. At this stage the yield was approximately 208 mg. These pools were loaded on to a S200 26/60 column in 1X PBS. The final yield was approximately 77 mg. The purity of the recombinant protein was determined to be greater than 95%.

Absolute size exclusion chromatography (aSEC) indicated that the protein was in a monomeric state, and mass spectrometry (MS) showed multiple modifications.

**Purification of Annexin 6 (ANXA6) from Freestyle^{™} CHO-S Cells.** The protein lysate was loaded on to an IMAC column and eluted by 500 mM imidazole. The yield at this point was approximately 78 mg. The relevant fractions were pooled and loaded on to an Ion Exchange Q (IExQ) column, and the indicated fractions were combined in two separate pools. The yield at this stage was approximately 24 mg (approximately 12 mg in each pool). The pools were run on an S200 26/60 column (SEC Run for Pool1 and Pool2). The final yield was determined to be: Pool1 = 2.25 mg and Pool2 = 1.9 mg.

Absolute size exclusion chromatography (aSEC) and MS did not show any difference between Pool1 and Pool2, and no difference was detected between the recombinant proteins expressed in Expi293F^{™} Cells and Freestyle^{™} CHO-S Cells.

### Production of rANXA6 protein in prokaryotic cells

The rANXA6 protein was also purified from prokaryotic cells. rANXA6 protein production was carried out in a 4 liter low endotoxin purification method in *E. coli* cells (Rosetta(DE3) competent cells) in TB media. The cells were grown at 30 C for 4 hours and 18 hours. Isopropyl β-D-1-thiogalactopyranoside (IPTG) inducer was used at 0.4 mM. The composition of the various buffers used is shown in Table 2. The total yield obtained using the method was 308 mg (approximately 2.5 mg/mL).

**Table 2. Buffer compositions.**

| **Buffer** | **Composition** |
|---|---|
| Lysis | 50 mM Tris-HCl pH 7.5, 150 mM NaCl, 5 mM Imidazole, 1% TX-100, 1 mM DTT, 1 mM EGTA, 1 mM PMSF, PI tab |
| Wash | 25 mM Tris-HCl pH 7.5, 150 mM NaCl, 5 mM Imidazole, 1 mM DTT, 1 mM PMSF, PI tablet |
| Elution | 50 mM Tris-HCl pH 7.5, 150 mM NaCl, 500 mM Imidazole, 1% TX-100, 1 mM DTT, 1 mM PMSF, PI tablet |

The recombinant protein was analyzed by Coomassie staining and immunoblot analysis, as shown in Figure 30.

**Purification of Annexin 6 (ANXA6) from *E.* coli Cells.** The protein lysate was loaded on to an IMAC column and eluted by 500 mM imidazole. The yield at this stage was determined to be approximately 546 mg. The relevant fractions were pooled and loaded on to an Ion Exchange Q (IExQ) column, and the indicated fractions were pooled. The yield at this stage was approximately 408 mg. These pools were loaded on to a S200 26/60 column in 1X PBS. After three runs the final yield was approximately 306 mg. The final endotoxin level was approximately 4 EU/mg/10.5 EU/mL. The purity of the recombinant protein was determined to be greater than 95%.

Absolute size exclusion chromatography (aSEC) indicated that the protein was in a monomeric state, and mass spectrometry (MS) showed multiple modifications.

### Example 6

This example details experiments comparing the function of rANXA6 produced in mammalian cells with rANXA6 produced in prokaryotic cells. These experiments were conducted to test whether the activity of an annexin protein (in this case, rANXA6) differed based on whether the annexin protein was produced in mammalian cells versus prokaryotic cells. The predicted posttranslational modification signature for annexin A6 was determined using PTMcode (http://ptmcode.embl.de) and is shown in Table 3. Various forms of posttranslational modification (PTM) identified through mass spectrometry (MS) (*e.g*., acetylation, nitrosylation, phosphorylation) are indicated in Table 3.

**Table 3. Posttranslational modifications of annexin A6 protein expressed in prokaryotic cells and mammalian cells.**

| **PTMcode prediction** | **AA** | **POSITION** | **Type** | **CHO** | ***E coli*⁴** | **CHO (Batch 1)⁵** | **CHO (Batch 2)⁵** | ***E coli* (Batch 1)⁵** | ***E coli* (Batch 2)⁵** | **HEK (Batch 1)⁵** | **HEK (Batch 2)⁵** |
|---|---|---|---|---|---|---|---|---|---|---|---|
| Acetylation³ | A³ | 2³ | | | | | | | | | |
| acetylation¹ | K¹ | 34¹ | | x¹ | x¹ | x¹ | x¹ | x¹ | x¹ | x¹ | x¹ |
| acetylation¹ | K¹ | 40¹ | | x¹ | | | | | | | |
| acetylation² | K² | 63² | | | x² | | | | | | |
| acetylation² | K² | 68² | | | x² | | | | | | |
| acetylation¹ | K¹ | 75¹ | | | x¹ | | | x¹ | x¹ | | |
| acetylation² | K² | 81² | | | x² | | | x² | x² | | |
| acetylation¹ | K¹ | 99¹ | | | x¹ | | | | | | |
| acetylation¹ | K¹ | 102¹ | | | | x¹ | | x¹ | x¹ | x¹ | |
| acetylation¹ | K¹ | 113¹ | | | x¹ | x¹ | | x¹ | x¹ | x¹ | |
| acetylation¹ | K¹ | 156¹ | | x¹ | | | | | | | |
| acetylation¹ | K¹ | 220¹ | | | x¹ | | | | | | |
| acetylation¹ | K¹ | 240' | | | x¹ | | | x¹ | x¹ | | |
| acetylation³ | K³ | 265³ | | | | | | | | | |
| acetylation² | K² | 306² | | | x² | | | x² | | | |
| acetylation³ | K³ | 314³ | | | | | | | | | |
| acetylation¹ | K¹ | 319¹ | | | x¹ | | | x¹ | | x¹ | |
| acetylation¹ | K¹ | 354¹ | | | x¹ | | | | | | |
| acetylation¹ | K¹ | 370¹ | | | x¹ | | | | | | |
| acetylation¹ | K¹ | 377¹ | | | x¹ | | | | x¹ | | |
| acetylation² | K² | 418² | | | x² | | | x² | x² | x² | |
| acetylation³ | K³ | 445³ | | | | | | | | | |
| acetylation¹ | K¹ | 446' | | | | | | | x¹ | | |
| acetylation³ | K³ | 483³ | | | | | | | | | |
| acetylation¹ | K¹ | 540¹ | | | x¹ | | | | | | |
| acetylation¹ | K¹ | 598' | | | x¹ | | x¹ | | x¹ | x¹ | x¹ |
| acetylation¹ | K¹ | 600' | | x¹ | x¹ | | x¹ | | | | x¹ |
| acetylation¹ | K¹ | 607¹ | | x¹ | x¹ | | | | x¹ | | |
| acetylation² | K² | 613² | | | | x² | | | | | |
| acetylation² | K² | 620² | | x² | x² | | | | x² | | |
| acetylation¹ | K¹ | 647¹ | | | x¹ | | | | | x¹ | |
| nitrosylation³ | C³ | 96³ | | | | | | | | | |
| nitrosylation³ | C³ | 114³ | | | | | | | | | |
| nitrosylation³ | C³ | 552³ | | | | | | | | | |
| phosphorylation³ | Y³ | 30³ | | | | | | | | | |
| phosphorylation³ | S3 | 51³ | | | | | | | | | |
| phosphorylation³ | Y³ | 95³ | | | | | | | | | |
| phosphorylation³ | S³ | 106³ | | | | | | | | | |
| phosphorylation³ | T3 | 110³ | | | | | | | | | |
| phosphorylation³ | Y³ | 201³ | | | | | | | | | |
| phosphorylation³ | S³ | 229³ | | | | | | | | | |
| phosphorylation³ | S³ | 251³ | | | | | | | | | |
| phosphorylation³ | T3 | 269³ | | | | | | | | | |
| phosphorylation¹ | T¹ | 273¹ | | | | | x¹ | | | | |
| phosphorylation¹ | S¹ | 321¹ | | x¹ | x¹ | | | | | | |
| phosphorylation¹ | Y¹ | 340¹ | | | x¹ | | | | | | |
| phosphorylation² | T2 | 381² | | | | | | | | | x² |
| phosphorylation³ | T3 | 391³ | | | | | | | | | |
| phosphorylation³ | T3 | 464³ | | | | | | | | | |
| phosphorylation³ | Y³ | 609³ | | | | | | | | | |
| phosphorylation³ | S³ | 628³ | | | | | | | | | |
| ubiquitination¹ | K¹ | 9¹ | | | | | | | | x¹ | |
| ubiquitination¹ | K¹ | 40¹ | | x¹ | x¹ | | | | x¹ | | |
| ubiquitination¹ | K¹ | 75¹ | | | x¹ | | x¹ | | | | |
| ubiquitination³ | K³ | 102³ | | | | | | | | | |
| ubiquitination¹ | K¹ | 156¹ | | x¹ | x¹ | | | | x¹ | | x¹ |
| ubiquitination¹ | K¹ | 220¹ | | | x¹ | | | | | | |
| ubiquitination¹ | K¹ | 240¹ | | | x¹ | | x¹ | | x¹ | | x¹ |
| ubiquitination¹ | K¹ | 319¹ | | | x¹ | | | | x¹ | | |
| ubiquitination¹ | K¹ | 354¹ | | | x¹ | | | | | | |
| ubiquitination¹ | K¹ | 370¹ | | | x¹ | | | | | | |
| ubiquitination² | K² | 377² | | | x | | | | | | |
| ubiquitination¹ | K¹ | 418¹ | | | x¹ | | x¹ | | x¹ | | x¹ |
| ubiquitination³ | K³ | 478³ | | | | | | | | | |
| ubiquitination¹ | K¹ | 483¹ | | | | | | | x¹ | | |
| ubiquitination¹ | K¹ | 540¹ | | | x¹ | | | | | | |
| ubiquitination¹ | K¹ | 568¹ | | | | | | | x¹ | | |
| ubiquitination¹ | K¹ | 580¹ | | | | | x¹ | | x¹ | | x¹ |
| ubiquitination¹ | K¹ | 598¹ | | | x¹ | | x¹ | | | | x¹ |
| ubiquitination¹ | K¹ | 600¹ | | x¹ | x¹ | | x¹ | | | | x¹ |
| ubiquitination | K¹ | 607¹ | | x¹ | x¹ | | | | x¹ | | x¹ |
| ubiquitination | K¹ | 620¹ | | x¹ | x¹ | | x¹ | | | x¹ | |
| ubiquitination | K¹ | 647¹ | | | x¹ | | | | | | |
| methylation¹ | R¹ | 11¹ | di¹ | | x¹ | | x¹ | x¹ | x¹ | | x¹ |
| methylation¹ | K¹ | 34¹ | mono/tri¹ | x¹ | x¹ | x¹ | x¹ | x¹ | x¹ | | x¹ |
| methylation¹ | K¹ | 40¹ | mono¹ | | | x¹ | | x¹ | x¹ | x¹ | |
| methylation¹ | K¹ | 63¹ | mono¹ | | | | | x¹ | | | |
| methylation¹ | K¹ | 68¹ | di¹ | | | x¹ | | | | | |
| methylation¹ | K¹ | 99¹ | mono¹ | | x¹ | | | x¹ | | | |
| methylation¹ | K¹ | 102¹ | di¹ | | | x¹ | | | | | |
| methylation¹ | K¹ | 113¹ | mono/tri¹ | | x¹ | x¹ | x¹ | x¹ | x¹ | x¹ | x¹ |
| methylation¹ | R¹ | 122¹ | mono¹ | | x¹ | | | | | | |
| methylation¹ | R¹ | 140¹ | mono¹ | | x¹ | | | | x¹ | x¹ | |
| methylation¹ | K¹ | 156¹ | di¹ | x¹ | x¹ | | | | x¹ | x¹ | |
| methylation¹ | R¹ | 166¹ | di¹ | | x¹ | | | x¹ | x¹ | | |
| methylation¹ | K¹ | 191¹ | mono¹ | x¹ | x¹ | | | | | | |
| methylation¹ | K¹ | 231¹ | mono¹ | | | | x¹ | | | | |
| methylation¹ | K/R¹ | 319¹ | tri¹ | | x¹ | | | | | x¹ | |
| methylation¹ | R¹ | 368¹ | di¹ | | | | | x¹ | | | |
| methylation¹ | K¹ | 370¹ | tri¹ | | x¹ | | | | | | |
| methylation¹ | K/R¹ | 418¹ | mono/di¹ | | | | | x¹ | | x¹ | |
| methylation¹ | K¹ | 456¹ | mono¹ | | | | | | x¹ | | |
| methylation¹ | K¹ | 478¹ | mono¹ | x¹ | x¹ | | | x¹ | | | |
| methylation¹ | K¹ | 483¹ | tri¹ | x¹ | x¹ | | | x¹ | | | |
| methylation¹ | K/R¹ | 520¹ | di¹ | | x¹ | | | x¹ | | | |
| methylation¹ | R¹ | 540¹ | mono/di¹ | | x¹ | x¹ | x¹ | x¹ | x¹ | x¹ | x¹ |
| methylation¹ | R¹ | 546¹ | mono/di¹ | | x¹ | x¹ | | | x¹ | x¹ | |
| methylation¹ | R¹ | 554¹ | mono¹ | | x¹ | x¹ | | | x¹ | x¹ | |
| methylation¹ | K¹ | 579¹ | mono¹ | | | | | | x¹ | | |
| methylation¹ | K/R¹ | 587¹ | mono/di¹ | | x¹ | | | x¹ | x¹ | | |
| methylation¹ | K¹ | 598¹ | di¹ | | x¹ | | | x¹ | | | |
| methylation¹ | K¹ | 600¹ | di¹ | | | | | x¹ | | | |
| methylation¹ | K¹ | 620¹ | di¹ | | | | | x¹ | | | |
| methylation¹ | K¹ | 639¹ | mono¹ | | | | | | | | x¹ |
| methylation¹ | K¹ | 644¹ | tri¹ | | x¹ | | | | | | |
| methylation¹ | K¹ | 647¹ | tri¹ | | x¹ | | | | | x¹ | |
| N-linked | | | | | | | None | | None | | None |
| glycosylation | | | | NA | NA | NA | found | NA | found | NA | found |

| | | | | | | | | | | | |
|---|---|---|---|---|---|---|---|---|---|---|---|
| ¹ = identified by mass spectrometry ² = *in silico* predicted and detected by mass spectrometry ³ = *in silico* predicted but not detected ⁴ = results in this column were obtained using annexin A6 protein obtained from R&D Systems, Inc. (Minneapolis, MN) catalog number 5186-A6-050 ⁵ = results in these columns were obtained using annexin A6 protein obtained from a contract research organization (CRO; Evotec SE (Princeton, NJ)) (see Example 5 for general protocols) Batch 1 = small milliliter (mL) sized production Batch 2 = medium liter-sized production | | | | | | | | | | | |

As shown in Table 3, the posttranslational modification signature of annexin A6 differs depending on whether the protein is expressed in prokaryotic cells or mammalian cells. It would be expected, therefore, that the altered posttranslational modification signature would result in a change in activity between the annexin A6 protein produced in prokaryotic cells versus mammalian cells. Unexpectedly, however, it was found that the annexin A6 protein produced in prokaryotic cells performed as well as the protein that was produced in mammalian cells in the myofiber injury experiments described below.

**Myofiber isolation, laser injury, and dye measurement.** For recombinant myofibers studies, myofibers were isolated from wildtype mice. Myofibers were incubated in Ringer's media with 13µg/ml or 130µg/ml human recombinant annexin, *E coli* or HEK cell produced. Bovine serum albumin (BSA) was used as a negative control. FM 4-64 (2.5µm) was added to the myofibers just prior to imaging. Fibers were subjected to laser-induced damage at room temperature using the Nikon A1R-MP multiphoton microscope. Imaging was performed using a 25×1.1NA objective directed by the NIS-Elements AR imaging software. FM 4-64 was excited using a 920nm wavelength laser and emission wavelengths of 575nm and 629nm were collected, respectively. To induce laser damage on isolated myofibers, a diffraction limited spot (diameter approximately 410nm) was created on the lateral membrane of the myofiber using a 920nm wavelength laser at 10-15% laser power for 1 second. FM 4-64 area was measured using FIJI from a single slice near the middle of the z-stack. Myofiber quality control was based on a number of characteristics including using adherent myofibers with intact sarcomere structure. Myofibers appeared devoid of tears or ruptures induced during the isolation protocol. The region of the myofiber selected for damage was linear and not located on a nucleus or neuromuscular junction.

Statistical analyses were performed with Prism (Graphpad, La Jolla, CA). Comparisons relied on ANOVA (1way ANOVA for 1 variable). P-values of less than or equal to 0.05 were considered significant. Error bars represent +/- standard error of the mean (SEM). Results are shown in Figure 31.

These experiments demonstrated that myofibers injured in the presence of extracellular recombinant annexin A6 (rANXA6), produced in *E coli* or mammalian cells, had similar beneficial effects on enhancing membrane repair and protecting against injury. This was demonstrated across multiple doses, 13mg/ml and 130mg/ml, measured as a reduction in FM 4-64 dye uptake after laser injury compared to BSA control treated myofibers.

### Example 7

This example was designed to test the necessity of the amino acid sequence VAAEIL (exon 21 of annexin A6) (SEQ ID NO: 47) in facilitating the localization and organization of the annexin repair cap at the site of membrane injury.

### Methods

**Animals.** Wildtype mice from the 129T2/SvEmsJ background were bred and housed in a specific pathogen free facility on a 12-hour light/dark cycle and fed ad libitum in accordance with the Northwestern University's Institutional Animal Care and Use Committee regulations. 129T2/SvEmsJ (129T2) mice were purchased from the Jackson Laboratory (Ben Harbor, ME; Stock # 002065). Two to three-month-old male and female were used for all wildtype mouse experiments.

**Plasmids.** Plasmids encoding annexin A6 with a carboxyl-terminal turboGFP tag were obtained from Origene (Rockville, MD). Subcloning of annexin A6 to replace the GFP tag with tdTomato (Addgene) was performed by Mutagenix (Suwanee, GA). Site directed mutagenesis was performed by Mutagenix on annexin A6-GFP to generate the construct lacking the VAAEIL (SEQ ID NO: 47) sequence. Plasmid DNA was isolated using the Qiagen endo-free Maxi prep kit (Qiagen #12362).

**Electroporation, myofiber isolation, laser injury, cap and vesicle measurement.** Flexor digitorum brevis (FDB) fibers were transfected with endo-free plasmid DNA by *in vivo* electroporation. Methods were described previously in Demonbreun et al JoVE 2015. Briefly, the hindlimb footpad was injected with 10µl of hyaluronidase (8units) (H4272, Sigma, St. Louis, MO). Two hours post injection up to 20µl of 2µg/µl endotoxin free plasmid was injected into the footpad. Electroporation was conducted by applying 20 pulses, 20 ms in duration/each, at 1Hz, at 100 V/cm. Animals were allowed to recover for a minimum of seven days and not more than ten days after electroporation to avoid examining injured muscle and to allow sufficient time for plasmid expression.

FDB muscle was removed and individual myofibers were isolated and imaged (Demonbreun et al., JoVE, 2015). Fibers were dissected and laser damaged as described (Demonbreun et al., JoVE2015; Swaggart et al., PNAS 2014). Briefly, fibers were dissociated in 0.2% BSA plus collagenase type II (Cat # 17101, Life Technologies, Grand Island, NY) for up to 90 minutes with intermittent trituration at 37 degrees in 10% CO₂. Fibers were then moved to Ringers solution and placed on MatTek confocal microscopy dishes (Cat # P35G-1.5-14-C, MatTek, Ashland MA). After one hour, fibers were adhered and the Ringers was replaced with 1ml of fresh Ringers solution. Imaging and ablation was performed on the Nikon A1R laser scanning confocal equipped with GaSP detectors through a 60x Apo lambda 1.4 NA objective driven by Nikon Elements AR software. A single pixel set as 120 nm (0.0144 µm²) was ablated using the 405 nm laser at 100% power for up to 5 seconds. Z-stack projections were acquired from consecutive acquisitions after the final time-lapse frame, approximately 4 minutes post damage, with a 0.125µM step size between slices. Z-stack renderings were constructed in FIJI. Fibers expressing similar levels of tagged protein were compared.

### Results

It was then questioned whether the presence of the VAAEIL (SEQ ID NO: 47) sequence in annexin A6 influenced annexin A6 localization upon muscle membrane injury. Myofibers were electroporated with full-length annexin A6-tdTomato and A6-GFP lacking VAAEIL (SEQ ID NO: 47) and subsequently laser-injured. Annexin A6 isoforms with and without the VAAEIL (SEQ ID NO: 47) sequence localized to the site of muscle membrane injury forming a repair cap (Figure 32). Annexin A6 lacking VAAEIL (SEQ ID NO: 47) partially colocalized with full-length annexin A6, forming a flatter repair cap at the membrane lesion. These data demonstrated that multiple annexin A6 isoforms, with and without the amino acids VAAEIL, participate in the muscle injury response.

### ADDITIONAL REFERENCES

Akerboom, J., T.W. Chen, T.J. Wardill, L. Tian, J.S. Marvin, S. Mutlu, N.C. Calderon, F. Esposti, B.G. Borghuis, X.R. Sun, A. Gordus, M.B. Orger, R. Portugues, F. Engert, J.J. Macklin, A. Filosa, A. Aggarwal, R.A. Kerr, R. Takagi, S. Kracun, E. Shigetomi, B.S. Khakh, H. Baier, L. Lagnado, S.S. Wang, C.I. Bargmann, B.E. Kimmel, V. Jayaraman, K. Svoboda, D.S. Kim, E.R. Schreiter, and L.L. Looger. 2012. Optimization of a GCaMP calcium indicator for neural activity imaging. J Neurosci. 32:13819-13840.
Avila-Sakar, A.J., C.E. Creutz, and R.H. Kretsinger. 1998. Crystal structure of bovine annexin VI in a calcium-bound state. Biochimica et biophysica acta. 1387:103-116.
Babbin, B.A., M.G. Laukoetter, P. Nava, S. Koch, W.Y. Lee, C.T. Capaldo, E. Peatman, E.A. Severson, R.J. Flower, M. Perretti, C.A. Parkos, and A. Nusrat. 2008. Annexin A1 regulates intestinal mucosal injury, inflammation, and repair. J Immunol. 181:5035-5044.
Bansal, D., K. Miyake, S.S. Vogel, S. Groh, C.C. Chen, R. Williamson, P.L. McNeil, and K.P. Campbell. 2003. Defective membrane repair in dysferlin-deficient muscular dystrophy. Nature. 423:168-172.
Bashir, R., S. Britton, T. Strachan, S. Keers, E. Vafiadaki, M. Lako, I. Richard, S. Marchand, N. Bourg, Z. Argov, M. Sadeh, I. Mahjneh, G. Marconi, M.R. Passos-Bueno, S. Moreira Ede, M. Zatz, J.S. Beckmann, and K. Bushby. 1998. A gene related to Caenorhabditis elegans spermatogenesis factor fer-1 is mutated in limb-girdle muscular dystrophy type 2B. Nat Genet. 20:37-42.
Bement, W.M., C.A. Mandato, and M.N. Kirsch. 1999. Wound-induced assembly and closure of an actomyosin purse string in Xenopus oocytes. Curr Biol. 9:579-587.
Benz, J., A. Bergner, A. Hofmann, P. Demange, P. Gottig, S. Liemann, R. Huber, and D. Voges. 1996. The structure of recombinant human annexin VI in crystals and membrane-bound. J Mol Biol. 260:638-643.
Bi, G.Q., J.M. Alderton, and R.A. Steinhardt. 1995. Calcium-regulated exocytosis is required for cell membrane resealing. The Journal of cell biology. 131:1747-1758.
Bizzarro, V., A. Petrella, and L. Parente. 2012. Annexin A1: novel roles in skeletal muscle biology. J Cell Physiol. 227:3007-3015.
Blackwood, R.A., and J.D. Ernst. 1990. Characterization of Ca2(+)-dependent phospholipid binding, vesicle aggregation and membrane fusion by annexins. The Biochemical journal. 266:195-200.
Boye, T.L., J.C. Jeppesen, K. Maeda, W. Pezeshkian, V. Solovyeva, J. Nylandsted, and A.C. Simonsen. 2018. Annexins induce curvature on free-edge membranes displaying distinct morphologies. Sci Rep. 8:10309.
Boye, T.L., K. Maeda, W. Pezeshkian, S.L. Sonder, S.C. Haeger, V. Gerke, A.C. Simonsen, and J. Nylandsted. 2017. Annexin A4 and A6 induce membrane curvature and constriction during cell membrane repair. Nat Commun. 8:1623.
Buzhynskyy, N., M. Golczak, J. Lai-Kee-Him, O. Lambert, B. Tessier, C. Gounou, R. Berat, A. Simon, T. Granier, J.M. Chevalier, S. Mazeres, J. Bandorowicz-Pikula, S. Pikula, and A.R. Brisson. 2009. Annexin-A6 presents two modes of association with phospholipid membranes. A combined QCM-D, AFM and cryo-TEM study. Journal of structural biology. 168:107-116.
Cai, C., H. Masumiya, N. Weisleder, N. Matsuda, M. Nishi, M. Hwang, J.K. Ko, P. Lin, A. Thornton, X. Zhao, Z. Pan, S. Komazaki, M. Brotto, H. Takeshima, and J. Ma. 2009. MG53 nucleates assembly of cell membrane repair machinery. Nat Cell Biol. 11:56-64.
Carmeille, R., S.A. Degrelle, L. Plawinski, F. Bouvet, C. Gounou, D. Evain-Brion, A.R. Brisson, and A. Bouter. 2015. Annexin-A5 promotes membrane resealing in human trophoblasts. Biochimica et biophysica acta. 1853:2033-2044.
Cagliani, R., F. Magri, A. Toscano, L. Merlini, F. Fortunato, C. Lamperti, C. Rodolico, A. Prelle, M. Sironi, M. Aguennouz, P. Ciscato, A. Uncini, M. Moggio, N. Bresolin, and G.P. Comi. 2005. Mutation finding in patients with dysferlin deficiency and role of the dysferlin interacting proteins annexin A1 and A2 in muscular dystrophies. Human mutation. 26:283.
Ceco, E., S. Bogdanovich, B. Gardner, T. Miller, A. DeJesus, J.U. Earley, M. Hadhazy, L.R. Smith, E.R. Barton, J.D. Molkentin, and E.M. McNally. 2014. Targeting latent TGFbeta release in muscular dystrophy. Science translational medicine. 6:259ra144.
Christmas, P., J. Callaway, J. Fallon, J. Jones, and H.T. Haigler. 1991. Selective secretion of annexin 1, a protein without a signal sequence, by the human prostate gland. The Journal of biological chemistry. 266:2499-2507.
Davenport, N.R., K.J. Sonnemann, K.W. Eliceiri, and W.M. Bement. 2016. Membrane dynamics during cellular wound repair. Mol Biol Cell. 27:2272-2285.
Defour, A., S. Medikayala, J.H. Van der Meulen, M.W. Hogarth, N. Holdreith, A. Malatras, W. Duddy, J. Boehler, K. Nagaraju, and J.K. Jaiswal. 2017. Annexin A2 links poor myofiber repair with inflammation and adipogenic replacement of the injured muscle. Human molecular genetics. 26:1979-1991.
de Laat, B., R.H. Derksen, I.J. Mackie, M. Roest, S. Schoormans, B.J. Woodhams, P.G. de Groot, and W.L. van Heerde. 2006. Annexin A5 polymorphism (-1C->T) and the presence of anti-annexin A5 antibodies in the antiphospholipid syndrome. Annals of the rheumatic diseases. 65:1468-1472.
Demonbreun, A.R., M.V. Allen, J.L. Warner, D.Y. Barefield, S. Krishnan, K.E. Swanson, J.U. Earley, and E.M. McNally. 2016a. Enhanced Muscular Dystrophy from Loss of Dysferlin Is Accompanied by Impaired Annexin A6 Translocation after Sarcolemmal Disruption. Am J Pathol. 186:1610-1622.
Demonbreun, A.R., and E.M. McNally. 2015. DNA Electroporation, Isolation and Imaging of Myofibers. Journal of visualized experiments : JoVE. 106:e53551.
Demonbreun, A.R., and E.M. McNally. 2016. Plasma Membrane Repair in Health and Disease. Curr Top Membr. 77:67-96.
Demonbreun, A.R., M. Quattrocelli, D.Y. Barefield, M.V. Allen, K.E. Swanson, and E.M. McNally. 2016b. An actin-dependent annexin complex mediates plasma membrane repair in muscle. The Journal of cell biology. 213:705-718.
Demonbreun, A.R., A.E. Rossi, M.G. Alvarez, K.E. Swanson, H.K. Deveaux, J.U. Earley, M. Hadhazy, R. Vohra, G.A. Walter, P. Pytel, and E.M. McNally. 2014. Dysferlin and myoferlin regulate transverse tubule formation and glycerol sensitivity. Am J Pathol. 184:248-259.
Demonbreun, A.R., K.E. Swanson, A.E. Rossi, H.K. Deveaux, J.U. Earley, M.V. Allen, P. Arya, S. Bhattacharyya, H. Band, P. Pytel, and E.M. McNally. 2015. Eps 15 Homology Domain (EHD)-1 Remodels Transverse Tubules in Skeletal Muscle. PLoS One. 10:e0136679.
Deora, A.B., G. Kreitzer, A.T. Jacovina, and K.A. Hajjar. 2004. An annexin 2 phosphorylation switch mediates p11-dependent translocation of annexin 2 to the cell surface. The Journal of biological chemistry. 279:43411-43418.
DiFranco, M., M. Quinonez, J. Capote, and J. Vergara. 2009. DNA transfection of mammalian skeletal muscles using in vivo electroporation. Journal of visualized experiments : JoVE. 32.
Duann, P., H. Li, P. Lin, T. Tan, Z. Wang, K. Chen, X. Zhou, K. Gumpper, H. Zhu, T. Ludwig, P.J. Mohler, B. Rovin, W.T. Abraham, C. Zeng, and J. Ma. 2015. MG53-mediated cell membrane repair protects against acute kidney injury. Science translational medicine. 7:279ra236.
Echigoya Y, Lim KRQ, Nakamura A, and Yokota T. Multiple Exon Skipping in the Duchenne Muscular Dystrophy Hot Spots: Prospects and Challenges. J Pers Med 8, 2018.
Fiehn, W., J.B. Peter, J.F. Mead, and M. Gan-Elepano. 1971. Lipids and fatty acids of sarcolemma, sarcoplasmic reticulum, and mitochondria from rat skeletal muscle. The Journal of biological chemistry. 246:5617-5620.
Flanigan, K.M., E. Ceco, K.M. Lamar, Y. Kaminoh, D.M. Dunn, J.R. Mendell, W.M. King, A. Pestronk, J.M. Florence, K.D. Mathews, R.S. Finkel, K.J. Swoboda, E. Gappmaier, M.T. Howard, J.W. Day, C. McDonald, E.M. McNally, and R.B. Weiss. 2013. LTBP4 genotype predicts age of ambulatory loss in duchenne muscular dystrophy. Ann Neurol. 73:481-488.
Gerke, V., C.E. Creutz, and S.E. Moss. 2005. Annexins: linking Ca2+ signalling to membrane dynamics. Nat Rev Mol Cell Biol. 6:449-461.
Gerke, V., and S.E. Moss. 2002. Annexins: from structure to function. Physiol Rev. 82:331-371.
Grewal, T., M. Hoque, J.R.W. Conway, M. Reverter, M. Wahba, S.S. Beevi, P. Timpson, C. Enrich, and C. Rentero. 2017. Annexin A6-A multifunctional scaffold in cell motility. Cell Adh Migr. 11:288-304.
Goulet, F., K.G. Moore, and A.C. Sartorelli. 1992. Glycosylation of annexin I and annexin II. Biochemical and biophysical research communications. 188:554-558.
Hack AA, et al. Gamma-sarcoglycan deficiency leads to muscle membrane defects and apoptosis independent of dystrophin. J Cell Biol. 1998;142(5):1279-1287.
Hannon, R., J.D. Croxtall, S.J. Getting, F. Roviezzo, S. Yona, M.J. Paul-Clark, F.N. Gavins, M. Perretti, J.F. Morris, J.C. Buckingham, and R.J. Flower. 2003. Aberrant inflammation and resistance to glucocorticoids in annexin 1-/- mouse. FASEB J. 17:253-255.
He, B., R.H. Tang, N. Weisleder, B. Xiao, Z. Yuan, C. Cai, H. Zhu, P. Lin, C. Qiao, J. Li, C. Mayer, J. Li, J. Ma, and X. Xiao. 2012. Enhancing muscle membrane repair by gene delivery of MG53 ameliorates muscular dystrophy and heart failure in delta-Sarcoglycan-deficient hamsters. Molecular therapy : the journal of the American Society of Gene Therapy. 20:727-735.
Hoffman, E.P., R.H. Brown, Jr., and L.M. Kunkel. 1987. Dystrophin: the protein product of the Duchenne muscular dystrophy locus. Cell. 51 :919-928.
Jennische, E., and H.A. Hansson. 1986. Postischemic skeletal muscle injury: patterns of injury in relation to adequacy of reperfusion. Exp Mol Pathol. 44:272-280.
Jia, Y., K. Chen, P. Lin, G. Lieber, M. Nishi, R. Yan, Z. Wang, Y. Yao, Y. Li, B.A. Whitson, P. Duann, H. Li, X. Zhou, H. Zhu, H. Takeshima, J.C. Hunter, R.L. McLeod, N. Weisleder, C. Zeng, and J. Ma. 2014. Treatment of acute lung injury by targeting MG53-mediated cell membrane repair. Nat Commun. 5:4387.
Jimenez, A.J., and F. Perez. 2017. Plasma membrane repair: the adaptable cell life-insurance. Curr Opin Cell Biol. 47:99-107.
Jost, M., C. Thiel, K. Weber, and V. Gerke. 1992. Mapping of three unique Ca(2+)-binding sites in human annexin II. Eur J Biochem. 207:923-930.
Kaetzel, M.A., Y.D. Mo, T.R. Mealy, B. Campos, W. Bergsma-Schutter, A. Brisson, J.R. Dedman, and B.A. Seaton. 2001. Phosphorylation mutants elucidate the mechanism of annexin IV-mediated membrane aggregation. Biochemistry. 40:4192-4199.
Lauritzen, S.P., T.L. Boye, and J. Nylandsted. 2015. Annexins are instrumental for efficient plasma membrane repair in cancer cells. Semin Cell Dev Biol. 45:32-38.
Lennon, N.J., A. Kho, B.J. Bacskai, S.L. Perlmutter, B.T. Hyman, and R.H. Brown, Jr. 2003. Dysferlin interacts with annexins A1 and A2 and mediates sarcolemmal wound-healing. The Journal of biological chemistry. 278:50466-50473.
Li D, Mastaglia FL, Fletcher S, and Wilton SD. Precision Medicine through Antisense Oligonucleotide-Mediated Exon Skipping. Trends Pharmacol Sci 39: 982-994, 2018.
Ling, Q., A.T. Jacovina, A. Deora, M. Febbraio, R. Simantov, R.L. Silverstein, B. Hempstead, W.H. Mark, and K.A. Hajjar. 2004. Annexin II regulates fibrin homeostasis and neoangiogenesis in vivo. The Journal of clinical investigation. 113:38-48.
Liu, J., H. Zhu, Y. Zheng, Z. Xu, L. Li, T. Tan, K.H. Park, J. Hou, C. Zhang, D. Li, R. Li, Z. Liu, N. Weisleder, D. Zhu, P. Lin, and J. Ma. 2015. Cardioprotection of recombinant human MG53 protein in a porcine model of ischemia and reperfusion injury. Journal of molecular and cellular cardiology. 80:10-19.
McDade, J.R., A. Archambeau, and D.E. Michele. 2014. Rapid actin-cytoskeleton-dependent recruitment of plasma membrane-derived dysferlin at wounds is critical for muscle membrane repair. FASEB J. 28:3660-3670.
McNeil, A.K., U. Rescher, V. Gerke, and P.L. McNeil. 2006. Requirement for annexin A1 in plasma membrane repair. The Journal of biological chemistry. 281:35202-35207.
McNeil, P.L., and R. Khakee. 1992. Disruptions of muscle fiber plasma membranes. Role in exercise-induced damage. Am J Pathol. 140:1097-1109.
McNeil, P.L., and T. Kirchhausen. 2005. An emergency response team for membrane repair. Nat Rev Mol Cell Biol. 6:499-505.
Min YL, Bassel-Duby R, and Olson EN. CRISPR Correction of Duchenne Muscular Dystrophy. Annu Rev Med, 2018.
Murphy, S., M. Zweyer, M. Henry, P. Meleady, R.R. Mundegar, D. Swandulla, and K. Ohlendieck. 2018. Proteomic analysis of the sarcolemma-enriched fraction from dystrophic mdx-4cv skeletal muscle. J Proteomics.
Petrof, B.J., J.B. Shrager, H.H. Stedman, A.M. Kelly, and H.L. Sweeney. 1993. Dystrophin protects the sarcolemma from stresses developed during muscle contraction. Proceedings of the National Academy of Sciences of the United States of America. 90:3710-3714.
Quattrocelli, M., D.Y. Barefield, J.L. Warner, A.H. Vo, M. Hadhazy, J.U. Earley, A.R. Demonbreun, and E.M. McNally. 2017a. Intermittent glucocorticoid steroid dosing enhances muscle repair without eliciting muscle atrophy. J Clin Invest. 127:2418-2432.
Quattrocelli, M., J. Capote, J.C. Ohiri, J.L. Warner, A.H. Vo, J.U. Earley, M. Hadhazy, A.R. Demonbreun, M.J. Spencer, and E.M. McNally. 2017b. Genetic modifiers of muscular dystrophy act on sarcolemmal resealing and recovery from injury. PLoS Genet. 13:e1007070.
Quattrocelli, M., I.M. Salamone, P.G. Page, J.L. Warner, A.R. Demonbreun, and E.M. McNally. 2017c. Intermittent Glucocorticoid Dosing Improves Muscle Repair and Function in Mice with Limb-Girdle Muscular Dystrophy. Am J Pathol. 187:2520-2535.
Reddy, A., E.V. Caler, and N.W. Andrews. 2001. Plasma membrane repair is mediated by Ca(2+)-regulated exocytosis of lysosomes. Cell. 106:157-169.
Rodriguez, A., P. Webster, J. Ortego, and N.W. Andrews. 1997. Lysosomes behave as Ca2+-regulated exocytic vesicles in fibroblasts and epithelial cells. J Cell Biol. 137:93-104.
Roostalu, U., and U. Strahle. 2012. In vivo imaging of molecular interactions at damaged sarcolemma. Dev Cell. 22:515-529.
Swaggart, K.A., A.R. Demonbreun, A.H. Vo, K.E. Swanson, E.Y. Kim, J.P. Fahrenbach, J. Holley-Cuthrell, A. Eskin, Z. Chen, K. Squire, A. Heydemann, A.A. Palmer, S.F. Nelson, and E.M. McNally. 2014. Annexin A6 modifies muscular dystrophy by mediating sarcolemmal repair. Proceedings of the National Academy of Sciences of the United States of America. 111:6004-6009.
Waddell, L.B., F.A. Lemckert, X.F. Zheng, J. Tran, F.J. Evesson, J.M. Hawkes, A. Lek, N.E. Street, P. Lin, N.F. Clarke, A.P. Landstrom, M.J. Ackerman, N. Weisleder, J. Ma, K.N. North, and S.T. Cooper. 2011. Dysferlin, annexin A1, and mitsugumin 53 are upregulated in muscular dystrophy and localize to longitudinal tubules of the T-system with stretch. J Neuropathol Exp Neurol. 70:302-313.
Wallner, B.P., R.J. Mattaliano, C. Hession, R.L. Cate, R. Tizard, L.K. Sinclair, C. Foeller, E.P. Chow, J.L. Browing, K.L. Ramachandran, and et al. 1986. Cloning and expression of human lipocortin, a phospholipase A2 inhibitor with potential anti-inflammatory activity. Nature. 320:77-81.
Weisleder, N., N. Takizawa, P. Lin, X. Wang, C. Cao, Y. Zhang, T. Tan, C. Ferrante, H. Zhu, P.J. Chen, R. Yan, M. Sterling, X. Zhao, M. Hwang, M. Takeshima, C. Cai, H. Cheng, H. Takeshima, R.P. Xiao, and J. Ma. 2012. Recombinant MG53 protein modulates therapeutic cell membrane repair in treatment of muscular dystrophy. Science translational medicine. 4:139ra185.
Yeung, T., B. Heit, J.F. Dubuisson, G.D. Fairn, B. Chiu, R. Inman, A. Kapus, M. Swanson, and S. Grinstein. 2009. Contribution of phosphatidylserine to membrane surface charge and protein targeting during phagosome maturation. The Journal of cell biology. 185:917-928.
Zaks, W.J., and C.E. Creutz. 1991. Ca(2+)-dependent annexin self-association on membrane surfaces. Biochemistry. 30:9607-9615.
Zhang Y, Long C, Bassel-Duby R, and Olson EN. Myoediting: Toward Prevention of Muscular Dystrophy by Therapeutic Genome Editing. Physiol Rev 98: 1205-1240, 2018.
Zweifach, A. 2000. FM1-43 reports plasma membrane phospholipid scrambling in T-lymphocytes. The Biochemical journal. 349:255-260.

## Claims

1. A composition for use in treating a cellular membrane injury comprising administering to a patient in need thereof a therapeutically effective amount of the composition, the composition comprising:
a protein having at least 90% sequence identity to annexin A6 as depicted in SEQ ID NO: 7, a protein having at least 90% sequence identity to annexin A6 as depicted in SEQ ID NO: 8 or a combination thereof, or a post-translationally modified form thereof, wherein the post-translationally modified form is biotinylated, glycosylated, PEGylated, and/or polysialylated, or
a polynucleotide capable of expressing an extracellular protein having at least 90% sequence identity to annexin A6 as depicted in SEQ ID NO: 7, a protein having at least 90% sequence identity to annexin A6 as depicted in SEQ ID NO: 8, or a combination thereof, or a modified form thereof.

2. A composition for use in delaying onset, enhancing recovery from cellular membrane injury, or preventing a cellular membrane injury comprising administering to a patient in need thereof a therapeutically effective amount of the composition, the composition comprising:
a protein having at least 90% sequence identity to annexin A6 as depicted in SEQ ID NO: 7, a protein having at least 90% sequence identity to annexin A6 as depicted in SEQ ID NO: 8, or a combination thereof, or a post-translationally modified form thereof, wherein the post-translationally modified form is biotinylated, glycosylated, PEGylated, and/or polysialylated, or
a polynucleotide capable of expressing an extracellular protein having at least 90% sequence identity to annexin A6 (SEQ ID NO: 7, SEQ ID NO: 8, or a combination thereof), or a modified form thereof.

3. The composition for use according to claim 1 or claim 2, wherein the protein or the extracellular protein has at least 95% or at least 99% sequence identity to annexin A6 as depicted in SEQ ID NO: 7, SEQ ID NO: 8 or a combination thereof or a post-translationally modified form thereof, wherein the post-translationally modified form is biotinylated, glycosylated, PEGylated, and/or polysialylated.

4. The composition for use according to claim 1 or claim 2, wherein the protein or the extracellular protein is annexin A6 as depicted inSEQ ID NO: 7, SEQ ID NO: 8 or a combination thereof.

5. The composition for use according to any one of claims 1-4, wherein the composition further comprises a protein having at least 90%, at least 95%, or at least 99% sequence identity to annexin A2 as depicted in SEQ ID NO: 2 or SEQ ID NO: 3 or a post-translationally modified form thereof, wherein the post-translationally modified form is biotinylated, glycosylated, PEGylated, and/or polysialylated, or the composition further comprises a polynucleotide capable of expressing an annexin protein having at least 90%, at least 95%, or at least 99% sequence identity to annexin A2 as depicted in SEQ ID NO: 2 or SEQ ID NO: 3 or a modified form thereof.

6. The composition for use according to any one of claims 1-5, wherein the composition further comprises a protein having at least 90%, at least 95%, or at least 99% sequence identity to annexin A1 as depicted in SEQ ID NO: 1 or a post-translationally modified form thereof, wherein the post-translationally modified form is biotinylated, glycosylated, PEGylated, and/or polysialylated, or the composition further comprises a polynucleotide capable of expressing an annexin protein having at least 90%, at least 95%, or at least 99% sequence identity to annexin A1 as depicted in SEQ ID NO: 1 or a modified form thereof.

7. The composition for use according to any one of claims 1-4, wherein the composition further comprises:
annexin A2 as depicted in SEQ ID NO: 2 or SEQ ID NO: 3 or a post-translationally modified form thereof, wherein the post-translationally modified form is biotinylated, glycosylated, PEGylated, and/or polysialylated; or a polynucleotide capable of expressing annexin A2 as depicted in SEQ ID NO: 2 or SEQ ID NO: 3; and/or
annexin A1 as depicted in SEQ ID NO: 1 or a post-translationally modified form thereof, wherein the post-translationally modified form is biotinylated, glycosylated, PEGylated, and/or polysialylated; or a polynucleotide capable of expressing annexin A1 as depicted in SEQ ID NO: 1.

8. The composition for use according to any one of claims 1-7, wherein the polynucleotide is contained in a vector, wherein the vector is a viral vector selected from the group consisting of a herpes virus vector, an adeno-associated virus (AAV) vector, an adeno virus vector, and a lentiviral vector, optionally wherein the AAV vector is recombinant AAV5, AAV6, AAV8, AAV9, or AAV74, optionally wherein the AAV74 vector is AAVrh74.

9. The composition for use according to any one of claims 1-8, wherein the use further comprises administering an effective amount of a second agent, wherein the second agent is selected from the group consisting of mitsugumin 53 (MG53), a modulator of latent TGF-β binding protein 4 (LTBP4), a modulator of transforming growth factor β (TGF-β) activity, a modulator of androgen response, a modulator of an inflammatory response, a promoter of muscle growth, a chemotherapeutic agent, a modulator of fibrosis, and a combination thereof.

10. The composition for use according to any one of claims 1-9, wherein purity of the annexin A6 in the composition is about 90% or higher as measured by standard release assay.

11. The composition for use according to any one of claims 1-10, wherein the composition has an endotoxin level that is less than about 0.50000 endotoxin units per milligram (EU/mg).

12. The composition for use according to any one of claims 1-11, wherein the patient suffers from an acute injury.

13. The composition for use according to claim 12, wherein the acute injury results from surgery, a burn, a toxin, a chemical, radiation-induced injury, acute myocardial injury, acute muscle injury, acute lung injury, acute epithelial injury, acute epidermal injury, acute kidney injury, acute liver injury, vascular injury, an excessive mechanical force, or trauma.

14. The composition for use according to any one of claims 1-11, wherein the patient suffers from a chronic disorder.

15. The composition for use according to claim 14, wherein the chronic disorder is Becker Muscular Dystrophy (BMD), Duchenne Muscular Dystrophy (DMD), Limb Girdle Muscular Dystrophy, congenital Muscular Dystrophy, Emery-Dreifuss Muscular Dystrophy (EDMD), Myotonic Dystrophy, Fascioscapulohumeral Dystrophy (FSHD), Oculopharyngeal Muscular Dystrophy, Distal Muscular Dystrophy, cystic fibrosis, pulmonary fibrosis, muscle atrophy, cerebral palsy, an epithelial disorder, an epidermal disorder, a kidney disorder, a liver disorder, sarcopenia, cardiomyopathy, Alzheimer's disease, stroke and ischemic injury, neural trauma, Huntington's disease, or Parkinson's disease.

16. The composition for use according to claim 15, wherein the cardiomyopathy is hypertrophic, dilated, congenital, arrhythmogenic, restrictive, ischemic, or heart failure.

## Patentansprüche

1. Zusammensetzung zur Verwendung bei der Behandlung einer Zellmembranverletzung, umfassend die Verabreichung einer therapeutisch wirksamen Menge der Zusammensetzung an einen Patienten, der diese benötigt, wobei die Zusammensetzung umfasst:
ein Protein mit mindestens 90% Sequenzidentität mit Annexin A6, wie in SEQ ID NO: 7 dargestellt, ein Protein mit mindestens 90% Sequenzidentität mit Annexin A6, wie in SEQ ID NO: 8 dargestellt, oder eine Kombination davon, oder eine posttranslational modifizierte Form davon, wobei die posttranslational modifizierte Form biotinyliert, glycosyliert, PEGyliert und/oder polysialyliert ist, oder
ein Polynukleotid, das in der Lage ist, ein extrazelluläres Protein mit mindestens 90% Sequenzidentität mit Annexin AG, wie in SEQ ID NO: 7 dargestellt, ein Protein mit mindestens 90% Sequenzidentität mit Annexin A6, wie in SEQ ID NO: 8 dargestellt, oder eine Kombination davon oder eine modifizierte Form davon zu exprimieren.

2. Zusammensetzung zur Verwendung bei der Verzögerung des Auftretens, der Verbesserung der Erholung von einer Zellmembranverletzung oder der Verhinderung einer Zellmembranverletzung, umfassend die Verabreichung einer therapeutisch wirksamen Menge der Zusammensetzung an einen Patienten, der diese benötigt, wobei die Zusammensetzung umfasst:
ein Protein mit mindestens 90 % Sequenzidentität mit Annexin A6, wie in SEQ ID NO: 7 dargestellt, ein Protein mit mindestens 90 % Sequenzidentität mit Annexin A6, wie in SEQ ID NO: 8 dargestellt, oder eine Kombination davon, oder eine posttranslational modifizierte Form davon, wobei die posttranslational modifizierte Form biotinyliert, glycosyliert, PEGyliert und/oder polysialyliert ist, oder
ein Polynukleotid, das in der Lage ist, ein extrazelluläres Protein zu exprimieren, das mindestens 90% Sequenzidentität mit Annexin A6 (SEQ ID NO: 7, SEQ ID NO: 8 oder einer Kombination davon) oder einer modifizierten Form davon aufweist.

3. Zusammensetzung zur Verwendung nach Anspruch 1 oder Anspruch 2, wobei das Protein oder das extrazelluläre Protein mindestens 95% oder mindestens 99% Sequenzidentität mit Annexin AB, wie in SEQ ID NO: 7, SEQ ID NO: 8 oder einer Kombination davon dargestellt, oder einer posttranslational modifizierten Form davon aufweist, wobei die posttranslational modifizierte Form biotinyliert, glycosyliert, PEGyliert und/oder polysialyliert ist.

4. Die Zusammensetzung zur Verwendung gemäß Anspruch 1 oder Anspruch 2, wobei das Protein oder das extrazelluläre Protein Annexin A6 ist, wie inSEQ ID NO: 7, SEQ ID NO: 8 oder einer Kombination davon dargestellt.

5. Zusammensetzung zur Verwendung nach einem der Ansprüche 1-4, wobei die Zusammensetzung ferner ein Protein mit mindestens 90%, mindestens 95% oder mindestens 99% Sequenzidentität mit Annexin A2, wie in SEQ ID NO: 2 oder SEQ ID NO: 3 oder eine posttranslational modifizierte Form davon, wobei die posttranslational modifizierte Form biotinyliert, glycosyliert, PEGyliert und/oder polysialyliert ist, oder die Zusammensetzung ferner ein Polynucleotid umfasst, das in der Lage ist, ein Annexin-Protein zu exprimieren, das mindestens 90%, mindestens 95% oder mindestens 99% Sequenzidentität mit Annexin A2, wie in SEQ ID NO: 2 oder SEQ ID NO: 3 dargestellt, oder einer modifizierten Form davon aufweist.

6. Zusammensetzung zur Verwendung nach einem der Ansprüche 1-5, wobei die Zusammensetzung ferner ein Protein mit mindestens 90%, mindestens 95% oder mindestens 99% Sequenzidentität mit Annexin A1, wie in SEQ ID NO: 1 oder eine posttranslational modifizierte Form davon, wobei die posttranslational modifizierte Form biotinyliert, glykosyliert, PEGyliert und/oder polysialyliert ist, oder die Zusammensetzung ferner ein Polynukleotid umfasst, das in der Lage ist, ein Annexin-Protein zu exprimieren, das mindestens 90 %, mindestens 95 % oder mindestens 99 % Sequenzidentität mit Annexin A1, wie in SEQ ID NO: 1 dargestellt, oder einer modifizierten Form davon aufweist.

7. Die Zusammensetzung zur Verwendung gemäß einem der Ansprüche 1-4, wobei die Zusammensetzung weiterhin umfasst:
Annexin A2, wie in SEQ ID NO: 2 oder SEQ ID NO: 3 dargestellt, oder eine posttranslational modifizierte Form davon, wobei die posttranslational modifizierte Form biotinyliert, glycosyliert, PEGyliert und/oder polysialyliert ist; oder ein Polynucleotid, das Annexin A2, wie in SEQ ID NO: 2 oder SEQ ID NO: 3 dargestellt, exprimieren kann; und/oder
Annexin A1, wie in SEQ ID NO: 1 dargestellt, oder eine posttranslational modifizierte Form davon, wobei die posttranslational modifizierte Form biotinyliert, glycosyliert, PEGyliert und/oder polysialyliert ist, oder ein Polynucleotid, das Annexin A1, wie in SEQ ID NO: 1 dargestellt, exprimieren kann.

8. Zusammensetzung zur Verwendung nach einem der Ansprüche 1-7, wobei das Polynukleotid in einem Vektor enthalten ist, wobei der Vektor ein viraler Vektor ist, der aus der Gruppe ausgewählt ist, die aus einem Herpesvirus-Vektor, einem Adenoassoziierten Virus (AAV)-Vektor, einem Adeno-Virus-Vektor und einem lentiviralen Vektor besteht, wobei es sich bei dem AAV-Vektor gegebenenfalls um rekombinantes AAVS, AAVB, AAVS, AAVY oder AAV74 handelt, wobei der AAV74-Vektor gegebenenfalls AAVrh74 ist.

9. Zusammensetzung zur Verwendung nach einem der Ansprüche 1-8, wobei die Verwendung ferner die Verabreichung einer wirksamen Menge eines zweiten Wirkstoffs umfasst, wobei der zweite Wirkstoff ausgewählt ist aus der Gruppe bestehend aus Mitsugumin 53 (MG53), einem Modulator des latenten TGF-B-Bindungsproteins 4 (LTBP4), einem Modulator der Aktivität des transformierenden Wachstumsfaktors (TGF-B), einem Modulator der Androgenreaktion, einem Modulator einer Entzündungsreaktion, einem Promotor des Muskelwachstums, einem chemotherapeutischen Wirkstoff, einem Modulator der Fibrose und einer Kombination davon.

10. Die Zusammensetzung zur Verwendung gemäß einem der Ansprüche 1-9, wobei die Reinheit des Annexin A6 in der Zusammensetzung etwa 90% oder mehr beträgt, gemessen durch einen Standard-Freisetzungsassay.

11. Die Zusammensetzung zur Verwendung nach einem der Ansprüche 1-10, wobei die Zusammensetzung einen Endotoxingehalt von weniger als etwa 0,50000 Endotoxineinheiten pro Milligramm (EU/mg) aufweist.

12. Die Zusammensetzung zur Verwendung nach einem der Ansprüche 1-11, wobei der Patient an einer akuten Verletzung leidet.

13. Die Zusammensetzung zur Verwendung nach Anspruch 12, wobei die akute Verletzung aus einer Operation, einer Verbrennung, einem Toxin, einer Chemikalie, einer strahleninduzierten Verletzung, einer akuten Myokardverletzung, einer akuten Muskelverletzung, einer akuten Lungenverletzung, einer akuten Epithelverletzung, einer akuten Epidermisverletzung, einer akuten Nierenverletzung, einer akuten Leberverletzung, einer Gefäßverletzung, einer übermäßigen mechanischen Kraft oder einem Trauma resultiert.

14. Die Zusammensetzung zur Verwendung nach einem der Ansprüche 1-11, wobei der Patient an einer chronischen Erkrankung leidet.

15. Zusammensetzung zur Verwendung nach Anspruch 14, wobei die chronische Erkrankung Becker-Muskeldystrophie (BMD), Duchenne-Muskeldystrophie (DMD), Gliedergürtel-Muskeldystrophie, kongenitale Muskeldystrophie, Emery-Dreifuss-Muskeldystrophie (EDMD), Myotonische Dystrophie, Fascioscapulohumeral-Dystrophie (FSHD), Okulopharyngeale Muskeldystrophie ist, Distale Muskeldystrophie, Mukoviszidose, Lungenfibrose, Muskelschwund, Zerebralparese, eine Epithelerkrankung, eine Epidermiserkrankung, eine Nierenerkrankung, eine Lebererkrankung, Sarkopenie, Kardiomyopathie, Alzheimer-Krankheit, Schlaganfall und ischämische Verletzungen, neurale Traumata, Chorea Huntington oder Parkinson-Krankheit.

16. Zusammensetzung zur Verwendung nach Anspruch 15, wobei die Kardiomyopathie hypertroph, dilatiert, kongenital, arrhythmogen, restriktiv, ischämisch oder Herzinsuffizienz ist.

## Revendications

1. Composition à utiliser dans le traitement d'une lésion de la membrane cellulaire comprenant l'administration à un patient qui en a besoin d'une quantité thérapeutiquement efficace de la composition, la composition comprenant :
une protéine ayant au moins 90 % d'identité de séquence avec l'annexine A6 telle que décrite dans SEQ ID NO : 7, une protéine ayant au moins 90 % d'identité de séquence avec l'annexine A6 telle que décrite dans SEQ ID NO : 8 ou une combinaison de ceux-ci, ou une forme modifiée au niveau post-translationnel de ceux-ci, dans laquelle la forme modifiée au niveau post-translationnel est biotinylée, glycosylée, PEGylée et/ou polysialylée, ou
un polynucléotide pouvant exprimer une protéine extracellulaire ayant une identité de séquence d'au moins 90 % avec l'annexine A6 telle que décrite dans SEQ ID NO : 7, une protéine ayant au moins 90 % d'identité de séquence avec l'annexine A6 telle que décrite dans SEQ ID NO : 8, ou une combinaison de ceux-ci, ou une forme modifiée de ceux-ci.

2. Composition à utiliser dans le retardement de l'apparition d'une lésion de la membrane cellulaire, l'amélioration de la guérison de cette lésion ou la prévention d'une lésion de la membrane cellulaire, comprenant l'administration à un patient qui en a besoin d'une quantité thérapeutiquement efficace de la composition, la composition comprenant :
une protéine ayant au moins 90 % d'identité de séquence avec l'annexine A6 telle que décrite dans SEQ ID NO : 7, une protéine ayant au moins 90 % d'identité de séquence avec l'annexine A6 telle que décrite dans SEQ ID NO : 8, ou une combinaison de ceux-ci, ou une forme modifiée au niveau post-translationnel de ceux-ci, dans laquelle la forme modifiée au niveau post-translationnel est biotinylée, glycosylée, PEGylée et/ou polysialylée, ou
un polynucléotide pouvant exprimer une protéine extracellulaire ayant une identité de séquence d'au moins 90 % avec l'annexine A6 (SEQ ID NO : 7, SEQ ID NO : 8, ou une combinaison de ceux-ci), ou une forme modifiée de ceux-ci.

3. Composition à utiliser selon la revendication 1 ou la revendication 2, dans laquelle la protéine ou la protéine extracellulaire présente une identité de séquence d'au moins 95 % ou d'au moins 99 % avec l'annexine A6 telle que décrite dans SEQ ID NO : 7, SEQ ID NO : 8 ou une combinaison de ceux-ci, ou une forme modifiée au niveau post-translationnel de ceux-ci, dans laquelle la forme modifiée au niveau post-translationnel est biotinylée, glycosylée, PEGylée et/ou polysialylée.

4. Composition à utiliser selon la revendication 1 ou la revendication 2, dans laquelle la protéine ou la protéine extracellulaire est l'annexine A6 telle que décrite dans SEQ ID NO : 7, SEQ ID NO : 8 ou une combinaison de ceux-ci.

5. Composition à utiliser selon l'une quelconque des revendications 1 à 4, dans laquelle la composition comprend en outre une protéine ayant au moins 90 %, au moins 95 % ou au moins 99 % d'identité de séquence avec l'annexine A2 telle que décrite dans SEQ ID NO : 2 ou SEQ ID NO : 3 ou une forme modifiée au niveau post-translationnel, dans laquelle la forme modifiée au niveau post-translationnel est biotinylée, glycosylée, PEGylée et/ou polysialylée, ou la composition comprend en outre un polynucléotide pouvant exprimer une protéine d'annexine ayant une identité de séquence d'au moins 90 %, d'au moins 95 % ou d'au moins 99 % avec l'annexine A2 telle que décrite dans SEQ ID NO : 2 ou SEQ ID NO : 3 ou une forme modifiée de celle-ci.

6. Composition à utiliser selon l'une quelconque des revendications 1 à 5, dans laquelle la composition comprend en outre une protéine ayant au moins 90 %, au moins 95 % ou au moins 99 % d'identité de séquence avec l'annexine A1 telle que décrite dans SEQ ID NO : 1 ou une forme modifiée au niveau post-translationnel, dans laquelle la forme modifiée au niveau post-translationnel est biotinylée, glycosylée, PEGylée et/ou polysialylée, ou la composition comprend en outre un polynucléotide pouvant exprimer une protéine d'annexine ayant une identité de séquence d'au moins 90 %, d'au moins 95 % ou d'au moins 99 % avec l'annexine A1 telle que décrite dans SEQ ID NO : 1 ou une forme modifiée de celle-ci.

7. Composition à utiliser selon l'une quelconque des revendications 1 à 4, dans laquelle la composition comprend en outre :
l'annexine A2 telle que décrite dans SEQ ID NO : 2 ou SEQ ID NO : 3 ou une forme modifiée au niveau post-translationnel de celle-ci, dans laquelle la forme modifiée au niveau post-translationnel est biotinylée, glycosylée, PEGylée et/ou polysialylée ; ou un polynucléotide pouvant exprimer l'annexine A2 telle que décrite dans SEQ ID NO : 2 ou SEQ ID NO : 3 ; et/ou
l'annexine A1 telle que décrite dans SEQ ID NO : 1 ou une forme modifiée au niveau post-translationnel de celle-ci, dans laquelle la forme modifiée au niveau post-translationnel est biotinylée, glycosylée, PEGylée et/ou polysialylée ; ou un polynucléotide pouvant exprimer l'annexine A1 telle que décrite dans SEQ ID NO : 1.

8. Composition à utiliser selon l'une quelconque des revendications 1 à 7, dans laquelle le polynucléotide est contenu dans un vecteur, le vecteur étant un vecteur viral choisi dans le groupe constitué d'un vecteur du virus de l'herpès, d'un vecteur du virus adéno-associé (AAV), d'un vecteur de l'adénovirus et d'un vecteur lentiviral, éventuellement dans lequel le vecteur AAV est un AAV5, AAV6, AAV8, AAV9 ou AAV74 recombinant, éventuellement dans lequel le vecteur AAV74 est l'AAVrh74.

9. Composition à utiliser selon l'une quelconque des revendications 1 à 8, dans laquelle l'utilisation comprend en outre l'administration d'une quantité efficace d'un second agent, le second agent étant choisi dans le groupe constitué de la mitsugumine 53 (MG53), d'un modulateur de la protéine latente de liaison TGF-β 4 (LTBP4), d'un modulateur de l'activité du facteur de croissance transformant β (TGF-β), d'un modulateur de la réponse androgénique, d'un modulateur d'une réponse inflammatoire, d'un promoteur de la croissance musculaire, d'un agent chimiothérapeutique, d'un modulateur de la fibrose, et d'une combinaison de ceux-ci.

10. Composition à utiliser selon l'une quelconque des revendications 1 à 9, dans laquelle la pureté de l'annexine A6 dans la composition est d'environ 90 % ou plus, telle que mesurée par un essai de libération standard.

11. Composition à utiliser selon l'une quelconque des revendications 1 à 10, dans laquelle la composition a un niveau d'endotoxine inférieur à environ 0,50000 unité d'endotoxine par milligramme (UE/mg).

12. Composition à utiliser selon l'une quelconque des revendications 1 à 11, dans laquelle le patient souffre d'une lésion aiguë.

13. Composition à utiliser selon la revendication 12, dans laquelle la lésion aiguë résulte d'une intervention chirurgicale, d'une brûlure, d'une toxine, d'un produit chimique, d'une lésion induite par des radiations, d'une lésion myocardique aiguë, d'une lésion musculaire aiguë, d'une lésion pulmonaire aiguë, d'une lésion épithéliale aiguë, d'une lésion épidermique aiguë, d'une lésion rénale aiguë, d'une lésion hépatique aiguë, d'une lésion vasculaire, d'une force mécanique excessive ou d'un traumatisme.

14. Composition à utiliser selon l'une quelconque des revendications 1 à 11, dans laquelle le patient souffre d'un trouble chronique.

15. Composition à utiliser selon la revendication 14, dans laquelle le trouble chronique est la dystrophie musculaire de Becker (BMD), la dystrophie musculaire de Duchenne (DMD), la dystrophie musculaire des ceintures, la dystrophie musculaire congénitale, la dystrophie musculaire d'Emery-Dreifuss (EDMD), la dystrophie myotonique, la dystrophie fascio-scapulo-humérale (FSHD), la dystrophie musculaire oculopharyngée, la dystrophie musculaire distale, la fibrose kystique, la fibrose pulmonaire, l'atrophie musculaire, la paralysie cérébrale, une maladie épithéliale, une maladie épidermique, une maladie rénale, une maladie hépatique, la sarcopénie, la cardiomyopathie, la maladie d'Alzheimer, les accidents vasculaires cérébraux et les lésions ischémiques, les traumatismes neuronaux, la maladie de Huntington ou la maladie de Parkinson.

16. Composition à utiliser selon la revendication 15, dans laquelle la cardiomyopathie est hypertrophique, dilatée, congénitale, arythmogène, restrictive, ischémique ou une insuffisance cardiaque.
